# EUROPEAN PATENT SPECIFICATION

(11) **EP 2 194 137 B1**
(45) Date of publication and mention of the grant of the patent: **21.12.2016**
(21) Application number: 10158183.3
(22) Date of filing: 18.04.2001
(51) Int. Cl.: C12N 7/01, C12N 7/02, C12N 7/04, C12N 15/867, C12N 15/49, C12N 5/10, C07K 14/16, C07K 14/155, A61K 48/00

(54) **Cells comprising codon optimized lentiviral particles**
Zellen beinhaltend Kodon-optimierte lentivirale Partikel
Cellules comprenant des particules lentivirales avec des codons optimisés

(30) Priority: 19.04.2000 GB 0009760
(43) Date of publication of application: 09.06.2010
(62) Divisional of application: 01921651.4
(73) Proprietor: Oxford BioMedica (UK) Limited, Oxford, OX4 6LT (GB)
(72) Inventor: Kingsman, Alan John, Oxford OX4 4GA (GB); Kim, Narry, Oxford OX4 4GA (GB); Kotsopoulou, Ekaterini, Oxford OX4 4GA (GB); Rohll, Jonathan, Oxford OX4 4GA (GB); Mitrophanous, Kyriacos, Oxford OX4 4GA (GB)
(74) Representative: D Young & Co LLP

(56) References cited:
- WO-A-00/15819
- WO-A-00/39302
- WO-A-00/65076
- WO-A-99/32646
- WO-A-99/41397
- WO-A1-00/55341
- J. ZUR MEGEDE ET AL.: "Increased expression and immunogenicity of sequence-modified HIV-1 gag gene" J. VIROL., vol. 74, no. 6, March 2000 (2000-03), pages 2628-2635, XP000980650
- E. KOTSOPOLOU ET AL.: "A rev-independent HIV 1-based vector that exploits a codon optimised HIV-1 gag-pol gene" J. VIROL., vol. 74, no. 10, 2000, pages 4839-4852, XP002140792
- R. WAGNER AT AL.: "Rev-idependent expression of synthetic gag-pol genes of HIV-1 and SIV: implications for the safety of lentiviral vectors" HUMAN GENE THERAPY, vol. 11, 2000, pages 2403-2413, XP001064159
- G. BUCHSCHACHER ET AL.: "Development of lentiviral vectors for gene therapy of human diseases" BLOOD, vol. 95, no. 6, 15 April 2000 (2000-04-15) , pages 2499-2504, XP000916373
- CHUNG ET AL.: "Development of new safer retrovirus packaging sequences and cell lines" J. CELL. BIOL. SUPPLEMENT, vol. 0, no. 21A, 1995, page 404, XP001064207
- ZUFFEREY R ET AL: "SELF-INACTIVATING LENTIVIRUS VECTOR FOR SAFE AND EFFICIENT IN VIVO GENE DELIVERY", JOURNAL OF VIROLOGY, THE AMERICAN SOCIETY FOR MICROBIOLOGY, US, vol. 72, no. 12, 1 December 1998 (1998-12-01), pages 9873-9880, XP002929451, ISSN: 0022-538X

## Description

### Field of the Invention

The present invention relates to methods of improving the safety of retroviral vectors capable of delivering therapeutic genes for use in gene therapy, and to novel nucleotide sequences for use in such methods.

### Background to the Invention

Retroviral vectors are now widely used as vehicles to deliver genes into cells. Their popularity stems from the fact that they are easy to produce and mediate stable integration of the gene that they carry into the genome of the target cell. This enables long-term expression of the delivered gene (1).

There has been considerable interest, for some time, in the development of retroviral vector systems based on lentiviruses. Lentiviruses are a small subgroup of complex retroviruses. They contain, in addition to the common retroviral genes (*gag, pol* and *env*)*,* genes which enable them to regulate their life cycle and to infect non-dividing cells (2). Vector systems based thereon are therefore of interest because of their potential use in the transfer of a gene of interest to non-dividing cells such as neurones. In addition, lentiviral vectors enable very stable long-term expression of the gene of interest. This has been shown to be at least three months for transduced rat neuronal cells while MLV based vectors were only able to express the gene of interest for six weeks.

The most commonly used lentivirus is the Human Immunodeficiency Virus (HIV), the etiologic agent of AIDS (acquired immune deficiency syndrome). HIV-based vectors have been shown to efficiently transduce non-diving cells (3) and can be used, for example, to target anti-HIV therapeutic genes to HIV susceptible cells. However, HIV vectors have a number of significant disadvantages that may limit their therapeutic application to certain diseases. In particular, HIV-1 is a human pathogen carrying potentially oncogenic proteins and sequences. There is the risk that introduction of vector particles produced in packaging cells which express HIV gag-pol will introduce these proteins into the patient leading to seroconversion.

Emphasis has therefore been placed on the safety of these vectors. One strategy looks at the design of production systems for retroviral vectors. A retrovirus vector system basically consists of two elements, a packaging cell line and a vector genome. The simplest packaging line consists of a provirus in which the ψ sequence (a determinant of RNA packaging reporting in HIV as lying between U5 and *gag*) has been deleted. When stably transfected into a cell, virus particles containing reverse transcriptase will be produced but virion RNA will not become packaged within these particles. The complementing component in a retrovirus vector system is the genome vector itself. The genome vector needs to contain a packaging sequence but much of the structural coding regions can be deleted. Often a selectable marker gene, or other nucleotide sequence of interest, is incorporated into the vector. Vector stocks of the packaging line can then be used to infect target cells. Provided the cell is successfully infected by the viral particle, the genome vector sequence will be reverse transcribed and integrated by the retroviral machinery. However, infection is an end process so no further replication or spread of the vector should occur.

As indicated above, however, problems are encountered in the design of safe and effective retroviral vectors. These include the possibility that recombination between the packaging vector and the packaging sequence can lead to the generation of wild type replication competent virus. Consequently efforts have been directed at improving the safety of packaging cell constructs.

In second generation packaging cell lines, in addition to deletion of the packaging sequence, the 3' LTR was also deleted so that two recombinations are necessary to generate a wild type virus.

In third generation packaging lines the *gag-pol* genes and *env* gene are placed on separate constructs that are sequentially introduced into the packaging cells to prevent recombination during transfection.

With regard to the packaging signal, EP 0 368 882A (Sodroski) discloses that in HIV it corresponds to the region between the 5' major splice donor and the *gag* initiation codon, and particularly corresponds to a segment just downstream of the 5' major splice donor, and about 14 bases upstream of the *gag* initiation codon. It is this region which Sodroski teaches should be deleted from the *gag-pol* cassette. WO97/12622 (Verma) describes that in HIV-1 a 39 bp internal deletion in the ψ sequence can be made between the 5' splice donor site and the starting codon of the *gag* gene.

Codon wobbling can be used to reduce recombination frequency while maintaining the primary protein sequence of the constructs, c.f. (4) in which the region of overlap between the *gag-pol* and *env* expression constructs was reduced to 61 bp extending over the common region between *pol* and *env* which are in different reading frames. Transversion mutations were introduced into the final 20 codons of *pol,* retaining the integrity of the coding region while reducing the homology with *env* to 55% in the overlap region. Similarly wobble mutations were introduced into the 3' of *env* and all sequences downstream of the *env* stop codon were deleted.

Efficient vectors usually contain part of *gag* on the genome vector to increase virion titre. Unlike the packaging sequence which can be in any position within a sequence to effect packaging, the *gag* sequence must be in its native position adjacent to ψ to have any effect.

It will be appreciated that whilst significant improvements in packaging cell and vector design have been made there is still scope for further refinement of current packaging lines.

WO 00/55341 and WO 99/41397 relate to viral vectors capable of delivering anti-viral inhibitory RNA molecules to target cells, particular for the treatment of AIDS and HIV infection.

WO 00/15819 relates to packaging cell lines for producing a viral accessory protein independent lentivirus-derived retroviral particle which comprises a coding sequence for lentivirus *gagpol* which has been codon optimised. Zufferey R et al., Journal of Virology, Dec 1998, p. 9873-9880 which describes self-inactivating lentivirus vectors for safe and efficient *in vivo* gene delivery.

### Summary of the Invention

It is therefore an aim of the invention to provide retroviral particles, in particular lentiviral particles, and particularly those which carry nucleotide constructs encoding therapeutic proteins, that have improved safety over the corresponding wild type viral particle. In our WO99/41397 we describe codon optimisation of the *gag-pol* genes as a means of overcoming the Rev/RRE requirement for export and to enhance RNA stability. We have now found however that the codon optimised *gag-pol* sequence overcomes potential recombination problems with vector genomes which carry part of a *gag* sequence with the aim of increasing titre. This strategy also avoids the need to use *gag* regions from different viruses in the packaging and vector genome constructs.

Another significant advantage provided by the invention is that the codon optimisation disrupts RNA secondary structures, such as the packaging signal, thus rendering the *gag-pol* mRNA non-packagable. Thus, the present invention allows retroviral sequence upstream of the *gag* initiation codon to be retained, in contrast to Sodroski and Verma, without significantly compromising safety.

### Statements of the Invention

According to one aspect of the present invention there is provided a method of producing a replication defective HIV vector comprising transfecting a producer cell with the following:
i) an HIV genome comprising a nucleotide of interest (NOI), a packaging signal, and RRE;
ii) a nucleotide sequence coding for HIV gag and pol proteins; and
iii) a nucleotide sequence coding for an env protein; characterised in that the nucleotide sequence coding for the HIV gag and pol proteins is codon optimised for expression in the producer cell with the exception of the sequence encompassing the frameshift site and wherein (ii) does not comprise RRE; wherein the producer cell expresses *rev* and wherein the HIV vector is a self-inactivating vector.

According to another aspect of the present invention there is provided an HIV vector particle producer cell comprising:
i) an HIV genome comprising at least one nucleotide sequence of interest, packaging signal, and RRE; and
ii) a nucleotide sequence coding for HIV gag and pol proteins; and
iii) a nucleotide sequence coding for an env protein; characterised in that the nucleotide sequence coding for the HIV gag and pol proteins is codon optimised for expression in the producer cell with the exception of the sequence encompassing the frameshift site and wherein (ii) does not comprise RRE; wherein the producer cell expresses *rev* and wherein the HIV vector is a self-inactivating vector.

We also provide novel codon optimised sequences as shown in SEQ ID NOS: 15 which may be used in the present invention. However, it will be appreciated that any convenient codon optimised *gag-pol* sequence may be employed in the invention.

The present invention also provides a pharmaceutical composition comprising a producer cell according to the present invention, together with a pharmaceutically acceptable diluent or carrier.

By "reducing" we mean that the chance of an event occurring is reduced compared to a comparable population havingg the wild-type *gag*-*pol* sequence. Within a population the chance of an event occurring may be prevented for an individual retrovirus vector or particle.

### Detailed Description of the Invention

Various preferred features and embodiments of the present invention will now be described by way of non-limiting example.

The present invention employs the concept of codon optimisation.

Codon optimisation has previously been described in our WO99/41397 as a means of overcoming the Rev/RRE requirement for export and to enhance RNA stability. The alterations to the coding sequences for the viral components improve the sequences for codon usage in the mammalian cells or other cells which are to act as the producer cells for retroviral vector particle production. This improvement in codon usage is referred to as "codon optimisation". Many viruses, including HIV and other lentiviruses, use a large number of rare codons and by changing these to correspond to commonly used mammalian codons, increased expression of the packaging components in mammalian producer cells can be achieved. Codon usage tables are known in the art for mammalian cells, as well as for a variety of other organisms.

By virtue of alterations in their sequences, the nucleotide sequences encoding the packaging components of the viral particles required for assembly of viral particles in the producer cells/packaging cells have RNA instability sequences (INS) eliminated from them. At the same time, the amino acid coding sequence for the packaging components is retained so that the viral components encoded by the sequences remain the same, or at least sufficiently similar that the function of the packaging components is not compromised.

The term "viral polypeptide required for the assembly of viral particles" means a polypeptide normally encoded by the viral genome to be packaged into viral particles, in the absence of which the viral genome cannot be packaged. For example, in the context of retroviruses such polypeptides would include *gag-pol* and *env.* The term "packaging component" is also included within this definition.

As discussed in our WO99/32646, the sequence requirements for packaging HIV vector genomes are complex. The HIV-1 packaging signal encompasses the splice donor site and contains a portion of the 5'-untranslated region of the *gag* gene, which has a putative secondary structure containing 4 short stem-loops. However, additional sequences elsewhere in the genome are also known to be important for efficient encapsidation of HIV. For example, the first 350 bps of the *gag* protein coding sequence may contribute to efficient packaging. Thus, for construction of HIV-1 vectors capable of expressing heterologous genes, a packaging signal extending to 350 bps of the *gag* protein-coding region has been used on the vector genome. We have now found that codon optimisation of the *gag* coding region on the packaging vector, at least in the region into which the packaging signal extends, also has the effect of disrupting packaging of the vector genome. Thus codon optimisation is a novel method of obtaining a replication defective viral particle.

Also as disclosed in WO99/32646, the structure of the packaging signal in equine lentiviruses is different from that of HIV. Instead of a short sequence of 4 stem loops together with a packaging signal extending to 350 bps of the *gag* protein-coding region, we have found that in equine lentiviruses the packaging signal may not extend as far into the *gag* protein-coding region as may have been thought.

In one aspect only codons relating to the packaging signal are codon optimised. Thus, in one aspect, codon optimisation extends to at least the first 350 bps of the *gag* protein coding region. In equine lentiviruses, at least, codon optimisation extends to at least nucleotide 300 of the gag coding region, more preferably to at least nucleotide 150 of the gag coding region. Although not optimal, codon optimisation could extend to, say, only the first 109 nucleotides of the *gag* coding region. It may also be possible for codon optimisation to extend to only the first codon of the *gag* coding region.

However, in the present invention the sequences are codon optimised in their entirety, with the exception of the sequence encompassing the frameshift site. The *gag-pol* gene comprises two overlapping reading frames encoding *gag* and *pol* proteins respectively. The expression of both proteins depends on a frameshift during translation. This frameshift occurs as a result of ribosome "slippage" during translation. This slippage is thought to be caused at least in part by ribosome-stalling RNA secondary structures. Such secondary structures exist downstream of the frameshift site in the *gag-pol* gene. For HIV, the region of overlap extends from nucleotide 1222 downstream of the beginning of *gag* (wherein nucleotide 1 is the A of the *gag* ATG) to the end of *gag* (nt 1503). Consequently, a 281 bp fragment spanning the frameshift site and the overlapping region of the two reading frames is preferably not codon optimised. Retaining this fragment will enable more efficient expression of the gag-pol proteins.

Derivations from optimal codon usage may be made, for example, in order to accommodate convenient restriction sites, and conservative amino acid changes may be introduced into the *gag-pol* proteins.

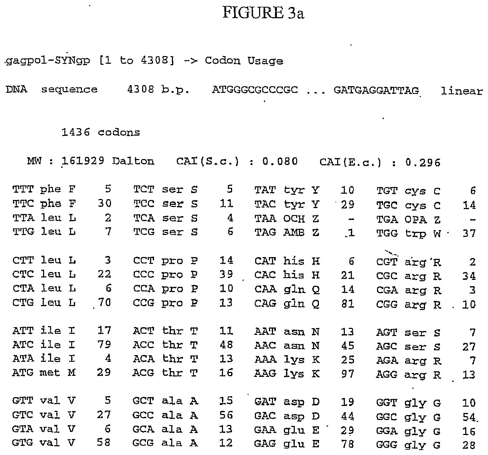

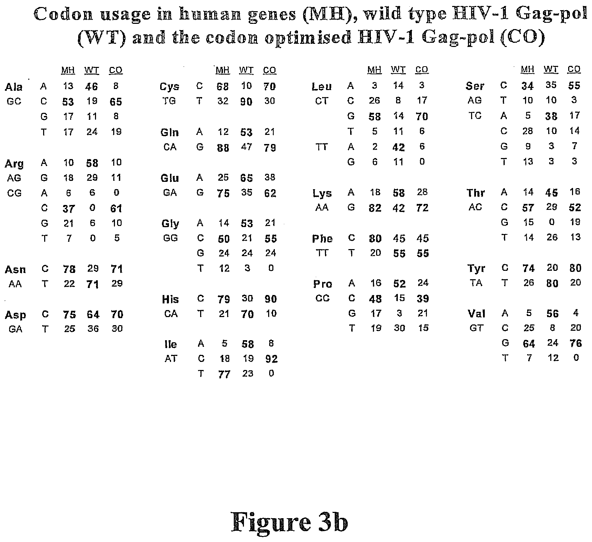

In a highly preferred embodiment, codon optimisation was based on lightly expressed mammalian genes. The third and sometimes the second and third base may be changed. An example of a codon usage table is given in Figure 3b.

Due to the degenerate nature of the Genetic Code, it will be appreciated that numerous *gag-pol* sequences can be achieved by a skilled worker. Also there are many retroviral variants described and which can be used as a starting point for generating a codon optimised *gag-pol* sequence. Lentiviral genomes can be quite variable. For example there are many quasi-species of HIV-1 which are still functional. This is also the case for EIAV. These variants may be used to enhance particular parts of the transduction process. Examples of HIV-1 variants may be found at http://hiv-web.lanl.gov. Details of EIAV clones may be found at the NCBI database: http://www.ncbi.nlm.nih.gov.

As codon optimisation may result in disruption of RNA secondary structures such as the packaging signal, it will be appreciated that any endogenous packaging signal upstream of the *gag* initiation codon could be retained without compromising safety.

An additional advantage of codon optimising packaging components is that this can increase gene expression. In particular, it can render *gag-pol* expression Rev independent. In order to enable the use of anti-*rev* or RRE factors in the retroviral vector, however, it would be necessary to render the viral vector generation system totally Rev/RRE independent (5). Thus, the genome also needs to be modified. This is achieved by optimising vector genome components. Advantageously, these modifications also lead to the production of a safer system absent of all accessory proteins both in the producer and in the transduced cell, and are described below.

As described above, the packaging components for a retroviral vector include expression products of *gag, pol* and *env* genes. In addition, efficient packaging depends on a short sequence of 4 stem loops followed by a partial sequence from *gag* and *env* (the "packaging signal"). Thus, inclusion of a deleted *gag* sequence in the retroviral vector genome (in addition to the full *gag* sequence on the packaging construct) will optimise vector titre. To date efficient packaging has been reported to require from 255 to 360 nucleotides of *gag* in vectors that still retain *env* sequences, or about 40 nucleotides of *gag* in a particular combination of splice donor mutation, *gag* and *env* deletions. We have surprisingly found that a deletion of up to 360 nucleotides in *gag* leads to an increase in vector titre. Further deletions resulted in lower titres. Additional mutations at the major splice donor site upstream of *gag* were found to disrupt packaging signal secondary structure and therefore lead to decreased vector titre. Thus, preferably, the retroviral vector genome includes a *gag* sequence from which up to 360 nucleotides have been removed.

We therefore allow the preparation of a so-called "minimal" system in which all of the accessory genes may be removed. In HIV these accessory genes, are *vpr, vif, tat, nef, vpu* and *rev.* Similarly, in other lentiviruses the analogous accessory genes normally present in the lentivirus may be removed. For the avoidance of doubt, however, we would mention that the present invention also extends to systems, particles and vectors in which one or more of these accessory genes is present and in any combination.

The term "viral vector" refers to a nucleotide construct comprising a viral genome capable of being transcribed in a host cell, which genome comprises sufficient viral genetic information to allow packaging of the viral RNA genome, in the presence of packaging components, into a viral particle capable of infecting a target cell. Infection of the target cell includes reverse transcription and integration into the target cell genome, where appropriate for particular viruses. The viral vector in use typically carries heterologous coding sequences (nucleotides of interest or "NOIs") which are to be delivered by the vector to the target cell, for example a first nucleotide sequence encoding a ribozyme. By "replication defective" we mean that a viral vector is incapable of independent replication to produce infectious viral particles within the final target cell.

The term "viral vector system" is intended to mean a kit of parts which can be used when combined with other necessary components for viral particle production to produce viral particles in host cells. For example, an NOI may typically be present in a plasmid vector construct suitable for cloning the NOI into a viral genome vector construct. When combined in a kit with a further nucleotide sequence, which will also typically be present in a separate plasmid vector construct, the resulting combination of plasmid containing the NOI and plasmid containing the further nucleotide sequence comprises the essential elements of the invention. Such a kit may then be used by the skilled person in the production of suitable viral vector genome constructs which when transfected into a host cell together with the plasmid containing the further nucleotide sequence, and optionally nucleic acid constructs encoding other components, required for viral assembly, will lead to the production of infectious viral particles.

Alternatively, the further nucleotide sequence may be stably present within a packaging cell line that is included in the kit.

The kit may include the other components needed to produce viral particles, such as host cells and other plasmids encoding essential viral polypeptides required for viral assembly. By way of example, the kit may contain (i) a plasmid containing an NOI and (ii) a plasmid containing a further nucleotide sequence encoding a modified retroviral *gag-pol* construct which has been codon optimised for expression in a producer of choice. Optional components would then be (a) a retroviral genome construct with suitable restriction enzyme recognition sites for cloning the NOI into the viral genome, optionally with at least a partial *gag* sequence; (b) a plasmid encoding a VSV-G env protein. Alternatively, nucleotide sequence encoding viral polypeptides required for assembly of viral particles may be provided in the kit as packaging cell lines comprising the nucleotide sequences, for example a VSV-G expressing cell line.

The term "viral vector production system" refers to the viral vector system described above wherein the NOI has already been inserted into a suitable viral vector genome.

In the present invention, several terms are used interchangeably. Thus, "virion", "virus", "viral, particle", "retroviral particle", "retrovirus", and "vector particle" mean virus and virus-like particles that are capable of introducing a nucleic acid into a cell through a viral-like entry mechanism. Such vector particles can, under certain circumstances, mediate the transfer of NOIs into the cells they infect. A retrovirus is capable of reverse transcribing its genetic material into DNA and incorporating this genetic material into a target cell's DNA upon transduction. Such cells are designated herein as "target cells".

As used herein the term "target cell" simply refers to a cell which the regulated retroviral vector of the present disclosure, whether native or targeted, is capable of infecting or transducing.

A lentiviral vector particle for use according to the invention will be capable of transducing cells which are slowly-dividing, and which non-lentiviruses such as MLV would not be able to efficiently transduce. Slowly-dividing cells divide once in about every three to four days including certain tumour cells. Although tumours contain rapidly dividing cells, some tumour cells especially those in the centre of the tumour, divide infrequently.

Alternatively the target cell may be a growth-arrested cell capable of undergoing cell division such as a cell in a central portion of a tumour mass or a stem cell such as a haematopoietic stem cell or a CD34-positive cell.

As a further alternative, the target cell may be a precursor of a differentiated cell such as a monocyte precursor, a CD33-positive cell, or a myeloid precursor.

As a further alternative, the target cell may be a differentiated cell such as a neuron, astrocyte, glial cell, microglial cell, macrophage, monocyte, epithelial cell, endothelial cell, hepatocyte, spermatocyte, spermatid or spermatozoa.

Target cells may be transduced either *in vitro* after isolation from a human individual or may be transduced directly *in vivo.*

Viral vectors for use according to the invention are HIV vectors.

The term "derivable" is used in its normal sense as meaning a nucleotide sequence such as an LTR or a part thereof which need not necessarily be obtained from a vector such as a retroviral vector but instead could be derived therefrom. By way of example, the sequence may be prepared synthetically or by use of recombinant DNA techniques.

Details on the genomic structure of some retroviruses may be found in the art. By way of example, details on HIV, and Mo-MLV may be found from the NCBI Genbank (Genome Accession Nos. AF033819 and AF033811, respectively). Details of HIV variants may also be found at http://hiv-web.lanl.gov. Details of EIAV variants may be found through http://www.ncbi.nlm.nih.gov.

The basic structure of a retrovirus genome is a 5' LTR and a 3' LTR, between or within which are located a packaging signal to enable the genome to be packaged, a primer binding site, integration sites to enable integration into a host cell genome and *gag, pol* and *env* genes encoding the packaging components - these are polypeptides required for the assembly of viral particles. More complex retroviruses have additional features, such as *rev* and RRE sequences in HIV, which enable the efficient export of RNA transcripts of the integrated provirus from the nucleus to the cytoplasm of an infected target cell.

In the provirus, these genes are flanked at both ends by regions called long terminal repeats (LTRs). The LTRs are responsible for proviral integration, and transcription. LTRs also serve as enhancer-promoter sequences and can control the expression of the viral genes. Encapsidation of the retroviral RNAs occurs by virtue of a *psi* sequence, which it has been disclosed in respect of HIV, at least, is located at the 5' end of the viral genome.

The LTRs themselves are identical sequences that can be divided into three elements, which are called U3, R and U5. U3 is derived from the sequence unique to the 3' end of the RNA. R is derived from a sequence repeated at both ends of the RNA and U5 is derived from the sequence unique to the 5' end of the RNA. The sizes of the three elements can vary considerably among different retroviruses.

In a defective retroviral vector genome *gag, pol* and *env* may be absent or not functional. The R regions at both ends of the RNA are repeated sequences. U5 and U3 represent unique sequences at the 5' and 3' ends of the RNA genome respectively.

As discussed above, in a typical retroviral vector for use in gene therapy, at least part of one or more of the *gag, pol* and *env* protein coding regions essential for replication may be removed from the viral vector. This makes the retroviral vector replication-defective. The removed portions may even be replaced by a nucleotide sequence of interest (NOI), as in the present invention, to generate a virus capable of integrating its genome into a host genome but wherein the modified viral genome is unable to propagate itself due to a lack of structural proteins. When integrated in the host genome, expression of the NOI occurs - resulting in, for example, a therapeutic and/or a diagnostic effect. Thus, the transfer of an NOI into a site of interest is typically achieved by: integrating the NOI into the recombinant viral vector; packaging the modified viral vector into a virion coat; and allowing transduction of a site of interest - such as a targeted cell or a targeted cell population.

A minimal retroviral genome for use in the present invention may therefore comprise (5') R - U5 - one or more NOIs - U3-R (3'). However, the plasmid vector used to produce the retroviral genome within a host cell/packaging cell will also include transcriptional regulatory control sequences operably linked to the retroviral genome to direct transcription of the genome in a host cell/packaging cell. These regulatory sequences may be the natural sequences associated with the transcribed retroviral sequence, i.e. the 5' U3 region, or they may be a heterologous promoter such as another viral promoter, for example the CMV promoter.

Some retroviral genomes require additional sequences for efficient virus production. For example, in the case of HIV, *rev* and RRE sequence should be included. However, we have found that the requirement for *rev* and RRE can be reduced or eliminated by codon optimisation. As expression of the codon optimised *gag-pol* is REV independent, RRE can be removed from the *gag-pol* expression cassette, thus removing any potential for recombination with any RRE contained on the vector genome.

Once the retroviral vector NOIs sequences need to be expressed. In a retrovirus, the promoter is located in the 5' LTR U3 region of the provirus. In retroviral vectors, the promoter driving expression of a therapeutic gene may be the native retroviral promoter in the 5' U3 region, or an alternative promoter engineered into the vector. The alternative promoter may physically replace the 5' U3 promoter native to the retrovirus, or it may be incorporated at a different place within the vector genome such as between the LTRs.

Thus, the NOI will also be operably linked to a transcriptional regulatory control sequence to allow transcription of the first nucleotide sequence to occur in the target cell. The control sequence will typically be active in mammalian cells. The control sequence may, for example, be a viral promoter such as the natural viral promoter or a CMV promoter or it may be a mammalian promoter. It is particularly preferred to use a promoter that is preferentially active in a particular cell type or tissue type in which the virus to be treated primarily infects. Thus, in one embodiment, a tissue-specific regulatory sequences may be used. The regulatory control sequences driving expression of the one or more first nucleotide sequences may be constitutive or regulated promoters.

The term "operably linked" denotes a relationship between a regulatory region (typically a promoter element, but may include an enhancer element) and the coding region of a gene, whereby the transcription of the coding region is under the control of the regulatory region.

As used herein, the term "enhancer" includes a DNA sequence which binds other protein components of the transcription initiation complex and thus facilitates the initiation of transcription directed by its associated promoter.

In one preferred embodiment of the present invention, the enhancer is an ischaemic like response element (ILRE).

The term "ischaemia like response element" - otherwise written as ILRE - includes an element that is responsive to or is active under conditions of ischaemia or conditions that are like ischaemia or are caused by ischaemia. By way of example, conditions that are like ischaemia or are caused by ischaemia include hypoxia and/or low glucose concentration(s).

The term "hypoxia" means a condition under which a particular organ or tissue receives an inadequate supply of oxygen.

Ischaemia can be an insufficient supply of blood to a specific organ or tissue. A consequence of decreased blood supply is an inadequate supply of oxygen to the organ or tissue (hypoxia). Prolonged hypoxia may result in injury-to the affected organ or tissue.

A preferred ILRE is a hypoxia response element (HRE).

In one preferred aspect of the present invention, there is hypoxia or ischaemia regulatable expression of the retroviral vector components. In this regard, hypoxia is a powerful regulator of gene expression in a wide range of different cell types and acts by the induction of the activity of hypoxia-inducible transcription factors such as hypoxia inducible factor-1 (HIV-1; 6), which bind to cognate DNA recognition sites, the hypoxia-responsive elements (HREs) on various gene promoters. Dachs *et al* (7) have used a multimeric form of the HRE from the mouse phosphoglycerate kinase-1 (PGK-1) gene (8) to control expression of both marker and therapeutic genes by human fibrosarcoma cells in response to hypoxia *in vitro* and within solid tumours *in vivo* (7 *ibid*)*.*

Hypoxia response enhancer elements (HREEs) have also been found in association with a number of genes including the erythropoietin (EPO) gene (9; 10). Other HREEs have been isolated from regulatory regions of both the muscle glycolytic enzyme pyruvate kinase (PKM) gene (11), the human muscle-specific β-enolase gene (ENO3; 12) and the endothelin-1 (ET-1) gene (13).

Preferably the HRE for use according to the present invention is selected from, for example, the erythropoietin HRE element (HREE1), muscle pyruvate kinase (PKM), HRE element, phosphoglycerate kinase (PGK) HRE, B-enolase (enolase 3; ENO3) HRE element, endothelin-1 (ET-1)HRE element and metallothionein II (MTII) HRE element.

Preferably the ILRE is used in combination with a transcriptional regulatory element, such as a promoter, which transcriptional regulatory element is preferably active in one or more selected cell type(s), preferably being only active in one cell type.

As outlined above, this combination aspect is called a responsive element.

Preferably the responsive element comprises at least the ILRE as herein defined.

Non-limiting examples of such a responsive element are presented as OBHRE1 and XiaMac. Another non-limiting example includes the ILRE in use in conjunction with an MLV promoter and/or a tissue restricted ischaemic responsive promoter. These responsive elements are disclosed in WO99/15684.

Other examples of suitable tissue restricted promoters/enhancers are those which are highly active in tumour cells such as a promoter/enhancer from a *MUC*1 gene, a *CEA* gene or a *5T4* antigen gene. The alpha-fetoprotein (AFP) promoter is also a tumour-specific promoter. One preferred promoter-enhancer combination is a human cytomegalovirus (hCMV) major immediate early (MIE) promoter/enhancer combination.

The term "promoter" is used in the normal sense of the art, e.g. an RNA polymerase binding site.

The promoter may be located in the retroviral 5' LTR to control the expression of a cDNA encoding an NOI, and/or gag-pol proteins.

Preferably the NOI and/or gag-pol proteins are capable of being expressed from the retrovirus genome such as from endogenous retroviral promoters in the long terminal repeat (LTR).

Preferably the NOI and/or gag-pol proteins are expressed from a heterologous promoter to which the heterologous gene or sequence, and/or codon optimised *gag-pol* sequence is operably linked.

Alternatively, the promoter may be an internal promoter.

Preferably the NOI is expressed from an internal promoter.

Vectors containing internal promoters have also been widely used to express multiple genes. An internal promoter makes it possible to exploit promoter/enhancer combinations other than those found in the viral LTR for driving gene expression. Multiple internal promoters can be included in a retroviral vector and it has proved possible to express at least three different cDNAs each from its own promoter (14). Internal ribosomal entry site (IRES) elements have also been used to allow translation of multiple coding regions from either a single mRNA or from fusion proteins that can then be expressed from an open reading frame.

The promoter may be constitutively efficient, or may be tissue or temporally restricted in their activity.

Preferably the promoter is a constitutive promoter such as CMV.

Preferably the promoters are tissue specific. That is, they are capable of driving transcription of a NOI or NOI(s) in one tissue while remaining largely "silent'' in other tissue types.

The term "tissue specific" means a promoter which is not restricted in activity to a single tissue type but which nevertheless shows selectivity in that they may be active in one group of tissues and less active or silent in another group.

The level of expression of an NOI or NOIs under the control of a particular promoter may be modulated by manipulating the promoter region. For example, different domains within a promoter region may possess different gene regulatory activities. The roles of these different regions are typically assessed using vector constructs having different variants of the promoter with specific regions deleted (that is, deletion analysis). This approach may be used to identify, for example, the smallest region capable of conferring tissue specificity or the smallest region conferring hypoxia sensitivity.

A number of tissue specific promoters, described above, may be particularly advantageous in practising the present invention. In most instances, these promoters may be isolated as convenient restriction digestion fragments suitable for cloning in a selected vector. Alternatively, promoter fragments may be isolated using the polymerase chain reaction. Cloning of the amplified fragments may be facilitated by incorporating restriction sites at the 5' end of the primers.

The NOI or NOIs may be under the expression control of an expression regulatory element, such as a promoter and enhancer.

Preferably the ischaemic responsive promoter is a tissue restricted ischaemic responsive promoter.

Preferably the tissue restricted ischaemic responsive promoter is a macrophage specific promoter restricted by repression.

Preferably the tissue restricted ischaemic responsive promoter is an endothelium specific promoter.

Preferably the regulated retroviral vector of the present invention is an ILRE regulated retroviral vector.

Preferably the regulated retroviral vector for use according to the present invention is an ILRE
regulated lentiviral vector.

Preferably the regulated retroviral vector for use according to the present invention is an
autoregulated hypoxia responsive lentiviral vector.

Preferably the regulated retroviral vector for use according to the present invention is regulated by
glucose concentration.

For example, the glucose-regulated proteins (grp's) such as grp78 and grp94 are highly conserved proteins known to be induced by glucose deprivation (15). The grp 78 gene is expressed at low levels in most normal healthy tissues under the influence of basal level promoter elements but has at least two critical "stress inducible regulatory elements" upstream of the TATA element (15 *ibid;* 16). Attachment to a truncated 632 base pair sequence of the 5'end of the grp78 promoter confers high inducibility to glucose deprivation on reporter genes *in vitro* (16 *ibid*)*.* Furthermore, this promoter sequence in retroviral vectors was capable of driving a high level expression of a reporter gene in tumour cells in murine fibrosarcomas, particularly in central relatively ischaemic/fibrotic sites (16 *ibid*)*.*

The regulated retroviral vector for use according to the present invention is a self-inactivating
(SIN) vector.

By way of example, self-inactivating retroviral vectors have been constructed by deleting the transcriptional enhancers or the enhancers and promoter in the U3 region of the 3' LTR. After a round of vector reverse transcription and integration, these changes are copied into both the 5' and the 3' LTRs producing a transcriptionally inactive provirus (17; 18; 19; 20). However, any promoter(s) internal to the LTRs in such vectors will still be transcriptionally active. This strategy has been employed to eliminate effects of the enhancers and promoters in the viral LTRs on transcription from internally placed genes. Such effects include increased transcription (21) or suppression of transcription (22). This strategy can also be used to eliminate downstream transcription from the 3' LTR into genomic DNA (23). This is of particular concern in human gene therapy where it is of critical importance to prevent the adventitious activation of an endogenous oncogene.

As discussed above, replication-defective retroviral vectors are typically propagated, for example to prepare suitable titres of the retroviral vector for subsequent transduction, by using a combination of a packaging or helper cell line and the recombinant vector. That is to say, that the three packaging proteins can be provided *in trans.*

In general a "packaging cell line" contains one or more of the retroviral *gag, pol* and *env* genes. In the present invention it contains codon optimised *gag-pol* genes, and an *env* gene. The packaging cell line produces the proteins required for packaging retroviral DNA but it cannot bring about encapsidation. Conventionally this has been achieved through lack of a *psi* region. However, when a recombinant vector carrying an NOI and a *psi* region is introduced into the packaging cell line, the helper proteins can package the *psi*-positive recombinant vector to produce the recombinant virus stock. This virus stock can be used to transduce cells to introduce the NOI into the genome of the target cells. Conventionally *a psi* packaging signal, called *psi* plus, has been used that contains additional sequences spanning from upstream of the splice donor to downstream of the *gag* start codon (24) since this has been shown to increase viral titres.

The recombinant virus whose genome lacks all genes required to make viral proteins can tranduce only once and cannot propagate. These viral vectors which are only capable of a single round of transduction of target cells are known as replication defective vectors. Hence, the NOI is introduced into the host/target cell genome without the generation of potentially harmful retrovirus. A summary of the available packaging lines is presented in Coffin *et al.,* 1997 (*bid*)*.*

The retroviral packaging cell line is preferably in the form of a transiently transfected cell line. Transient transfections may advantageously be used to measure levels of vector production when vectors are being developed. In this regard, transient transfection avoids the longer time required to generate stable vector-producing cell lines and may also be used if the vector or retroviral packaging components are toxic to cells. Components typically used to generate retroviral vectors include a plasmid encoding the gag-pol proteins, a plasmid encoding the env protein and a plasmid containing an NOI. Vector production involves transient transfection of one or more of these components into cells containing the other required components. If the vector encodes toxic genes or genes that interfere with the replication of the host cell, such as inhibitors of the cell cycle or genes that induce apotosis, it may be difficult to generate stable vector-producing cell lines, but transient transfection can be used to produce the vector before the cells die. Also, cell lines have been developed using transient transfection that produce vector titre levels that are comparable to the levels obtained from stable vector-producing cell lines (25).

Producer cells/packaging cells can be of any suitable cell type. Producer cells are generally mammalian cells but can be, for example, insect cells. A producer cell may be a packaging cell containing the virus structural genes, normally integrated into its genome into which the regulated retroviral vectors of the present invention are introduced. Alternatively the producer cell may be transfected with nucleic acid sequences encoding structural components, such as codon optimised *gag-pol* and *env* on one or more vectors such as plasmids, adenovirus vectors, herpes viral vectors or any method known to deliver functional DNA into target cells. The vectors for use according to the present invention are then introduced into the packaging cell by the methods of the present invention.

As used herein, the term "producer cell" or "vector producing cell" refers to a cell which contains all the elements necessary for production of regulated retroviral vector particles and regulated retroviral delivery systems.

Preferably, the producer cell is obtainable from a stable producer cell line. Preferably, the producer cell is obtainable from a derived stable producer cell line.

Preferably, the producer cell is obtainable from a derived producer cell line As used herein, the term "derived producer cell line" is a transduced producer cell line which has been screened and selected for high expression of a marker gene. Such cell lines contain retroviral insertions in integration sites that support high level expression from the retroviral genome. The term "derived producer cell line" is used interchangeably with the term "derived stable producer cell line" and the term "stable producer cell line

The derived producer cell line is an HIV producer cell line.

Preferably the envelope protein sequences, and nucleocapsid sequences are all stably integrated in the producer and/or packaging cell. However, one or more of these sequences could also exist in episomal form and gene expression could occur from the episome.

As used herein, the term "packaging cell" refers to a cell which contains those elements necessary for production of infectious recombinant virus which are lacking in a recombinant viral vector. Typically, such packaging cells contain one or more vectors which are capable of expressing viral structural proteins (such as codon optimised *gag- pol* and *env*) but they do not contain a packaging signal.

The term "packaging signal" which is referred to interchangeably as "packaging sequence" or *"psi"* is used in reference to the non-coding, *cis*-acting sequence required for encapsidation of retroviral RNA strands during viral particle formation. In HIV-1, this sequence has been mapped to loci extending from upstream of the major splice donor site (SD) to at least the *gag* start codon.

Packaging cell lines suitable for use with the above-described vector constructs may be readily prepared (see also WO 92/05266), and utilised to create producer cell lines for the production of retroviral vector particles. As already mentioned, a summary of the available packaging lines is presented in "Retroviruses" (1997 Cold Spring Harbour Laboratory Press Eds: JM Coffin, SM Hughes, HE Varmus pp 449).

Also as discussed above, simple packaging cell lines, comprising a provirus in which the packaging signal has been deleted, have been found to lead to the rapid production of undesirable replication competent viruses through recombination. In order to improve safety, second generation cell lines have been produced wherein the 3'LTR of the provirus is deleted. In such cells, two recombinations would be necessary to produce a wild type virus. A further improvement involves the introduction of the *gag*-*pol* genes and the *env* gene on separate constructs so-called third generation packaging cell lines. These constructs are introduced sequentially to prevent recombination during transfection (26; 27).

Preferably, the packaging cell lines are second generation packaging cell lines.

Preferably, the packaging cell lines are third generation packaging cell lines.

In these split-construct, third generation cell lines, a further reduction in recombination may be achieved by "codon wobbling". This technique, based on the redundancy of the genetic code, aims to reduce homology between the separate constructs, for example between the regions of overlap in the *gag-pol* and *env* open reading frames.

The packaging cell lines are useful for providing the gene products necessary to encapsidate and provide a membrane protein for a high titre regulated retrovirus vector and regulated nucleic gene delivery vehicle production. When regulated retrovirus sequences are introduced into the packaging cell lines, such sequences are encapsidated with the nucleocapsid (*gag-pol*) proteins and these units then bud through the cell membrane to become surrounded in cell membrane and to contain the envelope protein produced in the packaging cell line. These infectious regulated retroviruses are useful as infectious units *per se* or as gene delivery vectors.

The packaging cell may be a cell cultured *in vitro* such as a tissue culture cell line. Suitable cell lines include but are not limited to mammalian cells such as murine fibroblast derived cell lines or human cell lines. Preferably the packaging cell line is a human cell line, such as for example: HEK293, 293-T, TE671, HT1080.

Alternatively, the packaging cell may be a cell derived from the individual to be treated such as a monocyte, macrophage, blood cell or fibroblast. The cell may be isolated from an individual and the packaging and vector components administered *ex vivo* followed by re-administration of the autologous packaging cells.

It is highly desirable to use high-titre virus preparations in both experimental and practical applications. Techniques for increasing viral titre include using a *psi* plus packaging signal as discussed above and concentration of viral stocks. In addition, the use of different envelope proteins, such as the G protein from vesicular-stomatitis virus has improved titres following concentration to 10⁹ per ml (28). However, typically the envelope protein will be chosen such that the viral particle will preferentially infect cells that are infected with the virus which it desired to treat. For example where an HIV vector is being used to treat HIV infection, the *env* protein used will be the HIV *env* protein.

The process of producing a retroviral vector in which the envelope protein is not the native envelope of the retrovirus is known as "pseudotyping". Certain envelope proteins, such as MLV envelope protein and vesicular stomatitis virus G (VSV-G) protein, pseudotype retroviruses very well. Pseudotyping is not a new phenomenon and examples may be found in WO-A-98/05759, WO-A-98/05754, WO-A-97/17457, WO-A-96/09400, WO-A-91/00047 and (29).

As used herein, the term "high titre" means an effective amount of a retroviral vector or particle which is capable of transducing a target site such as a cell.

As used herein, the term "effective amount" means an amount of a regulated retroviral or lentiviral vector or vector particle which is sufficient to induce expression of an NOI at a target site.

Preferably the titre is from at least 10⁶ retrovirus particles per ml, such as from 10⁶ to 10⁷ per ml, more preferably at least 10⁷ retrovirus particles per ml.

In accordance with the present invention, it is possible to manipulate the viral genome or the regulated retroviral vector nucleotide sequence, so that viral genes are replaced or supplemented with one or more NOIs which may be heterologous NOIs.

The term "heterologous" refers to a nucleic acid sequence or protein sequence linked to a nucleic acid or protein sequence which it is not naturally linked.

With the present invention, the term NOI (i.e. nucleotide sequence of interest) includes any suitable nucleotide sequence, which need not necessarily be a complete naturally occurring DNA sequence. Thus, the DNA sequence can be, for example, a synthetic DNA sequence, a recombinant DNA sequence (i.e. prepared by use of recombinant DNA techniques), a cDNA sequence or a partial genomic DNA sequence, including combinations thereof. The DNA sequence need not be a coding region. If it is a coding region, it need not be an entire coding region. In addition, the DNA sequence can be in a sense orientation or in an anti-sense orientation. Preferably, it is in a sense orientation. Preferably, the DNA is or comprises cDNA.

The NOI(s) may be any one or more of selection gene(s), marker gene(s) and therapeutic gene(s).

As used herein, the term "selection gene" refers to the use of a NOI which encodes a selectable marker which may have an enzymatic activity that confers resistance to an antibiotic or drug upon the cell in which the selectable marker is expressed.

Many different selectable markers have been used successfully in retroviral vectors. These are reviewed in "retroviruses" (1997 Cold Spring Harbour Laboratory Press Eds: JM Coffin, SM Hughes, HE Varmus pp 444) and include, but are not limited to, the bacterial neomycin (neo) and hygromycin phosphotransferase genes which confer resistance to G418 and hygromycin respectively; a mutant mouse dihydrofolate reductase gene which confers resistance to methotrexate; the bacterial *gpt* gene which allows cells to grow in medium containing mycophenolic acid, xanthine and aminopterin; the bacterial *hisD* gene which allows cells to grow in medium without histidine but containing histidinol; the multidrug resistance gene (*mdr*) which confers resistance to a variety of drugs; and the bacterial genes which confer resistance to puromycin or phleomycin. All of these markers are dominant selectable and allow chemical selection of most cells expressing these genes. Other selectable markers are not dominant in that their use must be in conjunction with a cell line that lacks the relevant enzyme activity. Examples of non-dominant selectable markers include the thymidine kinase (*tk*) gene which is used in conjunction with *tk* cell lines.

Particularly preferred markers are blasticidin and neomycin, optionally operably linked to a thymidine kinase coding sequence typically under the transcriptional control of a strong viral promoter such the SV40 promoter.

In accordance with the present invention, suitable NOI sequences include those that are of therapeutic and/or diagnostic application such as, but are not limited to: sequences encoding cytokines, chemokines, hormones, antibodies, engineered immunoglobulin-like molecules, a single chain antibody, fusion proteins, enzymes, immune co-stimulatory molecules, immunomodulatory molecules, anti-sense RNA, a transdominant negative mutant of a target protein, a toxin, a conditional toxin, an antigen, a tumour suppressor protein and growth factors, membrane proteins, vasoactive proteins and peptides, anti-viral proteins and ribozymes, and derivatives therof (such as with an associated reporter group). When included, such coding sequences may be typically operatively linked to a suitable promoter, which may be a promoter driving expression of a ribozyme(s), or a different promoter or promoters, such as in one or more specific cell types.

Suitable NOIs for use in the invention in the treatment or prophylaxis of cancer include NOIs encoding proteins which: destroy the target cell (for example a ribosomal toxin), act as: tumour suppressors (such as wild-type p53); activators of anti-tumour immune mechanisms (such as cytokines, co-stimulatory molecules and immunoglobulins); inhibitors of angiogenesis; or which provide enhanced drug sensitivity (such as pro-drug activation enzymes); indirectly stimulate destruction of target cell by natural effector cells (for example, strong antigen to stimulate the immune system or convert a precursor substance to a toxic substance which destroys the target cell (for example a prodrug activating enzyme).

Examples of prodrugs include but are not limited to etoposide phosphate (used with alkaline phosphatase; 5-fluorocytosine (with cytosine deaminase); Doxorubin-N-p-hydroxyphenoxyacetamide (with Penicillin-V-Amidase); Para-N-bis (2-chloroethyl)aminobenzoyl glutamate (with Carboxypeptidase G2); Cephalosporin nitrogen mustard carbamates (with B-lactamase); SR4233 (with p450 reductase); Ganciclovir (with HSV thymidine kinase); mustard pro-drugs with nitroreductase and cyclophosphamide or ifosfamide (with cytochrome p450).

Suitable NOIs for use in the treatment or prevention of ischaemic heart disease include NOIs encoding plasminogen activators. Suitable NOIs for the treatment or prevention of rheumatoid arthritis or cerebral malaria include genes encoding anti-inflammatory proteins, antibodies directed against tumour necrosis factor (TNF) alpha, and anti-adhesion molecules (such as antibody molecules or receptors specific for adhesion molecules).

The expression products encoded by the NOIs may be proteins which are secreted from the cell. Alternatively the NOI expression products are not secreted and are active within the cell. In either event, it is preferred for the NOI expression product to demonstrate a bystander effect or a distant bystander effect; that is the production of the expression product in one cell leading to the killing of additional, related cells, either neighbouring or distant (e.g. metastatic), which possess a common phenotype. Encoded proteins could also destroy bystander tumour cells (for example with secreted antitumour antibody-ribosomal toxin fusion protein), indirectly stimulated destruction of bystander tumour cells (for example cytokines to stimulate the immune system or procoagulant proteins causing local vascular occlusion) or convert a precursor substance to a toxic substance which destroys bystander tumour cells (eg an enzyme which activates a prodrug to a diffusible drug). Also, the delivery of NOI(s) encoding antisense transcripts or ribozymes which interfere with expression of cellular genes for tumour persistence (for example against aberrant *myc* transcripts in Burkitts lymphoma or against *bcr-abl* transcripts in chronic myeloid leukemia. The use of combinations of such NOIs is also envisaged.

The NOI or NOIs for use in the present invention may also comprise one or more cytokine-encoding NOIs. Suitable cytokines and growth factors include but are not limited to: ApoE, Apo-SAA, BDNF, Cardiotrophin-1, EGF, ENA-78, Eotaxin, Eotaxin-2, Exodus-2, FGF-acidic, FGF-basic, fibroblast growth factor-10 (30). FLT3 ligand, Fractalkine (CX3C), GDNF, G-CSF, GM-CSF, GF-β1, insulin, IFN-γ, IGF-I, IGF-II, IL-1a, IL-1β, IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-18 (IGIF), Inhibin α, Inhibin β, IP-10, keratinocyte growth factor-2 (KGF-2), KGF, Leptin, LIF, Lymphotactin, Mullerian inhibitory substance, monocyte colony inhibitory factor, monocyte attractant protein (30 *ibid*)*,* M-CSF, MDC (67 a.a.), MDC (69 a.a.), MCP-1 (MCAF), MCP-2, MCP-3, MCP-4, MDC (67 a.a.), MDC (69 a.a.), MIG, MIP-1α, MIP-1β, MIP-3α, MIP-3β, MIP-4, myeloid progenitor inhibitor factor-1 (MPIF-1 ), NAP-2, Neurturin, Nerve growth factor, β-NGF, NT-3, NT-4, Oncostatin M, PDGF-AA, PDGF-AB, PDGF-BB, PF-4, RANTES, SDF1α, SDF1β, SCF, SCGF, stem cell factor (SCF), TARC, TGF-α, TGF-β, TGF-β2, TGF-β3, tumour necrosis factor (TNF), TNF-α, TNF-β, TNIL-1, TPO, VEGF, GCP-2, GRO/MGSA, GRO-β, GRO-γ, HCC1, 1-309.

The NOI or NOIs may be under the expression control of an expression regulatory element, such as a promoter and/or a promoter enhancer as known as "responsive elements".

When the regulated retroviral vector particles are used to transfer NOIs into cells which they transduce, such vector particles also designated "viral delivery systems" or "retroviral delivery systems". Viral vectors, including retroviral vectors, have been used to transfer NOIs efficiently by exploiting the viral transduction process. NOIs cloned into the retroviral genome can be delivered efficiently to cells susceptible to transduction by a retrovirus. Through other genetic manipulations, the replicative capacity of the retroviral genome can be destroyed. The vectors introduce new genetic material into a cell but are unable to replicate.

The regulated retroviral vector for use according to the present invention can be delivered by viral or non-viral techniques. Non-viral delivery systems include but are not limted to DNA transfection methods. Here, transfection includes a process using a non-viral vector to deliver a gene to a target mammalian cell.

Typical transfection methods include electroporation, DNA biolistics, lipid-mediated transfection, compacted DNA-mediated transfection, liposomes, immunoliposomes, lipofectin, cationic agent-mediated, cationic facial amphiphiles (CFAs) (31), multivalent cations such as spermine, cationic lipids or polylysine, 1, 2,-bis (oleoyloxy)-3-(trimethylammonio) propane (DOTAP)-cholesterol complexes (32) and combinations thereof.

Viral delivery systems include but are not limited to adenovirus vector, an adeno-associated viral (AAV) vector, a herpes viral vector, a retroviral vector, a lentiviral vector, or a baculoviral vector. These viral delivery systems may be configured as a split-intron vector. A split intron vector is described in WO 99/15683.

Other examples of vectors include *ex vivo* delivery systems, which include but are not limited to DNA transfection methods such as electroporation, DNA biolistics, lipid-mediated transfection, compacted DNA-mediated transfection.

The vector may be a plasmid DNA vector. Alternatively, the vector may be a recombinant viral vector. Suitable recombinant viral vectors include adenovirus vectors, adeno-associated viral (AAV) vectors, Herpes-virus vectors, or retroviral vectors, lentiviral vectors or a combination of adenoviral and lentiviral vectors. In the case of viral vectors, gene delivery is mediated by viral infection of a target cell.

If the features of adenoviruses are combined with the genetic stability of retro/lentiviruses then essentially the adenovirus can be used to transduce target cells to become transient retroviral producer cells that could stably infect neighbouring cells.

Also disclosed is a pharmaceutical composition for treating an individual by gene therapy, wherein the composition comprises a therapeutically effective amount of a regulated retroviral vector as described. The pharmaceutical composition may be for human or animal usage. Typically, a physician will determine the actual dosage which will be most suitable for an individual subject and it will vary with the age, weight and response of the particular patient.

The composition may optionally comprise a pharmaceutically acceptable carrier, diluent, excipient or adjuvant. The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s), and other carrier agents that may aid or increase the viral entry into the target site (such as for example a lipid delivery system).

Where appropriate, the pharmaceutical compositions can be administered by any one or more of: minipumps; inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intracavernosally, intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

The present invention is believed to have a wide therapeutic applicability - depending on *inter alia* the selection of the one or more NOIs.

For example, the present invention may be useful in the treatment of the disorders listed in WO-A-98/05635. For ease of reference, part of that list is now provided: cancer, inflammation or inflammatory disease, dermatological disorders, fever, cardiovascular effects, haemorrhage, coagulation and acute phase response, cachexia, anorexia, acute infection, HIV infection, shock states, graft-versus-host reactions, autoimmune disease, reperfusion injury, meningitis, migraine and aspirin-dependent anti-thrombosis; tumour growth, invasion and spread, angiogenesis, metastases, malignant, ascites and malignant pleural effusion; cerebral ischaemia, ischaemic heart disease, osteoarthritis, rheumatoid arthritis, osteoporosis, asthma, multiple sclerosis, neurodegeneration, Alzheimer's disease, atherosclerosis, stroke, vasculitis, Crohn's disease and ulcerative colitis; periodontitis, gingivitis; psoriasis, atopic dermatitis, chronic ulcers, epidermolysis bullosa; corneal ulceration, retinopathy and surgical wound healing; rhinitis, allergic conjunctivitis, eczema, anaphylaxis; restenosis, congestive heart failure, endometriosis, atherosclerosis or endosclerosis.

In addition, or in the alternative, the present invention may be useful in the treatment of disorders listed in WO-A-98/07859. For ease of reference, part of that list is now provided: cytokine and cell proliferation/differentiation activity; immunosuppressant or immunostimulant activity (e.g. for treating immune deficiency, including infection with human immune deficiency virus; regulation of lymphocyte growth; treating cancer and many autoimmune diseases, and to prevent transplant rejection or induce tumour immunity); regulation of haematopoiesis, e.g. treatment of myeloid or lymphoid diseases; promoting growth of bone, cartilage, tendon, ligament and nerve tissue, e.g. for healing wounds, treatment of burns, ulcers and periodontal disease and neurodegeneration; inhibition or activation of follicle-stimulating hormone (modulation of fertility); chemotactic/chemokinetic activity (e.g. for mobilising specific cell types to sites of injury or infection); haemostatic and thromtiolytic activity (e.g. for treating haemophilia and stroke); antiinflammatory activity (for treating e.g. septic shock or Crohn's disease); as antimicrobials; modulators of e.g. metabolism or behaviour; as analgesics; treating specific deficiency disorders; in treatment of e.g. psoriasis, in human or veterinary medicine.

In addition, or in the alternative, the present invention may be useful in the treatment of disorders listed in WO-A-98/09985. For ease of reference, part of that list is now provided: macrophage inhibitory and/or T cell inhibitory activity and thus, anti-inflammatory activity; anti-immune activity, i.e. inhibitory effects against a cellular and/or humoral immune response, including a response not associated with inflammation; inhibit the ability of macrophages and T cells to adhere to extracellular matrix components and fibronectin, as well as up-regulated fas receptor expression in T cells; inhibit unwanted immune reaction and inflammation including arthritis, including rheumatoid arthritis, inflammation associated with hypersensitivity, allergic reactions, asthma, systemic lupus erythematosus, collagen diseases and other autoimmune diseases, inflammation associated with atherosclerosis, arteriosclerosis, atherosclerotic heart disease, reperfusion injury, cardiac arrest, myocardial infarction, vascular inflammatory disorders, respiratory distress syndrome or other cardiopulmonary diseases, inflammation associated with peptic ulcer, ulcerative colitis and other diseases of the gastrointestinal tract, hepatic fibrosis, liver cirrhosis or other hepatic diseases, thyroiditis or other glandular diseases, glomerulonephritis or other renal and urologic diseases, otitis or other oto-rhino-laryngological diseases, dermatitis or other dermal diseases, periodontal diseases or other dental diseases, orchitis or epididimo-orchitis, infertility, orchidal trauma or other immune-related testicular diseases, placental dysfunction, placental insufficiency, habitual abortion, eclampsia, pre-eclampsia and other immune and/or inflammatory-related gynaecological diseases, posterior uveitis, intermediate uveitis, anterior uveitis, conjunctivitis, chorioretinitis, uveoretinitis, optic neuritis, intraocular inflammation, e.g. retinitis or cystoid macular oedema, sympathetic ophthalmia, scleritis, retinitis pigmentosa, immune and inflammatory components of degenerative fondus disease, inflammatory components of ocular trauma, ocular inflammation caused by infection, proliferative vitreo-retinopathies, acute ischaemic optic neuropathy, excessive scarring, e.g. following glaucoma filtration operation, immune and/or inflammation reaction against ocular implants and other immune and inflammatory-related ophthalmic diseases, inflammation associated with autoimmune diseases or conditions or disorders where, both in the central nervous system (CNS) or in any other organ, immune and/or inflammation suppression would be beneficial, Parkinson's disease, complication and/or side effects from treatment of Parkinson's disease, AIDS-related dementia complex HIV-related encephalopathy, Devic's disease, Sydenham chorea, Alzheimer's disease and other degenerative diseases, conditions or disorders of the CNS, inflammatory components of stokes, post-polio syndrome, immune and inflammatory components of psychiatric disorders, myelitis, encephalitis, subacute sclerosing pan-encephalitis, encephalomyelitis, acute neuropathy, subacute neuropathy, chronic neuropathy, Guillaim-Barre syndrome, Sydenham chora, myasthenia gravis, pseudo-tumour cerebri, Down's Syndrome, Huntington's disease, amyotrophic lateral sclerosis, inflammatory components of CNS compression or CNS trauma or infections of the CNS, inflammatory components of muscular atrophies and dystrophies, and immune and inflammatory related diseases, conditions or disorders of the central and peripheral nervous systems, post-traumatic inflammation, septic shock, infectious diseases, inflammatory complications or side effects of surgery, bone marrow transplantation or other transplantation complications and/or side effects, inflammatory and/or immune complications and side effects of gene therapy, e.g. due to infection with a viral carrier, or inflammation associated with AIDS, to suppress or inhibit a humoral and/or cellular immune response, to treat or ameliorate monocyte or leukocyte proliferative diseases, e.g. leukaemia, by reducing the amount of monocytes or lymphocytes, for the prevention and/or treatment of graft rejection in cases of transplantation of natural or artificial cells, tissue and organs such as cornea, bone marrow, organs, lenses, pacemakers, natural or artificial skin tissue.

The invention will now be further described by way of Examples, which are meant to serve to assist one of ordinary skill in the art in carrying out the invention and are not intended in any way to limit the scope of the invention. The Examples refer to the Figures. In the Figures:

### Description of the Figures

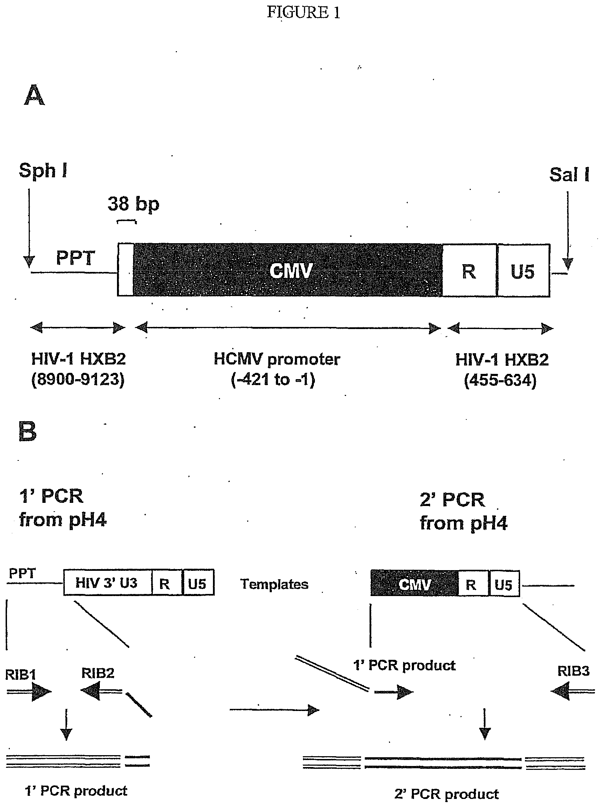

Figure 1 shows schematically how to create a suitable 3' LTR by PCR;

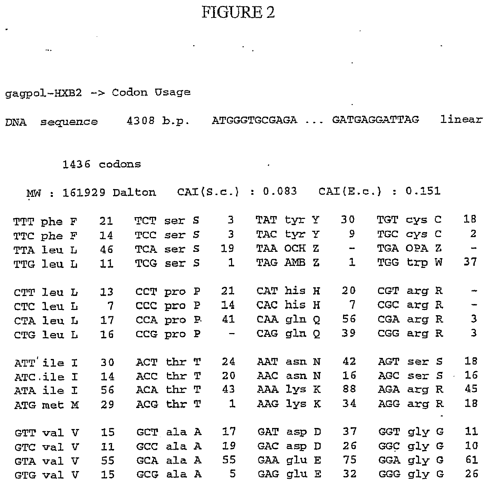

Figure 2 shows the codon usage table for wild type HIV *gag-pol* of strain HXB2 (accession number: K03455);
Figure 3a shows the codon usage table of the codon optimised sequence designated gagpol-SYNgp. Figure 3b shows a comparative codon usage table;

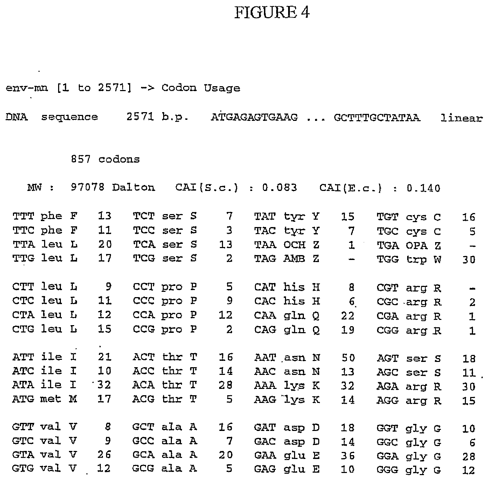

Figure 4 shows the codon usage table of the wild type HIV *env* called env-mn;

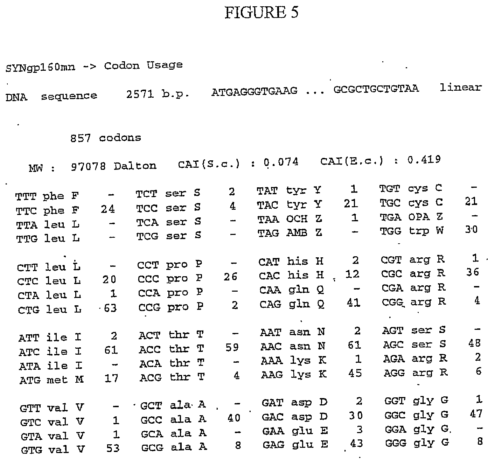

Figure 5 shows the codon usage table of the codon optimised sequence of HIV *env* designated SYNgp160mn;

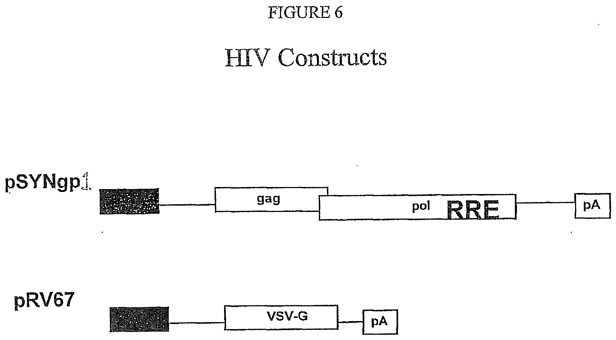

Figure 6 shows two plasmid constructs for use in the invention;

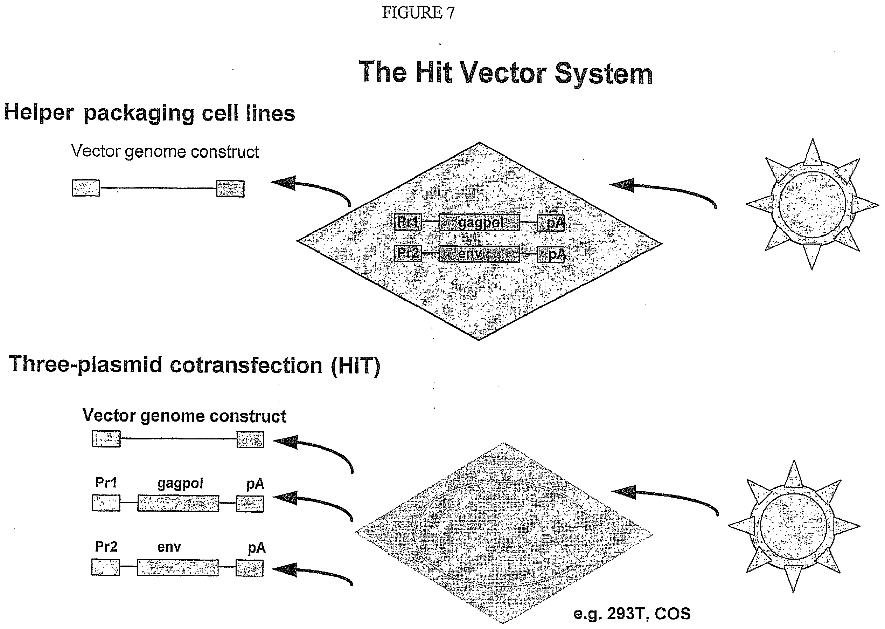

Figure 7 shows the principle behind two systems for producing retroviral vector particles;
Figure 8 shows a sequence comparison between the wild type HIV *gag-pol* sequence (pGP-RRE3) and the codon optimised *gag-pol* sequence (pSYNGP);
Figure 9 shows a sequence comparison between the wild type EIAV *gag-pol* sequence (WT) and the codon optimised *gag-pol* sequence (CO);
Figure 10 shows Rev independence of protein expression particle formation;
Figure 11 shows translation rates of wild-type (WT) and codon optimised *gag-pol;*
Figure 12 shows *gag-pol* mRNA levels in total and cytoplasmic fractions;
Figure 13 shows the effect of insertion of WT *gag* downstream of the codon optimised gene on RNA and protein levels;
Figure 14 shows the plasmids used to study the effect of HIV-1 *gag* on the codon optimised gene;
Figure 15 shows the effect on cytoplasmic RNA of insertion of HIV-1 *gag* upstream of the codon optimised gene;
Figure 16 shows the effect of Leptomycin B (LMB) on protein production;
Figure 17 shows the cytoplasmic RNA levels of the vector genomes;
Figure 18 shows transduction efficiency at MOI 1;
Figure 19 shows a schematic representation of pGP-RRE3;
Figure 20 shows a schematic representation of pSYNGP;
Figure 21 shows vector titres generated with different *gag-pol* constructs;
Figure 22 shows vector titres from the Rev/RRE (-) and (+) genomes;
Figure 23 shows vector titres from the pHS series of vector genomes;
Figure 24 shows vector titres for the pHS series of vector genomes in the presence or absence of Rev/RRE;
Figure 25 shows an analysis of *gag-pol* constructs;
Figure 26 shows a Western blot of 293T extracts;
Figure 27 is a schematic representation of pESYNGP;
Figure 28 is a schematic representation of LpESYNGP;
Figure 29 is a schematic representation of LpESYNGPRRE;
Figure 30 is a schematic representation of pESYNGPRRE;
Figure 31 is a schematic representation of pONY4.0Z;
Figure 32 is a schematic representation of pONY8.0Z;
Figure 33 is a schematic representation of pONY8.1 Z;
Figure 34 is a schematic representation of pONY3.1;
Figure 35 is a schematic representation of pCIneoERev;
Figure 36 is a schematic representation of pESYNREV;
Figures 37 and 38 show the effect of different vector constructs on viral vector titres;
Figures 39 and 40 show the effect of different vector constructs on RT activity;
Figure 41 shows the effect of the 5' leader sequence on viral vector titre;
Figure 42 shows viral vector titres when using pONY8.1Z;
Figure 43 shows a comparison between the sequences of pONY3.1 and codon optimised pONY3.2OPTI in the first 372 nucelotides of *gag*;
Figure 44 is a schematic representation of pIRES1hygESYNGP;
Figure 45 and 46 show the results of experiments to confirm that codon optimised *gag-pol* can be used in the production of packaging and producer cell lines;
Figures 47 and 48 show the results of experiments which confirm that RNA from codon optimised *gag-pol* is packaged less efficiently than that from the wild-type gene;
Figure 49 shows the results of an experiment which confirms that expression from pESYNGP and pESDSYNGP are similar;
Figure 50 is a schematic representation of pESDSYNGP; and
Figure 51 shows the results of an experiment which confirms that the efficiency of encapsidating *gag-pol* RNA in PEV-17 cells and B-241 cells in similar.

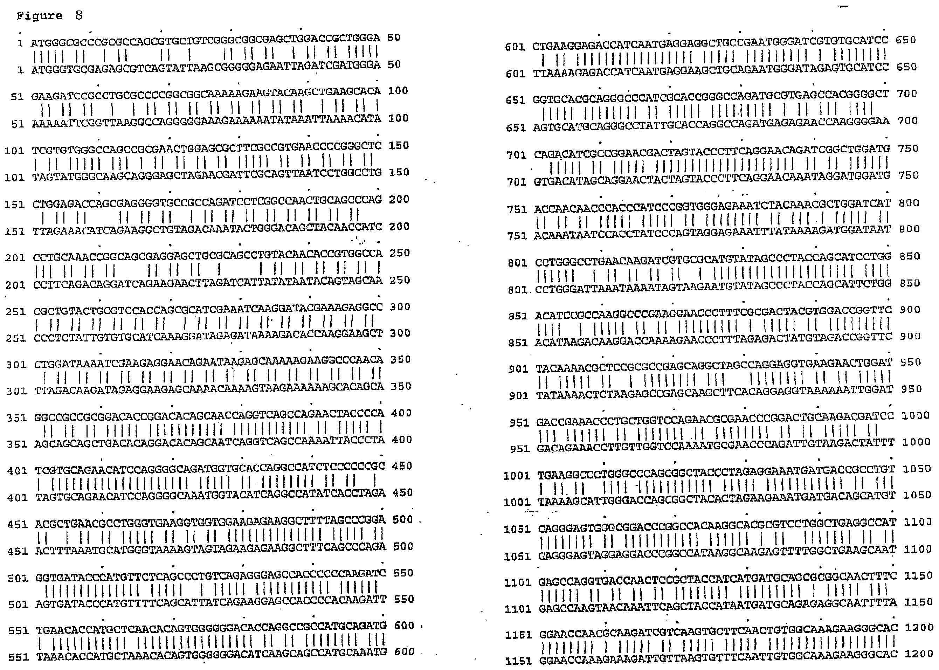

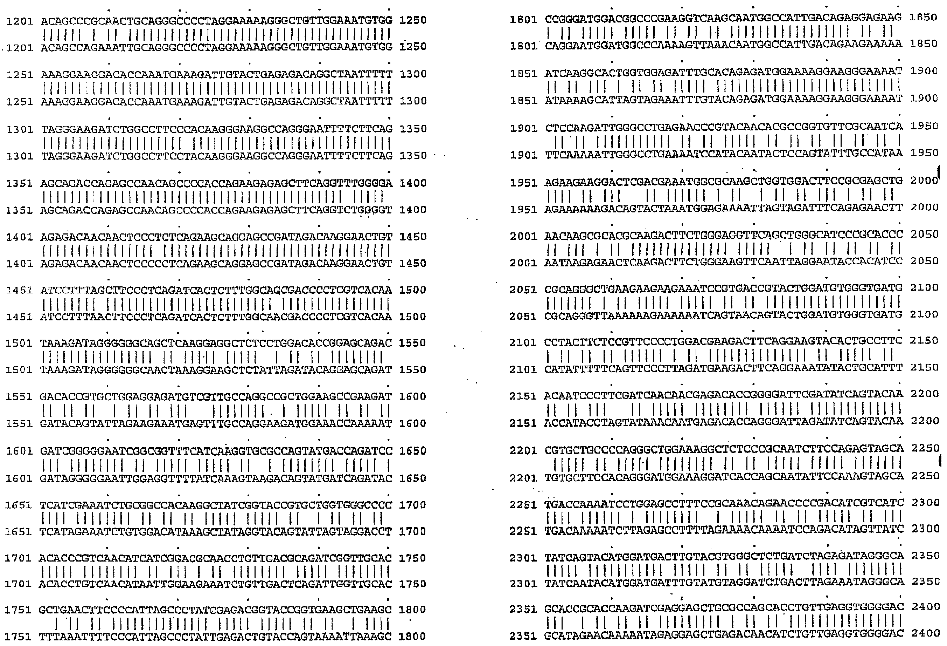

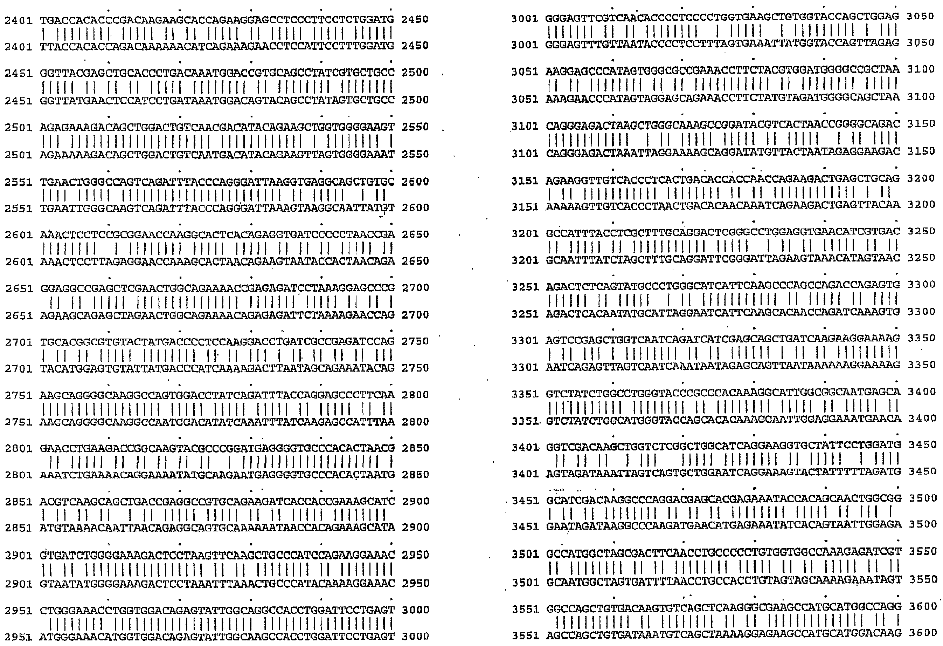

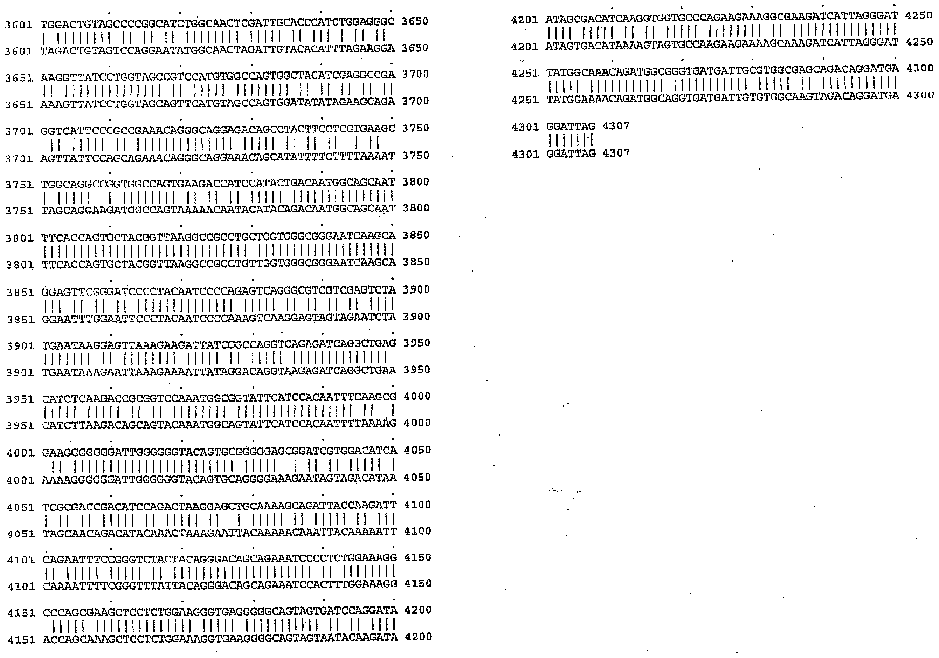

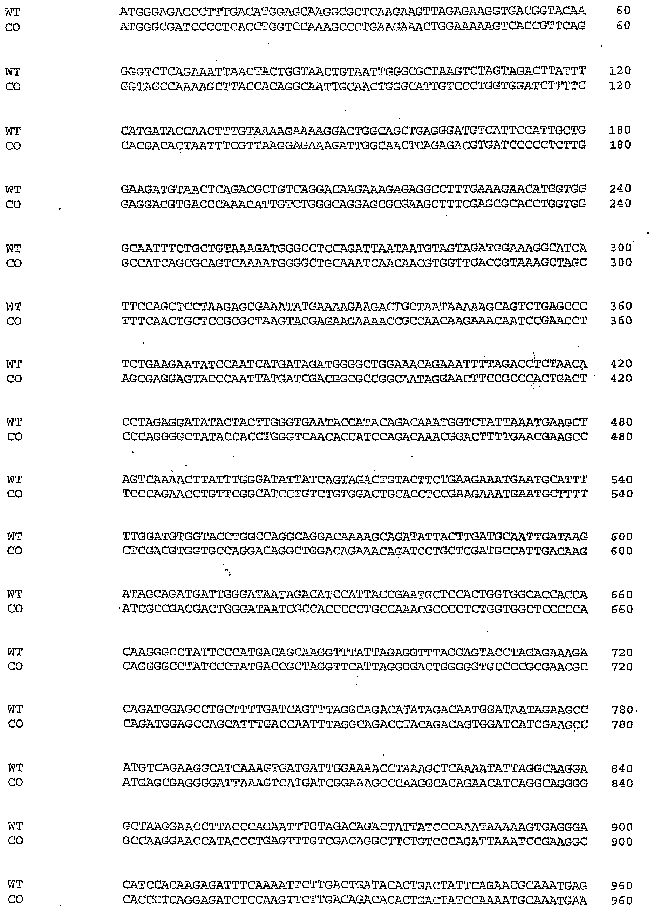

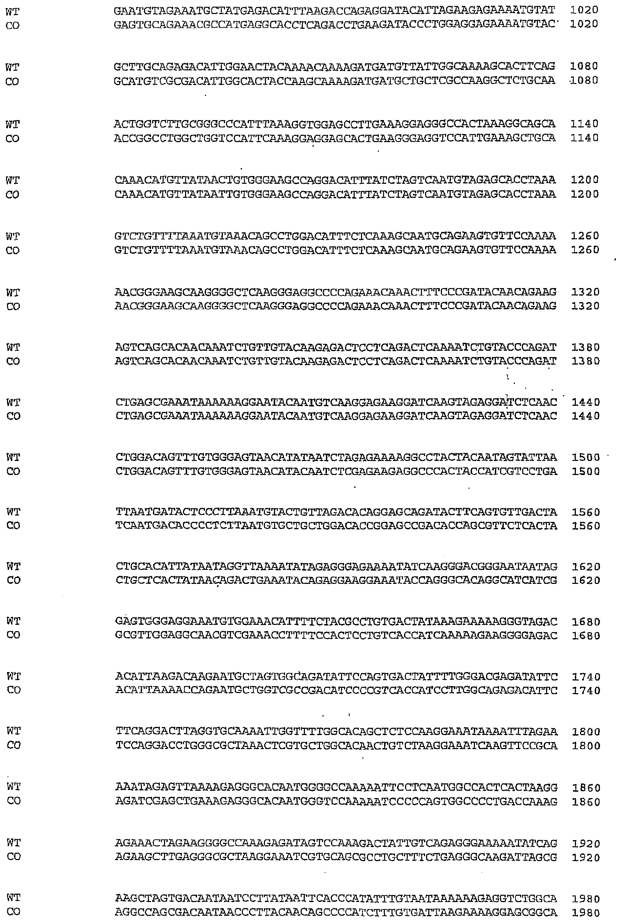

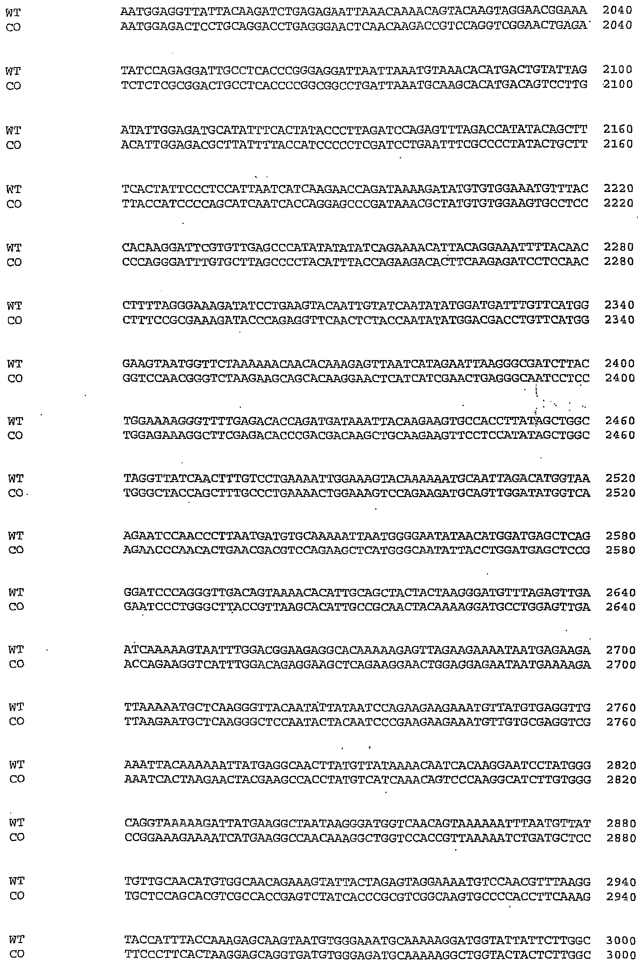

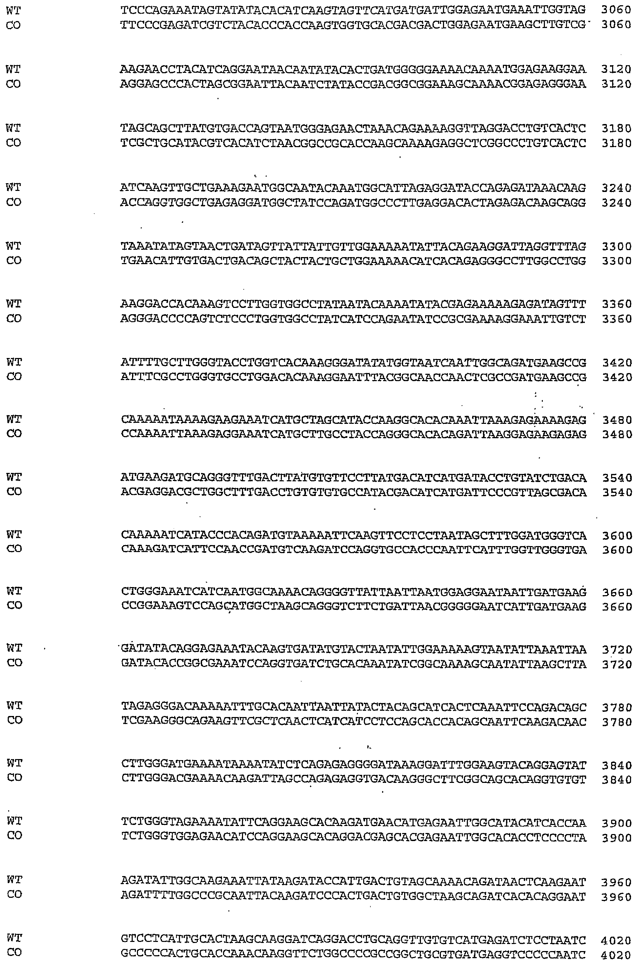

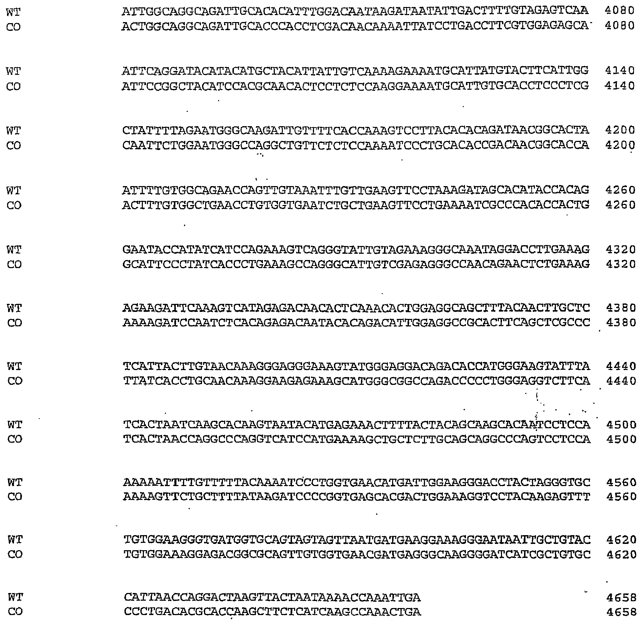

In more detail, Figure 8 shows a sequence comparison between the wild type HIV *gag-pol* sequence (pGP-RRE3) and the codon optimised *gag-pol* sequence (pSYNGP) wherein the upper sequence represents pSYNGP and the lower sequence represents pGP-RRE3.

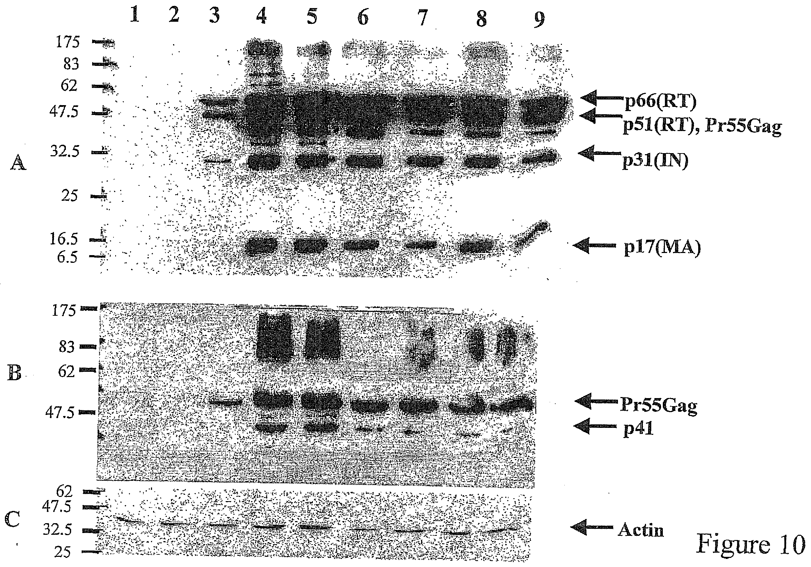

Figure 10 shows Rev independence of protein expression particle formation. 5µg of the *gag*-*pol* expression plasmids were transfected into 293T cells in the presence or absence of Rev (pCMV-Rev, 1µg) and protein levels were determined 48 hours post transfection in culture supernatants (A) and cell lysates (B). HIV-1 positive human serum was used to detect the gag-pol proteins. The blots were re-probed with an anti-actin antibody, as an internal control (C). The protein marker (New England Biolabs) sizes (in kDa) are shown on the side of the gel. Lanes: 1. Mock transfected 293T cells, 2. pGP-RRE3, 3. pGP-RRE3 + pCMV-Rev, 4. pSYNGP, 5. pSYNGP + pCMV-Rev, 6. pSYNGP-RRE, 7. pSYNGP-RRE +pCMV-Rev, 8. pSYNGP-ERR, 9. pSYNGP-ERR + pCMV-Rev.

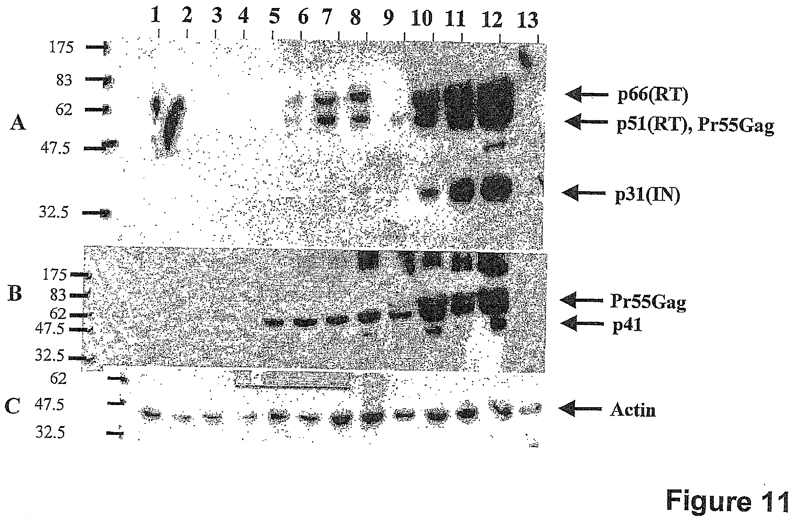

Figure 11 shows translation rates of WT and codon optimised *gag*-*pol*. 293T cells were transfected with 2µg pGP-RRE3 (+/- 1µg pCMV-Rev) or 2µg pSYNGP. Protein samples from culture supernatants (A) and cell extracts (B) were analysed by Western blotting 12, 25, 37 and 48 hours post-transfection. HIV-1 positive human serum was used to detect gag-pol proteins (A, B) and an anti-actin antibody was used as an internal control (C). The protein marker sizes are shown on the side of the gel (in kD). A Phosphorimager was used for quantification of the results. Lanes: 1. pGP-RRE3 12h, 2. pGP-RRE3 25h, 3. pGP-RRE3 37h, 4. pGP-RRE3 48h, 5. pGP-RRE3 + pCMV-Rev 12h, 6. pGP-RRE3 +pCMV-Rev 25h, 7. pGP-RRE3 + pCMV-Rev 37h, 8. pGP-RRE3 + pCMV-Rev 48h, 9. pSYNGP 12h, 10. pSYNGP 25h, 11. pSYNGP 37h, 12. pSYNGP 48h, 13. Mock transfected 293T cells.

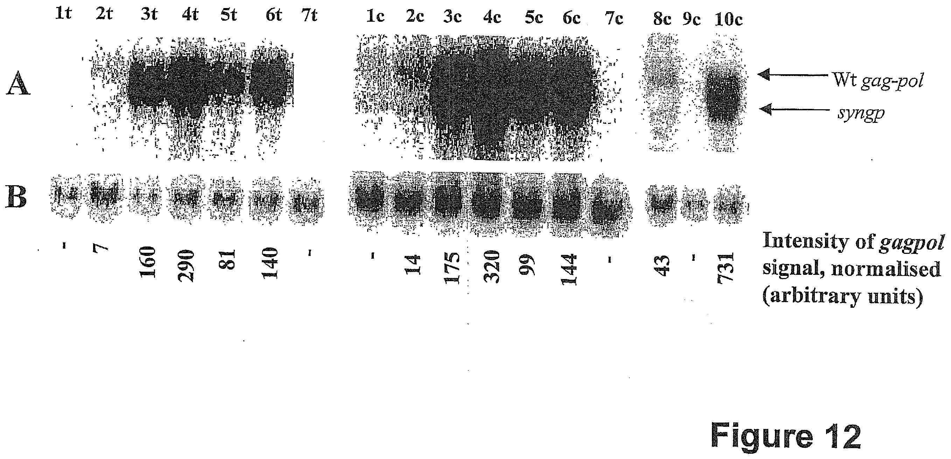

Figure 12 shows *gag-pol* mRNA levels in total and cytoplasmic fractions. Total and cytoplasmic RNA was extracted from 293T cells 36 hours after transfection with 5µg of the *gag-pol* expression plasmid (+/- 1µg pCMV-Rev) and mRNA levels were estimated by Northern blot analysis. A probe complementary to nt 1222-1503 of both the wild type and codon optimised gene was used. Panel A shows the band corresponding to the HIV-1 *gag*-*pol*. The sizes of the mRNAs are 4.4 kb for the codon optimised and 6 kb for the wild type gene. Panel B shows the band corresponding to human ubiquitin (internal control for normalisation of results). Quantification was performed using a Phosphorimager. Lane numbering: c indicates cytoplasmic fraction and t indicates total RNA fraction. Lanes: 1. pGP-RRE3 , 2. pGP-RRE3 + pCMV-Rev, 3. pSYNGP, 4. pSYNGP + pCMV-Rev, 5. pSYNGP-RRE, 6. pSYNGP-RRE + pCMV-Rev, 7. Mock transfected 293T cells, 8. pGP-RRE3 + pCMV-Rev, 9. Mock transfected 293T cells, 10. pSYNGP.

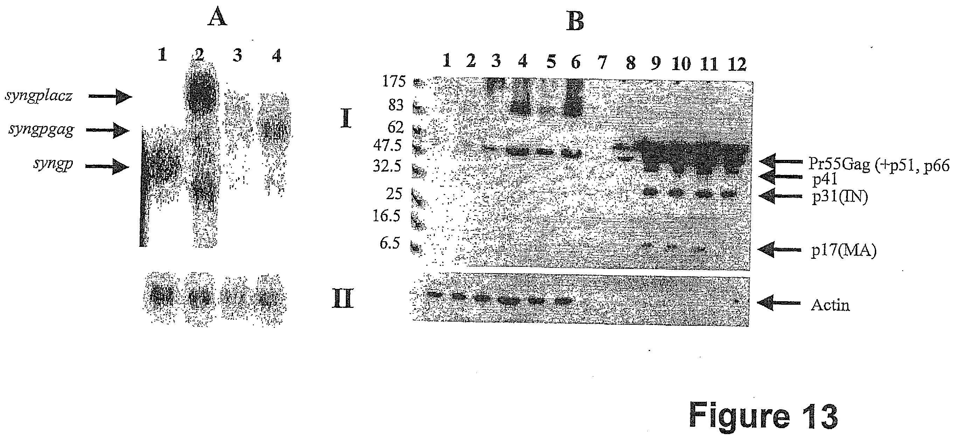

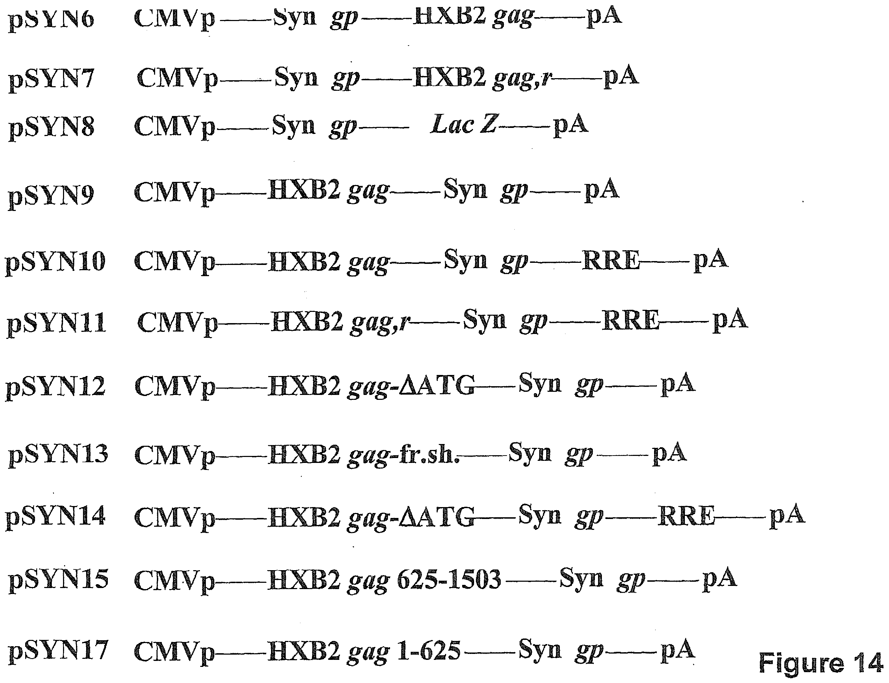

Figure 13 shows the effect of insertion of WT *gag* downstream of the codon optimised gene on RNA and protein levels. The wt *gag* sequence was inserted downstream of the codon optimised gene in both orientations (*Not*I site), resulting in plasmids pSYN6 (correct orientation, see Figure 14) and pSYN7 (reverse orientation, see Figure 14). The gene encoding for β-galactosidase (LacZ) was also inserted in the same site and the correct orientation (plasmid pSYN8, see Figure 14). 293T cells were transfected with 5µg of each plasmid and 48 hours post transfection mRNA and protein levels were determined as previously described by means of Northern and Western blot analysis respectively.

Northern blot analysis in cytoplasmic RNA fractions. The blot was probed with a probe complementary to nt 1510-2290 of the codon optimised gene (I) and was re-probed with a probe specific for human ubiquitin (II). Lanes: 1. pSYNGP, 2. pSYN8, 3. pSYN7, 4. pSYN6
Western blot analysis: HIV-1 positive human serum was used to detect the gag-pol proteins (I) and an anti-actin antibody was used as an internal control (II). Lanes: Cell lysates: 1. Mock transfected 293T cells, 2. pGP-RRE3 + pCMV-Rev, 3. pSYNGP, 4. pSYN6, 5. pSYN7, 6. pSYN8. Supernatants: 7. Mock transfected 293T cells, 8. pGP-RRE3 + pCMV-Rev, 9. pSYNGP, 10. pSYN6, 11. pSYN7, 12. pSYN8. The protein marker (New England Biolabs) sizes are shown on the side of the gel.

Figure 14 shows the plasmids used to study the effect of HIV-1 *gag* on the codon optimised gene. The backbone for all constructs was pCI-Neo. Syn *gp:* The codon optimised HIV-1 *gag-pol* gene. HXB2 *gag:* The wild type Hit-1 *gag* gene. HXB2 *gag,*r: The wild type HIV-1 *gag* gene in the reverse orientation. HXB2 *gag*ΔATG: The wild type HIV-1 *gag* gene without the *gag* ATG. HXB2 *gag*-fr.sh.: The wild type HIV-1 *gag* gene with a frameshift mutation. HXB2 *gag* 625-1503: Nucleotides 625-1503 of the wild type HIV-1 *gag* gene. HXB2 *gag* 1-625: Nucleotides 1-625 of the wild type HIV-1 *gag* gene.

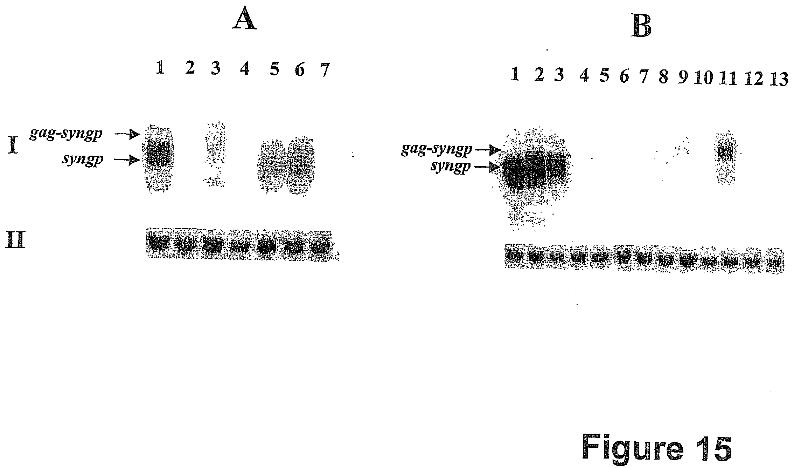

Figure 15 shows the effect on cytoplasmic RNA of insertion of HIV-1 *gag* upstream of the codon optimised gene. Cytoplasmic RNA was extracted 48 hours post transfection of 293T cells (5µg of each pSYN plasmid was used and 1 µg of pCMV-Rev was co-transfected in some cases). The probe that was used was designed to be complementary to nt 1510-2290 of the codon optimised gene (I). A probe specif for human ubiquitin was used as an internal control (II). Lanes: 1. pSYNGP, 2. pSYN9, 3. pSYN10 4. pSYN10 + pCMV-Rev, 5. pSYN11, 6. pSYN11 + pCMV-Rev, 7. pCMV-Rev.
Lanes: 1. pSYNGP, 2. pSYNGP-RRE, 3. pSYNGP-RRE + pCMV-Rev, 4. pSYN12, 5. pSYN14, 6. pSYN14 + pCMV-Rev, 7. pSYN13, 8. pSYN15, 9. pSYN17, 10. pGP-RRE3, 11. pSYN6, 12. pSYN9, 13. pCMV-Rev.

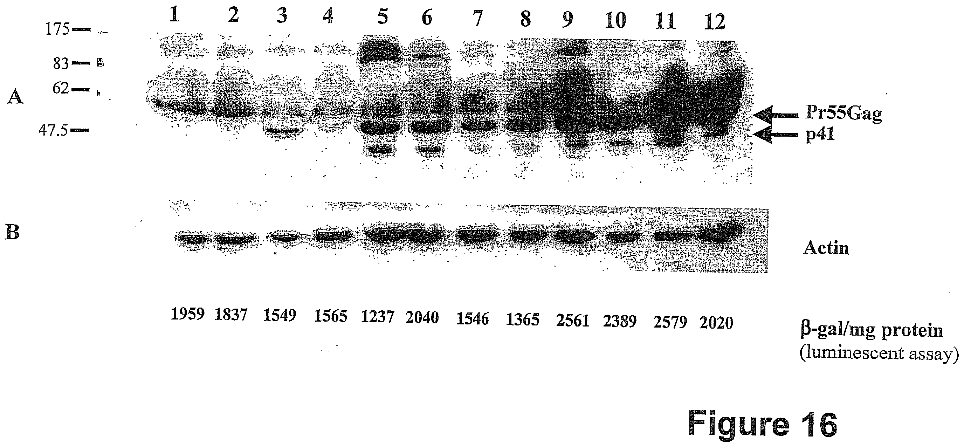

Figure 16 shows the effect of LMB on protein production. 293T cells were transfected with 1µg pCMV-Rev and 3µg of pGP-RRE3/pSYNGP/pSYNGP-RRE (+/- 1µg pCMV-Rev). Transfections were done in duplicate. 5 hours post transfection the medium was replaced with fresh medium in the first set and with fresh medium containing 7.5 nM LMB in the second. 20 hours later the cells were lysed and protein production was estimated by Western blot analysis. HIV-1 positive human serum was used to detect the gag-pol proteins (A) and an anti-actin antibody was used as an internal control (B). Lanes: 1. pGP-RRE3, 2. pGP-RRE3 + LMB, 3. pGP-RRE3 + pCMV-Rev, 4. pGP-RRE3 + pCMV-Rev + LMB, 5. pSYNGP, 6. pSYNGP + LMB, 7. pSYNGP + pCMV-Rev, 8. pSYNGP + pCMV-Rev + LMB, 9. pSYNGP-RRE, 10. pSYNGP-RRE + LMB, 11. pSYNGP-RRE + pCMV-Rev, 12. pSYNGP-RRE + pCMV-Rev + LMB.

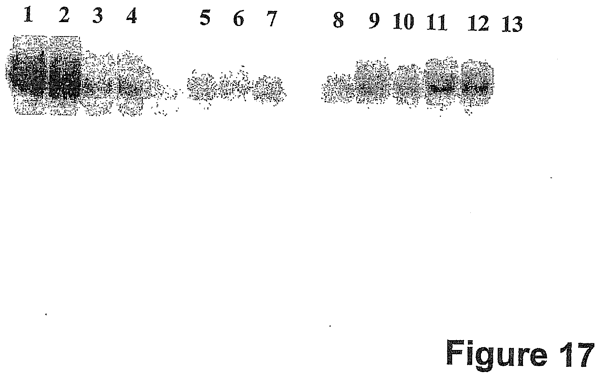

Figure 17 shows the cytoplasmic RNA levels of the vector genomes. 293T cells were transfected with 10 µg of each vector genome. Cytoplasmic RNA was extracted 48 hours post transfection. 20 µg of RNA were used from each sample for Northern blot analysis. The 700bp probe was designed to hybridise to all vector genome RNAs (see Materials and Methods). Lanes: 1. pH6nZ, 2. pH6nZ + pCMV-Rev, 3. pH6.1nZ, 4. pH6.1nZ + pCMV-Rev, 5. pHS1nZ, 6. pHS2nZ, 7. pHS3nZ, 8. pHS4nZ, 9. pHS5nZ, 10. pHS6nZ, 11. pHS7nZ, 12. pHS8nZ, 13. pCMV-Rev.

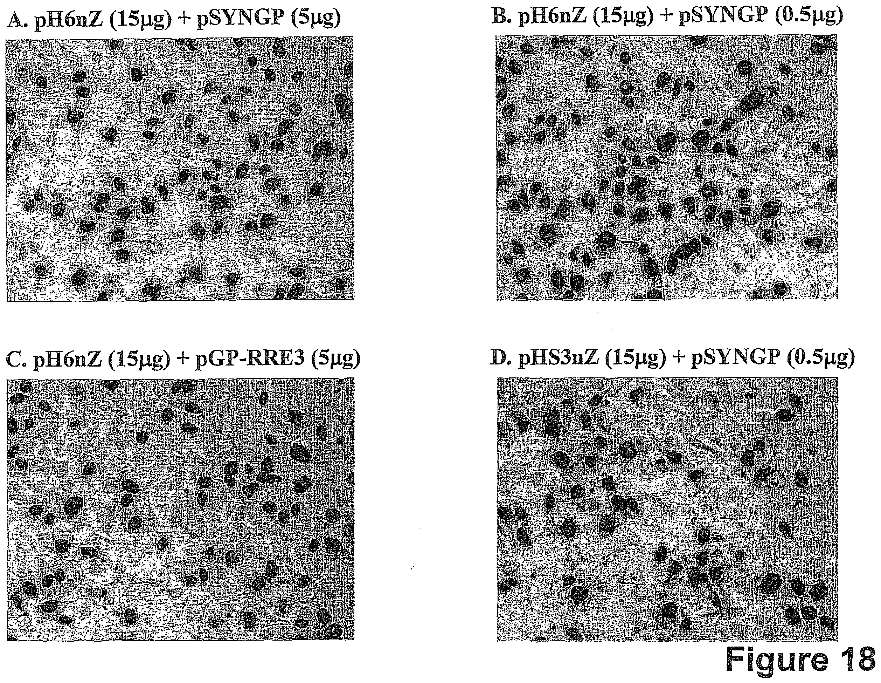

Figure 18 shows transduction efficiency at MOI 1. Viral stocks were generated by co-transfection of each *gag-pol* expression plasmid (5 or 0.5 µg), 15 µg pH6nZ or pHS3nZ (vector genome plasmid) and 5 µg pHCMVG (VSV envelope expression plasmid) on 293T cells. Virus was concentrated as previously described (45) and transduction efficiency was determined at m.o.i.'s 0:01-1 on HT1080 cells. There was a linear correlation of transduction efficiency and m.o.i. in all cases. An indicative picture at m.o.i. 1 is shown here. Transduction efficiency was >80% with either genome, either *gag-pol* and either high or low amounts of pSYNGP. Titres before concentration (I.U./ml): on 293T cells: A. 6.6x10⁵, B. 7.6x10⁵, C. 9.2x10⁵, D. 1.5x10⁵, on HT1080 cells: A. 6.0x10⁴, B. 9.9x10⁴, C. 8.0x10⁴, D. 2.9x10⁴. Titres after concentration (I.U./ml) on HT1080 cells: A. 6.0x10⁵, B. 2.0x1.0⁶, C. 1.4x10⁶, D. 2.0x10⁵.

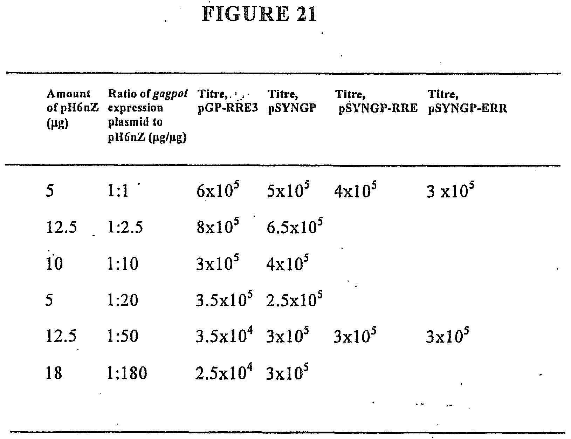

Figure 21 shows vector titers obtained with differed *gag-pol* constructs. Viral stocks were generated by co-transfection of each *gag-pol* expression plasmid, pH6nZ (vector genome plasmid) and pHCMVG (VSV envelope expression plasmid, 2.5µg for each transfection) on 293T cells. Titres (I.U./ml of virs stock) were measured on 293T cells by counting the number of blue colonies following X-Gal staining 48 hours after transduction. Experiments were performed at least twice and the variation between experiments was less than 15%.

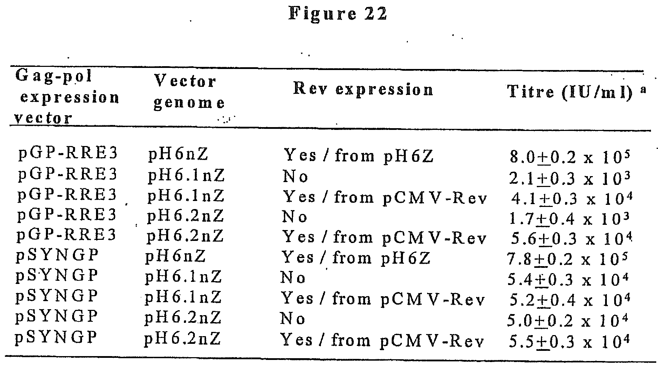

Figure 22 shows vector titres from the Rev/RRE (-) and (+) genomes. The retroviral vectors were generated as described in the Examples. Titres (I.U./ml of viral stock + SD) were determined in 293T cells.

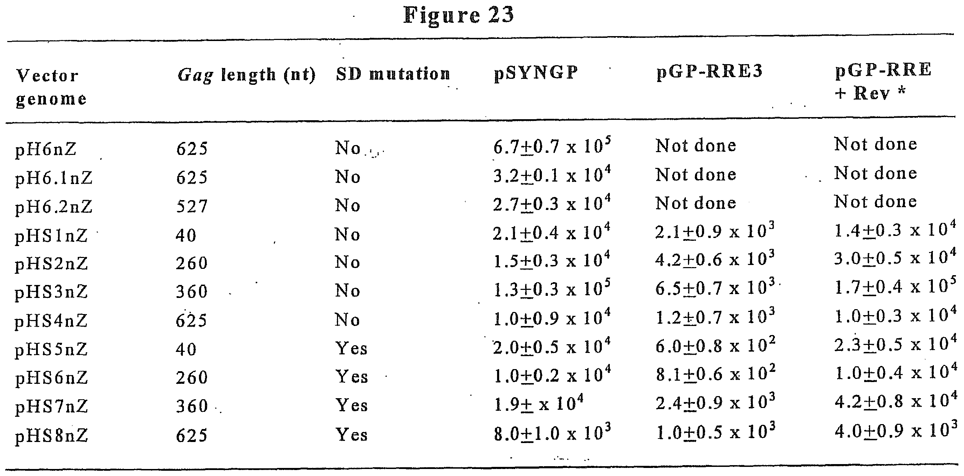

Figure 23 shows vector titres from the pHS series of vector genomes. The retroviral vector was generated as described in the Examples. Titres (I.U./m! of viral stock + SD) were determined in 293T cells. Rev is provided from pCMV-Rev. Note that pH6nZ expresses Rev and contains the RRE. None of the other genomes express Rev or contain the RRE. Expression from pSYNGP is Rev independent, whereas it is Rev dependent for pGP-RRE3.

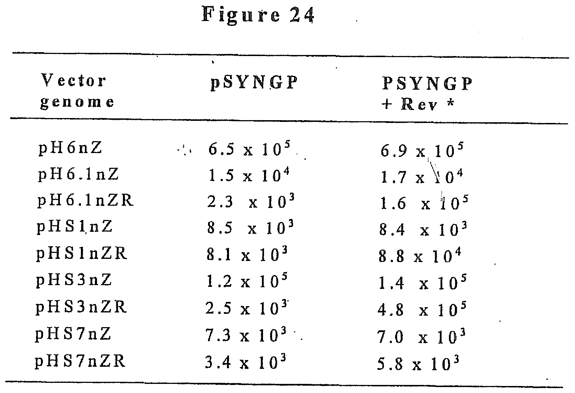

Figure 24 shows vector titres for the pHS series of vector genomes in the presence or absence of Rev/RRE. The retroviral vector was generated as described in the Examples. 5 μg of vector genome, 5 μg of pSYNGP and 2.5 μg of pHCMVG were used and titres (I.U./ml) were determined in 293T cells. Experiments were performed at least twice and the variation between experiments was less than 15%. Rev is provided from pCMV-Rev (1 μg). Note that pH6nZ expresses Rev and contains the RRE. None of the pHS genomes expresses Rev and only pHS1nZR, pHS3nZR, pHS7nZR and pH6.1nZR contain the RRE. gag-pol expression from pSYNGP is Rev independent.

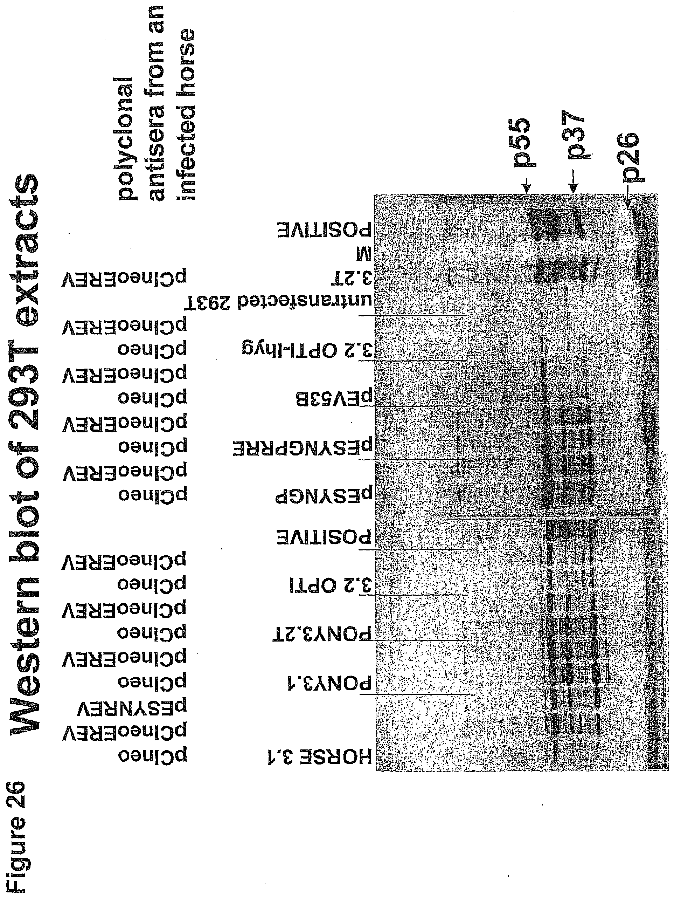

Figure 26 shows a Western blot of 293T extracts wherein 30:g of total cellular protein was separated by SDS/Page electrophoresis, transferred to nitro-cellulose and probed with anti EIAV antibodies. The secondary antibody was anti-Horse HRP (Sigma).

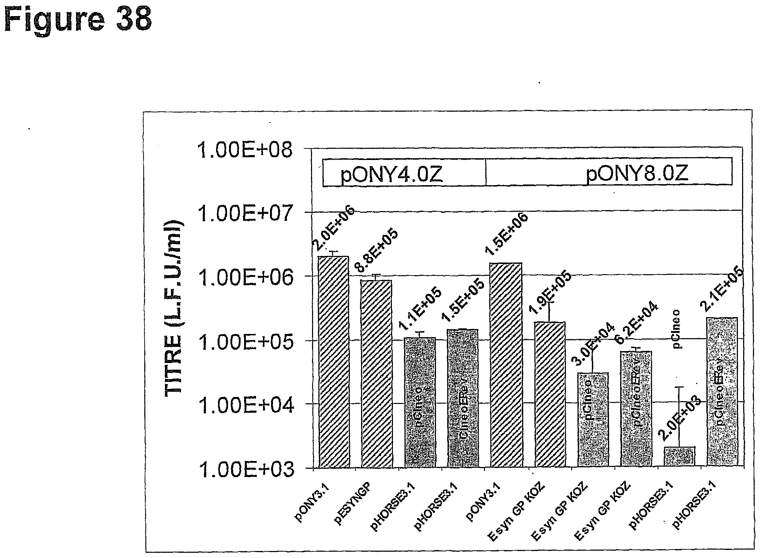

In Figure 38 the titres are shown in lacZ forming units (L.F.U.)/ml. The vectors used are indicated in boxes above the bars.
For ease of reference, we also set out the sequences listed in the accompanying Sequence Listing:
SEQ ID NO:1 shows the sequence of the wild-type *gag-pol* sequence for the strain HXB2 (accession no. K03455);
SEQ ID NO:2 shows the sequence of pSYNGP;
SEQ ID NO:3 shows the sequence of the Envelope gene for HIV-1 MN (Genbank accession no. M17449);
SEQ ID NO:4 shows the sequence of SYNgp-160mn - codon optimised env sequence;
SEQ ID NO:5 shows the sequence of pESYNGP;
SEQ ID NO:6 shows the sequence of LpESYNGP;
SEQ ID NO:7 shows the sequence of pESYNGPRRE;
SEQ ID NO:8 shows the sequence of LpESYNGPRRE;
SEQ ID NO:9 shows the sequence of pONY4.0Z;
SEQ ID NO:10 shows the sequence of pONY8.0Z;
SEQ ID NO:11 shows the sequence of pONY8.1Z;
SEQ ID NO:12 shows the sequence of pONY3.1;
SEQ ID NO: 13 shows the sequence of pCIneoERev;
SEQ ID NO:14 shows the sequence of pESYNREV;
SEQ ID NO:15 shows the sequence of codon optimised HIV *gag-pol;*
SEQ ID NO:16 shows the sequence of codon optimied EIAV *gag-pol;*
SEQ ID NO:17 shows the sequence of pIRES1hygESYNGP;
SEQ ID NO:18 shows the sequence of pESDSYNGP; and
SEQ ID NO:19 shows the sequence of pONY8.3G FB29(-).

### Example 1 - HIV

### Cell lines

293T cells (33) and HeLa cells (34) were maintained in Dubecco's modified Eagle's medium containing 10% (v/v) fetal calf serum and supplemented with L-glutamine and antibiotics (penicillin-streptomycin). 293T cells were obtained from D. Baltimore (Rockefeller University).

### HIV- 1 proviral clones

Proviral clones pWI3 (35) and pNL4-3 (36) were used.

### Construction of a Packaging System

In one of the present examples, a modified codon optimised HIV *env* sequence is used (SEQ I.D. No. 4). The corresponding *env* expression plasmid is designated pSYNgp160mn. The modified sequence contains extra motifs not used by (37). The extra sequences were taken from the HIV *env* sequence of strain MN and codon optimised. Any similar modification of the nucleic acid sequence would function similarly as long as it used codons corresponding to abundant tRNAs (38).

### Codon optimised HIV-1 gag-pol gene

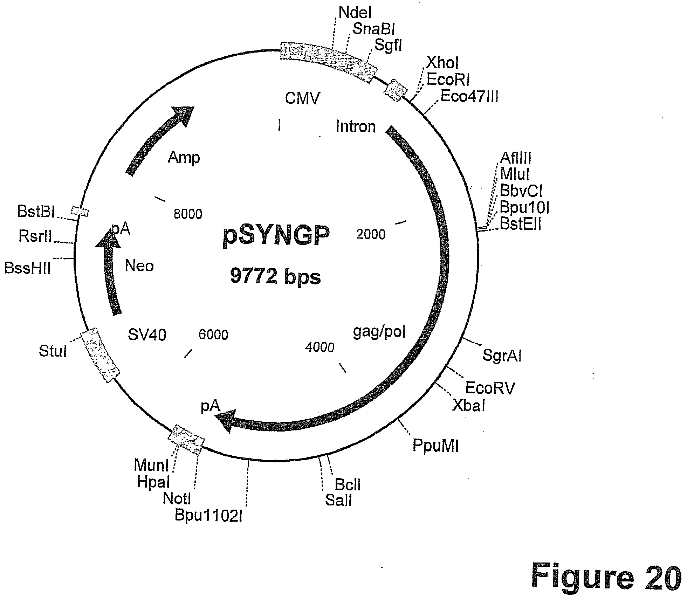

A codon optimised *gag-pol* gene, shown from nt 1108 to 5414 of SEQ ID NO: 2 was constructed by annealing a series of short overlapping oligonucleotides (approximately 30-40mers with 25% overlap, i.e. approximately 9 nucleotides). Oligonucleotides were purchased from R&D SYSTEMS (R&D Systems Europe Ltd, 4-10 The Quadrant, Barton Lane, Abingdon, OX14 3YS, UK). Codon optimisation was performed using the sequence of HXB-2 strain (AC: K03455) (39). The Kozak consensus sequence for optimal translation initiation (40) was also included. A fragment from base 1222 from the beginning of *gag* until the end of *gag* (1503) was not optimised in order to maintain the frameshift site and the overlap between the *gag* and *pol* reading frames. This was from clone pNL4-3. (When referring to base numbers within the *gag-pol* gene base 1 is the A of the *gag* ATG, which corresponds to base 790 from the beginning of the HXB2 sequence. When referring to sequences outside the *gag-pol* then the numbers refer to bases from the beginning of the HXB2 sequence, where base 1 corresponds to the beginning of the 5' LTR). Some deviations from optimisation were made in order to introduce convenient restriction sites. The final codon usage is shown in Figure 3b, which now resembles that of highly expressed human genes and is quite different from that of the wild type HIV-1 *gag-pol.* The gene was cloned into the mammalian expression vector pCIneo (Promega) in the *Eco*RI-*Not*I sites. The resulting plasmid was named pSYNGP (Figure 20, SEQ ID No 2). Sequencing of the gene in both strands verified the absence of any mistakes. A sequence comparison between the codon optimised and wild type HIV *gag-pol* sequence is shown in Figure 8.

### Rev/RRE constructs

The HIV-1 RRE sequence (bases 7769-8021 of the HXB2 sequence) was amplified by PCR from pWI3 proviral clone with primers bearing the *Not*I restriction site and was subsequently cloned into the *Not*I site of pSYNGP. The resulting plasmids were named pSYNGP-RRE (RRE in the correct orientation) and pSYNGP-ERR (RRE in the reverse orientation).

### Pseudotyped viral particles

In one form of the packaging system a synthetic *gag-pol* cassette is coexpressed with a heterologous envelope coding sequence. This could be for example VSV-G (44, 45), amphotropic MLV env (46, 47) or any other protein that would be incorporated into the HIV or EIAV particle (48). This includes molecules capable of targeting the vector to specif tissues.

### HIV-1 Vector genome constructs

pH6nZ is derived from pH4Z (49) by the addition of a single nucleotide to place an extra guanine residue that was missing from pH4Z at the 5= end of the vector genome transcript to optimise reverse transcription. In addition the gene coding for β-galactosidase (LacZ) was replaced by a gene encoding for a nuclear localising β-galactosidase. (We are grateful to Enca Martin-Rendon and Said Ismail for providing pH6nZ). In order to construct Rev(-) genome constructs the following modifications were made : a) A 1.8 kb *Pst*I *- Pst*I fragment was removed from pH6nZ, resulting in plasmid pH6.1nZ and b) an *Eco*NI (filled) - *Sph*I fragment was substituted with a *Spe*I (filled) - *Sph*I fragment from the same plasmid (pH6nZ), resulting in plasmid pH6.2nZ. In both cases sequences within *gag* (nt 1-625) were retained, as they have been shown to play a role in packaging *(93). Rev,* RRE and any other residual *env* sequences were removed. pH6.2nZ further contains the *env* splice acceptor, whereas pH6.1nZ does not.

A series of vectors encompassing further *gag* deletions plus or minus a mutant major splice donor (SD) (GT to CA mutation) were also derived from pH6Z. These were made by PCR with primers bearing a *Nar*I (5= primers) and an *Spe*I (3= primers) site. The PCR products were inserted into pH6Z at the *Nar*I *- Spe*I sites. The resulting vectors were named pHS1nZ (containing HIV-1 sequences up to *gag* 40), pHS2nZ (containing HIV-1 sequences up to *gag* 260), pHS3nZ (containing HIV-1 sequences up to *gag* 360), pHS4nZ (containing HIV-1 sequences up to *gag* 625), pHS5nZ (same as pHS1nZ but with a mutant SD), pHS6nZ (same as pHS2nZ but with a mutant SD), pHS7nZ (same as pHS3nZ but with a mutant SD) and pHS8nZ (same as pHS4nZ but with a mutant SD).

In addition, the RRE sequence (nt 7769-8021 of the HXB2 sequence) was inserted in the *Spe*I (filled) site of pH6.1nZ, pHS1nZ, pHS3nZ and pHS7nZ resulting in plasmids pH6.1nZR, pHS1nZR, pHS3nZR and pHS7nZR respectively.

Other modifications to the genome have been made including the generation of a SIN vector (by deletion of part of the 3= U3), the replacement of the LTRs with those from MLV or replacement of part of the 3=U3 with the MLV U3 region.

### Transient transfections, transductions and determination of viral titres

These were performed as previously described (49, 50). Briefly, 293T cells were seeded on 6cm dishes and 24 hours later they were transiently transfected by overnight calcium phosphate treatment. The medium was replaced 12 hours post-transfection and unless otherwise stated supernatants were harvested 48 hours post-transfection, filtered (through 0.22 or 0.45 μm filters) and titered by transduction of 293T cells. For this reason supernatant at appropriate dilutions of the original stock was added to 293T cells (plated onto 6 or 12 well plates 24 hours prior to transduction). 8 μg/ml Polybrene (Sigma) was added to each well and 48 hours post transduction viral titres were determined by X-gal staining.

### Luminescent β-galactosidase (β-gal) assays

These were performed on total cell extracts using a luminescent β-gal reporter system (CLONTECH). Untransfected 293T cells were used as negative control and 293T cells transfected with pCMV-β-gal (CLONTECH) were used as positive control.

### RNA analysis

Total or cytoplasmic RNA was extracted from 293T cells by using the RNeasy mini kit (QUIAGEN) 36-48 hours post-transfection. 5-10 μg of RNA was subjected to Northern blot analysis as previously described (51). Correct fractionation was verified by staining of the agarose gel. A probe complementary to bases 1222-1503 of the *gag-pol* gene was amplified by PCR from HIV-1 pNL4-3 proviral clone and was used to detect both the codon optimised and wild type *gag-pol* mRNAs. A second probe, complementary to nt 1510-2290 of the codon optimised gene was also amplified by PCR from plasmid pSYNGP and was used to detect the codon optimised genes only. A 732 bp fragment complementary to all vector genomes used in this study was prepared by an *Spe*I*-Avr*II digestion of pH6nZ. A probe specific for ubiquitin (CLONTECH) was used to normalise the results. All probes were labelled by random labelling (STRATAGENE) with α-³²P dCTP (Amersham). The results were quantitated by using a Storm PhosphorImager (Molecular Dynamics) and shown in Figure 12. In the total cellular fractions the 47S rRNA precursor could be clearly seen, whereas it was absent from the cytoplasmic fractions. As expected (52), Rev stimulates the cytoplasmic accumulation of wild type *gag-pol* mRNA (lanes 1c and 2c). RNA levels were 10-20 fold higher for the codon optimised gene compared to the wild type one, both in total and cytoplasmic fractions (compare lanes 3t-2t, 3c-2c, 10c-8c). The RRE sequence did not significantly destabilise the codon optimised RNAs since RNA levels were similar for codon optimised RNAs whether or not they contained the RRE sequence (compare lanes 3 and 5). Rev did not markedly enhance cytoplasmic accumulation of the codon optimised *gag-pol* mRNAs, even when they contained the RRE sequence (differences in RNA levels were less than 2-fold, compare lanes 3-4 or 5-6).

It appeared from a comparison of Figures 10 and 12 that all of the increase in protein expression from *syngp* could be accounted for by the increase in RNA levels. In order to investigate whether this was due to saturating levels of RNA in the cell, we transfected 0.1, 1 and 10 μg of the wild type or codon optimised expression vectors into 293T cells and compared protein production. In all cases protein production was 10-fold higher for the codon optimised gene for the same amount of transfected DNA, while increase in protein levels was proportional to the amount of transfected DNA for each individual gene. It seems likely therefore that the enhanced expression of the codon optimised gene can be mainly attributed to the enhanced RNA levels present in the cytoplasm and not to increased translation.

### Protein analysis

Total cell lysates were prepared from 293T cells 48 hours post-transfection (unless otherwise stated) with an alkaline lysis buffer. For extraction of proteins from cell supernatants the supernatant was first passed through a 0.22μm filter and the vector particles were collected by centrifugation of 1 ml of supernatant at 21,000 g for 30 minutes. Pellets were washed with PBS and then re-suspended in a small volume (2-10 ìl) of lysis buffer. Equal protein amounts were separated on a SDS 10-12% (v/v) polyacrylamide gel. Proteins were transferred to nitrocellulose membranes which were probed sequentially with a 1:500 dilution of HIV-1 positive human serum (AIDS Reagent Project, ADP508, Panel E) and a 1:1000 dilution of horseradish peroxidase labelled anti-human IgG (Sigma, A0176). Proteins were visualised using the ECL or ECL-plus western blotting detection reagent (Amersham). To verify equal protein loading, membranes were stripped and re-probed with a 1:1000 dilution of anti-actin antibody (Sigma, A2066), followed by a 1:2000 dilution of horseradish peroxidase labelled anti-rabbit IgG (Vector Laboratories, PI-1000).

### Expression of gag-pol gene products and vector particle production

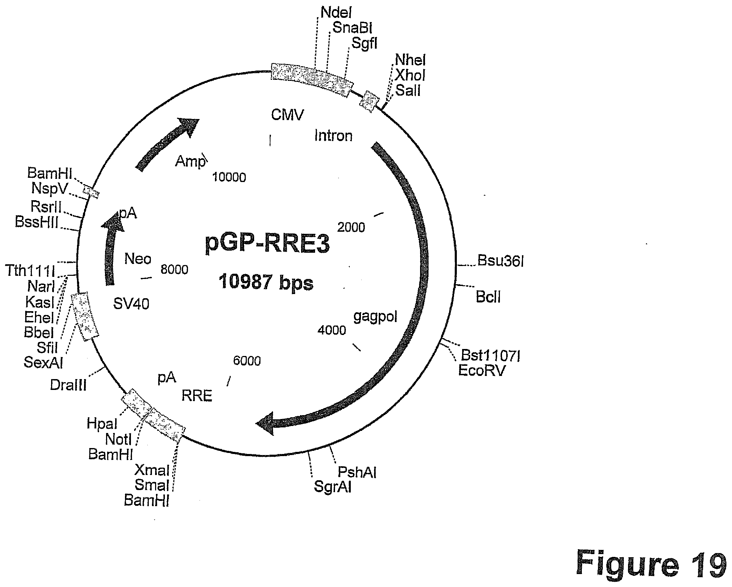

The wild type *gag-pol* (pGP-RRE3 B Figure 19) (49), and codon optimised expression vectors (pSYNGP, pSYNGP-RRE and pSYNGP-ERR) were transiently transfected into 293T cells. Transfections were performed in the presence or absence of a Rev expression vector, pCMV-Rev (53), in order to assess Rev-dependence for expression. Western blot analysis was performed on cell lysates and supernatants to assess protein production. The results are shown in Figure 10. As expected (54), expression of the wild type gene is observed only when Rev is provided *in trans* (lanes 2 and 3). In contrast, when the codon optimised *gag-pol* was used, there was high level expression in both the presence and absence of Rev (lanes 4 and 5), indicating that in this system there was no requirement for Rev. Protein levels were higher for the codon optimised gene than for the wild type *gag-pol* (compare lanes 4-9 with lane 3). The difference was more evident in the cell supernatants (approximately 10-fold higher protein levels for the codon optimised gene compared to the wild type one, quantitated by using a PhosphorImager) than in the cell lysates.

In previous studies where the RRE has been included in *gag-pol* expression vectors that had been engineered to remove INS sequences, inclusion of the RRE lead to a decrease in protein levels, that was restored by providing Rev *in trans* (55). In our hands, the presence of the RRE in the fully codon optimised *gag*-*pol* mRNA did not affect protein levels and provision of Rev *in trans* did not further enhance expression (lanes 6 and 7).

In order to compare translation rates between the wild type and codon optimised gene, protein production from the wild type and codon optimised expression vector was determined at several time intervals post transfection into 293T cells. Protein production and particle formation was determined by Western blot analysis and the results are shown in Figure 11. Protein production and particle formation was 10-fold higher for the codon optimised *gag-pol* at all time points.

To further determine whether this enhanced expression that was observed with the codon optimised gene was due to better translation or due to effects on the RNA, RNA analysis was carried out.

### The efficiency of vector production using the codon optimised gag-pol gene

To determine the effects of the codon optimised *gag-pol* on vector production, we used an HIV vector genome, pH6nZ and the VSV-G envelope expression plasmid pHCMVG (113), in combination with either pSYNGP, pSYNGP-RRE, pSYNGP-ERR or pGP-RRE3 as a source for the *gag*-*pol* in a plasmid ratio of 2 : I : 2 in a 3 plasmid co-transfection of 293T cells (49). Whole cell extracts and culture supernatants were evaluated by Western blot analysis for the presence of the *gag* and *gag-pol* gene products. Particle production was, as expected (Figure 10), 5-10 fold higher for the codon optimised genes when compared to the wild type.

To determine the effects of the codon optimised *gag-pol* gene on vector titres, several ratios of the vector components were used. The results are shown in Figure 21. Where the *gag-pol* was the limiting component in the system (as determined by the drop in titres observed with the wild type gene), titres were 10-fold higher for the codon optimised vectors. This is in agreement with the higher protein production observed for these vectors, but suggests that under normal conditions of vector production gag-pol is saturating and the codon optimisation gives no maximum yield advantage.

### The effect of HIV-1 gag INS sequences on the codon optimised gene is position dependent

It has previously been demonstrated that insertion of wild type HIV-1 *gag* sequences downstream of other RNAs, e.g. HIV-1 *tat* (56), HIV-1 *gag* (55) or CAT (57) can lead to a dramatic decrease in steady state mRNA levels, presumably as a result of the INS sequences. In other cases, e.g. for β-globin (58), it was shown that the effect was splice site dependent. Cellular AREs (AU-rich elements) that are found in the 3= UTR of labile mRNAs may confer mRNA destabilisation by inducing cytoplasmic deadenylation of the transcripts (59). To test whether HIV-1 *gag* INS sequences would destabilise the codon optimised RNA, the wild-type HIV-1 *gag* sequence, or parts of it (nt 1-625 or nt 625-1503), were amplified by PCR from the proviral clone pW13. All fragments were blunt ended and were inserted into pSYNGP or pSYNGP-RRE at either a blunted *Eco*R1 or *Not*I site (upstream or downstream of the codon optimised *gag-pol* gene repectively). As controls the wt HIV-1 *gag* in the reverse orientation (as INS sequences have been shown to act in an orientation dependent manner, (57) (pSYN7) and *lac*Z, excised from plasmid pCMV-βgal (CLONTECH) (in the correct orientation) (pSYN8) were also inserted in the same site. Contrary to our expectation, as shown in Figure 13, the wild type HIV-1 *gag* sequence did not appear to significantly affect RNA or protein levels of the codon optimised gene. We further constructed another series of plasmids (by PCR and from the same plasmids) where the wild type HIV-1 *gag* in the sense or reverse orientation, subfragments of *gag* (nt 1-625 or nt 625-1503), the wild type HIV-1 *gag* without the ATG or with a frameshift mutation 25 bases downstream of the ATG, or nt 72-1093 of LacZ (excised from plasmid pH6Z), or the first 1093 bases of *lacZ* with or without the ATG were inserted upstream of the codon optimised HIV-1 *gag-pol* gene in pSYNGP and/or pSYNGP-RRE (pSYN9-pSYN22, Figure 14). Northern blot analysis showed that insertion of the wild type HIV-1 *gag* gene upstream of the codon optimised HIV-1 *gag-pol* (pSYN9, pSYN10) lead to diminished RNA levels in the presence or absence of Rev/RRE (Figure 15A, lanes 1-4 and Figure 15B, lanes 1+12). The effect was not dependent on translation as insertion of a wild type HIV-1 *gag* lacking the ATG or with a frameshift mutation (pSYN12, pSYN13 and pSYN14) also diminished RNA levels (Figure 15B, lanes 1-7). Western blot analysis verified that there was no HIV-1 gag translation product for pSYN12-14. However, it is possible that, as the wt HIV-1 *gag* exhibits such an adverse codon usage, it may act as a non-translatable long 5= leader for *syngp,* and if this is the case, then the ATG mutation should not have any effects.

Insertion of smaller parts of the wild type HIV-1 *gag* gene (pSYN15 and pSYN17) also lead to a decrease in RNA levels (Figure 15B, lanes 1-3 and 8-9), but not to levels as low as when the whole *gag* sequence was used (lanes 1-3, 4-7 and 8-9 in Figure 15B). This indicates that the effect of INS sequences is dependent on their size. Insertion of the wild type HIV-1 *gag* in the reverse orientation (pSYN11) had no effect on RNA levels (Figure 15A, lanes 1 and 5-6). However a splicing event seemed to take place in that case, as indicated by the size of the RNA (equal to the size of the codon optimised *gag-pol* RNA) and by the translation product (gag-pol, in equal amounts compared to pSYNGP, as verified by Western blot analysis).

These data indicate therefore that wild type HIV-1 *gag* instability sequences act in a position and size dependent manner, probably irrespective of translation. It should also be noted that the RRE was unable to rescue the destabilised RNAs through interactiοn with Rev.

### Construction of an HIV-1 based vector system that lacks all the accessory proteins

Until now several HIV-1 based vector systems have been reported that lack all accessory proteins but Rev (49, 60). We wished to investigate whether the codon optimised gene would permit the construction of an HIV-1 based vector system that lacks all accessory proteins. We initially deleted *rev*/RRE and any residual *env* sequences, but kept the first 625 nucleotides of *gag,* as they have been shown to play a role in efficient packaging (61). Two vector genome constructs were made, pH6.1nZ (retaining only HIV sequences up to nt 625 of *gag*) and pH6.2nZ (same as pH6.1nZ, but also retaining the *env* splice acceptor). These were derived from a conventional HIV vector genome that contains RRE and expresses Rev (pH6nZ). Our 3-plasmid vector system now expressed only HIV-1 gag-pol and the VSV-G envelope proteins. Vector particle titres were determined as described in the previous section. A ratio of 2 : 2 : 1 of vector genome (pH6Z or pH6.1nZ or pH6.2nZ) : gag-pol expression vector (pGP-RRE3 or pSYNGP) : pHCMV-G was used. Transfections were performed in the presence or absence of pCMV-Rev, as gag-pol expression was still Rev dependent for the wild type gene. The results are summarised in Figure 22 and indicate that an HIV vector could be produced in the total absence of Rev, but that maximum titres were compromised at 20-fold lower than could be achieved in the presence of Rev. As gag-pol expression should be the same for pSYNGP with pH6nZ or pH6.1nZ or pH6.2nZ (since it is Rev independent), as well as for pGP-RRE3 when Rev is provided *in trans,* we suspected that the vector genome retained a requirement for Rev and was therefore limiting the titres. To confirm this, Northern blot analysis was performed on cytoplasmic RNA prepared from cells transfected with pH6nZ or pH6.1nZ in the presence or absence of pCMV-Rev. As can be seen in Figure 17, lanes 1-4, the levels of cytoplasmic RNA derived from pH6nZ were 5-10 fold higher than those obtained with pH6.1 nZ (compare lanes 1-2 to lanes 3-4). These data support the notion that RNA produced from the vector genome requires the Rev/RRE system to ensure high cytoplasmic levels. This may be due to inefficient nuclear export of the RNA, as INS sequences residing within *gag* were still present.

Further deletions in the *gag* sequences of the vector genome might therefore be necessary to restore titres. To date efficient packaging has been reported to require 360 (62) or 255 (63) nucleotides of *gag* in vectors that still retain *env* sequences, or about 40 nucleotides of *gag* in a particular combination of splice donor mutation, *gag* and *env* deletions (64, 63). In an attempt to remove the requirement for Rev/RRE in our vector genome without compromising efficient packaging we constructed a series of vectors derived from pH6nZ containing progressively larger deletions of HIV-1 sequences (only sequences upstream and within *gag* were retained) plus and minus a mutant major splice donor (SD) (GT to CA mutation). Vector particle titres were determined as before and the results are summarized in Figure 23. As can be seen, deletion of up to nt 360 in *gag* (vector pHS3nZ) resulted in an increase in titres (compared to pH6.1nZ or pH6.2nZ) and only a 5-fold decrease (titres were 1.3-1.7 x 10⁵) compared to pH6nZ. Further deletions resulted in titres lower than pHS3nZ and similar to pH6.1nZ. In addition, the SD mutation did not have a positive effect on vector titres and in the case of pHS3nZ it resulted in a 10-fold decrease in titres (compare titres for pHS3nZ and pHS7nZ in Figure 23). Northern blot analysis on cytoplasmic RNA (Figure 17, lanes 1 and 5-12) showed that RNA levels were indeed higher for pH6nZ, which could account for the maximum titres observed with this vector. RNA levels were equal for pHS1nZ (lane 5), pHS2nZ (lane 6) and pHS3nZ (lane 7) whereas titres were 5-8 ford higher for pHS3nZ. It is possible that further deletions (than that found in pHS3nZ) in *gag* might result in less efficient packaging (as for HIV-1 the packaging signal extends in *gag*) and therefore even though all 3 vectors produce similar amounts of RNA only pHS3nZ retains maximum packaging efficiency. It is also interesting to note that the SD mutation resulted in increased RNA levels in the cytoplasm (compare lanes 6 and 10, 7 and 11 or 8 and 12 in Figure 17) but equal or decreased titres (Figure 23). The GT dinucleotide that was mutated is in the stem of SL2 of the packaging signal (65). It has been reported that SL2 might not be very important for HIV-1 RNA encapsidation (65, 66), whereas SL3 is of great importance (67). Folding of the wild type and SD-mutant vector sequences with the RNAdraw software program revealed that the mutation alters significantly the secondary structure of the RNA and not only of SL2. It is likely therefore that although the SD mutation enhances cytoplasmic RNA levels it does not increase titres as it alters the secondary structure of the packaging signal.

To investigate whether the titre differences that were observed with the Rev minus vectors were indeed due to Rev dependence of the genomes, the RRE sequence (nt 7769 - 8021 of the HXB2 sequence) was inserted in the *Spe*I site (downstream of the *gag* sequence and just upstream of the internal CMV promoter) of pH6.1nZ, pHS1nZ, pHS3nZ and pHS7nZ, resulting in plasmids pH6.1nZR, pHS1nZR, pHS3nZR and pHS7nZR respectively. Vector particle titres were determined with pSYNGP and pHCMVG in the presence or absence of Rev (pCMV-Rev) as before and the results are summarised in Figure 24. In the absence of Rev titres were further compromised for pH6.1nZR (7-fold compared to pH6.1nZ), pHS3nZR (6-fold compared to pHS3nZ) and pHS7nZR (2.5-fold compared to pHS7nZ). This was expected, as the RRE also acts as an instability sequence (68) and so it would be expected to confer Rev-dependence. In the presence of Rev titres were restored to the maximum titres observed for pH6nZ in the case of pHS3nZR (5 x 10⁵) and pH6.1nZR (2 x 10⁵). Titres were not restored for pHS7nZR in the presence of Rev. This supports the hypothesis that the SD mutation in pHS7nZ affects the structure of the packaging signal and thus the packaging ability of this vector genome, as in this case Rev may be able to stimulate vector genome RNA levels, as for pHS3nZR and pH6.1nZR, but it can not affect the secondary structure of the packaging signal. For vector pHS1nZ inclusion of the RRE did not lead to a decrease in titres. This could be due to the fact that pHS1nZ contains only 40 nucleotides of *gag* sequences and therefore even with the RRE the size of instability sequences is not higher than for pHS2nZ that gives equal titres to pHS1nZ. Rev was able to partially restore titres for pHS1nZR (10-fold increase when compared to pHS1nZ and 8-fold lower than pH6nZ) but not fully as in the case of pHS3nZ. This is also in agreement with the hypothesis that 40 nucleotides of HIV-1 *gag* sequences might not be sufficient for efficient vector RNA packaging and this could account for the partial and not complete restoration in titres observed with pHS1nZR in the presence of Rev.

In addition, end-point titres were determined for pHS3nZ and pH6nZ with pSYNGP in HeLa and HT1080 human cell lines. In both cases titres followed the pattern observed in 293T cells, with titres being 2-3 fold lower for pHS3nZ than for pH6nZ (See Figure 10). Finally, transduction efficiency of vector produced with pHS3nZ or pH6nZ and different amounts of pSYNGP or pGP-RRE3 at different m.o.i.=s (and as high as 1) was determined in HT1080 cells. This experiment was performed as the high level gag-pol expression from pSYNGP may result in interference by genome-empty particles at high vector concentrations. As expected for VSVG pseudotyped retroviral particles (69) transduction efficiencies correlated with the m.o.i.=s, whether high or low amounts of pSYNGP were used and with pH6nZ or pHS3nZ. For m.o.i. 1 transduction efficiency was approximately 50-60% in all cases (Figure 18). The above data indicate that no interference due to genome-empty particles is observed in this experimental system.

### The codon optimised gag-pol gene does not use the exportin-1 nuclear export pathway

Rev mediates the export of unspliced and singly spliced HIV-1 mRNAs via the nuclear export receptor exportin-1 (CRM1) (70, 71, 72, 73, 74). Leptomycin B (LMB) has been shown to inhibit leucine-rich NES mediated nuclear export by disrupting the formation of the exportin-1/NES/RanGTP complex (75, 72). In particular, LMB inhibits nucleo-cytoplasmic translocation of Rev and Rev-dependent HIV mRNAs (76). To investigate whether exportin-1 mediates the export of the codon optimised *gag-pol* constructs, the effect of LMB on protein production was tested. Western blot analysis was performed on cell lysates from cells transfected with the *gag-pol* constructs (+/- pCMV-Rev) and treated or not with LMB (7.5 nM, for 20 hours, beginning treatment 5 hours post-transfection). To confirm that LMB had no global effects on transport, the expression of β-gal from the control plasmid pCMV-βGal was also measured. An actin internal control was used to account for protein variations between samples. The results are shown in Figure 16. As expected (76), the wild type *gag-pol* was not expressed in the presence of LMB (compare lanes 3 and 4), whereas LMB had no effect on protein production from the codon optimised *gag-pol,* irrespective of the presence of the RRE in the transcript and the provision of Rev *in trans* (compare lanes 5 and 6, 7 and 8, 9 and 10, 11 and 12, 5-6 and 11-12). The resistance of the expression of the codon-optimised *gag-pol* to inhibition by LMB indicates that the exportin-1 pathway is not used and therefore an alternative export pathway must be used. This offers a possible explanation for the Rev independent expression. The fact that the presence of a nonfunctional Rev/RRE interaction did not affect expression implies that the RRE does not necessarily act as an inhibitory (e.g. nuclear retention) signal *per se,* which is in agreement with previous observations (5, 58).

In conclusion, this is the first report of an HIV-1 based vector system, composed of pSYNGP, pHS3nZ and pHCMVG, where significant vector production can be achieved in the absence of all accessory proteins. These data indicate that in order to achieve maximum titres the HIV vector genome must be configured to retain efficient packaging and that this requires the retention of *gag* sequences and a splice donor. By reducing the *gag* sequence to 360 nt in pHS3nZ and combining this with pSYNGP it is possible to achieve titre of at least 10⁵ I.U./ml that is only 5-fold lower than the maximum levels achieved in the presence of Rev.

### Example 2 - EIAV (not according to the invention)

### Codon-optimised EIAV gag-pol expression cassettes

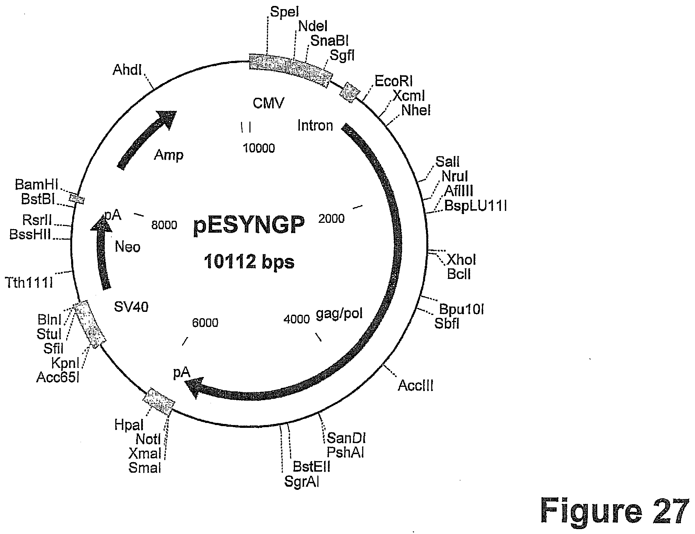

We also examined if the codon-optimisation process would alter the properties of the *gag-pol* gene of the non-primate lentivirus EIAV. The sequence is of the codon-optimised gene is shown from nt1103 to 5760 of SEQ ID NO:5 (Figure 9). The wild type and the codon-optimised sequences are denoted WT and CO, respectively. The codon usage was changed to that of highly expressed mammalian genes. pESYNGP (Figure 27 and SEQ ID NO:5) was made by transferring an *Xba*I*-Not*I fragment from a plasmid containing a codon-optimised EIAV gag/pol gene, synthesised by Operon Technologies Inc., Alameda, CA, into pCIneo (Promega). The gene was supplied in a proprietary plasmid backbone, GeneOp. The fragment transferred to pCIneo includes sequences flanking the codon-optimised EIAV gag/pol ORF: tctagaGAATTCGCCACC**ATG- EIAV gag/pol-TGA**ACCCGGGgcggccgc. The ATG start and TGA stop codons are shown in bold and the recognition sequences for *Xba*I and *Not*I sites in lower case.

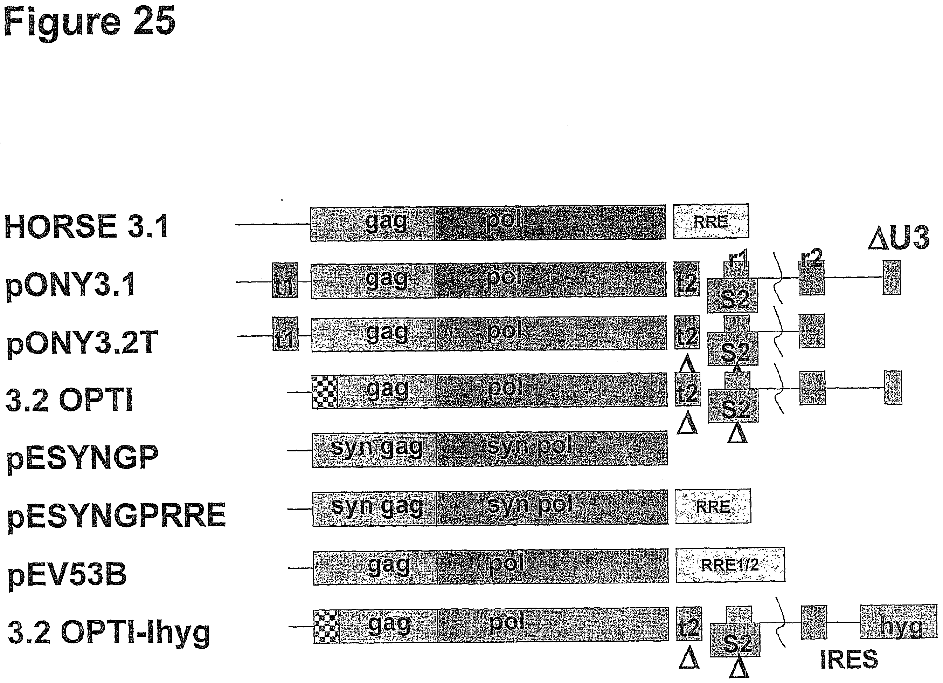

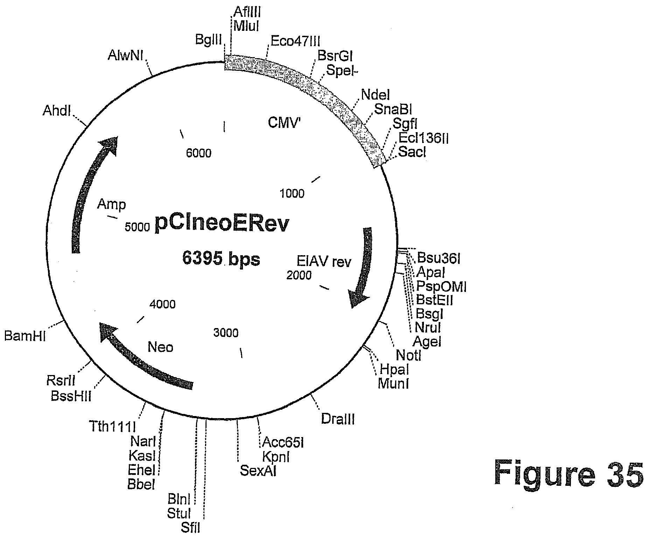

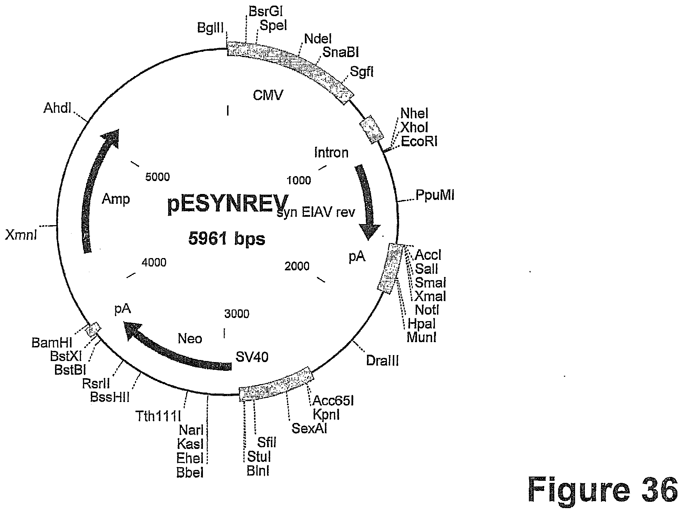

The expression of Gag/Pol from the codon-optimised gene was assessed with respect to that from various wild type EIAV gag/pol expression constructs by transient transfection of HEK 293T cells (Figure 25). Transfections were carried out using the calcium phosphate technique, using equal moles of each Gag/Pol expression plasmid together with a plasmid which expressed EIAV Rev either from the wild type sequence or from a codon-optimised version of the gene: pCIneoEREV (WO 99/32646) (Figure 35 and SEQ ID NO:13) or pESYNREV (Figure 36 and SEQ ID NO:14), respectively. pESYNREV is a pCIneo-basted plasmid (Promega) which was made by introducing the *EcoR*I to *Sal*I fragment from a synthetic EIAV REV plasmid, made by Operon Technologies Alameda, CA. The plasmid backbone was the proprietary plasmid GeneOp in which was inserted a codon-optimised EIAV REV gene flanked by *Eco*RI and *Sal*I recognition sequences and a Kozak consensus sequence to drive efficient translation of the gene. The mass of DNA on each transfection was equalised by addition of pCIneo plasmid. In transfections in which a Rev expression plasmid was omitted, a similar mass of pCIneo (Promega) was used instead (lanes labelled pCIneo). Cytoplasmic extracts were prepared 48 hours post transfection and 15µg amounts of protein were fractionated by SDS-PAGE and then transferred to Hybond ECL. The Western blot was probed with a polyclonal antisera from an EIAV-infected horse and then with a secondary antibody, anti-horse horse-radish peroxidase conjugate. Development of the blot was carried out using the ECL kit (Amersham). Positive controls for the blotting and development procedure, and cytoplasmic extract from untransfected HEK 293T cells are as indicated. The positions of various EIAV proteins are indicated.

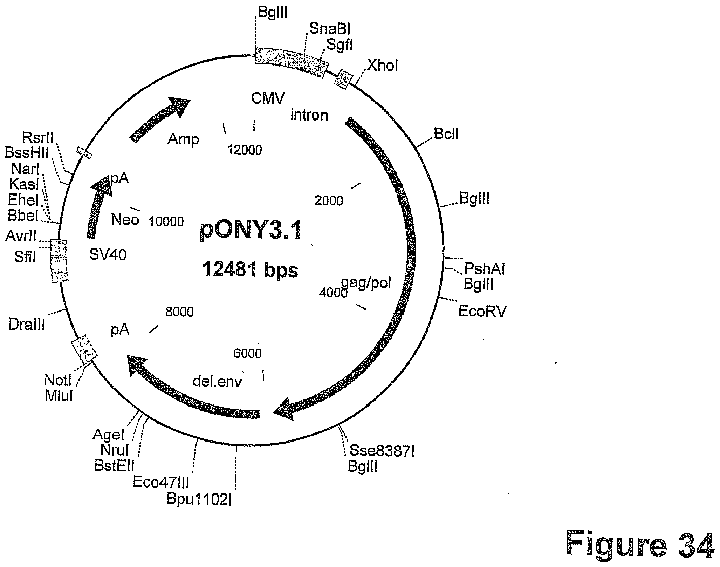

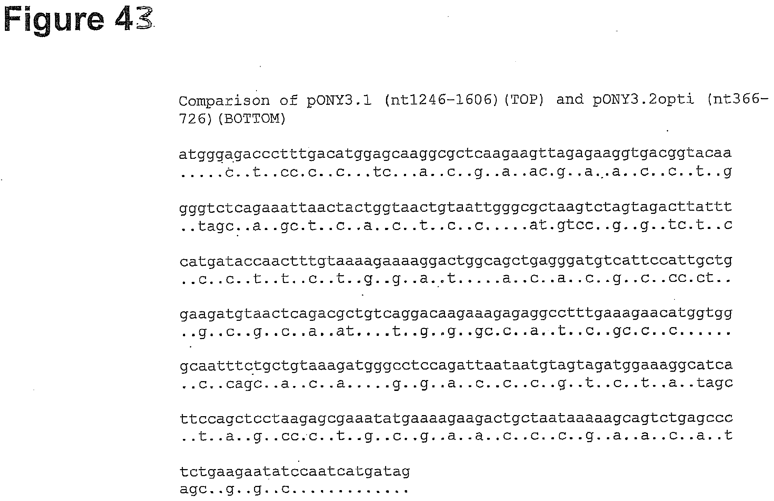

Expression from wild type *gag*/*pol* was achieved from various plasmids (see Figure 25). pONY3.2T is a derivative of pONY3.1 (WO99/32646)(Figure 34 and SEQ ID NO:12) in which mutations which ablate expression of Tat and S2 have been made. In addition, the EIAV sequence is truncated downstream of the second exon of *rev.* Specifically, expression of Tat is ablated by an 83nt deletion in exon 2 of tat which corresponds with respect to the wild type EIAV sequence, Acc. No. U01866, to deletion of nt 5234-5316 inclusive. S2 ORF expression is ablated by a 51 nt deletion, corresponding to nt 5346-5396 of Acc. No. U01866. The EIAV sequence is deleted downstream of a position corresponding to nt 7815 of Acc. No. U01866. These alterations do not alter *rev,* hence expression of this gene is expressed as for pONY3.1. pONY3.2 OPTI is a derivative of pONY3.1 which has the same deletions for ablation of Tat and S2 expression as described above. In addition, the first 372nt of *gag* have been 'codon-optimised' for expression in human cells. The sequence of the wild type and codon-optimised sequences present in pONY3.2OPTI in this region are compared in Figure 43. Base differences between the sequences are indicated. The region which was codon-optimised represents the region of overlap between the vector and wild-type gag/pol expression constructs. Reduction of homology within this region would be expected to improve the safety profile of the vector system due to the reduced chances of recombination between the vector genome and the gag/pol transcripts. 3.2 OPTI-Ihyg is a derivative of 3.2 OPTI in which the *Sna*BI-*Not*I fragment of 3.2 OPTI is transferred to pIRES1hygro (Clontech) prepared for ligation by digestion with the same sites. The *gag*/*pol* gene is thus placed upstream of the IRES hygromycin phosphotransferase. Of note is the fact that the resulting construct contains the intron from pCIneo, not from pIRES1hygro. pEV53B is a derivative of PEV53A (WO 98/51810) in which the EIAV-derived sequence upstream of the Gag initiation codon is reduced to include only the major splice donor and surrounding seqeunces: CAG/GTAAGA**TG**, where the Gag initiation codon is shown in bold face.

The results (Figure 26) shown the Rev-dependence of Gag/Pol expression from pHORSE3.1 (WO 99/32646), which has an EIAV derived leader sequence starting just downstream of the primer binding site and an RRE placed downstream of *gag*/*pol* composed of the two EIAV sequences reported to have RRE activity. Expression was enhanced by the same amount when Rev expression was driven by wild type (pCIneoERev) (Figure 35) or codon-optimised (pESYNREV) (Figure 36) genes. This result confirms the functionality of the codon-optimised Rev expression plasmid.

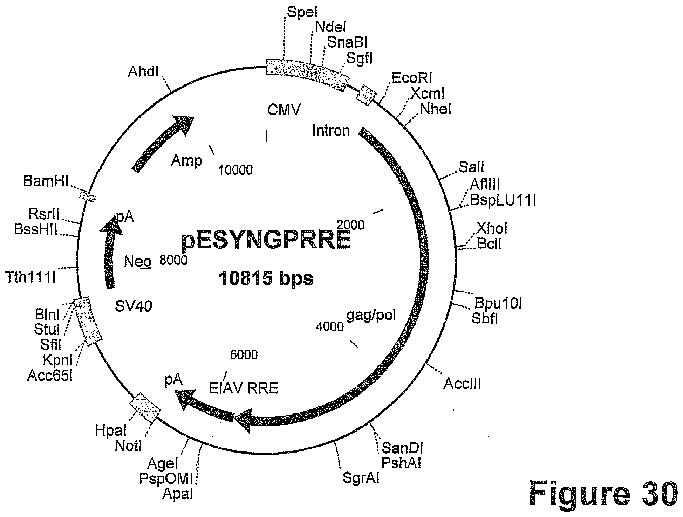

In contrast to expression of Gag/Pol from pONY3.1, expression from pESYNGP was not influenced by the presence of Rev, however it was slightly lower than from pONY3.1 or pON3.2T. Expression from pESYNGPRRE (Figure 30 and SEQ ID NO:7), in which the EIAV RRE sequence present in pHORSE3.1 is placed downstream of *gag*/*pol,* appeared slightly lower than from pESYNGP. The levels of expression from 3.2 OPTI and 3.2OPTI-Ihyg were significantly lower than from pESYNGP or pONY3.1, even in the presence of Rev. This result suggested that there may be determinants of Gag/Pol expression within the first 372nt of the *gag* and showed that 3.2 OPTI was unlikely to be useful as a basis for EIAV vector production. Furthermore it demonstrates that codon-optimisation of only certain regions of the whole gag/pol gene may not lead to high levels of Rev-independent expression.

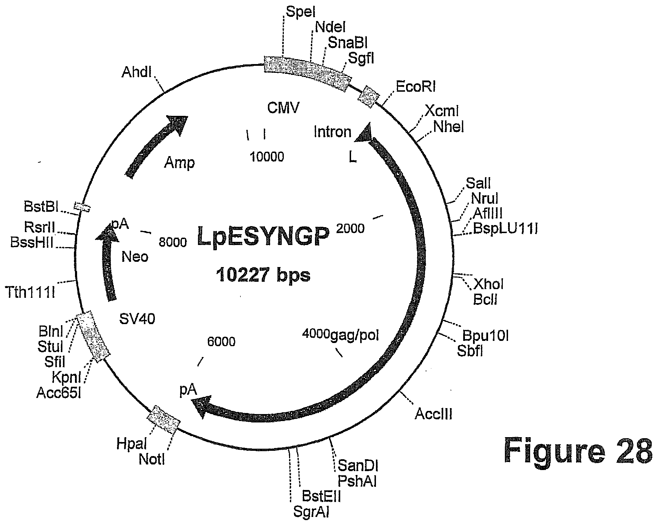

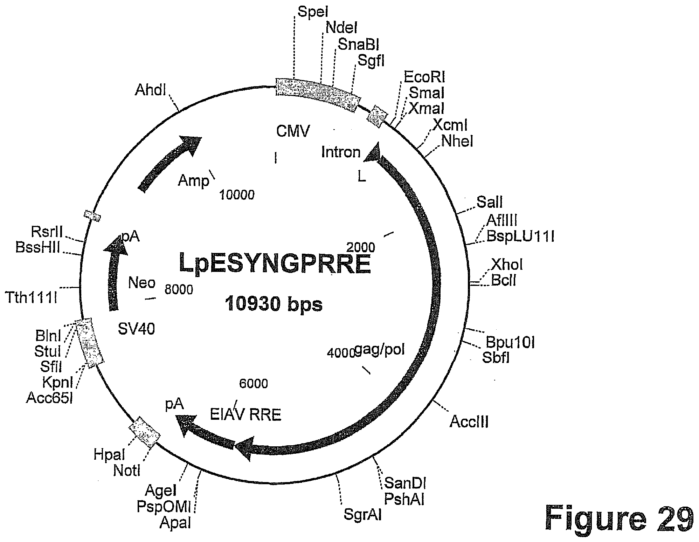

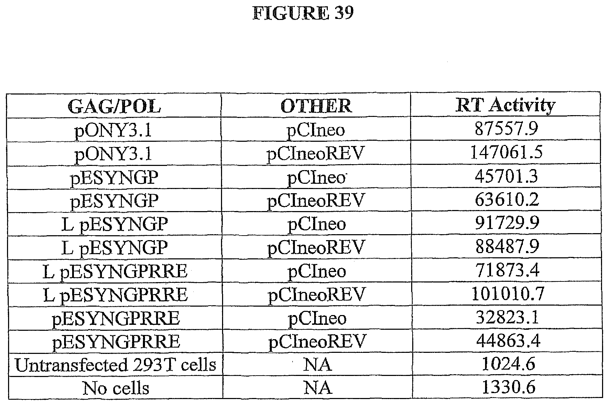

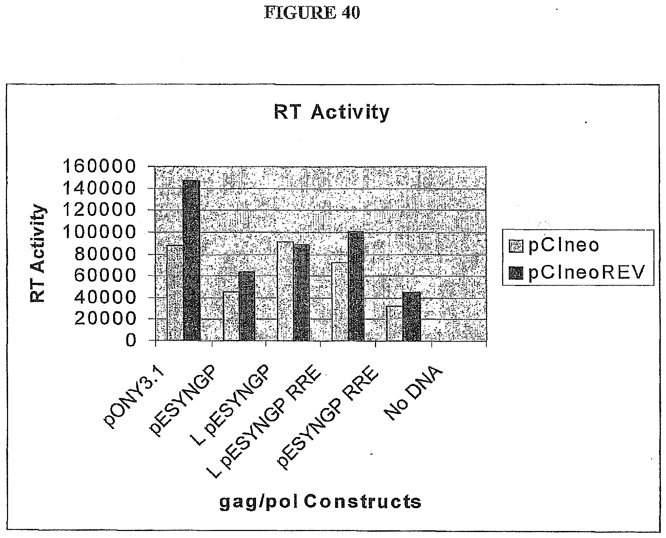

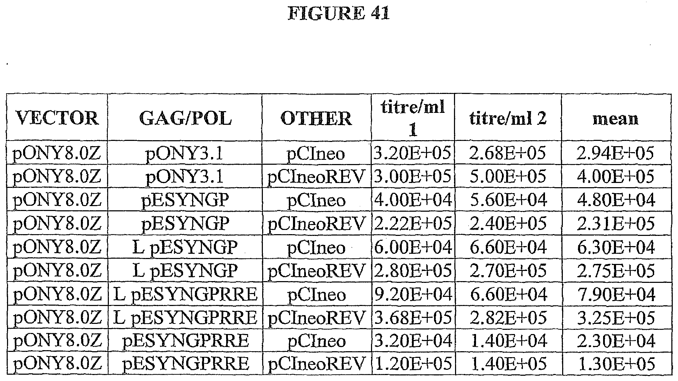

We have previously demonstrated (43) that the 5' leader (121bp upstream of the ATG start codon) and the RRE sequence (43) are important for high expression of the wild type EIAV *gag-pol.* Three constructs were made that contained either the leader sequence (LpESYNGP), the leader and RRE sequences (LpESYNGPRRE) or the RRE sequence (pESYNGPRRE). The sequences of these constructs are shown in SEQ ID NOS:6-8 and Figures 28-30. They were transfected into 293T cells in either the presence or absence of Rev expression plasmid. The cell supernatant was then measured for reverse transcriptase activity (RT), using a conventional RT assay, to evaluate which construct generated the highest amount of *gag-pol* mRNA. The results are shown in Figures 39 and 40. It is clear from these results that the 5' leader leads to an increase in RT activity. The ability of these Gag/Pol expression constructs to support formation of infectious vector particles was also tested by transient transfection of HEK 293 cells. The results of this analysis of show that all of the constructs could provide functional EIAV Gag/Pol, and show the Rev dependence of titre with the pONY8.0Z vector genome plasmid, which does not encode any EIAV proteins (Figure 41).

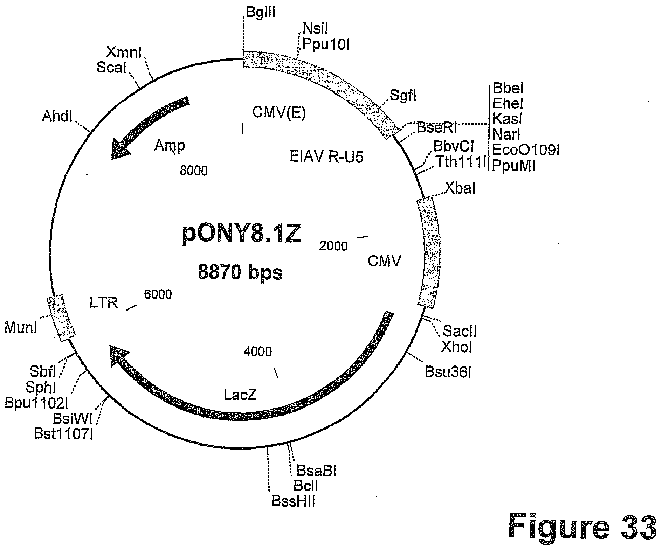

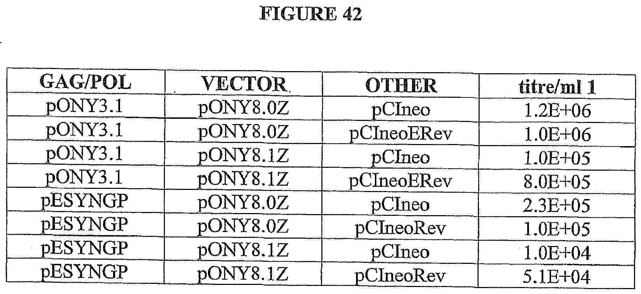

The ability of pESYNGP to act in concert with a minimal EIAV vector genome plasmid pONY8.1Z (Figure 33, SEQ ID NO:11) was evaluated (Figure 42). The result shows that the titres obtained with pESYNGP and pONY8.1Z are about 10-fold lower than from pONY3.1 and pONY8.1Z. This reduced titre reflects the lack of Rev protein in the system rather than a deficiency of Gag/Pol production which we have already shown is independent of Rev expression.

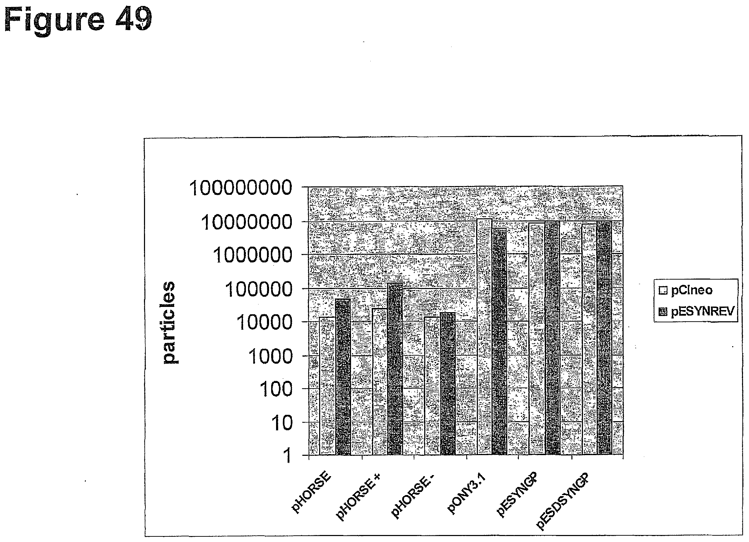

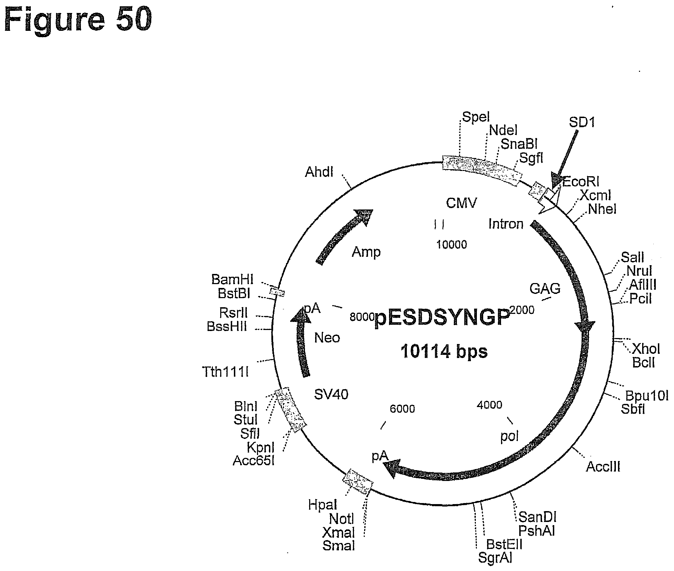

Expression of EIAY Gag/Pol was also tested from pESDSYNGP (Figure 50 and SEQ ID NO:18) in which the Kozak consensus sequence of Gag is replaced by the natural EIAV splice donor. pESDSYNGP was made from pESYNGP by exchange of the 306bp *Eco*RI-*Nhe*I fragment, which runs from just upstream of the start codon for gag/pol to approximately 300 base pairs inside the gag/pol ORF with a 308bp *Eco*RI*-Nhe*I fragment derived by digestion of a PCR product made using pESYNGP as template and using the following primers: SD FOR [GGCTAGAGAATTCCAGGTAAGATGGGCGATCCCCTCACCTGG] and SD REV [TTGGGTACTCCTCGCTAGGTTC]. This manipulation replaces the Kozak concensus sequence upstream of the ATG in pESYNGP with the splice donor found in EIAV. The sequence between the *Eco*RI site and the ATG of gag/pol is thus CAGGTAAG, exactly as found in the natural viral sequence. Therefore the mRNA is deleted with respect to sequences upstream but not downstream of the splice donor. The performance of pESDSYNGP was assessed relative to pESYNGP and other expression plasmids by measurement of reverse transcriptase activity in supernatants from transiently transfected HEK 293T cells using a Taqman-based version of the product enhanced reverse transcriptase (PERT) assay. In this method, reverse transcriptase associated with vector particles is released by mild detergent treatment and used to synthesize cDNA using MS2 bacteriophage RNA as template. MS2 RNA template and primer are present in excess hence the amount of cDNA is proportional to the amount ofRT released from the particles. Therefore, the amount of cDNA synthesised is proportional to the number of particles. MS2 cDNA is then quantitated using Taqman technology. The assay is carried out on test samples in parallel with a vector stock of known titre and estimated particle content. The use of the standard allows creation of a 'standard curve' and allows the relative RT content of various samples to be calculated. The results of this analysis are shown in Figure 49. The results show that Gag/Pol expression is virtually identical from pESYNGP and pESDSYNGP. The results also indicate that expression is not significantly enhanced by Rev. The activity of the Rev expression plasmid is confirmed by the result obtained with pHORSE +, in which there is an RRE downstream of the wild type EIAV *gag*/*pol,* and that shows a 6-fold enhancement of expression in the presence of Rev. We also noted that the expression from pHORSE was enhanced 3-fold in the presence of Rev. Since this construct has no RRE it suggests that Rev may be having a non-specific enhancing effect on expression, possibly as a result of being expressed at high levels in this experimental system.

The ability of pESYNGP to participate in the formation of infectious viral vector particles, when co-transfected with plasmids for the vector genome and envelope was assessed by transient transfection of HEK 293T, as described previously (49, 50). Briefly, 293T cells were seeded on 6cm dishes (1.2 x 10⁶/dish) and 24 hours later they were transfected by the calcium phosphate procedure. The medium was replaced 12 hours post-transfection and supernatants were harvested 48 hours post-transfection, filtered (0.45 µm filters) and titered by transduction of D17, canine osteosarcoma cells, in the presence of 8 µg/ml Polybrene (Sigma). Cells were seeded at 0.9 x 10⁵ /well in 12 well plates 24 hours prior to use in titration assays. Dilutions of supernatant were made in complete media (DMEM/10%FBS) and 0.5ml aliquots plated out onto the D17 cells. 4 hours after addition of the vector the media was supplemented, with a further 1 ml of media. Transduction was assessed by X-gal staining of cells 48 hours after addition of viral dilutions.

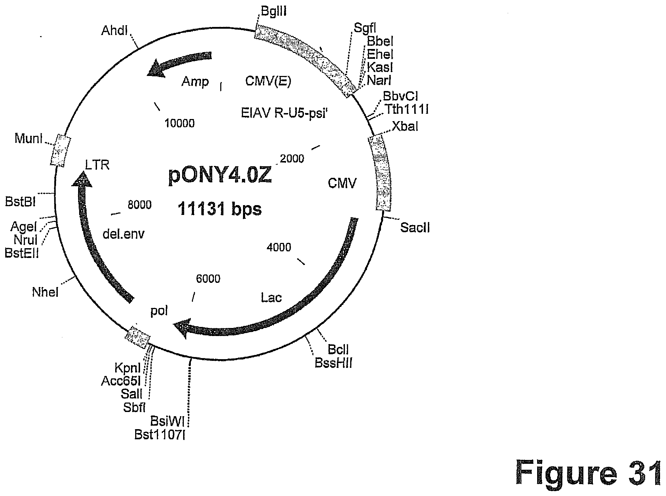

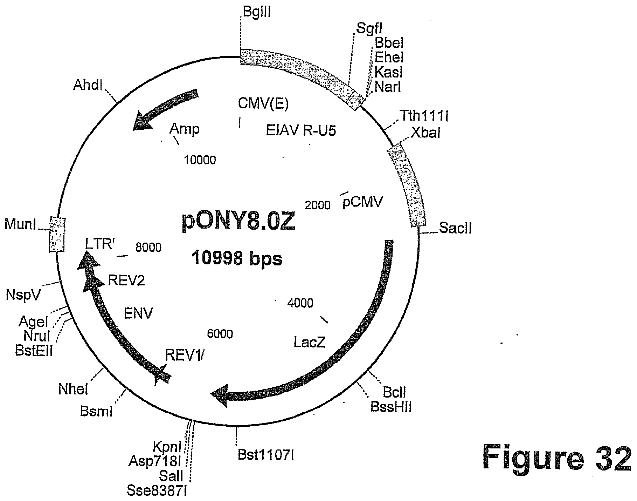

The vector genomes used for these experiments were pONY4.0Z (Figure 31 and SEQ ID NO:9) and pONY8.0Z (Figure 32 and SEQ ID NO:10).

pONY4.0Z (WO 99/32646) was derived from pONY2.11Z by replacement of the U3 region in the 5'LTR with the cytomegalovirus immediate early promoter (pCMV). This was carried out in such a way that the first base of the transcript derived from this CMV promoter corresponds to the first base of the R region. This manipulation results in the production of high levels of vector genome in transduced cells, particularly HEK 293T cells, and has been described previously (50). pONY4.0Z expresses all EIAV proteins except for envelope, expression of which is ablated by a deletion of 736nt between the *Hind*III sites present in *env.*

pONY8.0Z was derived from pONY4.0Z by introducing mutations which 1) prevented expression of TAT by an 83nt deletion in the exon 2 of tat) prevented S2 ORF expression by a 51 nt deletion 3) prevented REV expression by deletion of a single base within exon 1 of rev and 4) prevented expression of the N-terminal portion of gag by insertion of T in ATG start codons, thereby changing the sequence to ATTG from ATG. With respect to the wild type EIAV sequence Acc. No. U01866 these correspond to deletion of nt 5234-5316 inclusive, nt 5346-5396 inclusive and nt 5538. The insertion of T residues was after nt 526 and 543.

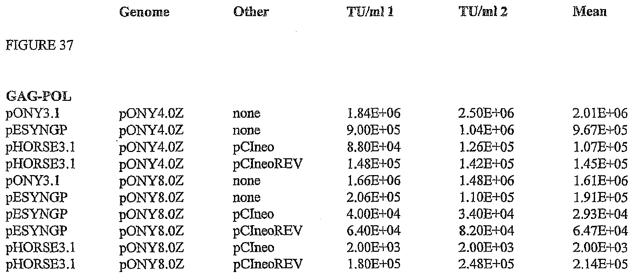

The results of this analysis are shown tabulated in Figure 37, and graphically in Figure 38. Transfections were carried out with only 3 plasmids (vector genome, gag/pol expression plasmid and VSV-G expression plasmid) - diagonal lined bars, or with four plasmids, which included the previous set of plasmids together with an additional plasmid encoding Rev or a similar plasmid not coding a functional protein - filled bars. The result show that high titres of vector can be achieved using pESYNGP to supply EIAV Gag/Pol. The highest titres were obtained using the Rev-expressing vector genome plasmid, pONY4.0Z, and they were only slightly lower than observed when Gag/Pol was supplied by pONY3.1. Lower titres were observed with pONY8.0Z vector genome plasmid with pESYNGP than with pONY3.1. This is due to the Rev expression requirement of pONY8.0Z. Rev is expressed by pONY3.1, but not pESYNGP. These results confirm the utility of the codon-optimised Gag/Pol expression plasmid.

### Use of the synthetic EIAV gag/pol gene in construction of cell lines which stably express EIAV gag/pol.

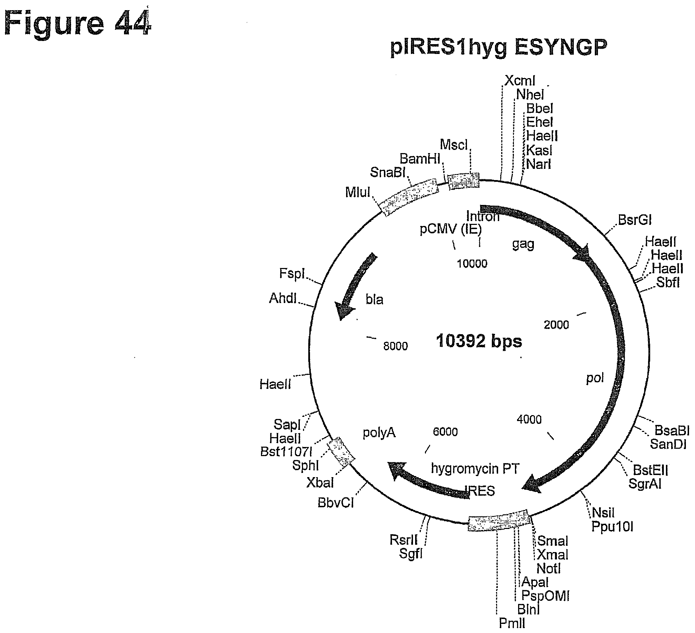

Cells lines which express high amounts of EIAV Gag/pol are required for the construction of packaging and producer cells for EIAV vectors. As a first step in their construction HEK 293 cells were stably transfected with pIRES1hyg ESYNGP (Figure 44 and SEQ ID NO:17), in which EIAV Gag/pol expression is driven by a CMV promoter, and is linked to an ORF for expression of hygromycin phosphotransferase by an EMCV IRES. pIRES1hyg ESYNGP was made as follows. The synthetic EIAV gag/pol gene and flanking sequences was transferred from pESYNGP into pIRES1hygro expression vector (Clontech). First, pESYNGP was digested with *Eco*RI*,* and the ends filled in by treatment with T4DNA polymerase and then digested with *Not*I*.* pIRES1hygro was prepared for ligation with this fragment by digestion with *Nsi*I*,* the ends trimmed flush by treatment with T4 DNA polymerase, then digested with *Not*I*.* Prior to transfection into HEK 293 cells pIRES1hyg ESYNGP was digested with *Ahd*I which linearises the plasmid.

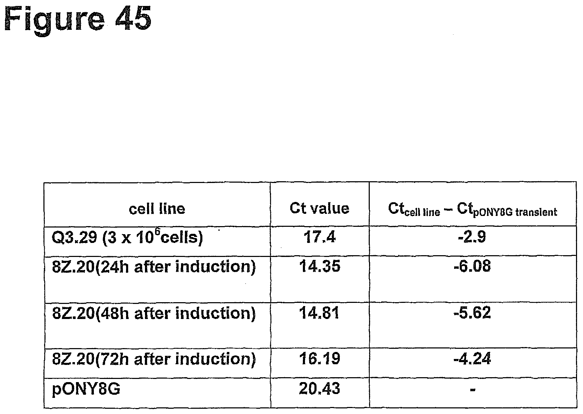

Clonal cell lines,were derived by serial dilution and analysed for expression of Gag/Pol by a Taqman-based product enhanced reverse trariscriptase (PERT) assay. Data for the cell line Q3.29, which expressed the highest level of Gag/Pol is shown. The analysis showed that the level of expression from the codon-optimised EIAV Gag/Pol cassette in Q3.29 was very similar to that seen for an EIAV producer line, 8Z.20, in which Gag/Pol is expressed from the pEV53B wild type expression cassette, that produced vector particles at titres of almost 10⁶ transducing units per ml. (Figure 45). Assuming exponential amplification during the assay, a difference of Ct value of 1.0 corresponds to a difference of 2-fold in concentration of the reverse transcriptase released from the particles. Therefore the difference in Gag/Pol expression between Q3.29 and 8Z.20 cells is approximately 2-8 fold. Furthermore the Ct values observed indicate that the level of expression of Gag/Pol is significantly higher than in samples of pONY8G vector particles with a titre of 2 x 10⁶ transducing units per ml on D17 cells, but made by transient transfection of HEK 293T cells. These data indicate that the codon-optimised EIAV Gag/Pol construct can be used in the construction of EIAV packaging and producer lines and confirms the previous result that expression is independent of Rev expression.

The Q3.29 cell line was then tested for its ability to support production of infectious vector particles when transfected with a vector genome plasmid, pONY8.0Z, and the VSV-G envelope expression plasmid, pRV67 and the EIAV REV expression plasmid, pESYNREV. In addition we also evaluated the performance of a plasmid pONY8.3G FB29 (-) which is modified form of the pONY8G vector genome plasmid. PONY8G is a standard EIAV vector genome used for comparison purposes. The modifications and construction of pONY8.3G FB29 (-) (SEQ ID NO:19) are described in PCT/GB00/03837 and briefly are 1) the introduction of loxP recognition sites upstream and downstream of the vector genome cassette 2) the placement of an expression cassette for codon-optimised REV, derived from pESYNREV, and driven by the FB29 U3 promoter downstream of the vector genome cassette and orientated so that the direction of transcription was towards the vector genome cassette. The REV expression cassette is located upstream of the 3' loxP site. Thus the pONY8.3G FB29 - plasmid carries expression cassettes for the vector genome RNA and for EIAV Rev.

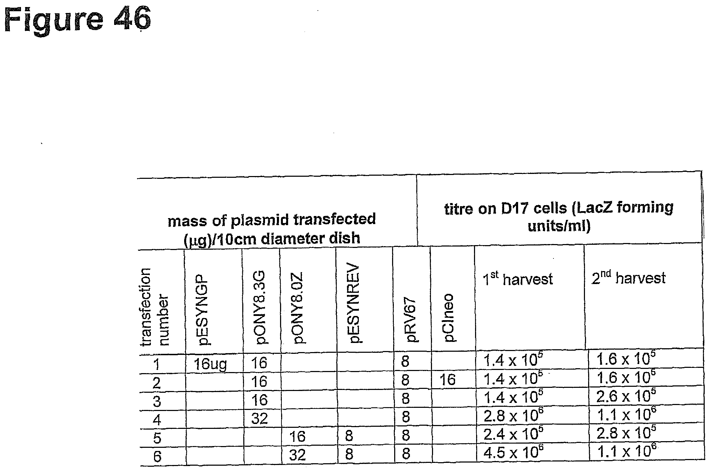

The titres were established by limiting dilution on D17 canine osteosarcoma cells and are shown in Figure 46

The titres obtained from transfections 2-6 were up to 4.5 x 10⁶ transducing units per ml indicating levels of Gag/Pol expression sufficient to support titres at least this high. The titres obtained were not higher when additional Gag/Pol was supplied (transfection 1) indicating that Gag/Pol expression was not the limitation on titre.

### Improved safety profile due to Gag/Pol expression from a codon-optimised expression construct

RCR formation takes place by recombination between different components of the vector system or by recombination of vector system components with nucleotide sequences present in the producer cells. Although recombination at the DNA level during construction of producer cell lines is possible (perhaps leading to insertional activation of endogenous retroelements or retroviruses) it is thought that recombination to produce RCR occurs mainly between RNA's undergoing reverse transcription, hence occurs within the mature vector particles. In consequence, recombination will be more likely to occur between RNA's which contain packaging signals, such as the vector genome and the *gag*/*pol* mRNA. Usually however the *gag*/*pol* transcript is modified so that it is deleted with respect to some or all defined packaging elements, thereby reducing the chances of its involvement in recombination.

The codon-optimisation process used to create the HIV and EIAV Gag/Pol expression plasmid, pSYNGP and pESYNGP, also results in disruption of sequences and structures that direct packaging as a result of introducing changes at approximately every 3^{rd} nucleotide position. We have obtained evidence for the lower level of incorporation of the codon-optimised RNA derived from pESYNGP into virions.

The packaging of mRNA's derived from a wild type *gag*/*pol* pEV53B expression cassette, and from the codon-optimised EIAV *gag*/*pol* expression cassette, pESYNGP, was compared. Medium was collected from a HEK 293 based cell-lines which were stably transfected with either pEV53B (cell line B-241), or with pESYNGP. Both cell lines produce vector particles which do not contain vector RNA and do not have envelopes. In some experiments, an EIAV vector genome plasmid (pECG3-CZW) was transfected into the cells to serve as an internal positive control for hybridisation and for the presence of particles capable of packaging RNA. pECG3-CZW is a derivative of pEC-LacZ (WO 98/51810) and was made from the latter by 1) reduction of *gag* sequences so that only the first 200nt of *gag,* rather than the first 577nt, was included and 2) inclusion of the woodchuck hepatitis virus post-transcriptional regulatory element (WHV PRE) (corresponding to nt 901-1800 of Acc. No. J04514) into the *Not*I site downstream of the LacZ reporter gene.

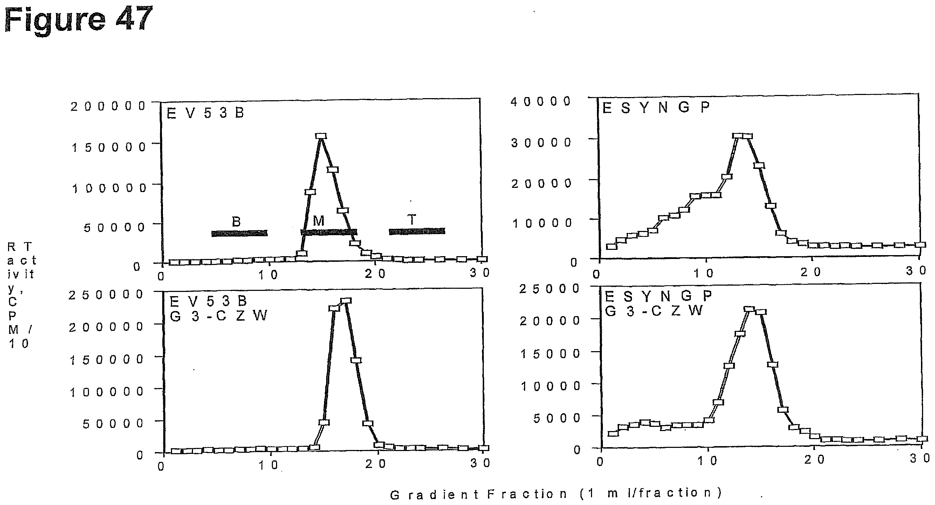

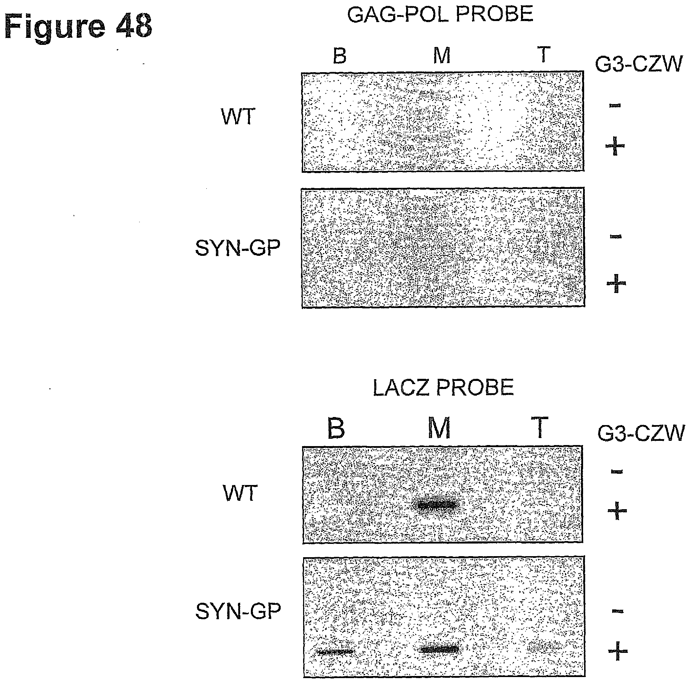

Viral particles derived from each of the cell lines were then partially purified from the medium by equilibrium density gradient centrifugation. To do this 10 ml of medium from producer cells, harvested at 24 hours after induction with sodium butyrate, was layered onto a 20-60% (w/w) sucrose gradient in TNE buffer (pH 7.4) and centrifuged for 24 hours at 25,000 rpm and 4° C in a SW28 rotor. Fractions were collected from the bottom and 10 µl of each fraction assayed for reverse transcriptase activity to locate viral particles. The results of this analysis are shown in (Figure 47) where the profile of reverse transcriptase activity is shown as a function of gradient fraction. In these figures, the top of the gradient is on the right. It should be noted that the levels of RT activity from the pESYNGP-expressing cell were significantly lower than from pEV53B expressing cells. To determine the RNA content of the purified virions, aliquots from the top, middle or bottom fractions were pooled (as indicated by the bars labeled T, M and B) and the RNA from each fraction was subjected to slot-blot hybridization analysis. Using a probe specific for a common region of wild type and synthetic *gag*/*pol,* encapsidation of RNA was easily detectable in the peak fractions (M) of virions synthesized from the wild type construct (pEV53B), but was not detected from virions synthesized from the synthetic Gag/Pol construct (pESYNGP)(Figure 48). The control for the presence of capsid capable of carrying out encapsidation was the EIAV G3-CZW vector genome which was readily detected in peak fractions from cells expressing either the wild type or synthetic gag/pol proteins. Even taking into account the different levels of expression from the wild type and synthetic Gag/Pol expression constructs this result indicates that the RNA from the codon-optimised *gag*/*pol* gene is packaged significantly less efficiently than the wild type gene and represents a significant improvement to the safety profile of the system. Of further note is that the RNA transcribed from pEV53B was packaged. This RNA is deleted with respect to sequences upstream of the splice donor sequence (CAG/GTAAG) and yet was still packaged. This points to the localisation of major packaging determinants within the gag coding region and is in contrast to the collected observations on the location of the packaging signal of HIV-1.

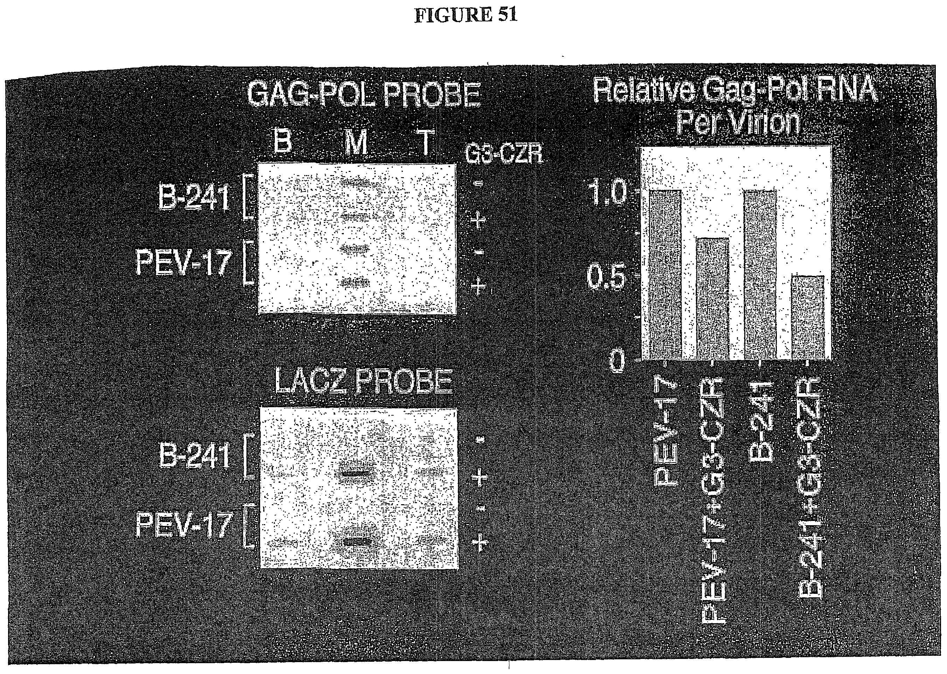

In additional experiments we have shown that the packaging of transcripts from pEV53B is only slightly lower than from pEV53A (Figure 51). This indicates further that major packaging sequences are located within the gag coding region. In these experiments cell line B-241 expressed pEV53B RNA and PEV-17 expressed pEV53A RNA. The EIAV vector genome used to confirm the presence of packaging competent vector particles was G3-CZR, which is the same as G3-CZW, described above, except for the replacement of the woodchuck post-transcriptional regulatory element with a sequence containing the EIAV RRE elements. Methodology was as described above.

### References

1. Miller, N., and J. Whelan. 1997. Hum Gene Ther. 8:803-15.
2. Lewis & Emerman. 1993. J. Virol. 68:510.
3. Naldini, L., U. Blomer, P. Gallay, D. Ory, R. Mulligan, F. H. Gage, 1. M. Verma, and D. Trono. 1996. Science. 272:263-7.
4. Morgenstern & Land. 1990. Nucleic Acids Res. 18: 3587-3596.
5. Chang, D. D., and P. A. Sharp. 1989. Cell. 59:789-795.
6. Wang & Semenza. 1993. Proc Natl Acad Sci. 90:430.
7. Dachs et al. 1997. Nature Med. 5:515.
8. Firth et al. 1994. Proc Natl Acad Sci. 90: 6496-6500.
9. Madan et al. 1993. Proc Natl Acad Sci. 90:3928.
10. Semenza & Wang. 1992. Mol Cell Biol. 1992. 12: 5447-5454.
11. Takenaka et al. 1989. J Biol Chem. 264: 2363-2367.
12. Peshavaria & Day. 1991. Biochem J. 275: 427-433.
13. Inou et al. 1989. J Biol Chem. 264: 14954-14959.
14. Overell et al. 1988. Mol Cell Biol. 8: 1803-1808.
15. Attenello & Lee. 1984. Science. 226: 187-190.
16. Gazit et al. 1985. Cancer Res. 55: 1660-1663.
17. Yu et al. 1986. Proc Natl Acad Sci. 83: 3194-3198.
18. Dougherty & Temin. 1987. Proc Natl Acad Sci. 84: 1197-1201.
19. Hawley et al. 1987. Proc Natl Acad Sci. 84: 2406-2410.
20. Yee, J. K., A. Miyanohara, P. LaPorte, K. Bouic, J. C. Burns, and T. Friedmann. 1994. Proc. Natl. Acad. Sci. USA. 91:9564-8.
21. Jolley et al. 1983. Nucleic Acids Res. 11: 1855-1872.
22. Emerman & Tenim. 1984. Cell. 39: 449-467.
23. Herman & Coffin. 1987. Science. 236: 845-848.
24. Bender et al., 1987, J Virol 61: 1639-1646.
25. Pear et al., 1993, Proc Natl Acad Sci 90: 8392-8396.
26. Danos & Mulligan. 1998. Proc Natl Acad Sci. 85: 6460-6464.
27. Markowitz et al. 1988. Virology. 167: 400-406.
28. Cosset et al., 1995, J. Virol. 69: 7430-7436.
29. Mebatsion et al. 1997. Cell. 90: 841-847.
30. Marshall. 1998. Nature Biotechnology. 16: 129.
31. Nature Biotechnology. 1996. 14:556.
32. Wolff & Trubetskoy. 1998. Nature Biotechnology. 16: 421.
33. DuBridge, R. B., P. Tang, H. C. Hsia, P.-M. Leong, J. H. Miller, and M. P. Calos. 1987. Mol. Cell. Biol. 7:379-387.
34. Gey, G. O., W. D. Coffman, and M. T. Kubicek. 1952. Cancer res. 12:264.
35. Kim, S. Y., R. Byrn, J. Groopman, and D. Baltimore. 1989. J. Virol. 63:3708-3713.
36. Adachi, A., H. Gendelman, S. Koenig, T. Folks, R. Willey, A. Rabson, and M. Martin. 1986. J. Virol. 59:284-291.
37. Haas, J., E.-C. Park, and B. Seed. 1996. Current Biology. 6:315.
38. Zolotukhin, S., M. Potter, W. W. Hauswirth, J. Guy, and N. Muzyczka. 1996. A "humanized" green fluorescent protein cDNA adapted for high-level expression in mammalian cells. J Virol. 70:4646-54.
39. Fisher, A., E. Collalti, L. Ratner, R. Gallo, and F. Wong-Staal. 1985. Nature. 316:262-265.
40. Kozak, M. 1992. [Review]. Annu. Rev. Cell Biol. 8:197-225.
41. Cassan, M., N. Delaunay, C. Vaquero, and J. P. Rousset. 1994. J. Virol. 68:1501-8.
42. Parkin, N. T., M. Chamorro, and H. E. Varmus. 1992. J. Virol. 66:5147-51. 68:3888-3895.
43. Mitrophanous K, Yoon S, Rohll J, Patil D, Wilkes F, Kim V, Kingsman S, Kingsman A, Mazarakis N, 1999. Gene Ther. 6 (11): 1808-18
44. Ory, D. S., B. A. Neugeboren, and R. C. Mulligan. 1996. Proc Natl Acad Sci USA. 93:11400-6.
45. Zhu, Z. H., S. S. Chen, and A. S. Huang. 1990. J Acquir Immune Defic Syndr. 3:215-9.
46. Chesebro, B., K. Wehrly, and W. Maury. 1990. J Virol. 64:4553-7.
47. Spector, D. H., E. Wade, D. A. Wright, V. Koval, C. Clark, D. Jaquish, and S. A. Spector. 1990. J Virol. 64:2298-2308.
48. Valsesia Wittmann, S., A. Drynda, G. Deleage, M. Aumailley, J. M. Heard, O. Danos, G. Verdier, and F. L. Cosset. 1994. J Virol. 68:4609-19.
49. Kim, V. N., K. Mitrophanous, S. M. Kingsman, and K. A. J. 1998. J Virol 72: 811-816.
50. Soneoka, Y., P. M. Cannon, E. E. Ramsdale, J. C. Griffiths, G. Romano, S. M. Kingsman, and A. J. Kingsman. 1995. Nucleic Acids Res. 23:628-33.
51. Sagerstrom, C., and H. Sive. 1996. RNA blot analysis, p. 83-104. In P. Krieg (ed.), A laboratory guide to RNA: isolation, analysis and synthesis, vol. 1. Wiley-Liss Inc., New York.
52. Malim, M. H., J. Hauber, S. Y. Le, J. V. Maizel, and B. R. Cullen. 1989. Nature. 338:254-7.
53. Felber, B. K., M. Hadzopoulou Cladaras, C. Cladaras, T. Copeland, and G. N. Pavlakis. 1989. Proc. Natl. Acad. Sci. USA.. 86:1495-1499.
54. Hadzopoulou Cladaras, M., B. K. Felber, C. Cladaras, A. Athanassopoulos, A. Tse, and G. N. Pavlakis. 1989. J. Virol. 63:1265-74.
55. Schneider, R., M. Campbell, G. Nasioulas, B. K. Felber, and G. N. Pavlakis. 1997. J Virol. 71 :4892-903.
56. Schwartz, S., B. K. Felber, and G. N. Pavlakis. 1992. J. Virol. 66:150-159.
57. Maldarelli, F., M. A. Martin, and K. Strebel. 1991 J. Virol. 65:5732-5743.
58. Mikaelian, I., M. Krieg, M. Gait, and J. Karn. 1996. J. Mol. Biol. 257:246-264.
59. Xu, N., C.-Y. Chen, and A.-B. Shyu. 1997. Mol. Cell. Biol. 17:4611-4621.
60. Naldini, L. 1998. Curr. Opin. Biotechnol. 9:457-463.
61. Parolin, C., T. Dorfman, G. Palu, H. Gottlinger, and J. Sodroski. 1994J. Virol.
62. Dull, T., R. Zufferey, M. Kelly, R. Mandel, M. Nguyen, D. Trono, and L. Naldini. 1998. J. Virol. 72:8463-8471.
63. Cui, Y., T. Iwakama, and L.-J. Chang. 1999. J. Virol. 73:6171-6176.
64. Chang, L.-J., V. Urlacher, T. Iwakama, Y. Cui, and J. Zucali. 1999. Gene Ther. 6:715-728.
65. Harrison, G., G. Miele, E. Hunter, and A. Lever. 1998. J. Virol. 72:5886-5896.
66. McBride, M. S., and A. T. Panganiban. 1997. J. Virol. 71:2050-8.
67. Lever, A., H. Gottlinger, W. Haseltine, and J. Sodroski. 1989 J. Virol. 63:4085-7.
68. Brighty, D., and M. Rosenberg. 1994. Proc. Natl. Acad. Sci. USA. 91:8314-8318.
69. Arai, T., M. Takada, M. Ui, and H. Iba. 1999. Virology. 260:109-115.
70. Fornerod, M., M. Ohno, M. Yoshida, and I. W. Mattaj. 1997. Cell. 90:1051-1060.
71. Fridell, R. A., H. P. Bogerd, and B. R. Cullen. 1996. Proc. Natl. Acad. Sci. U S A. 93:4421-4.
72. Pollard, V., and M. Malim. 1998. Annu. Rev. Microbiol. 52:491-532.
73. Stade, K., C. S. Ford, C. Guthrie, and K. Weis. 1997. Cell. 90:1041-1050.
74. Ullman, K. S., M. Powers, A" and D. J. Forbes. 1997. Cell. 90:967-970.
75. Otero, G. C., M. E. Harris, J. E. Donello, and T. J. Hope. 1998. J. Virol. 72:7593-7597.
76. Wolff et al. 1997. Chem Biol. 4: 139-147.

## Claims

1. A method of producing a replication defective HIV vector comprising transfecting a producer cell with the following:
i) an HIV genome comprising a nucleotide of interest (NOI), a packaging signal, and RRE;
ii) a nucleotide sequence coding for HIV gag and pol proteins; and
iii) a nucleotide sequence coding for an env protein;
**characterised in that** the nucleotide sequence coding for the HIV gag and pol proteins is codon optimised for expression in the producer cell with the exception of the sequence encompassing the frameshift site and wherein (ii) does not comprise RRE; wherein the producer cell expresses *rev* and wherein the HIV vector is a self-inactivating vector.

2. A method according to claim 1, wherein the codon optimised nucleotide sequence has the sequence shown in SEQ ID NO: 15.

3. An HIV vector particle producer cell comprising:
i) an HIV, genome comprising at least one nucleotide sequence of interest, packaging signal, and RRE; and
ii) a nucleotide sequence coding for HIV gag and pol proteins; and
iii) a nucleotide sequence coding for an env protein;
**characterised in that** the nucleotide sequence coding for the HIV gag and pol proteins is codon optimised for expression in the producer cell with the exception of the sequence encompassing the frameshift site and wherein (ii) does not comprise RRE; wherein the producer cell expresses *rev* and wherein the HIV vector is a self-inactivating vector.

4. A producer cell according to claim 3, wherein the codon optimised nucleotide sequence has the sequence shown in SEQ ID NO: 15.

5. A producer cell according to claim 3 wherein the envelope gene is codon optimised.

6. A producer cell according to any of claims 3 to 5 wherein the nucleotide of interest is selected from a therapeutic gene, a marker gene and a selection gene.

7. A producer cell according to any one of claims 3 to 6 for use in a method of producing HIV vector particles.

8. A pharmaceutical composition comprising the producer cell of any one of claims 3 to 7, together with a pharmaceutically acceptable carrier or diluent.

## Patentansprüche

1. Verfahren zum Herstellen eines replikations-defizienten HIV-Vektors, umfassend das Transfizieren einer Produzentenzelle mit Folgendem:
i) einem HIV-Genom umfassend ein Nukleotid von Interesse (NVI), ein Verpackungssignal und RRE;
ii) einer Nukleotidsequenz, die für die HIV-Proteine gag und pol codiert; und
iii) einer Nukleotidsequenz, die für ein env-Protein codiert;
**dadurch gekennzeichnet, dass** die Nukleotidsequenz, die für die HIV-Proteine gag und pol codiert, für die Expression in der Produzentenzelle Codon-optimiert ist, mit Ausnahme der Sequenz umfassend die Leserasterverschiebungs-Stelle, und wobei (ii) nicht RRE umfasst; wobei die Produzentenzelle *rev* exprimiert und wobei der HIV-Vektor ein selbstinaktivierender Vektor ist.

2. Verfahren nach Anspruch 1, wobei die Codon-optimierte Nukleotidsequenz die in SEQ ID NO: 15 dargestellte Sequenz aufweist.

3. HIV-Vektorpartikel-Produzentenzelle, die Folgendes umfasst:
i) ein HIV-Genom umfassend wenigstens eine Nukleotidsequenz von Interesse, ein Verpackungssignal und RRE; und
ii) eine Nukleotidsequenz, die für die HIV-Proteine gag und pol codiert; und
iii) eine Nukleotidsequenz, die für ein env-Protein codiert;
**dadurch gekennzeichnet, dass** die Nukleotidsequenz, die für die HIV-Proteine gag und pol codiert, für die Expression in der Produzentenzelle Codon-optimiert ist, mit Ausnahme der Sequenz umfassend die Leserasterverschiebungs-Stelle, und wobei (ii) nicht RRE umfasst; wobei die Produzentenzelle *rev* exprimiert und wobei der HIV-Vektor ein selbstinaktivierender Vektor ist.

4. Produzentenzelle nach Anspruch 3, wobei die Codon-optimierte Nukleotidsequenz die in SEQ ID NO: 15 dargestellte Sequenz aufweist.

5. Produzentenzelle nach Anspruch 3, wobei das HüllGen Codon-optimiert ist.

6. Produzentenzelle nach einem der Ansprüche 3 bis 5, wobei das Nukleotid von Interesse aus einem therapeutischen Gen, einem Markergen und einem Selektionsgen ausgewählt ist.

7. Produzentenzelle nach einem der Ansprüche 3 bis 6 zur Verwendung in einem Verfahren zur Erzeugung von HIV-Vektorpartikeln.

8. Pharmazeutische Zusammensetzung, die die Produzentenzelle nach einem der Ansprüche 3 bis 7 zusammen mit einem pharmazeutisch unbedenklichen Träger oder Verdünnungsmittel umfasst.

## Revendications

1. Procédé de production d'un vecteur de VIH déficient en réplication comprenant la transfection d'une cellule productrice avec les éléments suivants :
i) un génome de VIH comprenant un nucléotide d'intérêt (NOI), un signal d'encapsulation, et RRE ;
ii) une séquence nucléotidique codant pour les protéines gag et pol de VIH ; et
iii) une séquence nucléotidique codant pour une protéine env ;
**caractérisé en ce que** la séquence nucléotidique codant pour les protéines gag et pol de VIH est optimisée en codon pour expression dans la cellule productrice à l'exception de la séquence comprenant le site de déphasage et où (ii) ne comprend pas RRE ; la cellule productrice exprimant rev et le vecteur de VIH étant un vecteur auto-inactivateur.

2. Procédé selon la revendication 1, dans lequel la séquence nucléotidique optimisée en codon présente la séquence décrite dans SEQ ID NO: 15.

3. Cellule productrice de particule de vecteur de VIH comprenant :
i) un génome de VIH comprenant au moins une séquence nucléotidique d'intérêt, un signal d'encapsulation et RRE ; et
ii) une séquence nucléotidique codant pour les protéines gag et pol de VIH ; et
iii) une séquence nucléotidique codant pour une protéine env ;
**caractérisée en ce que** la séquence nucléotidique codant pour les protéines gag et pol de VIH est optimisée en codon pour l'expression dans la cellule productrice à l'exception de la séquence comprenant le site de déphasage et où (ii) ne comprend pas RRE ; la cellule productrice exprimant rev et le vecteur de VIH étant un vecteur auto-inactivateur.

4. Cellule productrice selon la revendication 3, dans laquelle la séquence nucléotidique optimisée en codon a la séquence décrite dans SEQ ID NO: 15.

5. Cellule productrice selon la revendication 3 dans laquelle le gène d'enveloppe est optimisé en codon.

6. Cellule productrice selon l'une quelconque des revendications 3 à 5 dans laquelle le nucléotide d'intérêt est choisi parmi un gène thérapeutique, un gène marqueur et un gène de sélection.

7. Cellule productrice selon l'une quelconque des revendications 3 à 6 pour utilisation dans un procédé de production de particules de vecteur de VIH.

8. Composition pharmaceutique comprenant la cellule productrice de l'une quelconque des revendications 3 à 7, conjointement avec un véhicule ou diluant pharmaceutiquement acceptable.


### SEQUENCE LISTING

<110> Oxford Biomedica (UK) Limited
<120> Method
<130> P008827EPA
<150> GB 0009760.0
   <151> 2000-04-19
<160> 37
<170> PatentIn version 3.5
<210> 1
   <211> 4307
   <212> DNA
   <213> Human immunodeficiency virus
<400> 1
   atgggtgcga gagcgtcagt attaagcggg ggagaattag atcgatggga aaaaattcgg 60
   ttaaggccag ggggaaagaa aaaatataaa ttaaaacata tagtatgggc aagcagggag 120
   ctagaacgat tcgcagttaa tcctggcctg ttagaaacat cagaaggctg tagacaaata 180
   ctgggacagc tacaaccatc ccttcagaca ggatcagaag aacttagatc attatataat 240
   acagtagcaa ccctctattg tgtgcatcaa aggatagaga taaaagacac caaggaagct 300
   ttagacaaga tagaggaaga gcaaaacaaa agtaagaaaa aagcacagca agcagcagct 360
   gacacaggac acagcaatca ggtcagccaa aattacccta tagtgcagaa catccagggg 420
   caaatggtac atcaggccat atcacctaga actttaaatg catgggtaaa agtagtagaa 480
   gagaaggctt tcagcccaga agtgataccc atgttttcag cattatcaga aggagccacc 540
   ccacaagatt taaacaccat gctaaacaca gtggggggac atcaagcagc catgcaaatg 600
   ttaaaagaga ccatcaatga ggaagctgca gaatgggata gagtgcatcc agtgcatgca 660
   gggcctattg caccaggcca gatgagagaa ccaaggggaa gtgacatagc aggaactact 720
   agtacccttc aggaacaaat aggatggatg acaaataatc cacctatccc agtaggagaa 780
   atttataaaa gatggataat cctgggatta aataaaatag taagaatgta tagccctacc 840
   agcattctgg acataagaca aggaccaaag gaacccttta gagactatgt agaccggttc 900
   tataaaactc taagagccga gcaagcttca caggaggtaa aaaattggat gacagaaacc 960
   ttgttggtcc aaaatgcgaa cccagattgt aagactattt taaaagcatt gggaccagcg 1020
   gctacactag aagaaatgat gacagcatgt cagggagtag gaggacccgg ccataaggca 1080
   agagttttgg ctgaagcaat gagccaagta acaaattcag ctaccataat gatgcagaga 1140
   ggcaatttta ggaaccaaag aaagattgtt aagtgtttca attgtggcaa agaagggcac 1200
   acagccagaa attgcagggc ccctaggaaa aagggctgtt ggaaatgtgg aaaggaagga 1260
   caccaaatga aagattgtac tgagagacag gctaattttt tagggaagat ctggccttcc 1320
   tacaagggaa ggccagggaa ttttcttcag agcagaccag agccaacagc cccaccagaa 1380
   gagagcttca ggtctggggt agagacaaca actccccctc agaagcagga gccgatagac 1440
   aaggaactgt atcctttaac ttccctcagg tcactctttg gcaacgaccc ctcgtcacaa 1500
   taaagatagg ggggcaacta aaggaagctc tattagatac aggagcagat gatacagtat 1560
   tagaagaaat gagtttgcca ggaagatgga aaccaaaaat gataggggga attggaggtt 1620
   ttatcaaagt aagacagtat gatcagatac tcatagaaat ctgtggacat aaagctatag 1680
   gtacagtatt agtaggacct acacctgtca acataattgg aagaaatctg ttgactcaga 1740
   ttggttgcac tttaaatttt cccattagcc ctattgagac tgtaccagta aaattaaagc 1800
   caggaatgga tggcccaaaa gttaaacaat ggccattgac agaagaaaaa ataaaagcat 1860
   tagtagaaat ttgtacagag atggaaaagg aaggaaaaat ttcaaaaatt gggcctgaaa 1920
   atccatacaa tactccagta tttgccataa agaaaaaaga cagtactaaa tggagaaaat 1980
   tagtagattt cagagaactt aataagagaa ctcaagactt ctgggaagtt caattaggaa 2040
   taccacatcc cgcagggtta aaaaagaaaa aatcagtaac agtactggat gtgggtgatg 2100
   catatttttc agttccctta gatgaagact tcaggaagta tactgcattt accataccta 2160
   gtataaacaa tgagacacca gggattagat atcagtacaa tgtgcttcca cagggatgga 2220
   aaggatcacc agcaatattc caaagtagca tgacaaaaat cttagagcct tttagaaaac 2280
   aaaatccaga catagttatc tatcaataca tggatgattt gtatgtagga tctgacttag 2340
   aaatagggca gcatagaaca aaaatagagg agctgagaca acatctgttg aggtggggac 2400
   ttaccacacc agacaaaaaa catcagaaag aacctccatt cctttggatg ggttatgaac 2460
   tccatcctga taaatggaca gtacagccta tagtgctgcc agaaaaagac agctggactg 2520
   tcaatgacat acagaagtta gtggggaaat tgaattgggc aagtcagatt tacccaggga 2580
   ttaaagtaag gcaattatgt aaactcctta gaggaaccaa agcactaaca gaagtaatac 2640
   cactaacaga agaagcagag ctagaactgg cagaaaacag agagattcta aaagaaccag 2700
   tacatggagt gtattatgac ccatcaaaag acttaatagc agaaatacag aagcaggggc 2760
   aaggccaatg gacatatcaa atttatcaag agccatttaa aaatctgaaa acaggaaaat 2820
   atgcaagaat gaggggtgcc cacactaatg atgtaaaaca attaacagag gcagtgcaaa 2880
   aaataaccac agaaagcata gtaatatggg gaaagactcc taaatttaaa ctgcccatac 2940
   aaaaggaaac atgggaaaca tggtggacag agtattggca agccacctgg attcctgagt 3000
   gggagtttgt taatacccct cccttagtga aattatggta ccagttagag aaagaaccca 3060
   tagtaggagc agaaaccttc tatgtagatg gggcagctaa cagggagact aaattaggaa 3120
   aagcaggata tgttactaat agaggaagac aaaaagttgt caccctaact gacacaacaa 3180
   atcagaagac tgagttacaa gcaatttatc tagctttgca ggattcggga ttagaagtaa 3240
   acatagtaac agactcacaa tatgcattag gaatcattca agcacaacca gatcaaagtg 3300
   aatcagagtt agtcaatcaa ataatagagc agttaataaa aaaggaaaag gtctatctgg 3360
   catgggtacc agcacacaaa ggaattggag gaaatgaaca agtagataaa ttagtcagtg 3420
   ctggaatcag gaaagtacta tttttagatg gaatagataa ggcccaagat gaacatgaga 3480
   aatatcacag taattggaga gcaatggcta gtgattttaa cctgccacct gtagtagcaa 3540
   aagaaatagt agccagctgt gataaatgtc agctaaaagg agaagccatg catggacaag 3600
   tagactgtag tccaggaata tggcaactag attgtacaca tttagaagga aaagttatcc 3660
   tggtagcagt tcatgtagcc agtggatata tagaagcaga agttattcca gcagaaacag 3720
   ggcaggaaac agcatatttt cttttaaaat tagcaggaag atggccagta aaaacaatac 3780
   atactgacaa tggcagcaat ttcaccggtg ctacggttag ggccgcctgt tggtgggcgg 3840
   gaatcaagca ggaatttgga attccctaca atccccaaag tcaaggagta gtagaatcta 3900
   tgaataaaga attaaagaaa attataggac aggtaagaga tcaggctgaa catcttaaga 3960
   cagcagtaca aatggcagta ttcatccaca attttaaaag aaaagggggg attggggggt 4020
   acagtgcagg ggaaagaata gtagacataa tagcaacaga catacaaact aaagaattac 4080
   aaaaacaaat tacaaaaatt caaaattttc gggtttatta cagggacagc agaaattcac 4140
   tttggaaagg accagcaaag ctcctctgga aaggtgaagg ggcagtagta atacaagata 4200
   atagtgacat aaaagtagtg ccaagaagaa aagcaaagat cattagggat tatggaaaac 4260
   agatggcagg tgatgattgt gtggcaagta gacaggatga ggattag              4307
<210> 2
   <211> 9772
   <212> DNA
   <213> Artificial Sequence
<220>
   <223> pSYNGP
<400> 2
   tcaatattgg ccattagcca tattattcat tggttatata gcataaatca atattggcta 60
   ttggccattg catacgttgt atctatatca taatatgtac atttatattg gctcatgtcc 120
   aatatgaccg ccatgttggc attgattatt gactagttat taatagtaat caattacggg 180
   gtcattagtt catagcccat atatggagtt ccgcgttaca taacttacgg taaatggccc 240
   gcctggctga ccgcccaacg acccccgccc attgacgtca ataatgacgt atgttcccat 300
   agtaacgcca atagggactt tccattgacg tcaatgggtg gagtatttac ggtaaactgc 360
   ccacttggca gtacatcaag tgtatcatat gccaagtccg ccccctattg acgtcaatga 420
   cggtaaatgg cccgcctggc attatgccca gtacatgacc ttacgggact ttcctacttg 480
   gcagtacatc tacgtattag tcatcgctat taccatggtg atgcggtttt ggcagtacac 540
   caatgggcgt ggatagcggt ttgactcacg gggatttcca agtctccacc ccattgacgt 600
   caatgggagt ttgttttggc accaaaatca acgggacttt ccaaaatgtc gtaacaactg 660
   cgatcgcccg ccccgttgac gcaaatgggc ggtaggcgtg tacggtggga ggtctatata 720
   agcagagctc gtttagtgaa ccgtcagatc actagaagct ttattgcggt agtttatcac 780
   agttaaattg ctaacgcagt cagtgcttct gacacaacag tctcgaactt aagctgcagt 840
   gactctctta aggtagcctt gcagaagttg gtcgtgaggc actgggcagg taagtatcaa 900
   ggttacaaga caggtttaag gagaccaata gaaactgggc ttgtcgagac agagaagact 960
   cttgcgtttc tgataggcac ctattggtct tactgacatc cactttgcct ttctctccac 1020
   aggtgtccac tcccagttca attacagctc ttaaggctag agtacttaat acgactcact 1080
   ataggctagc ctcgagaatt cgccaccatg ggcgcccgcg ccagcgtgct gtcgggcggc 1140
   gagctggacc gctgggagaa gatccgcctg cgccccggcg gcaaaaagaa gtacaagctg 1200
   aagcacatcg tgtgggccag ccgcgaactg gagcgcttcg ccgtgaaccc cgggctcctg 1260
   gagaccagcg aggggtgccg ccagatcctc ggccaactgc agcccagcct gcaaaccggc 1320
   agcgaggagc tgcgcagcct gtacaacacc gtggccacgc tgtactgcgt ccaccagcgc 1380
   atcgaaatca aggatacgaa agaggccctg gataaaatcg aagaggaaca gaataagagc 1440
   aaaaagaagg cccaacaggc cgccgcggac accggacaca gcaaccaggt cagccagaac 1500
   taccccatcg tgcagaacat ccaggggcag atggtgcacc aggccatctc cccccgcacg 1560
   ctgaacgcct gggtgaaggt ggtggaagag aaggctttta gcccggaggt gatacccatg 1620
   ttctcagccc tgtcagaggg agccaccccc caagatctga acaccatgct caacacagtg 1680
   gggggacacc aggccgccat gcagatgctg aaggagacca tcaatgagga ggctgccgaa 1740
   tgggatcgtg tgcatccggt gcacgcaggg cccatcgcac cgggccagat gcgtgagcca 1800
   cggggctcag acatcgccgg aacgactagt acccttcagg aacagatcgg ctggatgacc 1860
   aacaacccac ccatcccggt gggagaaatc tacaaacgct ggatcatcct gggcctgaac 1920
   aagatcgtgc gcatgtatag ccctaccagc atcctggaca tccgccaagg cccgaaggaa 1980
   ccctttcgcg actacgtgga ccggttctac aaaacgctcc gcgccgagca ggctagccag 2040
   gaggtgaaga actggatgac cgaaaccctg ctggtccaga acgcgaaccc ggactgcaag 2100
   acgatcctga aggccctggg cccagcggct accctagagg aaatgatgac cgcctgtcag 2160
   ggagtgggcg gacccggcca caaggcacgc gtcctggctg aggccatgag ccaggtgacc 2220
   aactccgcta ccatcatgat gcagcgcggc aactttcgga accaacgcaa gatcgtcaag 2280
   tgcttcaact gtggcaaaga agggcacaca gcccgcaact gcagggcccc taggaaaaag 2340
   ggctgttgga aatgtggaaa ggaaggacac caaatgaaag attgtactga gagacaggct 2400
   aattttttag ggaagatctg gccttcccac aagggaaggc cagggaattt tcttcagagc 2460
   agaccagagc caacagcccc accagaagag agcttcaggt ttggggaaga gacaacaact 2520
   ccctctcaga agcaggagcc gatagacaag gaactgtatc ctttagcttc cctcagatca 2580
   ctctttggca gcgacccctc gtcacaataa agataggggg gcagctcaag gaggctctcc 2640
   tggacaccgg agcagacgac accgtgctgg aggagatgtc gttgccaggc cgctggaagc 2700
   cgaagatgat cgggggaatc ggcggtttca tcaaggtgcg ccagtatgac cagatcctca 2760
   tcgaaatctg cggccacaag gctatcggta ccgtgctggt gggccccaca cccgtcaaca 2820
   tcatcggacg caacctgttg acgcagatcg gttgcacgct gaacttcccc attagcccta 2880
   tcgagacggt accggtgaag ctgaagcccg ggatggacgg cccgaaggtc aagcaatggc 2940
   cattgacaga ggagaagatc aaggcactgg tggagatttg cacagagatg gaaaaggaag 3000
   ggaaaatctc caagattggg cctgagaacc cgtacaacac gccggtgttc gcaatcaaga 3060
   agaaggactc gacgaaatgg cgcaagctgg tggacttccg cgagctgaac aagcgcacgc 3120
   aagacttctg ggaggttcag ctgggcatcc cgcaccccgc agggctgaag aagaagaaat 3180
   ccgtgaccgt actggatgtg ggtgatgcct acttctccgt tcccctggac gaagacttca 3240
   ggaagtacac tgccttcaca atcccttcga tcaacaacga gacaccgggg attcgatatc 3300
   agtacaacgt gctgccccag ggctggaaag gctctcccgc aatcttccag agtagcatga 3360
   ccaaaatcct ggagcctttc cgcaaacaga accccgacat cgtcatctat cagtacatgg 3420
   atgacttgta cgtgggctct gatctagaga tagggcagca ccgcaccaag atcgaggagc 3480
   tgcgccagca cctgttgagg tggggactga ccacacccga caagaagcac cagaaggagc 3540
   ctcccttcct ctggatgggt tacgagctgc accctgacaa atggaccgtg cagcctatcg 3600
   tgctgccaga gaaagacagc tggactgtca acgacataca gaagctggtg gggaagttga 3660
   actgggccag tcagatttac ccagggatta aggtgaggca gctgtgcaaa ctcctccgcg 3720
   gaaccaaggc actcacagag gtgatccccc taaccgagga ggccgagctc gaactggcag 3780
   aaaaccgaga gatcctaaag gagcccgtgc acggcgtgta ctatgacccc tccaaggacc 3840
   tgatcgccga gatccagaag caggggcaag gccagtggac ctatcagatt taccaggagc 3900
   ccttcaagaa cctgaagacc ggcaagtacg cccggatgag gggtgcccac actaacgacg 3960
   tcaagcagct gaccgaggcc gtgcagaaga tcaccaccga aagcatcgtg atctggggaa 4020
   agactcctaa gttcaagctg cccatccaga aggaaacctg ggaaacctgg tggacagagt 4080
   attggcaggc cacctggatt cctgagtggg agttcgtcaa cacccctccc ctggtgaagc 4140
   tgtggtacca gctggagaag gagcccatag tgggcgccga aaccttctac gtggatgggg 4200
   ccgctaacag ggagactaag ctgggcaaag ccggatacgt cactaaccgg ggcagacaga 4260
   aggttgtcac cctcactgac accaccaacc agaagactga gctgcaggcc atttacctcg 4320
   ctttgcagga ctcgggcctg gaggtgaaca tcgtgacaga ctctcagtat gccctgggca 4380
   tcattcaagc ccagccagac cagagtgagt ccgagctggt caatcagatc atcgagcagc 4440
   tgatcaagaa ggaaaaggtc tatctggcct gggtacccgc ccacaaaggc attggcggca 4500
   atgagcaggt cgacaagctg gtctcggctg gcatcaggaa ggtgctattc ctggatggca 4560
   tcgacaaggc ccaggacgag cacgagaaat accacagcaa ctggcgggcc atggctagcg 4620
   acttcaacct gccccctgtg gtggccaaag agatcgtggc cagctgtgac aagtgtcagc 4680
   tcaagggcga agccatgcat ggccaggtgg actgtagccc cggcatctgg caactcgatt 4740
   gcacccatct ggagggcaag gttatcctgg tagccgtcca tgtggccagt ggctacatcg 4800
   aggccgaggt cattcccgcc gaaacagggc aggagacagc ctacttcctc ctgaagctgg 4860
   caggccggtg gccagtgaag accatccata ctgacaatgg cagcaatttc accagtgcta 4920
   cggttaaggc cgcctgctgg tgggcgggaa tcaagcagga gttcgggatc ccctacaatc 4980
   cccagagtca gggcgtcgtc gagtctatga ataaggagtt aaagaagatt atcggccagg 5040
   tcagagatca ggctgagcat ctcaagaccg cggtccaaat ggcggtattc atccacaatt 5100
   tcaagcggaa gggggggatt ggggggtaca gtgcggggga gcggatcgtg gacatcatcg 5160
   cgaccgacat ccagactaag gagctgcaaa agcagattac caagattcag aatttccggg 5220
   tctactacag ggacagcaga aatcccctct ggaaaggccc agcgaagctc ctctggaagg 5280
   gtgagggggc agtagtgatc caggataata gcgacatcaa ggtggtgccc agaagaaagg 5340
   cgaagatcat tagggattat ggcaaacaga tggcgggtga tgattgcgtg gcgagcagac 5400
   aggatgagga ttaggaattg ggctagagcg gccgcttccc tttagtgagg gttaatgctt 5460
   cgagcagaca tgataagata cattgatgag tttggacaaa ccacaactag aatgcagtga 5520
   aaaaaatgct ttatttgtga aatttgtgat gctattgctt tatttgtaac cattataagc 5580
   tgcaataaac aagttaacaa caacaattgc attcatttta tgtttcaggt tcagggggag 5640
   atgtgggagg ttttttaaag caagtaaaac ctctacaaat gtggtaaaat ccgataagga 5700
   tcgatccggg ctggcgtaat agcgaagagg cccgcaccga tcgcccttcc caacagttgc 5760
   gcagcctgaa tggcgaatgg acgcgccctg tagcggcgca ttaagcgcgg cgggtgtggt 5820
   ggttacgcgc agcgtgaccg ctacacttgc cagcgcccta gcgcccgctc ctttcgcttt 5880
   cttcccttcc tttctcgcca cgttcgccgg ctttccccgt caagctctaa atcgggggct 5940
   ccctttaggg ttccgattta gagctttacg gcacctcgac cgcaaaaaac ttgatttggg 6000
   tgatggttca cgtagtgggc catcgccctg atagacggtt tttcgccctt tgacgttgga 6060
   gtccacgttc tttaatagtg gactcttgtt ccaaactgga acaacactca accctatctc 6120
   ggtctattct tttgatttat aagggatttt gccgatttcg gcctattggt taaaaaatga 6180
   gctgatttaa caaatattta acgcgaattt taacaaaata ttaacgttta caatttcgcc 6240
   tgatgcggta ttttctcctt acgcatctgt gcggtatttc acaccgcata cgcggatctg 6300
   cgcagcacca tggcctgaaa taacctctga aagaggaact tggttaggta ccttctgagg 6360
   cggaaagaac cagctgtgga atgtgtgtca gttagggtgt ggaaagtccc caggctcccc 6420
   agcaggcaga agtatgcaaa gcatgcatct caattagtca gcaaccaggt gtggaaagtc 6480
   cccaggctcc ccagcaggca gaagtatgca aagcatgcat ctcaattagt cagcaaccat 6540
   agtcccgccc ctaactccgc ccatcccgcc cctaactccg cccagttccg cccattctcc 6600
   gccccatggc tgactaattt tttttattta tgcagaggcc gaggccgcct cggcctctga 6660
   gctattccag aagtagtgag gaggcttttt tggaggccta ggcttttgca aaaagcttga 6720
   ttcttctgac acaacagtct cgaacttaag gctagagcca ccatgattga acaagatgga 6780
   ttgcacgcag gttctccggc cgcttgggtg gagaggctat tcggctatga ctgggcacaa 6840
   cagacaatcg gctgctctga tgccgccgtg ttccggctgt cagcgcaggg gcgcccggtt 6900
   ctttttgtca agaccgacct gtccggtgcc ctgaatgaac tgcaggacga ggcagcgcgg 6960
   ctatcgtggc tggccacgac gggcgttcct tgcgcagctg tgctcgacgt tgtcactgaa 7020
   gcgggaaggg actggctgct attgggcgaa gtgccggggc aggatctcct gtcatctcac 7080
   cttgctcctg ccgagaaagt atccatcatg gctgatgcaa tgcggcggct gcatacgctt 7140
   gatccggcta cctgcccatt cgaccaccaa gcgaaacatc gcatcgagcg agcacgtact 7200
   cggatggaag ccggtcttgt cgatcaggat gatctggacg aagagcatca ggggctcgcg 7260
   ccagccgaac tgttcgccag gctcaaggcg cgcatgcccg acggcgagga tctcgtcgtg 7320
   acccatggcg atgcctgctt gccgaatatc atggtggaaa atggccgctt ttctggattc 7380
   atcgactgtg gccggctggg tgtggcggac cgctatcagg acatagcgtt ggctacccgt 7440
   gatattgctg aagagcttgg cggcgaatgg gctgaccgct tcctcgtgct ttacggtatc 7500
   gccgctcccg attcgcagcg catcgccttc tatcgccttc ttgacgagtt cttctgagcg 7560
   ggactctggg gttcgaaatg accgaccaag cgacgcccaa cctgccatca cgatggccgc 7620
   aataaaatat ctttattttc attacatctg tgtgttggtt ttttgtgtga atcgatagcg 7680
   ataaggatcc gcgtatggtg cactctcagt acaatctgct ctgatgccgc atagttaagc 7740
   cagccccgac acccgccaac acccgctgac gcgccctgac gggcttgtct gctcccggca 7800
   tccgcttaca gacaagctgt gaccgtctcc gggagctgca tgtgtcagag gttttcaccg 7860
   tcatcaccga aacgcgcgag acgaaagggc ctcgtgatac gcctattttt ataggttaat 7920
   gtcatgataa taatggtttc ttagacgtca ggtggcactt ttcggggaaa tgtgcgcgga 7980
   acccctattt gtttattttt ctaaatacat tcaaatatgt atccgctcat gagacaataa 8040
   ccctgataaa tgcttcaata atattgaaaa aggaagagta tgagtattca acatttccgt 8100
   gtcgccctta ttcccttttt tgcggcattt tgccttcctg tttttgctca cccagaaacg 8160
   ctggtgaaag taaaagatgc tgaagatcag ttgggtgcac gagtgggtta catcgaactg 8220
   gatctcaaca gcggtaagat ccttgagagt tttcgccccg aagaacgttt tccaatgatg 8280
   agcactttta aagttctgct atgtggcgcg gtattatccc gtattgacgc cgggcaagag 8340
   caactcggtc gccgcataca ctattctcag aatgacttgg ttgagtactc accagtcaca 8400
   gaaaagcatc ttacggatgg catgacagta agagaattat gcagtgctgc cataaccatg 8460
   agtgataaca ctgcggccaa cttacttctg acaacgatcg gaggaccgaa ggagctaacc 8520
   gcttttttgc acaacatggg ggatcatgta actcgccttg atcgttggga accggagctg 8580
   aatgaagcca taccaaacga cgagcgtgac accacgatgc ctgtagcaat ggcaacaacg 8640
   ttgcgcaaac tattaactgg cgaactactt actctagctt cccggcaaca attaatagac 8700
   tggatggagg cggataaagt tgcaggacca cttctgcgct cggcccttcc ggctggctgg 8760
   tttattgctg ataaatctgg agccggtgag cgtgggtctc gcggtatcat tgcagcactg 8820
   gggccagatg gtaagccctc ccgtatcgta gttatctaca cgacggggag tcaggcaact 8880
   atggatgaac gaaatagaca gatcgctgag ataggtgcct cactgattaa gcattggtaa 8940
   ctgtcagacc aagtttactc atatatactt tagattgatt taaaacttca tttttaattt 9000
   aaaaggatct aggtgaagat cctttttgat aatctcatga ccaaaatccc ttaacgtgag 9060
   ttttcgttcc actgagcgtc agaccccgta gaaaagatca aaggatcttc ttgagatcct 9120
   ttttttctgc gcgtaatctg ctgcttgcaa acaaaaaaac caccgctacc agcggtggtt 9180
   tgtttgccgg atcaagagct accaactctt tttccgaagg taactggctt cagcagagcg 9240
   cagataccaa atactgtcct tctagtgtag ccgtagttag gccaccactt caagaactct 9300
   gtagcaccgc ctacatacct cgctctgcta atcctgttac cagtggctgc tgccagtggc 9360
   gataagtcgt gtcttaccgg gttggactca agacgatagt taccggataa ggcgcagcgg 9420
   tcgggctgaa cggggggttc gtgcacacag cccagcttgg agcgaacgac ctacaccgaa 9480
   ctgagatacc tacagcgtga gctatgagaa agcgccacgc ttcccgaagg gagaaaggcg 9540
   gacaggtatc cggtaagcgg cagggtcgga acaggagagc gcacgaggga gcttccaggg 9600
   ggaaacgcct ggtatcttta tagtcctgtc gggtttcgcc acctctgact tgagcgtcga 9660
   tttttgtgat gctcgtcagg ggggcggagc ctatggaaaa acgccagcaa cgcggccttt 9720
   ttacggttcc tggccttttg ctggcctttt gctcacatgg ctcgacagat ct         9772
<210> 3
   <211> 2571
   <212> DNA
   <213> Human immunodeficiency virus
<400> 3
   atgagagtga aggggatcag gaggaattat cagcactggt ggggatgggg cacgatgctc 60
   cttgggttat taatgatctg tagtgctaca gaaaaattgt gggtcacagt ctattatggg 120
   gtacctgtgt ggaaagaagc aaccaccact ctattttgtg catcagatgc taaagcatat 180
   gatacagagg tacataatgt ttgggccaca caagcctgtg tacccacaga ccccaaccca 240
   caagaagtag aattggtaaa tgtgacagaa aattttaaca tgtggaaaaa taacatggta 300
   gaacagatgc atgaggatat aatcagttta tgggatcaaa gcctaaagcc atgtgtaaaa 360
   ttaaccccac tctgtgttac tttaaattgc actgatttga ggaatactac taataccaat 420
   aatagtactg ctaataacaa tagtaatagc gagggaacaa taaagggagg agaaatgaaa 480
   aactgctctt tcaatatcac cacaagcata agagataaga tgcagaaaga atatgcactt 540
   ctttataaac ttgatatagt atcaatagat aatgatagta ccagctatag gttgataagt 600
   tgtaatacct cagtcattac acaagcttgt ccaaagatat cctttgagcc aattcccata 660
   cactattgtg ccccggctgg ttttgcgatt ctaaaatgta acgataaaaa gttcagtgga 720
   aaaggatcat gtaaaaatgt cagcacagta caatgtacac atggaattag gccagtagta 780
   tcaactcaac tgctgttaaa tggcagtcta gcagaagaag aggtagtaat tagatctgag 840
   aatttcactg ataatgctaa aaccatcata gtacatctga atgaatctgt acaaattaat 900
   tgtacaagac ccaactacaa taaaagaaaa aggatacata taggaccagg gagagcattt 960
   tatacaacaa aaaatataat aggaactata agacaagcac attgtaacat tagtagagca 1020
   aaatggaatg acactttaag acagatagtt agcaaattaa aagaacaatt taagaataaa 1080
   acaatagtct ttaatcaatc ctcaggaggg gacccagaaa ttgtaatgca cagttttaat 1140
   tgtggagggg aatttttcta ctgtaataca tcaccactgt ttaatagtac ttggaatggt 1200
   aataatactt ggaataatac tacagggtca aataacaata tcacacttca atgcaaaata 1260
   aaacaaatta taaacatgtg gcaggaagta ggaaaagcaa tgtatgcccc tcccattgaa 1320
   ggacaaatta gatgttcatc aaatattaca gggctactat taacaagaga tggtggtaag 1380
   gacacggaca cgaacgacac cgagatcttc agacctggag gaggagatat gagggacaat 1440
   tggagaagtg aattatataa atataaagta gtaacaattg aaccattagg agtagcaccc 1500
   accaaggcaa agagaagagt ggtgcagaga gaaaaaagag cagcgatagg agctctgttc 1560
   cttgggttct taggagcagc aggaagcact atgggcgcag cgtcagtgac gctgacggta 1620
   caggccagac tattattgtc tggtatagtg caacagcaga acaatttgct gagggccatt 1680
   gaggcgcaac agcatatgtt gcaactcaca gtctggggca tcaagcagct ccaggcaaga 1740
   gtcctggctg tggaaagata cctaaaggat caacagctcc tggggttttg gggttgctct 1800
   ggaaaactca tttgcaccac tactgtgcct tggaatgcta gttggagtaa taaatctctg 1860
   gatgatattt ggaataacat gacctggatg cagtgggaaa gagaaattga caattacaca 1920
   agcttaatat actcattact agaaaaatcg caaacccaac aagaaaagaa tgaacaagaa 1980
   ttattggaat tggataaatg ggcaagtttg tggaattggt ttgacataac aaattggctg 2040
   tggtatataa aaatattcat aatgatagta ggaggcttgg taggtttaag aatagttttt 2100
   gctgtacttt ctatagtgaa tagagttagg cagggatact caccattgtc gttgcagacc 2160
   cgccccccag ttccgagggg acccgacagg cccgaaggaa tcgaagaaga aggtggagag 2220
   agagacagag acacatccgg tcgattagtg catggattct tagcaattat ctgggtcgac 2280
   ctgcggagcc tgttcctctt cagctaccac cacagagact tactcttgat tgcagcgagg 2340
   attgtggaac ttctgggacg cagggggtgg gaagtcctca aatattggtg gaatctccta 2400
   cagtattgga gtcaggaact aaagagtagt gctgttagct tgcttaatgc cacagctata 2460
   gcagtagctg aggggacaga tagggttata gaagtactgc aaagagctgg tagagctatt 2520
   ctccacatac ctacaagaat aagacagggc ttggaaaggg ctttgctata a          2571
<210> 4
   <211> 2571
   <212> DNA
   <213> Artificial Sequence
<220>
   <223> SYNgp-160mn - codon optimised env sequence
<400> 4
   atgagggtga aggggatccg ccgcaactac cagcactggt ggggctgggg cacgatgctc 60
   ctggggctgc tgatgatctg cagcgccacc gagaagctgt gggtgaccgt gtactacggc 120
   gtgcccgtgt ggaaggaggc caccaccacc ctgttctgcg ccagcgacgc caaggcgtac 180
   gacaccgagg tgcacaacgt gtgggccacc caggcgtgcg tgcccaccga ccccaacccc 240
   caggaggtgg agctcgtgaa cgtgaccgag aacttcaaca tgtggaagaa caacatggtg 300
   gagcagatgc atgaggacat catcagcctg tgggaccaga gcctgaagcc ctgcgtgaag 360
   ctgacccccc tgtgcgtgac cctgaactgc accgacctga ggaacaccac caacaccaac 420
   aacagcaccg ccaacaacaa cagcaacagc gagggcacca tcaagggcgg cgagatgaag 480
   aactgcagct tcaacatcac caccagcatc cgcgacaaga tgcagaagga gtacgccctg 540
   ctgtacaagc tggatatcgt gagcatcgac aacgacagca ccagctaccg cctgatctcc 600
   tgcaacacca gcgtgatcac ccaggcctgc cccaagatca gcttcgagcc catccccatc 660
   cactactgcg cccccgccgg cttcgccatc ctgaagtgca acgacaagaa gttcagcggc 720
   aagggcagct gcaagaacgt gagcaccgtg cagtgcaccc acggcatccg gccggtggtg 780
   agcacccagc tcctgctgaa cggcagcctg gccgaggagg aggtggtgat ccgcagcgag 840
   aacttcaccg acaacgccaa gaccatcatc gtgcacctga atgagagcgt gcagatcaac 900
   tgcacgcgtc ccaactacaa caagcgcaag cgcatccaca tcggccccgg gcgcgccttc 960
   tacaccacca agaacatcat cggcaccatc cgccaggccc actgcaacat ctctagagcc 1020
   aagtggaacg acaccctgcg ccagatcgtg agcaagctga aggagcagtt caagaacaag 1080
   accatcgtgt tcaaccagag cagcggcggc gaccccgaga tcgtgatgca cagcttcaac 1140
   tgcggcggcg aattcttcta ctgcaacacc agccccctgt tcaacagcac ctggaacggc 1200
   aacaacacct ggaacaacac caccggcagc aacaacaata ttaccctcca gtgcaagatc 1260
   aagcagatca tcaacatgtg gcaggaggtg ggcaaggcca tgtacgcccc ccccatcgag 1320
   ggccagatcc ggtgcagcag caacatcacc ggtctgctgc tgacccgcga cggcggcaag 1380
   gacaccgaca ccaacgacac cgaaatcttc cgccccggcg gcggcgacat gcgcgacaac 1440
   tggagatctg agctgtacaa gtacaaggtg gtgacgatcg agcccctggg cgtggccccc 1500
   accaaggcca agcgccgcgt ggtgcagcgc gagaagcggg ccgccatcgg cgccctgttc 1560
   ctgggcttcc tgggggcggc gggcagcacc atgggggccg ccagcgtgac cctgaccgtg 1620
   caggcccgcc tgctcctgag cggcatcgtg cagcagcaga acaacctcct ccgcgccatc 1680
   gaggcccagc agcatatgct ccagctcacc gtgtggggca tcaagcagct ccaggcccgc 1740
   gtgctggccg tggagcgcta cctgaaggac cagcagctcc tgggcttctg gggctgctcc 1800
   ggcaagctga tctgcaccac cacggtaccc tggaacgcct cctggagcaa caagagcctg 1860
   gacgacatct ggaacaacat gacctggatg cagtgggagc gcgagatcga taactacacc 1920
   agcctgatct acagcctgct ggagaagagc cagacccagc aggagaagaa cgagcaggag 1980
   ctgctggagc tggacaagtg ggcgagcctg tggaactggt tcgacatcac caactggctg 2040
   tggtacatca aaatcttcat catgattgtg ggcggcctgg tgggcctccg catcgtgttc 2100
   gccgtgctga gcatcgtgaa ccgcgtgcgc cagggctaca gccccctgag cctccagacc 2160
   cggccccccg tgccgcgcgg gcccgaccgc cccgagggca tcgaggagga gggcggcgag 2220
   cgcgaccgcg acaccagcgg caggctcgtg cacggcttcc tggcgatcat ctgggtcgac 2280
   ctccgcagcc tgttcctgtt cagctaccac caccgcgacc tgctgctgat cgccgcccgc 2340
   atcgtggaac tcctaggccg ccgcggctgg gaggtgctga agtactggtg gaacctcctc 2400
   cagtattgga gccaggagct gaagtccagc gccgtgagcc tgctgaacgc caccgccatc 2460
   gccgtggccg agggcaccga ccgcgtgatc gaggtgctcc agagggccgg gagggcgatc 2520
   ctgcacatcc ccacccgcat ccgccagggg ctcgagaggg cgctgctgta a          2571
<210> 5
   <211> 10112
   <212> DNA
   <213> Artificial Sequence
<220>
   <223> pESYNGP
<400> 5
   tcaatattgg ccattagcca tattattcat tggttatata gcataaatca atattggcta 60
   ttggccattg catacgttgt atctatatca taatatgtac atttatattg gctcatgtcc 120
   aatatgaccg ccatgttggc attgattatt gactagttat taatagtaat caattacggg 180
   gtcattagtt catagcccat atatggagtt ccgcgttaca taacttacgg taaatggccc 240
   gcctggctga ccgcccaacg acccccgccc attgacgtca ataatgacgt atgttcccat 300
   agtaacgcca atagggactt tccattgacg tcaatgggtg gagtatttac ggtaaactgc 360
   ccacttggca gtacatcaag tgtatcatat gccaagtccg ccccctattg acgtcaatga 420
   cggtaaatgg cccgcctggc attatgccca gtacatgacc ttacgggact ttcctacttg 480
   gcagtacatc tacgtattag tcatcgctat taccatggtg atgcggtttt ggcagtacac 540
   caatgggcgt ggatagcggt ttgactcacg gggatttcca agtctccacc ccattgacgt 600
   caatgggagt ttgttttggc accaaaatca acgggacttt ccaaaatgtc gtaacaactg 660
   cgatcgcccg ccccgttgac gcaaatgggc ggtaggcgtg tacggtggga ggtctatata 720
   agcagagctc gtttagtgaa ccgtcagatc actagaagct ttattgcggt agtttatcac 780
   agttaaattg ctaacgcagt cagtgcttct gacacaacag tctcgaactt aagctgcagt 840
   gactctctta aggtagcctt gcagaagttg gtcgtgaggc actgggcagg taagtatcaa 900
   ggttacaaga caggtttaag gagaccaata gaaactgggc ttgtcgagac agagaagact 960
   cttgcgtttc tgataggcac ctattggtct tactgacatc cactttgcct ttctctccac 1020
   aggtgtccac tcccagttca attacagctc ttaaggctag agtacttaat acgactcact 1080
   ataggctaga gaattcgcca ccatgggcga tcccctcacc tggtccaaag ccctgaagaa 1140
   actggaaaaa gtcaccgttc agggtagcca aaagcttacc acaggcaatt gcaactgggc 1200
   attgtccctg gtggatcttt tccacgacac taatttcgtt aaggagaaag attggcaact 1260
   cagagacgtg atccccctct tggaggacgt gacccaaaca ttgtctgggc aggagcgcga 1320
   agctttcgag cgcacctggt gggccatcag cgcagtcaaa atggggctgc aaatcaacaa 1380
   cgtggttgac ggtaaagcta gctttcaact gctccgcgct aagtacgaga agaaaaccgc 1440
   caacaagaaa caatccgaac ctagcgagga gtacccaatt atgatcgacg gcgccggcaa 1500
   taggaacttc cgcccactga ctcccagggg ctataccacc tgggtcaaca ccatccagac 1560
   aaacggactt ttgaacgaag cctcccagaa cctgttcggc atcctgtctg tggactgcac 1620
   ctccgaagaa atgaatgctt ttctcgacgt ggtgccagga caggctggac agaaacagat 1680
   cctgctcgat gccattgaca agatcgccga cgactgggat aatcgccacc ccctgccaaa 1740
   cgcccctctg gtggctcccc cacaggggcc tatccctatg accgctaggt tcattagggg 1800
   actgggggtg ccccgcgaac gccagatgga gccagcattt gaccaattta ggcagaccta 1860
   cagacagtgg atcatcgaag ccatgagcga ggggattaaa gtcatgatcg gaaagcccaa 1920
   ggcacagaac atcaggcagg gggccaagga accataccct gagtttgtcg acaggcttct 1980
   gtcccagatt aaatccgaag gccaccctca ggagatctcc aagttcttga cagacacact 2040
   gactatccaa aatgcaaatg aagagtgcag aaacgccatg aggcacctca gacctgaaga 2100
   taccctggag gagaaaatgt acgcatgtcg cgacattggc actaccaagc aaaagatgat 2160
   gctgctcgcc aaggctctgc aaaccggcct ggctggtcca ttcaaaggag gagcactgaa 2220
   gggaggtcca ttgaaagctg cacaaacatg ttataattgt gggaagccag gacatttatc 2280
   tagtcaatgt agagcaccta aagtctgttt taaatgtaaa cagcctggac atttctcaaa 2340
   gcaatgcaga agtgttccaa aaaacgggaa gcaaggggct caagggaggc cccagaaaca 2400
   aactttcccg atacaacaga agagtcagca caacaaatct gttgtacaag agactcctca 2460
   gactcaaaat ctgtacccag atctgagcga aataaaaaag gaatacaatg tcaaggagaa 2520
   ggatcaagta gaggatctca acctggacag tttgtgggag taacatacaa tctcgagaag 2580
   aggcccacta ccatcgtcct gatcaatgac acccctctta atgtgctgct ggacaccgga 2640
   gccgacacca gcgttctcac tactgctcac tataacagac tgaaatacag aggaaggaaa 2700
   taccagggca caggcatcat cggcgttgga ggcaacgtcg aaaccttttc cactcctgtc 2760
   accatcaaaa agaaggggag acacattaaa accagaatgc tggtcgccga catccccgtc 2820
   accatccttg gcagagacat tctccaggac ctgggcgcta aactcgtgct ggcacaactg 2880
   tctaaggaaa tcaagttccg caagatcgag ctgaaagagg gcacaatggg tccaaaaatc 2940
   ccccagtggc ccctgaccaa agagaagctt gagggcgcta aggaaatcgt gcagcgcctg 3000
   ctttctgagg gcaagattag cgaggccagc gacaataacc cttacaacag ccccatcttt 3060
   gtgattaaga aaaggagcgg caaatggaga ctcctgcagg acctgaggga actcaacaag 3120
   accgtccagg tcggaactga gatctctcgc ggactgcctc accccggcgg cctgattaaa 3180
   tgcaagcaca tgacagtcct tgacattgga gacgcttatt ttaccatccc cctcgatcct 3240
   gaatttcgcc cctatactgc ttttaccatc cccagcatca atcaccagga gcccgataaa 3300
   cgctatgtgt ggaagtgcct cccccaggga tttgtgctta gcccctacat ttaccagaag 3360
   acacttcaag agatcctcca acctttccgc gaaagatacc cagaggttca actctaccaa 3420
   tatatggacg acctgttcat ggggtccaac gggtctaaga agcagcacaa ggaactcatc 3480
   atcgaactga gggcaatcct cctggagaaa ggcttcgaga cacccgacga caagctgcaa 3540
   gaagttcctc catatagctg gctgggctac cagctttgcc ctgaaaactg gaaagtccag 3600
   aagatgcagt tggatatggt caagaaccca acactgaacg acgtccagaa gctcatgggc 3660
   aatattacct ggatgagctc cggaatccct gggcttaccg ttaagcacat tgccgcaact 3720
   acaaaaggat gcctggagtt gaaccagaag gtcatttgga cagaggaagc tcagaaggaa 3780
   ctggaggaga ataatgaaaa gattaagaat gctcaagggc tccaatacta caatcccgaa 3840
   gaagaaatgt tgtgcgaggt cgaaatcact aagaactacg aagccaccta tgtcatcaaa 3900
   cagtcccaag gcatcttgtg ggccggaaag aaaatcatga aggccaacaa aggctggtcc 3960
   accgttaaaa atctgatgct cctgctccag cacgtcgcca ccgagtctat cacccgcgtc 4020
   ggcaagtgcc ccaccttcaa agttcccttc actaaggagc aggtgatgtg ggagatgcaa 4080
   aaaggctggt actactcttg gcttcccgag atcgtctaca cccaccaagt ggtgcacgac 4140
   gactggagaa tgaagcttgt cgaggagccc actagcggaa ttacaatcta taccgacggc 4200
   ggaaagcaaa acggagaggg aatcgctgca tacgtcacat ctaacggccg caccaagcaa 4260
   aagaggctcg gccctgtcac tcaccaggtg gctgagagga tggctatcca gatggccctt 4320
   gaggacacta gagacaagca ggtgaacatt gtgactgaca gctactactg ctggaaaaac 4380
   atcacagagg gccttggcct ggagggaccc cagtctccct ggtggcctat catccagaat 4440
   atccgcgaaa aggaaattgt ctatttcgcc tgggtgcctg gacacaaagg aatttacggc 4500
   aaccaactcg ccgatgaagc cgccaaaatt aaagaggaaa tcatgcttgc ctaccagggc 4560
   acacagatta aggagaagag agacgaggac gctggctttg acctgtgtgt gccatacgac 4620
   atcatgattc ccgttagcga cacaaagatc attccaaccg atgtcaagat ccaggtgcca 4680
   cccaattcat ttggttgggt gaccggaaag tccagcatgg ctaagcaggg tcttctgatt 4740
   aacgggggaa tcattgatga aggatacacc ggcgaaatcc aggtgatctg cacaaatatc 4800
   ggcaaaagca atattaagct tatcgaaggg cagaagttcg ctcaactcat catcctccag 4860
   caccacagca attcaagaca accttgggac gaaaacaaga ttagccagag aggtgacaag 4920
   ggcttcggca gcacaggtgt gttctgggtg gagaacatcc aggaagcaca ggacgagcac 4980
   gagaattggc acacctcccc taagattttg gcccgcaatt acaagatccc actgactgtg 5040
   gctaagcaga tcacacagga atgcccccac tgcaccaaac aaggttctgg ccccgccggc 5100
   tgcgtgatga ggtcccccaa tcactggcag gcagattgca cccacctcga caacaaaatt 5160
   atcctgacct tcgtggagag caattccggc tacatccacg caacactcct ctccaaggaa 5220
   aatgcattgt gcacctccct cgcaattctg gaatgggcca ggctgttctc tccaaaatcc 5280
   ctgcacaccg acaacggcac caactttgtg gctgaacctg tggtgaatct gctgaagttc 5340
   ctgaaaatcg cccacaccac tggcattccc tatcaccctg aaagccaggg cattgtcgag 5400
   agggccaaca gaactctgaa agaaaagatc caatctcaca gagacaatac acagacattg 5460
   gaggccgcac ttcagctcgc ccttatcacc tgcaacaaag gaagagaaag catgggcggc 5520
   cagaccccct gggaggtctt catcactaac caggcccagg tcatccatga aaagctgctc 5580
   ttgcagcagg cccagtcctc caaaaagttc tgcttttata agatccccgg tgagcacgac 5640
   tggaaaggtc ctacaagagt tttgtggaaa ggagacggcg cagttgtggt gaacgatgag 5700
   ggcaagggga tcatcgctgt gcccctgaca cgcaccaagc ttctcatcaa gccaaactga 5760
   acccggggcg gccgcttccc tttagtgagg gttaatgctt cgagcagaca tgataagata 5820
   cattgatgag tttggacaaa ccacaactag aatgcagtga aaaaaatgct ttatttgtga 5880
   aatttgtgat gctattgctt tatttgtaac cattataagc tgcaataaac aagttaacaa 5940
   caacaattgc attcatttta tgtttcaggt tcagggggag atgtgggagg ttttttaaag 6000
   caagtaaaac ctctacaaat gtggtaaaat ccgataagga tcgatccggg ctggcgtaat 6060
   agcgaagagg cccgcaccga tcgcccttcc caacagttgc gcagcctgaa tggcgaatgg 6120
   acgcgccctg tagcggcgca ttaagcgcgg cgggtgtggt ggttacgcgc agcgtgaccg 6180
   ctacacttgc cagcgcccta gcgcccgctc ctttcgcttt cttcccttcc tttctcgcca 6240
   cgttcgccgg ctttccccgt caagctctaa atcgggggct ccctttaggg ttccgattta 6300
   gagctttacg gcacctcgac cgcaaaaaac ttgatttggg tgatggttca cgtagtgggc 6360
   catcgccctg atagacggtt tttcgccctt tgacgttgga gtccacgttc tttaatagtg 6420
   gactcttgtt ccaaactgga acaacactca accctatctc ggtctattct tttgatttat 6480
   aagggatttt gccgatttcg gcctattggt taaaaaatga gctgatttaa caaatattta 6540
   acgcgaattt taacaaaata ttaacgttta caatttcgcc tgatgcggta ttttctcctt 6600
   acgcatctgt gcggtatttc acaccgcata cgcggatctg cgcagcacca tggcctgaaa 6660
   taacctctga aagaggaact tggttaggta ccttctgagg cggaaagaac cagctgtgga 6720
   atgtgtgtca gttagggtgt ggaaagtccc caggctcccc agcaggcaga agtatgcaaa 6780
   gcatgcatct caattagtca gcaaccaggt gtggaaagtc cccaggctcc ccagcaggca 6840
   gaagtatgca aagcatgcat ctcaattagt cagcaaccat agtcccgccc ctaactccgc 6900
   ccatcccgcc cctaactccg cccagttccg cccattctcc gccccatggc tgactaattt 6960
   ttttatttta tgcagaggcc gaggccgcct cggcctctga gctattccag aagtagtgag 7020
   gaggcttttt tggaggccta ggcttttgca aaaagcttga ttcttctgac acaacagtct 7080
   cgaacttaag gctagagcca ccatgattga acaagatgga ttgcacgcag gttctccggc 7140
   cgcttgggtg gagaggctat tcggctatga ctgggcacaa cagacaatcg gctgctctga 7200
   tgccgccgtg ttccggctgt cagcgcaggg gcgcccggtt ctttttgtca agaccgacct 7260
   gtccggtgcc ctgaatgaac tgcaggacga ggcagcgcgg ctatcgtggc tggccacgac 7320
   gggcgttcct tgcgcagctg tgctcgacgt tgtcactgaa gcgggaaggg actggctgct 7380
   attgggcgaa gtgccggggc aggatctcct gtcatctcac cttgctcctg ccgagaaagt 7440
   atccatcatg gctgatgcaa tgcggcggct gcatacgctt gatccggcta cctgcccatt 7500
   cgaccaccaa gcgaaacatc gcatcgagcg agcacgtact cggatggaag ccggtcttgt 7560
   cgatcaggat gatctggacg aagagcatca ggggctcgcg ccagccgaac tgttcgccag 7620
   gctcaaggcg cgcatgcccg acggcgagga tctcgtcgtg acccatggcg atgcctgctt 7680
   gccgaatatc atggtggaaa atggccgctt ttctggattc atcgactgtg gccggctggg 7740
   tgtggcggac cgctatcagg acatagcgtt ggctacccgt gatattgctg aagagcttgg 7800
   cggcgaatgg gctgaccgct tcctcgtgct ttacggtatc gccgctcccg attcgcagcg 7860
   catcgccttc tatcgccttc ttgacgagtt cttctgagcg ggactctggg gttcgaaatg 7920
   accgaccaag cgacgcccaa cctgccatca cgatggccgc aataaaatat ctttattttc 7980
   attacatctg tgtgttggtt ttttgtgtga atcgatagcg ataaggatcc gcgtatggtg 8040
   cactctcagt acaatctgct ctgatgccgc atagttaagc cagccccgac acccgccaac 8100
   acccgctgac gcgccctgac gggcttgtct gctcccggca tccgcttaca gacaagctgt 8160
   gaccgtctcc gggagctgca tgtgtcagag gttttcaccg tcatcaccga aacgcgcgag 8220
   acgaaagggc ctcgtgatac gcctattttt ataggttaat gtcatgataa taatggtttc 8280
   ttagacgtca ggtggcactt ttcggggaaa tgtgcgcgga acccctattt gtttattttt 8340
   ctaaatacat tcaaatatgt atccgctcat gagacaataa ccctgataaa tgcttcaata 8400
   atattgaaaa aggaagagta tgagtattca acatttccgt gtcgccctta ttcccttttt 8460
   tgcggcattt tgccttcctg tttttgctca cccagaaacg ctggtgaaag taaaagatgc 8520
   tgaagatcag ttgggtgcac gagtgggtta catcgaactg gatctcaaca gcggtaagat 8580
   ccttgagagt tttcgccccg aagaacgttt tccaatgatg agcactttta aagttctgct 8640
   atgtggcgcg gtattatccc gtattgacgc cgggcaagag caactcggtc gccgcataca 8700
   ctattctcag aatgacttgg ttgagtactc accagtcaca gaaaagcatc ttacggatgg 8760
   catgacagta agagaattat gcagtgctgc cataaccatg agtgataaca ctgcggccaa 8820
   cttacttctg acaacgatcg gaggaccgaa ggagctaacc gcttttttgc acaacatggg 8880
   ggatcatgta actcgccttg atcgttggga accggagctg aatgaagcca taccaaacga 8940
   cgagcgtgac accacgatgc ctgtagcaat ggcaacaacg ttgcgcaaac tattaactgg 9000
   cgaactactt actctagctt cccggcaaca attaatagac tggatggagg cggataaagt 9060
   tgcaggacca cttctgcgct cggcccttcc ggctggctgg tttattgctg ataaatctgg 9120
   agccggtgag cgtgggtctc gcggtatcat tgcagcactg gggccagatg gtaagccctc 9180
   ccgtatcgta gttatctaca cgacggggag tcaggcaact atggatgaac gaaatagaca 9240
   gatcgctgag ataggtgcct cactgattaa gcattggtaa ctgtcagacc aagtttactc 9300
   atatatactt tagattgatt taaaacttca tttttaattt aaaaggatct aggtgaagat 9360
   cctttttgat aatctcatga ccaaaatccc ttaacgtgag ttttcgttcc actgagcgtc 9420
   agaccccgta gaaaagatca aaggatcttc ttgagatcct ttttttctgc gcgtaatctg 9480
   ctgcttgcaa acaaaaaaac caccgctacc agcggtggtt tgtttgccgg atcaagagct 9540
   accaactctt tttccgaagg taactggctt cagcagagcg cagataccaa atactgtcct 9600
   tctagtgtag ccgtagttag gccaccactt caagaactct gtagcaccgc ctacatacct 9660
   cgctctgcta atcctgttac cagtggctgc tgccagtggc gataagtcgt gtcttaccgg 9720
   gttggactca agacgatagt taccggataa ggcgcagcgg tcgggctgaa cggggggttc 9780
   gtgcacacag cccagcttgg agcgaacgac ctacaccgaa ctgagatacc tacagcgtga 9840
   gctatgagaa agcgccacgc ttcccgaagg gagaaaggcg gacaggtatc cggtaagcgg 9900
   cagggtcgga acaggagagc gcacgaggga gcttccaggg ggaaacgcct ggtatcttta 9960
   tagtcctgtc gggtttcgcc acctctgact tgagcgtcga tttttgtgat gctcgtcagg 10020
   ggggcggagc ctatggaaaa acgccagcaa cgcggccttt ttacggttcc tggccttttg 10080
   ctggcctttt gctcacatgg ctcgacagat ct                               10112
<210> 6
   <211> 10227
   <212> DNA
   <213> Artificial Sequence
<220>
   <223> LpESYNGP
<400> 6
<210> 7
   <211> 10815
   <212> DNA
   <213> Artificial Sequence
<220>
   <223> pESYNGPRRE
<400> 7
   tcaatattgg ccattagcca tattattcat tggttatata gcataaatca atattggcta 60
   ttggccattg catacgttgt atctatatca taatatgtac atttatattg gctcatgtcc 120
   aatatgaccg ccatgttggc attgattatt gactagttat taatagtaat caattacggg 180
   gtcattagtt catagcccat atatggagtt ccgcgttaca taacttacgg taaatggccc 240
   gcctggctga ccgcccaacg acccccgccc attgacgtca ataatgacgt atgttcccat 300
   agtaacgcca atagggactt tccattgacg tcaatgggtg gagtatttac ggtaaactgc 360
   ccacttggca gtacatcaag tgtatcatat gccaagtccg ccccctattg acgtcaatga 420
   cggtaaatgg cccgcctggc attatgccca gtacatgacc ttacgggact ttcctacttg 480
   gcagtacatc tacgtattag tcatcgctat taccatggtg atgcggtttt ggcagtacac 540
   caatgggcgt ggatagcggt ttgactcacg gggatttcca agtctccacc ccattgacgt 600
   caatgggagt ttgttttggc accaaaatca acgggacttt ccaaaatgtc gtaacaactg 660
   cgatcgcccg ccccgttgac gcaaatgggc ggtaggcgtg tacggtggga ggtctatata 720
   agcagagctc gtttagtgaa ccgtcagatc actagaagct ttattgcggt agtttatcac 780
   agttaaattg ctaacgcagt cagtgcttct gacacaacag tctcgaactt aagctgcagt 840
   gactctctta aggtagcctt gcagaagttg gtcgtgaggc actgggcagg taagtatcaa 900
   ggttacaaga caggtttaag gagaccaata gaaactgggc ttgtcgagac agagaagact 960
   cttgcgtttc tgataggcac ctattggtct tactgacatc cactttgcct ttctctccac 1020
   aggtgtccac tcccagttca attacagctc ttaaggctag agtacttaat acgactcact 1080
   ataggctaga gaattcgcca ccatgggcga tcccctcacc tggtccaaag ccctgaagaa 1140
   actggaaaaa gtcaccgttc agggtagcca aaagcttacc acaggcaatt gcaactgggc 1200
   attgtccctg gtggatcttt tccacgacac taatttcgtt aaggagaaag attggcaact 1260
   cagagacgtg atccccctct tggaggacgt gacccaaaca ttgtctgggc aggagcgcga 1320
   agctttcgag cgcacctggt gggccatcag cgcagtcaaa atggggctgc aaatcaacaa 1380
   cgtggttgac ggtaaagcta gctttcaact gctccgcgct aagtacgaga agaaaaccgc 1440
   caacaagaaa caatccgaac ctagcgagga gtacccaatt atgatcgacg gcgccggcaa 1500
   taggaacttc cgcccactga ctcccagggg ctataccacc tgggtcaaca ccatccagac 1560
   aaacggactt ttgaacgaag cctcccagaa cctgttcggc atcctgtctg tggactgcac 1620
   ctccgaagaa atgaatgctt ttctcgacgt ggtgccagga caggctggac agaaacagat 1680
   cctgctcgat gccattgaca agatcgccga cgactgggat aatcgccacc ccctgccaaa 1740
   cgcccctctg gtggctcccc cacaggggcc tatccctatg accgctaggt tcattagggg 1800
   actgggggtg ccccgcgaac gccagatgga gccagcattt gaccaattta ggcagaccta 1860
   cagacagtgg atcatcgaag ccatgagcga ggggattaaa gtcatgatcg gaaagcccaa 1920
   ggcacagaac atcaggcagg gggccaagga accataccct gagtttgtcg acaggcttct 1980
   gtcccagatt aaatccgaag gccaccctca ggagatctcc aagttcttga cagacacact 2040
   gactatccaa aatgcaaatg aagagtgcag aaacgccatg aggcacctca gacctgaaga 2100
   taccctggag gagaaaatgt acgcatgtcg cgacattggc actaccaagc aaaagatgat 2160
   gctgctcgcc aaggctctgc aaaccggcct ggctggtcca ttcaaaggag gagcactgaa 2220
   gggaggtcca ttgaaagctg cacaaacatg ttataattgt gggaagccag gacatttatc 2280
   tagtcaatgt agagcaccta aagtctgttt taaatgtaaa cagcctggac atttctcaaa 2340
   gcaatgcaga agtgttccaa aaaacgggaa gcaaggggct caagggaggc cccagaaaca 2400
   aactttcccg atacaacaga agagtcagca caacaaatct gttgtacaag agactcctca 2460
   gactcaaaat ctgtacccag atctgagcga aataaaaaag gaatacaatg tcaaggagaa 2520
   ggatcaagta gaggatctca acctggacag tttgtgggag taacatacaa tctcgagaag 2580
   aggcccacta ccatcgtcct gatcaatgac acccctctta atgtgctgct ggacaccgga 2640
   gccgacacca gcgttctcac tactgctcac tataacagac tgaaatacag aggaaggaaa 2700
   taccagggca caggcatcat cggcgttgga ggcaacgtcg aaaccttttc cactcctgtc 2760
   accatcaaaa agaaggggag acacattaaa accagaatgc tggtcgccga catccccgtc 2820
   accatccttg gcagagacat tctccaggac ctgggcgcta aactcgtgct ggcacaactg 2880
   tctaaggaaa tcaagttccg caagatcgag ctgaaagagg gcacaatggg tccaaaaatc 2940
   ccccagtggc ccctgaccaa agagaagctt gagggcgcta aggaaatcgt gcagcgcctg 3000
   ctttctgagg gcaagattag cgaggccagc gacaataacc cttacaacag ccccatcttt 3060
   gtgattaaga aaaggagcgg caaatggaga ctcctgcagg acctgaggga actcaacaag 3120
   accgtccagg tcggaactga gatctctcgc ggactgcctc accccggcgg cctgattaaa 3180
   tgcaagcaca tgacagtcct tgacattgga gacgcttatt ttaccatccc cctcgatcct 3240
   gaatttcgcc cctatactgc ttttaccatc cccagcatca atcaccagga gcccgataaa 3300
   cgctatgtgt ggaagtgcct cccccaggga tttgtgctta gcccctacat ttaccagaag 3360
   acacttcaag agatcctcca acctttccgc gaaagatacc cagaggttca actctaccaa 3420
   tatatggacg acctgttcat ggggtccaac gggtctaaga agcagcacaa ggaactcatc 3480
   atcgaactga gggcaatcct cctggagaaa ggcttcgaga cacccgacga caagctgcaa 3540
   gaagttcctc catatagctg gctgggctac cagctttgcc ctgaaaactg gaaagtccag 3600
   aagatgcagt tggatatggt caagaaccca acactgaacg acgtccagaa gctcatgggc 3660
   aatattacct ggatgagctc cggaatccct gggcttaccg ttaagcacat tgccgcaact 3720
   acaaaaggat gcctggagtt gaaccagaag gtcatttgga cagaggaagc tcagaaggaa 3780
   ctggaggaga ataatgaaaa gattaagaat gctcaagggc tccaatacta caatcccgaa 3840
   gaagaaatgt tgtgcgaggt cgaaatcact aagaactacg aagccaccta tgtcatcaaa 3900
   cagtcccaag gcatcttgtg ggccggaaag aaaatcatga aggccaacaa aggctggtcc 3960
   accgttaaaa atctgatgct cctgctccag cacgtcgcca ccgagtctat cacccgcgtc 4020
   ggcaagtgcc ccaccttcaa agttcccttc actaaggagc aggtgatgtg ggagatgcaa 4080
   aaaggctggt actactcttg gcttcccgag atcgtctaca cccaccaagt ggtgcacgac 4140
   gactggagaa tgaagcttgt cgaggagccc actagcggaa ttacaatcta taccgacggc 4200
   ggaaagcaaa acggagaggg aatcgctgca tacgtcacat ctaacggccg caccaagcaa 4260
   aagaggctcg gccctgtcac tcaccaggtg gctgagagga tggctatcca gatggccctt 4320
   gaggacacta gagacaagca ggtgaacatt gtgactgaca gctactactg ctggaaaaac 4380
   atcacagagg gccttggcct ggagggaccc cagtctccct ggtggcctat catccagaat 4440
   atccgcgaaa aggaaattgt ctatttcgcc tgggtgcctg gacacaaagg aatttacggc 4500
   aaccaactcg ccgatgaagc cgccaaaatt aaagaggaaa tcatgcttgc ctaccagggc 4560
   acacagatta aggagaagag agacgaggac gctggctttg acctgtgtgt gccatacgac 4620
   atcatgattc ccgttagcga cacaaagatc attccaaccg atgtcaagat ccaggtgcca 4680
   cccaattcat ttggttgggt gaccggaaag tccagcatgg ctaagcaggg tcttctgatt 4740
   aacgggggaa tcattgatga aggatacacc ggcgaaatcc aggtgatctg cacaaatatc 4800
   ggcaaaagca atattaagct tatcgaaggg cagaagttcg ctcaactcat catcctccag 4860
   caccacagca attcaagaca accttgggac gaaaacaaga ttagccagag aggtgacaag 4920
   ggcttcggca gcacaggtgt gttctgggtg gagaacatcc aggaagcaca ggacgagcac 4980
   gagaattggc acacctcccc taagattttg gcccgcaatt acaagatccc actgactgtg 5040
   gctaagcaga tcacacagga atgcccccac tgcaccaaac aaggttctgg ccccgccggc 5100
   tgcgtgatga ggtcccccaa tcactggcag gcagattgca cccacctcga caacaaaatt 5160
   atcctgacct tcgtggagag caattccggc tacatccacg caacactcct ctccaaggaa 5220
   aatgcattgt gcacctccct cgcaattctg gaatgggcca ggctgttctc tccaaaatcc 5280
   ctgcacaccg acaacggcac caactttgtg gctgaacctg tggtgaatct gctgaagttc 5340
   ctgaaaatcg cccacaccac tggcattccc tatcaccctg aaagccaggg cattgtcgag 5400
   agggccaaca gaactctgaa agaaaagatc caatctcaca gagacaatac acagacattg 5460
   gaggccgcac ttcagctcgc ccttatcacc tgcaacaaag gaagagaaag catgggcggc 5520
   cagaccccct gggaggtctt catcactaac caggcccagg tcatccatga aaagctgctc 5580
   ttgcagcagg cccagtcctc caaaaagttc tgcttttata agatccccgg tgagcacgac 5640
   tggaaaggtc ctacaagagt tttgtggaaa ggagacggcg cagttgtggt gaacgatgag 5700
   ggcaagggga tcatcgctgt gcccctgaca cgcaccaagc ttctcatcaa gccaaactga 5760
   acccgacgaa tcccaggggg aatctcaacc cctattaccc aacagtcaga aaaatctaag 5820
   tgtgaggaga acacaatgtt tcaaccttat tgttataata atgacagtaa gaacagcatg 5880
   gcagaatcga aggaagcaag agaccaagaa atgaacctga aagaagaatc taaagaagaa 5940
   aaaagaagaa atgactggtg gaaaataggt atgtttctgt tatgcttagc cagggccctc 6000
   tggaaggtga ccagtggtgc agggtcctcc ggcagtcgtt acctgaagaa aaaattccat 6060
   cacaaacatg catcgcgaga agacacctgg gaccaggccc aacacaacat acacctagca 6120
   ggcgtgaccg gtggatcagg ggacaaatac tacaagcaga agtactccag gaacgactgg 6180
   aatggagaat cagaggagta caacaggcgg ccaaagagct gggtgaagtc aatcgaggca 6240
   tttggagaga gctatatttc cgagaagacc aaaggggaga tttctcagcc tggggcggct 6300
   atcaacgagc acaagaacgg ctctgggggg aacaatcctc accaagggtc cttagacctg 6360
   gagattcgaa gcgaaggagg aaacatttat gactgttgca ttaaagccca agaaggaact 6420
   ctcgctatcc cttgctgtgg atttccctta tggctatttt gggggtcggg gcggccgctt 6480
   ccctttagtg agggttaatg cttcgagcag acatgataag atacattgat gagtttggac 6540
   aaaccacaac tagaatgcag tgaaaaaaat gctttatttg tgaaatttgt gatgctattg 6600
   ctttatttgt aaccattata agctgcaata aacaagttaa caacaacaat tgcattcatt 6660
   ttatgtttca ggttcagggg gagatgtggg aggtttttta aagcaagtaa aacctctaca 6720
   aatgtggtaa aatccgataa ggatcgatcc gggctggcgt aatagcgaag aggcccgcac 6780
   cgatcgccct tcccaacagt tgcgcagcct gaatggcgaa tggacgcgcc ctgtagcggc 6840
   gcattaagcg cggcgggtgt ggtggttacg cgcagcgtga ccgctacact tgccagcgcc 6900
   ctagcgcccg ctcctttcgc tttcttccct tcctttctcg ccacgttcgc cggctttccc 6960
   cgtcaagctc taaatcgggg gctcccttta gggttccgat ttagagcttt acggcacctc 7020
   gaccgcaaaa aacttgattt gggtgatggt tcacgtagtg ggccatcgcc ctgatagacg 7080
   gtttttcgcc ctttgacgtt ggagtccacg ttctttaata gtggactctt gttccaaact 7140
   ggaacaacac tcaaccctat ctcggtctat tcttttgatt tataagggat tttgccgatt 7200
   tcggcctatt ggttaaaaaa tgagctgatt taacaaatat ttaacgcgaa ttttaacaaa 7260
   atattaacgt ttacaatttc gcctgatgcg gtattttctc cttacgcatc tgtgcggtat 7320
   ttcacaccgc atacgcggat ctgcgcagca ccatggcctg aaataacctc tgaaagagga 7380
   acttggttag gtaccttctg aggcggaaag aaccagctgt ggaatgtgtg tcagttaggg 7440
   tgtggaaagt ccccaggctc cccagcaggc agaagtatgc aaagcatgca tctcaattag 7500
   tcagcaacca ggtgtggaaa gtccccaggc tccccagcag gcagaagtat gcaaagcatg 7560
   catctcaatt agtcagcaac catagtcccg cccctaactc cgcccatccc gcccctaact 7620
   ccgcccagtt ccgcccattc tccgccccat ggctgactaa ttttttttat ttatgcagag 7680
   gccgaggccg cctcggcctc tgagctattc cagaagtagt gaggaggctt ttttggaggc 7740
   ctaggctttt gcaaaaagct tgattcttct gacacaacag tctcgaactt aaggctagag 7800
   ccaccatgat tgaacaagat ggattgcacg caggttctcc ggccgcttgg gtggagaggc 7860
   tattcggcta tgactgggca caacagacaa tcggctgctc tgatgccgcc gtgttccggc 7920
   tgtcagcgca ggggcgcccg gttctttttg tcaagaccga cctgtccggt gccctgaatg 7980
   aactgcagga cgaggcagcg cggctatcgt ggctggccac gacgggcgtt ccttgcgcag 8040
   ctgtgctcga cgttgtcact gaagcgggaa gggactggct gctattgggc gaagtgccgg 8100
   ggcaggatct cctgtcatct caccttgctc ctgccgagaa agtatccatc atggctgatg 8160
   caatgcggcg gctgcatacg cttgatccgg ctacctgccc attcgaccac caagcgaaac 8220
   atcgcatcga gcgagcacgt actcggatgg aagccggtct tgtcgatcag gatgatctgg 8280
   acgaagagca tcaggggctc gcgccagccg aactgttcgc caggctcaag gcgcgcatgc 8340
   ccgacggcga ggatctcgtc gtgacccatg gcgatgcctg cttgccgaat atcatggtgg 8400
   aaaatggccg cttttctgga ttcatcgact gtggccggct gggtgtggcg gaccgctatc 8460
   aggacatagc gttggctacc cgtgatattg ctgaagagct tggcggcgaa tgggctgacc 8520
   gcttcctcgt gctttacggt atcgccgctc ccgattcgca gcgcatcgcc ttctatcgcc 8580
   ttcttgacga gttcttctga gcgggactct ggggttcgaa atgaccgacc aagcgacgcc 8640
   caacctgcca tcacgatggc cgcaataaaa tatctttatt ttcattacat ctgtgtgttg 8700
   gttttttgtg tgaatcgata gcgataagga tccgcgtatg gtgcactctc agtacaatct 8760
   gctctgatgc cgcatagtta agccagcccc gacacccgcc aacacccgct gacgcgccct 8820
   gacgggcttg tctgctcccg gcatccgctt acagacaagc tgtgaccgtc tccgggagct 8880
   gcatgtgtca gaggttttca ccgtcatcac cgaaacgcgc gagacgaaag ggcctcgtga 8940
   tacgcctatt tttataggtt aatgtcatga taataatggt ttcttagacg tcaggtggca 9000
   cttttcgggg aaatgtgcgc ggaaccccta tttgtttatt tttctaaata cattcaaata 9060
   tgtatccgct catgagacaa taaccctgat aaatgcttca ataatattga aaaaggaaga 9120
   gtatgagtat tcaacatttc cgtgtcgccc ttattccctt ttttgcggca ttttgccttc 9180
   ctgtttttgc tcacccagaa acgctggtga aagtaaaaga tgctgaagat cagttgggtg 9240
   cacgagtggg ttacatcgaa ctggatctca acagcggtaa gatccttgag agttttcgcc 9300
   ccgaagaacg ttttccaatg atgagcactt ttaaagttct gctatgtggc gcggtattat 9360
   cccgtattga cgccgggcaa gagcaactcg gtcgccgcat acactattct cagaatgact 9420
   tggttgagta ctcaccagtc acagaaaagc atcttacgga tggcatgaca gtaagagaat 9480
   tatgcagtgc tgccataacc atgagtgata acactgcggc caacttactt ctgacaacga 9540
   tcggaggacc gaaggagcta accgcttttt tgcacaacat gggggatcat gtaactcgcc 9600
   ttgatcgttg ggaaccggag ctgaatgaag ccataccaaa cgacgagcgt gacaccacga 9660
   tgcctgtagc aatggcaaca acgttgcgca aactattaac tggcgaacta cttactctag 9720
   cttcccggca acaattaata gactggatgg aggcggataa agttgcagga ccacttctgc 9780
   gctcggccct tccggctggc tggtttattg ctgataaatc tggagccggt gagcgtgggt 9840
   ctcgcggtat cattgcagca ctggggccag atggtaagcc ctcccgtatc gtagttatct 9900
   acacgacggg gagtcaggca actatggatg aacgaaatag acagatcgct gagataggtg 9960
   cctcactgat taagcattgg taactgtcag accaagttta ctcatatata ctttagattg 10020
   atttaaaact tcatttttaa tttaaaagga tctaggtgaa gatccttttt gataatctca 10080
   tgaccaaaat cccttaacgt gagttttcgt tccactgagc gtcagacccc gtagaaaaga 10140
   tcaaaggatc ttcttgagat cctttttttc tgcgcgtaat ctgctgcttg caaacaaaaa 10200
   aaccaccgct accagcggtg gtttgtttgc cggatcaaga gctaccaact ctttttccga 10260
   aggtaactgg cttcagcaga gcgcagatac caaatactgt ccttctagtg tagccgtagt 10320
   taggccacca cttcaagaac tctgtagcac cgcctacata cctcgctctg ctaatcctgt 10380
   taccagtggc tgctgccagt ggcgataagt cgtgtcttac cgggttggac tcaagacgat 10440
   agttaccgga taaggcgcag cggtcgggct gaacgggggg ttcgtgcaca cagcccagct 10500
   tggagcgaac gacctacacc gaactgagat acctacagcg tgagctatga gaaagcgcca 10560
   cgcttcccga agggagaaag gcggacaggt atccggtaag cggcagggtc ggaacaggag 10620
   agcgcacgag ggagcttcca gggggaaacg cctggtatct ttatagtcct gtcgggtttc 10680
   gccacctctg acttgagcgt cgatttttgt gatgctcgtc aggggggcgg agcctatgga 10740
   aaaacgccag caacgcggcc tttttacggt tcctggcctt ttgctggcct tttgctcaca 10800
   tggctcgaca gatct                                                 10815
<210> 8
   <211> 10930
   <212> DNA
   <213> Artificial Sequence
<220>
   <223> LpESYNGPRRE
<400> 8
   tcaatattgg ccattagcca tattattcat tggttatata gcataaatca atattggcta 60
   ttggccattg catacgttgt atctatatca taatatgtac atttatattg gctcatgtcc 120
   aatatgaccg ccatgttggc attgattatt gactagttat taatagtaat caattacggg 180
   gtcattagtt catagcccat atatggagtt ccgcgttaca taacttacgg taaatggccc 240
   gcctggctga ccgcccaacg acccccgccc attgacgtca ataatgacgt atgttcccat 300
   agtaacgcca atagggactt tccattgacg tcaatgggtg gagtatttac ggtaaactgc 360
   ccacttggca gtacatcaag tgtatcatat gccaagtccg ccccctattg acgtcaatga 420
   cggtaaatgg cccgcctggc attatgccca gtacatgacc ttacgggact ttcctacttg 480
   gcagtacatc tacgtattag tcatcgctat taccatggtg atgcggtttt ggcagtacac 540
   caatgggcgt ggatagcggt ttgactcacg gggatttcca agtctccacc ccattgacgt 600
   caatgggagt ttgttttggc accaaaatca acgggacttt ccaaaatgtc gtaacaactg 660
   cgatcgcccg ccccgttgac gcaaatgggc ggtaggcgtg tacggtggga ggtctatata 720
   agcagagctc gtttagtgaa ccgtcagatc actagaagct ttattgcggt agtttatcac 780
   agttaaattg ctaacgcagt cagtgcttct gacacaacag tctcgaactt aagctgcagt 840
   gactctctta aggtagcctt gcagaagttg gtcgtgaggc actgggcagg taagtatcaa 900
   ggttacaaga caggtttaag gagaccaata gaaactgggc ttgtcgagac agagaagact 960
   cttgcgtttc tgataggcac ctattggtct tactgacatc cactttgcct ttctctccac 1020
   aggtgtccac tcccagttca attacagctc ttaaggctag agtacttaat acgactcact 1080
   ataggctaga gaattcgaga ggggcgcaga ccctacctgt tgaacctggc tgatcgtagg 1140
   atccccggga cagcagagga gaacttacag aagtcttctg gaggtgttcc tggccagaac 1200
   acaggaggac aggtaagatg ggcgatcccc tcacctggtc caaagccctg aagaaactgg 1260
   aaaaagtcac cgttcagggt agccaaaagc ttaccacagg caattgcaac tgggcattgt 1320
   ccctggtgga tcttttccac gacactaatt tcgttaagga gaaagattgg caactcagag 1380
   acgtgatccc cctcttggag gacgtgaccc aaacattgtc tgggcaggag cgcgaagctt 1440
   tcgagcgcac ctggtgggcc atcagcgcag tcaaaatggg gctgcaaatc aacaacgtgg 1500
   ttgacggtaa agctagcttt caactgctcc gcgctaagta cgagaagaaa accgccaaca 1560
   agaaacaatc cgaacctagc gaggagtacc caattatgat cgacggcgcc ggcaatagga 1620
   acttccgccc actgactccc aggggctata ccacctgggt caacaccatc cagacaaacg 1680
   gacttttgaa cgaagcctcc cagaacctgt tcggcatcct gtctgtggac tgcacctccg 1740
   aagaaatgaa tgcttttctc gacgtggtgc caggacaggc tggacagaaa cagatcctgc 1800
   tcgatgccat tgacaagatc gccgacgact gggataatcg ccaccccctg ccaaacgccc 1860
   ctctggtggc tcccccacag gggcctatcc ctatgaccgc taggttcatt aggggactgg 1920
   gggtgccccg cgaacgccag atggagccag catttgacca atttaggcag acctacagac 1980
   agtggatcat cgaagccatg agcgagggga ttaaagtcat gatcggaaag cccaaggcac 2040
   agaacatcag gcagggggcc aaggaaccat accctgagtt tgtcgacagg cttctgtccc 2100
   agattaaatc cgaaggccac cctcaggaga tctccaagtt cttgacagac acactgacta 2160
   tccaaaatgc aaatgaagag tgcagaaacg ccatgaggca cctcagacct gaagataccc 2220
   tggaggagaa aatgtacgca tgtcgcgaca ttggcactac caagcaaaag atgatgctgc 2280
   tcgccaaggc tctgcaaacc ggcctggctg gtccattcaa aggaggagca ctgaagggag 2340
   gtccattgaa agctgcacaa acatgttata attgtgggaa gccaggacat ttatctagtc 2400
   aatgtagagc acctaaagtc tgttttaaat gtaaacagcc tggacatttc tcaaagcaat 2460
   gcagaagtgt tccaaaaaac gggaagcaag gggctcaagg gaggccccag aaacaaactt 2520
   tcccgataca acagaagagt cagcacaaca aatctgttgt acaagagact cctcagactc 2580
   aaaatctgta cccagatctg agcgaaataa aaaaggaata caatgtcaag gagaaggatc 2640
   aagtagagga tctcaacctg gacagtttgt gggagtaaca tacaatctcg agaagaggcc 2700
   cactaccatc gtcctgatca atgacacccc tcttaatgtg ctgctggaca ccggagccga 2760
   caccagcgtt ctcactactg ctcactataa cagactgaaa tacagaggaa ggaaatacca 2820
   gggcacaggc atcatcggcg ttggaggcaa cgtcgaaacc ttttccactc ctgtcaccat 2880
   caaaaagaag ggagacacaa ttaaaaccag aatgctggtc gccgacatcc ccgtcaccat 2940
   ccttggcaga gacattctcc aggacctggg cgctaaactc gtgctggcac aactgtctaa 3000
   ggaaatcaag ttccgcaaga tcgagctgaa agagggcaca atgggtccaa aaatccccca 3060
   gtggcccctg accaaagaga agcttgaggg cgctaaggaa atcgtgcagc gcctgctttc 3120
   tgagggcaag attagcgagg ccagcgacaa taacccttac aacagcccca tctttgtgat 3180
   taagaaaagg agcggcaaat ggagactcct gcaggacctg agggaactca acaagaccgt 3240
   ccaggtcgga actgagatct ctcgcggact gcctcacccc ggcggcctga ttaaatgcaa 3300
   gcacatgaca gtccttgaca ttggagacgc ttattttacc atccccctcg atcctgaatt 3360
   tcgcccctat actgctttta ccatccccag catcaatcac caggagcccg ataaacgcta 3420
   tgtgtggaag tgcctccccc agggatttgt gcttagcccc tacatttacc agaagacact 3480
   tcaagagatc ctccaacctt tccgcgaaag atacccagag gttcaactct accaatatat 3540
   ggacgacctg ttcatggggt ccaacgggtc taagaagcag cacaaggaac tcatcatcga 3600
   actgagggca atcctcctgg agaaaggctt cgagacaccc gacgacaagc tgcaagaagt 3660
   tcctccatat agctggctgg gctaccagct ttgccctgaa aactggaaag tccagaagat 3720
   gcagttggat atggtcaaga acccaacact gaacgacgtc cagaagctca tgggcaatat 3780
   tacctggatg agctccggaa tccctgggct taccgttaag cacattgccg caactacaaa 3840
   aggatgcctg gagttgaacc agaaggtcat ttggacagag gaagctcaga aggaactgga 3900
   ggagaataat gaaaagatta agaatgctca agggctccaa tactacaatc ccgaagaaga 3960
   aatgttgtgc gaggtcgaaa tcactaagaa ctacgaagcc acctatgtca tcaaacagtc 4020
   ccaaggcatc ttgtgggccg gaaagaaaat catgaaggcc aacaaaggct ggtccaccgt 4080
   taaaaatctg atgctcctgc tccagcacgt cgccaccgag tctatcaccc gcgtcggcaa 4140
   gtgccccacc ttcaaagttc ccttcactaa ggagcaggtg atgtgggaga tgcaaaaagg 4200
   ctggtactac tcttggcttc ccgagatcgt ctacacccac caagtggtgc acgacgactg 4260
   gagaatgaag cttgtcgagg agcccactag cggaattaca atctataccg acggcggaaa 4320
   gcaaaacgga gagggaatcg ctgcatacgt cacatctaac ggccgcacca agcaaaagag 4380
   gctcggccct gtcactcacc aggtggctga gaggatggct atccagatgg cccttgagga 4440
   cactagagac aagcaggtga acattgtgac tgacagctac tactgctgga aaaacatcac 4500
   agagggcctt ggcctggagg gaccccagtc tccctggtgg cctatcatcc agaatatccg 4560
   cgaaaaggaa attgtctatt tcgcctgggt gcctggacac aaaggaattt acggcaacca 4620
   actcgccgat gaagccgcca aaattaaaga ggaaatcatg cttgcctacc agggcacaca 4680
   gattaaggag aagagagacg aggacgctgg ctttgacctg tgtgtgccat acgacatcat 4740
   gattcccgtt agcgacacaa agatcattcc aaccgatgtc aagatccagg tgccacccaa 4800
   ttcatttggt tgggtgaccg gaaagtccag catggctaag cagggtcttc tgattaacgg 4860
   gggaatcatt gatgaaggat acaccggcga aatccaggtg atctgcacaa atatcggcaa 4920
   aagcaatatt aagcttatcg aagggcagaa gttcgctcaa ctcatcatcc tccagcacca 4980
   cagcaattca agacaacctt gggacgaaaa caagattagc cagagaggtg acaagggctt 5040
   cggcagcaca ggtgtgttct gggtggagaa catccaggaa gcacaggacg agcacgagaa 5100
   ttggcacacc tcccctaaga ttttggcccg caattacaag atcccactga ctgtggctaa 5160
   gcagatcaca caggaatgcc cccactgcac caaacaaggt tctggccccg ccggctgcgt 5220
   gatgaggtcc cccaatcact ggcaggcaga ttgcacccac ctcgacaaca aaattatcct 5280
   gaccttcgtg gagagcaatt ccggctacat ccacgcaaca ctcctctcca aggaaaatgc 5340
   attgtgcacc tccctcgcaa ttctggaatg ggccaggctg ttctctccaa aatccctgca 5400
   caccgacaac ggcaccaact ttgtggctga acctgtggtg aatctgctga agttcctgaa 5460
   aatcgcccac accactggca ttccctatca ccctgaaagc cagggcattg tcgagagggc 5520
   caacagaact ctgaaagaaa agatccaatc tcacagagac aatacacaga cattggaggc 5580
   cgcacttcag ctcgccctta tcacctgcaa caaaggaaga gaaagcatgg gcggccagac 5640
   cccctgggag gtcttcatca ctaaccaggc ccaggtcatc catgaaaagc tgctcttgca 5700
   gcaggcccag tcctccaaaa agttctgctt ttataagatc cccggtgagc acgactggaa 5760
   aggtcctaca agagttttgt ggaaaggaga cggcgcagtt gtggtgaacg atgagggcaa 5820
   ggggatcatc gctgtgcccc tgacacgcac caagcttctc atcaagccaa actgaacccg 5880
   acgaatccca gggggaatct caacccctat tacccaacag tcagaaaaat ctaagtgtga 5940
   ggagaacaca atgtttcaac cttattgtta taataatgac agtaagaaca gcatggcaga 6000
   atcgaaggaa gcaagagacc aagaaatgaa cctgaaagaa gaatctaaag aagaaaaaag 6060
   aagaaatgac tggtggaaaa taggtatgtt tctgttatgc ttagccaggg ccctctggaa 6120
   ggtgaccagt ggtgcagggt cctccggcag tcgttacctg aagaaaaaat tccatcacaa 6180
   acatgcatcg cgagaagaca cctgggacca ggcccaacac aacatacacc tagcaggcgt 6240
   gaccggtgga tcaggggaca aatactacaa gcagaagtac tccaggaacg actggaatgg 6300
   agaatcagag gagtacaaca ggcggccaaa gagctgggtg aagtcaatcg aggcatttgg 6360
   agagagctat atttccgaga agaccaaagg ggagatttct cagcctgggg cggctatcaa 6420
   cgagcacaag aacggctctg gggggaacaa tcctcaccaa gggtccttag acctggagat 6480
   tcgaagcgaa ggaggaaaca tttatgactg ttgcattaaa gcccaagaag gaactctcgc 6540
   tatcccttgc tgtggatttc ccttatggct attttggggg tcggggcggc cgcttccctt 6600
   tagtgagggt taatgcttcg agcagacatg ataagataca ttgatgagtt tggacaaacc 6660
   acaactagaa tgcagtgaaa aaaatgcttt atttgtgaaa tttgtgatgc tattgcttta 6720
   tttgtaacca ttataagctg caataaacaa gttaacaaca acaattgcat tcattttatg 6780
   tttcaggttc agggggagat gtgggaggtt ttttaaagca agtaaaacct ctacaaatgt 6840
   ggtaaaatcc gataaggatc gatccgggct ggcgtaatag cgaagaggcc cgcaccgatc 6900
   gcccttccca acagttgcgc agcctgaatg gcgaatggac gcgccctgta gcggcgcatt 6960
   aagcgcggcg ggtgtggtgg ttacgcgcag cgtgaccgct acacttgcca gcgccctagc 7020
   gcccgctcct ttcgctttct tcccttcctt tctcgccacg ttcgccggct ttccccgtca 7080
   agctctaaat cgggggctcc ctttagggtt ccgatttaga gctttacggc acctcgaccg 7140
   caaaaaactt gatttgggtg atggttcacg tagtgggcca tcgccctgat agacggtttt 7200
   tcgccctttg acgttggagt ccacgttctt taatagtgga ctcttgttcc aaactggaac 7260
   aacactcaac cctatctcgg tctattcttt tgatttataa gggattttgc cgatttcggc 7320
   ctattggtta aaaaatgagc tgatttaaca aatatttaac gcgaatttta acaaaatatt 7380
   aacgtttaca atttcgcctg atgcggtatt ttctccttac gcatctgtgc ggtatttcac 7440
   accgcatacg cggatctgcg cagcaccatg gcctgaaata acctctgaaa gaggaacttg 7500
   gttaggtacc ttctgaggcg gaaagaacca gctgtggaat gtgtgtcagt tagggtgtgg 7560
   aaagtcccca ggctccccag caggcagaag tatgcaaagc atgcatctca attagtcagc 7620
   aaccaggtgt ggaaagtccc caggctcccc agcaggcaga agtatgcaaa gcatgcatct 7680
   caattagtca gcaaccatag tcccgcccct aactccgccc atcccgcccc taactccgcc 7740
   cagttccgcc cattctccgc cccatggctg actaattttt tttatttatg cagaggccga 7800
   ggccgcctcg gcctctgagc tattccagaa gtagtgagga ggcttttttg gaggcctagg 7860
   cttttgcaaa aagcttgatt cttctgacac aacagtctcg aacttaaggc tagagccacc 7920
   atgattgaac aagatggatt gcacgcaggt tctccggccg cttgggtgga gaggctattc 7980
   ggctatgact gggcacaaca gacaatcggc tgctctgatg ccgccgtgtt ccggctgtca 8040
   gcgcaggggc gcccggttct ttttgtcaag accgacctgt ccggtgccct gaatgaactg 8100
   caggacgagg cagcgcggct atcgtggctg gccacgacgg gcgttccttg cgcagctgtg 8160
   ctcgacgttg tcactgaagc gggaagggac tggctgctat tgggcgaagt gccggggcag 8220
   gatctcctgt catctcacct tgctcctgcc gagaaagtat ccatcatggc tgatgcaatg 8280
   cggcggctgc atacgcttga tccggctacc tgcccattcg accaccaagc gaaacatcgc 8340
   atcgagcgag cacgtactcg gatggaagcc ggtcttgtcg atcaggatga tctggacgaa 8400
   gagcatcagg ggctcgcgcc agccgaactg ttcgccaggc tcaaggcgcg catgcccgac 8460
   ggcgaggatc tcgtcgtgac ccatggcgat gcctgcttgc cgaatatcat ggtggaaaat 8520
   ggccgctttt ctggattcat cgactgtggc cggctgggtg tggcggaccg ctatcaggac 8580
   atagcgttgg ctacccgtga tattgctgaa gagcttggcg gcgaatgggc tgaccgcttc 8640
   ctcgtgcttt acggtatcgc cgctcccgat tcgcagcgca tcgccttcta tcgccttctt 8700
   gacgagttct tctgagcggg actctggggt tcgaaatgac cgaccaagcg acgcccaacc 8760
   tgccatcacg atggccgcaa taaaatatct ttattttcat tacatctgtg tgttggtttt 8820
   ttgtgtgaat cgatagcgat aaggatccgc gtatggtgca ctctcagtac aatctgctct 8880
   gatgccgcat agttaagcca gccccgacac ccgccaacac ccgctgacgc gccctgacgg 8940
   gcttgtctgc tcccggcatc cgcttacaga caagctgtga ccgtctccgg gagctgcatg 9000
   tgtcagaggt tttcaccgtc atcaccgaaa cgcgcgagac gaaagggcct cgtgatacgc 9060
   ctatttttat aggttaatgt catgataata atggtttctt agacgtcagg tggcactttt 9120
   cggggaaatg tgcgcggaac ccctatttgt ttatttttct aaatacattc aaatatgtat 9180
   ccgctcatga gacaataacc ctgataaatg cttcaataat attgaaaaag gaagagtatg 9240
   agtattcaac atttccgtgt cgcccttatt cccttttttg cggcattttg ccttcctgtt 9300
   tttgctcacc cagaaacgct ggtgaaagta aaagatgctg aagatcagtt gggtgcacga 9360
   gtgggttaca tcgaactgga tctcaacagc ggtaagatcc ttgagagttt tcgccccgaa 9420
   gaacgttttc caatgatgag cacttttaaa gttctgctat gtggcgcggt attatcccgt 9480
   attgacgccg ggcaagagca actcggtcgc cgcatacact attctcagaa tgacttggtt 9540
   gagtactcac cagtcacaga aaagcatctt acggatggca tgacagtaag agaattatgc 9600
   agtgctgcca taaccatgag tgataacact gcggccaact tacttctgac aacgatcgga 9660
   ggaccgaagg agctaaccgc ttttttgcac aacatggggg atcatgtaac tcgccttgat 9720
   cgttgggaac cggagctgaa tgaagccata ccaaacgacg agcgtgacac cacgatgcct 9780
   gtagcaatgg caacaacgtt gcgcaaacta ttaactggcg aactacttac tctagcttcc 9840
   cggcaacaat taatagactg gatggaggcg gataaagttg caggaccact tctgcgctcg 9900
   gcccttccgg ctggctggtt tattgctgat aaatctggag ccggtgagcg tgggtctcgc 9960
   ggtatcattg cagcactggg gccagatggt aagccctccc gtatcgtagt tatctacacg 10020
   acggggagtc aggcaactat ggatgaacga aatagacaga tcgctgagat aggtgcctca 10080
   ctgattaagc attggtaact gtcagaccaa gtttactcat atatacttta gattgattta 10140
   aaacttcatt tttaatttaa aaggatctag gtgaagatcc tttttgataa tctcatgacc 10200
   aaaatccctt aacgtgagtt ttcgttccac tgagcgtcag accccgtaga aaagatcaaa 10260
   ggatcttctt gagatccttt ttttctgcgc gtaatctgct gcttgcaaac aaaaaaacca 10320
   ccgctaccag cggtggtttg tttgccggat caagagctac caactctttt tccgaaggta 10380
   actggcttca gcagagcgca gataccaaat actgtccttc tagtgtagcc gtagttaggc 10440
   caccacttca agaactctgt agcaccgcct acatacctcg ctctgctaat cctgttacca 10500
   gtggctgctg ccagtggcga taagtcgtgt cttaccgggt tggactcaag acgatagtta 10560
   ccggataagg cgcagcggtc gggctgaacg gggggttcgt gcacacagcc cagcttggag 10620
   cgaacgacct acaccgaact gagataccta cagcgtgagc tatgagaaag cgccacgctt 10680
   cccgaaggga gaaaggcgga caggtatccg gtaagcggca gggtcggaac aggagagcgc 10740
   acgagggagc ttccaggggg aaacgcctgg tatctttata gtcctgtcgg gtttcgccac 10800
   ctctgacttg agcgtcgatt tttgtgatgc tcgtcagggg ggcggagcct atggaaaaac 10860
   gccagcaacg cggccttttt acggttcctg gccttttgct ggccttttgc tcacatggct 10920
   cgacagatct                                                        10930
<210> 9
   <211> 11131
   <212> DNA
   <213> Artificial Sequence
<220>
   <223> pONY4.0Z
<400> 9
   ctaaattgta agcgttaata ttttgttaaa attcgcgtta aatttttgtt aaatcagctc 60
   attttttaac caataggccg aaatcggcaa aatcccttat aaatcaaaag aatagaccga 120
   gatagggttg agtgttgttc cagtttggaa caagagtcca ctattaaaga acgtggactc 180
   caacgtcaaa gggcgaaaaa ccgtctatca gggcgatggc ccactacgtg aaccatcacc 240
   ctaatcaagt tttttggggt cgaggtgccg taaagcacta aatcggaacc ctaaagggag 300
   cccccgattt agagcttgac ggggaaagcc aacctggctt atcgaaatta atacgactca 360
   ctatagggag accggcagat cttgaataat aaaatgtgtg tttgtccgaa atacgcgttt 420
   tgagatttct gtcgccgact aaattcatgt cgcgcgatag tggtgtttat cgccgataga 480
   gatggcgata ttggaaaaat tgatatttga aaatatggca tattgaaaat gtcgccgatg 540
   tgagtttctg tgtaactgat atcgccattt ttccaaaagt gatttttggg catacgcgat 600
   atctggcgat agcgcttata tcgtttacgg gggatggcga tagacgactt tggtgacttg 660
   ggcgattctg tgtgtcgcaa atatcgcagt ttcgatatag gtgacagacg atatgaggct 720
   atatcgccga tagaggcgac atcaagctgg cacatggcca atgcatatcg atctatacat 780
   tgaatcaata ttggccatta gccatattat tcattggtta tatagcataa atcaatattg 840
   gctattggcc attgcatacg ttgtatccat atcgtaatat gtacatttat attggctcat 900
   gtccaacatt accgccatgt tgacattgat tattgactag ttattaatag taatcaatta 960
   cggggtcatt agttcatagc ccatatatgg agttccgcgt tacataactt acggtaaatg 1020
   gcccgcctgg ctgaccgccc aacgaccccc gcccattgac gtcaataatg acgtatgttc 1080
   ccatagtaac gccaataggg actttccatt gacgtcaatg ggtggagtat ttacggtaaa 1140
   ctgcccactt ggcagtacat caagtgtatc atatgccaag tccgccccct attgacgtca 1200
   atgacggtaa atggcccgcc tggcattatg cccagtacat gaccttacgg gactttccta 1260
   cttggcagta catctacgta ttagtcatcg ctattaccat ggtgatgcgg ttttggcagt 1320
   acaccaatgg gcgtggatag cggtttgact cacggggatt tccaagtctc caccccattg 1380
   acgtcaatgg gagtttgttt tggcaccaaa atcaacggga ctttccaaaa tgtcgtaaca 1440
   actgcgatcg cccgccccgt tgacgcaaat gggcggtagg cgtgtacggt gggaggtcta 1500
   tataagcaga gctcgtttag tgaaccgggc actcagattc tgcggtctga gtcccttctc 1560
   tgctgggctg aaaaggcctt tgtaataaat ataattctct actcagtccc tgtctctagt 1620
   ttgtctgttc gagatcctac agttggcgcc cgaacaggga cctgagaggg gcgcagaccc 1680
   tacctgttga acctggctga tcgtaggatc cccgggacag cagaggagaa cttacagaag 1740
   tcttctggag gtgttcctgg ccagaacaca ggaggacagg taagatggga gaccctttga 1800
   catggagcaa ggcgctcaag aagttagaga aggtgacggt acaagggtct cagaaattaa 1860
   ctactggtaa ctgtaattgg gcgctaagtc tagtagactt atttcatgat accaactttg 1920
   taaaagaaaa ggactggcag ctgagggatg tcattccatt gctggaagat gtaactcaga 1980
   cgctgtcagg acaagaaaga gaggcctttg aaagaacatg gtgggcaatt tctgctgtaa 2040
   agatgggcct ccagattaat aatgtagtag atggaaaggc atcattccag ctcctaagag 2100
   cgaaatatga aaagaagact gctaataaaa agcagtctga gccctctgaa gaatatctct 2160
   agaactagtg gatcccccgg gctgcaggag tggggaggca cgatggccgc tttggtcgag 2220
   gcggatccgg ccattagcca tattattcat tggttatata gcataaatca atattggcta 2280
   ttggccattg catacgttgt atccatatca taatatgtac atttatattg gctcatgtcc 2340
   aacattaccg ccatgttgac attgattatt gactagttat taatagtaat caattacggg 2400
   gtcattagtt catagcccat atatggagtt ccgcgttaca taacttacgg taaatggccc 2460
   gcctggctga ccgcccaacg acccccgccc attgacgtca ataatgacgt atgttcccat 2520
   agtaacgcca atagggactt tccattgacg tcaatgggtg gagtatttac ggtaaactgc 2580
   ccacttggca gtacatcaag tgtatcatat gccaagtacg ccccctattg acgtcaatga 2640
   cggtaaatgg cccgcctggc attatgccca gtacatgacc ttatgggact ttcctacttg 2700
   gcagtacatc tacgtattag tcatcgctat taccatggtg atgcggtttt ggcagtacat 2760
   caatgggcgt ggatagcggt ttgactcacg gggatttcca agtctccacc ccattgacgt 2820
   caatgggagt ttgttttggc accaaaatca acgggacttt ccaaaatgtc gtaacaactc 2880
   cgccccattg acgcaaatgg gcggtaggca tgtacggtgg gaggtctata taagcagagc 2940
   tcgtttagtg aaccgtcaga tcgcctggag acgccatcca cgctgttttg acctccatag 3000
   aagacaccgg gaccgatcca gcctccgcgg ccccaagctt cagctgctcg aggatctgcg 3060
   gatccgggga attccccagt ctcaggatcc accatggggg atcccgtcgt tttacaacgt 3120
   cgtgactggg aaaaccctgg cgttacccaa cttaatcgcc ttgcagcaca tccccctttc 3180
   gccagctggc gtaatagcga agaggcccgc accgatcgcc cttcccaaca gttgcgcagc 3240
   ctgaatggcg aatggcgctt tgcctggttt ccggcaccag aagcggtgcc ggaaagctgg 3300
   ctggagtgcg atcttcctga ggccgatact gtcgtcgtcc cctcaaactg gcagatgcac 3360
   ggttacgatg cgcccatcta caccaacgta acctatccca ttacggtcaa tccgccgttt 3420
   gttcccacgg agaatccgac gggttgttac tcgctcacat ttaatgttga tgaaagctgg 3480
   ctacaggaag gccagacgcg aattattttt gatggcgtta actcggcgtt tcatctgtgg 3540
   tgcaacgggc gctgggtcgg ttacggccag gacagtcgtt tgccgtctga atttgacctg 3600
   agcgcatttt tacgcgccgg agaaaaccgc ctcgcggtga tggtgctgcg ttggagtgac 3660
   ggcagttatc tggaagatca ggatatgtgg cggatgagcg gcattttccg tgacgtctcg 3720
   ttgctgcata aaccgactac acaaatcagc gatttccatg ttgccactcg ctttaatgat 3780
   gatttcagcc gcgctgtact ggaggctgaa gttcagatgt gcggcgagtt gcgtgactac 3840
   ctacgggtaa cagtttcttt atggcagggt gaaacgcagg tcgccagcgg caccgcgcct 3900
   ttcggcggtg aaattatcga tgagcgtggt ggttatgccg atcgcgtcac actacgtctg 3960
   aacgtcgaaa acccgaaact gtggagcgcc gaaatcccga atctctatcg tgcggtggtt 4020
   gaactgcaca ccgccgacgg cacgctgatt gaagcagaag cctgcgatgt cggtttccgc 4080
   gaggtgcgga ttgaaaatgg tctgctgctg ctgaacggca agccgttgct gattcgaggc 4140
   gttaaccgtc acgagcatca tcctctgcat ggtcaggtca tggatgagca gacgatggtg 4200
   caggatatcc tgctgatgaa gcagaacaac tttaacgccg tgcgctgttc gcattatccg 4260
   aaccatccgc tgtggtacac gctgtgcgac cgctacggcc tgtatgtggt ggatgaagcc 4320
   aatattgaaa cccacggcat ggtgccaatg aatcgtctga ccgatgatcc gcgctggcta 4380
   ccggcgatga gcgaacgcgt aacgcgaatg gtgcagcgcg atcgtaatca cccgagtgtg 4440
   atcatctggt cgctggggaa tgaatcaggc cacggcgcta atcacgacgc gctgtatcgc 4500
   tggatcaaat ctgtcgatcc ttcccgcccg gtgcagtatg aaggcggcgg agccgacacc 4560
   acggccaccg atattatttg cccgatgtac gcgcgcgtgg atgaagacca gcccttcccg 4620
   gctgtgccga aatggtccat caaaaaatgg ctttcgctac ctggagagac gcgcccgctg 4680
   atcctttgcg aatacgccca cgcgatgggt aacagtcttg gcggtttcgc taaatactgg 4740
   caggcgtttc gtcagtatcc ccgtttacag ggcggcttcg tctgggactg ggtggatcag 4800
   tcgctgatta aatatgatga aaacggcaac ccgtggtcgg cttacggcgg tgattttggc 4860
   gatacgccga acgatcgcca gttctgtatg aacggtctgg tctttgccga ccgcacgccg 4920
   catccagcgc tgacggaagc aaaacaccag cagcagtttt tccagttccg tttatccggg 4980
   caaaccatcg aagtgaccag cgaatacctg ttccgtcata gcgataacga gctcctgcac 5040
   tggatggtgg cgctggatgg taagccgctg gcaagcggtg aagtgcctct ggatgtcgct 5100
   ccacaaggta aacagttgat tgaactgcct gaactaccgc agccggagag cgccgggcaa 5160
   ctctggctca cagtacgcgt agtgcaaccg aacgcgaccg catggtcaga agccgggcac 5220
   atcagcgcct ggcagcagtg gcgtctggcg gaaaacctca gtgtgacgct ccccgccgcg 5280
   tcccacgcca tcccgcatct gaccaccagc gaaatggatt tttgcatcga gctgggtaat 5340
   aagcgttggc aatttaaccg ccagtcaggc tttctttcac agatgtggat tggcgataaa 5400
   aaacaactgc tgacgccgct gcgcgatcag ttcacccgtg caccgctgga taacgacatt 5460
   ggcgtaagtg aagcgacccg cattgaccct aacgcctggg tcgaacgctg gaaggcggcg 5520
   ggccattacc aggccgaagc agcgttgttg cagtgcacgg cagatacact tgctgatgcg 5580
   gtgctgatta cgaccgctca cgcgtggcag catcagggga aaaccttatt tatcagccgg 5640
   aaaacctacc ggattgatgg tagtggtcaa atggcgatta ccgttgatgt tgaagtggcg 5700
   agcgatacac cgcatccggc gcggattggc ctgaactgcc agctggcgca ggtagcagag 5760
   cgggtaaact ggctcggatt agggccgcaa gaaaactatc ccgaccgcct tactgccgcc 5820
   tgttttgacc gctgggatct gccattgtca gacatgtata ccccgtacgt cttcccgagc 5880
   gaaaacggtc tgcgctgcgg gacgcgcgaa ttgaattatg gcccacacca gtggcgcggc 5940
   gacttccagt tcaacatcag ccgctacagt caacagcaac tgatggaaac cagccatcgc 6000
   catctgctgc acgcggaaga aggcacatgg ctgaatatcg acggtttcca tatggggatt 6060
   ggtggcgacg actcctggag cccgtcagta tcggcggaat tccagctgag cgccggtcgc 6120
   taccattacc agttggtctg gtgtcaaaaa taataataac cgggcagggg ggatccgcag 6180
   atccggctgt ggaatgtgtg tcagttaggg tgtggaaagt ccccaggctc cccagcaggc 6240
   agaagtatgc aaagcatgcc tgcaggaatt cgatatcaag cttatcgata ccgtcgacct 6300
   cgaggggggg cccggtaccc agcttttgtt ccctttagtg agggttaatt gcgcgggaag 6360
   tatttatcac taatcaagca caagtaatac atgagaaact tttactacag caagcacaat 6420
   cctccaaaaa attttgtttt tacaaaatcc ctggtgaaca tgattggaag ggacctacta 6480
   gggtgctgtg gaagggtgat ggtgcagtag tagttaatga tgaaggaaag ggaataattg 6540
   ctgtaccatt aaccaggact aagttactaa taaaaccaaa ttgagtattg ttgcaggaag 6600
   caagacccaa ctaccattgt cagctgtgtt tcctgaggtc tctaggaatt gattacctcg 6660
   atgcttcatt aaggaagaag aataaacaaa gactgaaggc aatccaacaa ggaagacaac 6720
   ctcaatattt gttataaggt ttgatatatg ggagtatttg gtaaaggggt aacatggtca 6780
   gcatcgcatt ctatggggga atcccagggg gaatctcaac ccctattacc caacagtcag 6840
   aaaaatctaa gtgtgaggag aacacaatgt ttcaacctta ttgttataat aatgacagta 6900
   agaacagcat ggcagaatcg aaggaagcaa gagaccaaga aatgaacctg aaagaagaat 6960
   ctaaagaaga aaaaagaaga aatgactggt ggaaaatagg tatgtttctg ttatgcttag 7020
   caggaactac tggaggaata ctttggtggt atgaaggact cccacagcaa cattatatag 7080
   ggttggtggc gataggggga agattaaacg gatctggcca atcaaatgct atagaatgct 7140
   ggggttcctt cccggggtgt agaccatttc aaaattactt cagttatgag accaatagaa 7200
   gcatgcatat ggataataat actgctacat tattagaagc tttaaccaat ataactgctc 7260
   tataaataac aaaacagaat tagaaacatg gaagttagta aagacttctg gcataactcc 7320
   tttacctatt tcttctgaag ctaacactgg actaattaga cataagagag attttggtat 7380
   aagtgcaata gtggcagcta ttgtagccgc tactgctatt gctgctagcg ctactatgtc 7440
   ttatgttgct ctaactgagg ttaacaaaat aatggaagta caaaatcata cttttgaggt 7500
   agaaaatagt actctaaatg gtatggattt aatagaacga caaataaaga tattatatgc 7560
   tatgattctt caaacacatg cagatgttca actgttaaag gaaagacaac aggtagagga 7620
   gacatttaat ttaattggat gtatagaaag aacacatgta ttttgtcata ctggtcatcc 7680
   ctggaatatg tcatggggac atttaaatga gtcaacacaa tgggatgact gggtaagcaa 7740
   aatggaagat ttaaatcaag agatactaac tacacttcat ggagccagga acaatttggc 7800
   acaatccatg ataacattca atacaccaga tagtatagct caatttggaa aagacctttg 7860
   gagtcatatt ggaaattgga ttcctggatt gggagcttcc attataaaat atatagtgat 7920
   gtttttgctt atttatttgt tactaacctc ttcgcctaag atcctcaggg ccctctggaa 7980
   ggtgaccagt ggtgcagggt cctccggcag tcgttacctg aagaaaaaat tccatcacaa 8040
   acatgcatcg cgagaagaca cctgggacca ggcccaacac aacatacacc tagcaggcgt 8100
   gaccggtgga tcaggggaca aatactacaa gcagaagtac tccaggaacg actggaatgg 8160
   agaatcagag gagtacaaca ggcggccaaa gagctgggtg aagtcaatcg aggcatttgg 8220
   agagagctat atttccgaga agaccaaagg ggagatttct cagcctgggg cggctatcaa 8280
   cgagcacaag aacggctctg gggggaacaa tcctcaccaa gggtccttag acctggagat 8340
   tcgaagcgaa ggaggaaaca tttatgactg ttgcattaaa gcccaagaag gaactctcgc 8400
   tatcccttgc tgtggatttc ccttatggct attttgggga ctagtaatta tagtaggacg 8460
   catagcaggc tatggattac gtggactcgc tgttataata aggatttgta ttagaggctt 8520
   aaatttgata tttgaaataa tcagaaaaat gcttgattat attggaagag ctttaaatcc 8580
   tggcacatct catgtatcaa tgcctcagta tgtttagaaa aacaaggggg gaactgtggg 8640
   gtttttatga ggggttttat aaatgattat aagagtaaaa agaaagttgc tgatgctctc 8700
   ataaccttgt ataacccaaa ggactagctc atgttgctag gcaactaaac cgcaataacc 8760
   gcatttgtga cgcgagttcc ccattggtga cgcgttaact tcctgttttt acagtatata 8820
   agtgcttgta ttctgacaat tgggcactca gattctgcgg tctgagtccc ttctctgctg 8880
   ggctgaaaag gcctttgtaa taaatataat tctctactca gtccctgtct ctagtttgtc 8940
   tgttcgagat cctacagagc tcatgccttg gcgtaatcat ggtcatagct gtttcctgtg 9000
   tgaaattgtt atccgctcac aattccacac aacatacgag ccggaagcat aaagtgtaaa 9060
   gcctggggtg cctaatgagt gagctaactc acattaattg cgttgcgctc actgcccgct 9120
   ttccagtcgg gaaacctgtc gtgccagctg cattaatgaa tcggccaacg cgcggggaga 9180
   ggcggtttgc gtattgggcg ctcttccgct tcctcgctca ctgactcgct gcgctcggtc 9240
   gttcggctgc ggcgagcggt atcagctcac tcaaaggcgg taatacggtt atccacagaa 9300
   tcaggggata acgcaggaaa gaacatgtga gcaaaaggcc agcaaaaggc caggaaccgt 9360
   aaaaaggccg cgttgctggc gtttttccat aggctccgcc cccctgacga gcatcacaaa 9420
   aatcgacgct caagtcagag gtggcgaaac ccgacaggac tataaagata ccaggcgttt 9480
   ccccctggaa gctccctcgt gcgctctcct gttccgaccc tgccgcttac cggatacctg 9540
   tccgcctttc tcccttcggg aagcgtggcg ctttctcata gctcacgctg taggtatctc 9600
   agttcggtgt aggtcgttcg ctccaagctg ggctgtgtgc acgaaccccc cgttcagccc 9660
   gaccgctgcg ccttatccgg taactatcgt cttgagtcca acccggtaag acacgactta 9720
   tcgccactgg cagcagccac tggtaacagg attagcagag cgaggtatgt aggcggtgct 9780
   acagagttct tgaagtggtg gcctaactac ggctacacta gaaggacagt atttggtatc 9840
   tgcgctctgc tgaagccagt taccttcgga aaaagagttg gtagctcttg atccggcaaa 9900
   caaaccaccg ctggtagcgg tggttttttt gtttgcaagc agcagattac gcgcagaaaa 9960
   aaaggatctc aagaagatcc tttgatcttt tctacggggt ctgacgctca gtggaacgaa 10020
   aactcacgtt aagggatttt ggtcatgaga ttatcaaaaa ggatcttcac ctagatcctt 10080
   ttaaattaaa aatgaagttt taaatcaatc taaagtatat atgagtaaac ttggtctgac 10140
   agttaccaat gcttaatcag tgaggcacct atctcagcga tctgtctatt tcgttcatcc 10200
   atagttgcct gactccccgt cgtgtagata actacgatac gggagggctt accatctggc 10260
   cccagtgctg caatgatacc gcgagaccca cgctcaccgg ctccagattt atcagcaata 10320
   aaccagccag ccggaagggc cgagcgcaga agtggtcctg caactttatc cgcctccatc 10380
   cagtctatta attgttgccg ggaagctaga gtaagtagtt cgccagttaa tagtttgcgc 10440
   aacgttgttg ccattgctac aggcatcgtg gtgtcacgct cgtcgtttgg tatggcttca 10500
   ttcagctccg gttcccaacg atcaaggcga gttacatgat cccccatgtt gtgcaaaaaa 10560
   gcggttagct ccttcggtcc tccgatcgtt gtcagaagta agttggccgc agtgttatca 10620
   ctcatggtta tggcagcact gcataattct cttactgtca tgccatccgt aagatgcttt 10680
   tctgtgactg gtgagtactc aaccaagtca ttctgagaat agtgtatgcg gcgaccgagt 10740
   tgctcttgcc cggcgtcaat acgggataat accgcgccac atagcagaac tttaaaagtg 10800
   ctcatcattg gaaaacgttc ttcggggcga aaactctcaa ggatcttacc gctgttgaga 10860
   tccagttcga tgtaacccac tcgtgcaccc aactgatctt cagcatcttt tactttcacc 10920
   agcgtttctg ggtgagcaaa aacaggaagg caaaatgccg caaaaaaggg aataagggcg 10980
   acacggaaat gttgaatact catactcttc ctttttcaat attattgaag catttatcag 11040
   ggttattgtc tcatgagcgg atacatattt gaatgtattt agaaaaataa acaaataggg 11100
   gttccgcgca catttccccg aaaagtgcca c                               11131
<210> 10
   <211> 10998
   <212> DNA
   <213> Artificial Sequence
<220>
   <223> pONY8.0Z
<400> 10
   agatcttgaa taataaaatg tgtgtttgtc cgaaatacgc gttttgagat ttctgtcgcc 60
   gactaaattc atgtcgcgcg atagtggtgt ttatcgccga tagagatggc gatattggaa 120
   aaattgatat ttgaaaatat ggcatattga aaatgtcgcc gatgtgagtt tctgtgtaac 180
   tgatatcgcc atttttccaa aagtgatttt tgggcatacg cgatatctgg cgatagcgct 240
   tatatcgttt acgggggatg gcgatagacg actttggtga cttgggcgat tctgtgtgtc 300
   gcaaatatcg cagtttcgat ataggtgaca gacgatatga ggctatatcg ccgatagagg 360
   cgacatcaag ctggcacatg gccaatgcat atcgatctat acattgaatc aatattggcc 420
   attagccata ttattcattg gttatatagc ataaatcaat attggctatt ggccattgca 480
   tacgttgtat ccatatcgta atatgtacat ttatattggc tcatgtccaa cattaccgcc 540
   atgttgacat tgattattga ctagttatta atagtaatca attacggggt cattagttca 600
   tagcccatat atggagttcc gcgttacata acttacggta aatggcccgc ctggctgacc 660
   gcccaacgac ccccgcccat tgacgtcaat aatgacgtat gttcccatag taacgccaat 720
   agggactttc cattgacgtc aatgggtgga gtatttacgg taaactgccc acttggcagt 780
   acatcaagtg tatcatatgc caagtccgcc ccctattgac gtcaatgacg gtaaatggcc 840
   cgcctggcat tatgcccagt acatgacctt acgggacttt cctacttggc agtacatcta 900
   cgtattagtc atcgctatta ccatggtgat gcggttttgg cagtacacca atgggcgtgg 960
   atagcggttt gactcacggg gatttccaag tctccacccc attgacgtca atgggagttt 1020
   gttttggcac caaaatcaac gggactttcc aaaatgtcgt aacaactgcg atcgcccgcc 1080
   ccgttgacgc aaatgggcgg taggcgtgta cggtgggagg tctatataag cagagctcgt 1140
   ttagtgaacc gggcactcag attctgcggt ctgagtccct tctctgctgg gctgaaaagg 1200
   cctttgtaat aaatataatt ctctactcag tccctgtctc tagtttgtct gttcgagatc 1260
   ctacagttgg cgcccgaaca gggacctgag aggggcgcag accctacctg ttgaacctgg 1320
   ctgatcgtag gatccccggg acagcagagg agaacttaca gaagtcttct ggaggtgttc 1380
   ctggccagaa cacaggagga caggtaagat tgggagaccc tttgacattg gagcaaggcg 1440
   ctcaagaagt tagagaaggt gacggtacaa gggtctcaga aattaactac tggtaactgt 1500
   aattgggcgc taagtctagt agacttattt catgatacca actttgtaaa agaaaaggac 1560
   tggcagctga gggatgtcat tccattgctg gaagatgtaa ctcagacgct gtcaggacaa 1620
   gaaagagagg cctttgaaag aacatggtgg gcaatttctg ctgtaaagat gggcctccag 1680
   attaataatg tagtagatgg aaaggcatca ttccagctcc taagagcgaa atatgaaaag 1740
   aagactgcta ataaaaagca gtctgagccc tctgaagaat atctctagaa ctagtggatc 1800
   ccccgggctg caggagtggg gaggcacgat ggccgctttg gtcgaggcgg atccggccat 1860
   tagccatatt attcattggt tatatagcat aaatcaatat tggctattgg ccattgcata 1920
   cgttgtatcc atatcataat atgtacattt atattggctc atgtccaaca ttaccgccat 1980
   gttgacattg attattgact agttattaat agtaatcaat tacggggtca ttagttcata 2040
   gcccatatat ggagttccgc gttacataac ttacggtaaa tggcccgcct ggctgaccgc 2100
   ccaacgaccc ccgcccattg acgtcaataa tgacgtatgt tcccatagta acgccaatag 2160
   ggactttcca ttgacgtcaa tgggtggagt atttacggta aactgcccac ttggcagtac 2220
   atcaagtgta tcatatgcca agtacgcccc ctattgacgt caatgacggt aaatggcccg 2280
   cctggcatta tgcccagtac atgaccttat gggactttcc tacttggcag tacatctacg 2340
   tattagtcat cgctattacc atggtgatgc ggttttggca gtacatcaat gggcgtggat 2400
   agcggtttga ctcacgggga tttccaagtc tccaccccat tgacgtcaat gggagtttgt 2460
   tttggcacca aaatcaacgg gactttccaa aatgtcgtaa caactccgcc ccattgacgc 2520
   aaatgggcgg taggcatgta cggtgggagg tctatataag cagagctcgt ttagtgaacc 2580
   gtcagatcgc ctggagacgc catccacgct gttttgacct ccatagaaga caccgggacc 2640
   gatccagcct ccgcggcccc aagcttcagc tgctcgagga tctgcggatc cggggaattc 2700
   cccagtctca ggatccacca tgggggatcc cgtcgtttta caacgtcgtg actgggaaaa 2760
   ccctggcgtt acccaactta atcgccttgc agcacatccc cctttcgcca gctggcgtaa 2820
   tagcgaagag gcccgcaccg atcgcccttc ccaacagttg cgcagcctga atggcgaatg 2880
   gcgctttgcc tggtttccgg caccagaagc ggtgccggaa agctggctgg agtgcgatct 2940
   tcctgaggcc gatactgtcg tcgtcccctc aaactggcag atgcacggtt acgatgcgcc 3000
   catctacacc aacgtaacct atcccattac ggtcaatccg ccgtttgttc ccacggagaa 3060
   tccgacgggt tgttactcgc tcacatttaa tgttgatgaa agctggctac aggaaggcca 3120
   gacgcgaatt atttttgatg gcgttaactc ggcgtttcat ctgtggtgca acgggcgctg 3180
   ggtcggttac ggccaggaca gtcgtttgcc gtctgaattt gacctgagcg catttttacg 3240
   cgccggagaa aaccgcctcg cggtgatggt gctgcgttgg agtgacggca gttatctgga 3300
   agatcaggat atgtggcgga tgagcggcat tttccgtgac gtctcgttgc tgcataaacc 3360
   gactacacaa atcagcgatt tccatgttgc cactcgcttt aatgatgatt tcagccgcgc 3420
   tgtactggag gctgaagttc agatgtgcgg cgagttgcgt gactacctac gggtaacagt 3480
   ttctttatgg cagggtgaaa cgcaggtcgc cagcggcacc gcgcctttcg gcggtgaaat 3540
   tatcgatgag cgtggtggtt atgccgatcg cgtcacacta cgtctgaacg tcgaaaaccc 3600
   gaaactgtgg agcgccgaaa tcccgaatct ctatcgtgcg gtggttgaac tgcacaccgc 3660
   cgacggcacg ctgattgaag cagaagcctg cgatgtcggt ttccgcgagg tgcggattga 3720
   aaatggtctg ctgctgctga acggcaagcc gttgctgatt cgaggcgtta accgtcacga 3780
   gcatcatcct ctgcatggtc aggtcatgga tgagcagacg atggtgcagg atatcctgct 3840
   gatgaagcag aacaacttta acgccgtgcg ctgttcgcat tatccgaacc atccgctgtg 3900
   gtacacgctg tgcgaccgct acggcctgta tgtggtggat gaagccaata ttgaaaccca 3960
   cggcatggtg ccaatgaatc gtctgaccga tgatccgcgc tggctaccgg cgatgagcga 4020
   acgcgtaacg cgaatggtgc agcgcgatcg taatcacccg agtgtgatca tctggtcgct 4080
   ggggaatgaa tcaggccacg gcgctaatca cgacgcgctg tatcgctgga tcaaatctgt 4140
   cgatccttcc cgcccggtgc agtatgaagg cggcggagcc gacaccacgg ccaccgatat 4200
   tatttgcccg atgtacgcgc gcgtggatga agaccagccc ttcccggctg tgccgaaatg 4260
   gtccatcaaa aaatggcttt cgctacctgg agagacgcgc ccgctgatcc tttgcgaata 4320
   cgcccacgcg atgggtaaca gtcttggcgg tttcgctaaa tactggcagg cgtttcgtca 4380
   gtatccccgt ttacagggcg gcttcgtctg ggactgggtg gatcagtcgc tgattaaata 4440
   tgatgaaaac ggcaacccgt ggtcggctta cggcggtgat tttggcgata cgccgaacga 4500
   tcgccagttc tgtatgaacg gtctggtctt tgccgaccgc acgccgcatc cagcgctgac 4560
   ggaagcaaaa caccagcagc agtttttcca gttccgttta tccgggcaaa ccatcgaagt 4620
   gaccagcgaa tacctgttcc gtcatagcga taacgagctc ctgcactgga tggtggcgct 4680
   ggatggtaag ccgctggcaa gcggtgaagt gcctctggat gtcgctccac aaggtaaaca 4740
   gttgattgaa ctgcctgaac taccgcagcc ggagagcgcc gggcaactct ggctcacagt 4800
   acgcgtagtg caaccgaacg cgaccgcatg gtcagaagcc gggcacatca gcgcctggca 4860
   gcagtggcgt ctggcggaaa acctcagtgt gacgctcccc gccgcgtccc acgccatccc 4920
   gcatctgacc accagcgaaa tggatttttg catcgagctg ggtaataagc gttggcaatt 4980
   taaccgccag tcaggctttc tttcacagat gtggattggc gataaaaaac aactgctgac 5040
   gccgctgcgc gatcagttca cccgtgcacc gctggataac gacattggcg taagtgaagc 5100
   gacccgcatt gaccctaacg cctgggtcga acgctggaag gcggcgggcc attaccaggc 5160
   cgaagcagcg ttgttgcagt gcacggcaga tacacttgct gatgcggtgc tgattacgac 5220
   cgctcacgcg tggcagcatc aggggaaaac cttatttatc agccggaaaa cctaccggat 5280
   tgatggtagt ggtcaaatgg cgattaccgt tgatgttgaa gtggcgagcg atacaccgca 5340
   tccggcgcgg attggcctga actgccagct ggcgcaggta gcagagcggg taaactggct 5400
   cggattaggg ccgcaagaaa actatcccga ccgccttact gccgcctgtt ttgaccgctg 5460
   ggatctgcca ttgtcagaca tgtatacccc gtacgtcttc ccgagcgaaa acggtctgcg 5520
   ctgcgggacg cgcgaattga attatggccc acaccagtgg cgcggcgact tccagttcaa 5580
   catcagccgc tacagtcaac agcaactgat ggaaaccagc catcgccatc tgctgcacgc 5640
   ggaagaaggc acatggctga atatcgacgg tttccatatg gggattggtg gcgacgactc 5700
   ctggagcccg tcagtatcgg cggaattcca gctgagcgcc ggtcgctacc attaccagtt 5760
   ggtctggtgt caaaaataat aataaccggg caggggggat ccgcagatcc ggctgtggaa 5820
   tgtgtgtcag ttagggtgtg gaaagtcccc aggctcccca gcaggcagaa gtatgcaaag 5880
   catgcctgca ggaattcgat atcaagctta tcgataccgt cgacctcgag ggggggcccg 5940
   gtacccagct tttgttccct ttagtgaggg ttaattgcgc gggaagtatt tatcactaat 6000
   caagcacaag taatacatga gaaactttta ctacagcaag cacaatcctc caaaaaattt 6060
   tgtttttaca aaatccctgg tgaacatgat tggaagggac ctactagggt gctgtggaag 6120
   ggtgatggtg cagtagtagt taatgatgaa ggaaagggaa taattgctgt accattaacc 6180
   aggactaagt tactaataaa accaaattga gtattgttgc aggaagcaag acccaactac 6240
   cattgtcagc tgtgtttcct gacctcaata tttgttataa ggtttgatat gaatcccagg 6300
   gggaatctca acccctatta cccaacagtc agaaaaatct aagtgtgagg agaacacaat 6360
   gtttcaacct tattgttata ataatgacag taagaacagc atggcagaat cgaaggaagc 6420
   aagagaccaa gaatgaacct gaaagaagaa tctaaagaag aaaaaagaag aaatgactgg 6480
   tggaaaatag gtatgtttct gttatgctta gcaggaacta ctggaggaat actttggtgg 6540
   tatgaaggac tcccacagca acattatata gggttggtgg cgataggggg aagattaaac 6600
   ggatctggcc aatcaaatgc tatagaatgc tggggttcct tcccggggtg tagaccattt 6660
   caaaattact tcagttatga gaccaataga agcatgcata tggataataa tactgctaca 6720
   ttattagaag ctttaaccaa tataactgct ctataaataa caaaacagaa ttagaaacat 6780
   ggaagttagt aaagacttct ggcataactc ctttacctat ttcttctgaa gctaacactg 6840
   gactaattag acataagaga gattttggta taagtgcaat agtggcagct attgtagccg 6900
   ctactgctat tgctgctagc gctactatgt cttatgttgc tctaactgag gttaacaaaa 6960
   taatggaagt acaaaatcat acttttgagg tagaaaatag tactctaaat ggtatggatt 7020
   taatagaacg acaaataaag atattatatg ctatgattct tcaaacacat gcagatgttc 7080
   aactgttaaa ggaaagacaa caggtagagg agacatttaa tttaattgga tgtatagaaa 7140
   gaacacatgt attttgtcat actggtcatc cctggaatat gtcatgggga catttaaatg 7200
   agtcaacaca atgggatgac tgggtaagca aaatggaaga tttaaatcaa gagatactaa 7260
   ctacacttca tggagccagg aacaatttgg cacaatccat gataacattc aatacaccag 7320
   atagtatagc tcaatttgga aaagaccttt ggagtcatat tggaaattgg attcctggat 7380
   tgggagcttc cattataaaa tatatagtga tgtttttgct tatttatttg ttactaacct 7440
   cttcgcctaa gatcctcagg gccctctgga aggtgaccag tggtgcaggg tcctccggca 7500
   gtcgttacct gaagaaaaaa ttccatcaca aacatgcatc gcgagaagac acctgggacc 7560
   aggcccaaca caacatacac ctagcaggcg tgaccggtgg atcaggggac aaatactaca 7620
   agcagaagta ctccaggaac gactggaatg gagaatcaga ggagtacaac aggcggccaa 7680
   agagctgggt gaagtcaatc gaggcatttg gagagagcta tatttccgag aagaccaaag 7740
   gggagatttc tcagcctggg gcggctatca acgagcacaa gaacggctct ggggggaaca 7800
   atcctcacca agggtcctta gacctggaga ttcgaagcga aggaggaaac atttatgact 7860
   gttgcattaa agcccaagaa ggaactctcg ctatcccttg ctgtggattt cccttatggc 7920
   tattttgggg actagtaatt atagtaggac gcatagcagg ctatggatta cgtggactcg 7980
   ctgttataat aaggatttgt attagaggct taaatttgat atttgaaata atcagaaaaa 8040
   tgcttgatta tattggaaga gctttaaatc ctggcacatc tcatgtatca atgcctcagt 8100
   atgtttagaa aaacaagggg ggaactgtgg ggtttttatg aggggtttta taaatgatta 8160
   taagagtaaa aagaaagttg ctgatgctct cataaccttg tataacccaa aggactagct 8220
   catgttgcta ggcaactaaa ccgcaataac cgcatttgtg acgcgagttc cccattggtg 8280
   acgcgttaac ttcctgtttt tacagtatat aagtgcttgt attctgacaa ttgggcactc 8340
   agattctgcg gtctgagtcc cttctctgct gggctgaaaa ggcctttgta ataaatataa 8400
   ttctctactc agtccctgtc tctagtttgt ctgttcgaga tcctacagag ctcatgcctt 8460
   ggcgtaatca tggtcatagc tgtttcctgt gtgaaattgt tatccgctca caattccaca 8520
   caacatacga gccggaagca taaagtgtaa agcctggggt gcctaatgag tgagctaact 8580
   cacattaatt gcgttgcgct cactgcccgc tttccagtcg ggaaacctgt cgtgccagct 8640
   gcattaatga atcggccaac gcgcggggag aggcggtttg cgtattgggc gctcttccgc 8700
   ttcctcgctc actgactcgc tgcgctcggt cgttcggctg cggcgagcgg tatcagctca 8760
   ctcaaaggcg gtaatacggt tatccacaga atcaggggat aacgcaggaa agaacatgtg 8820
   agcaaaaggc cagcaaaagg ccaggaaccg taaaaaggcc gcgttgctgg cgtttttcca 8880
   taggctccgc ccccctgacg agcatcacaa aaatcgacgc tcaagtcaga ggtggcgaaa 8940
   cccgacagga ctataaagat accaggcgtt tccccctgga agctccctcg tgcgctctcc 9000
   tgttccgacc ctgccgctta ccggatacct gtccgccttt ctcccttcgg gaagcgtggc 9060
   gctttctcat agctcacgct gtaggtatct cagttcggtg taggtcgttc gctccaagct 9120
   gggctgtgtg cacgaacccc ccgttcagcc cgaccgctgc gccttatccg gtaactatcg 9180
   tcttgagtcc aacccggtaa gacacgactt atcgccactg gcagcagcca ctggtaacag 9240
   gattagcaga gcgaggtatg taggcggtgc tacagagttc ttgaagtggt ggcctaacta 9300
   cggctacact agaaggacag tatttggtat ctgcgctctg ctgaagccag ttaccttcgg 9360
   aaaaagagtt ggtagctctt gatccggcaa acaaaccacc gctggtagcg gtggtttttt 9420
   tgtttgcaag cagcagatta cgcgcagaaa aaaaggatct caagaagatc ctttgatctt 9480
   ttctacgggg tctgacgctc agtggaacga aaactcacgt taagggattt tggtcatgag 9540
   attatcaaaa aggatcttca cctagatcct tttaaattaa aaatgaagtt ttaaatcaat 9600
   ctaaagtata tatgagtaaa cttggtctga cagttaccaa tgcttaatca gtgaggcacc 9660
   tatctcagcg atctgtctat ttcgttcatc catagttgcc tgactccccg tcgtgtagat 9720
   aactacgata cgggagggct taccatctgg ccccagtgct gcaatgatac cgcgagaccc 9780
   acgctcaccg gctccagatt tatcagcaat aaaccagcca gccggaaggg ccgagcgcag 9840
   aagtggtcct gcaactttat ccgcctccat ccagtctatt aattgttgcc gggaagctag 9900
   agtaagtagt tcgccagtta atagtttgcg caacgttgtt gccattgcta caggcatcgt 9960
   ggtgtcacgc tcgtcgtttg gtatggcttc attcagctcc ggttcccaac gatcaaggcg 10020
   agttacatga tcccccatgt tgtgcaaaaa agcggttagc tccttcggtc ctccgatcgt 10080
   tgtcagaagt aagttggccg cagtgttatc actcatggtt atggcagcac tgcataattc 10140
   tcttactgtc atgccatccg taagatgctt ttctgtgact ggtgagtact caaccaagtc 10200
   attctgagaa tagtgtatgc ggcgaccgag ttgctcttgc ccggcgtcaa tacgggataa 10260
   taccgcgcca catagcagaa ctttaaaagt gctcatcatt ggaaaacgtt cttcggggcg 10320
   aaaactctca aggatcttac cgctgttgag atccagttcg atgtaaccca ctcgtgcacc 10380
   caactgatct tcagcatctt ttactttcac cagcgtttct gggtgagcaa aaacaggaag 10440
   gcaaaatgcc gcaaaaaagg gaataagggc gacacggaaa tgttgaatac tcatactctt 10500
   cctttttcaa tattattgaa gcatttatca gggttattgt ctcatgagcg gatacatatt 10560
   tgaatgtatt tagaaaaata aacaaatagg ggttccgcgc acatttcccc gaaaagtgcc 10620
   acctaaattg taagcgttaa tattttgtta aaattcgcgt taaatttttg ttaaatcagc 10680
   tcatttttta accaataggc cgaaatcggc aaaatccctt ataaatcaaa agaatagacc 10740
   gagatagggt tgagtgttgt tccagtttgg aacaagagtc cactattaaa gaacgtggac 10800
   tccaacgtca aagggcgaaa aaccgtctat cagggcgatg gcccactacg tgaaccatca 10860
   ccctaatcaa gttttttggg gtcgaggtgc cgtaaagcac taaatcggaa ccctaaaggg 10920
   agcccccgat ttagagcttg acggggaaag ccaacctggc ttatcgaaat taatacgact 10980
   cactataggg agaccggc                                               10998
<210> 11
   <211> 8870
   <212> DNA
   <213> Artificial Sequence
<220>
   <223> pONY8.1Z
<400> 11
   agatcttgaa taataaaatg tgtgtttgtc cgaaatacgc gttttgagat ttctgtcgcc 60
   gactaaattc atgtcgcgcg atagtggtgt ttatcgccga tagagatggc gatattggaa 120
   aaattgatat ttgaaaatat ggcatattga aaatgtcgcc gatgtgagtt tctgtgtaac 180
   tgatatcgcc atttttccaa aagtgatttt tgggcatacg cgatatctgg cgatagcgct 240
   tatatcgttt acgggggatg gcgatagacg actttggtga cttgggcgat tctgtgtgtc 300
   gcaaatatcg cagtttcgat ataggtgaca gacgatatga ggctatatcg ccgatagagg 360
   cgacatcaag ctggcacatg gccaatgcat atcgatctat acattgaatc aatattggcc 420
   attagccata ttattcattg gttatatagc ataaatcaat attggctatt ggccattgca 480
   tacgttgtat ccatatcgta atatgtacat ttatattggc tcatgtccaa cattaccgcc 540
   atgttgacat tgattattga ctagttatta atagtaatca attacggggt cattagttca 600
   tagcccatat atggagttcc gcgttacata acttacggta aatggcccgc ctggctgacc 660
   gcccaacgac ccccgcccat tgacgtcaat aatgacgtat gttcccatag taacgccaat 720
   agggactttc cattgacgtc aatgggtgga gtatttacgg taaactgccc acttggcagt 780
   acatcaagtg tatcatatgc caagtccgcc ccctattgac gtcaatgacg gtaaatggcc 840
   cgcctggcat tatgcccagt acatgacctt acgggacttt cctacttggc agtacatcta 900
   cgtattagtc atcgctatta ccatggtgat gcggttttgg cagtacacca atgggcgtgg 960
   atagcggttt gactcacggg gatttccaag tctccacccc attgacgtca atgggagttt 1020
   gttttggcac caaaatcaac gggactttcc aaaatgtcgt aacaactgcg atcgcccgcc 1080
   ccgttgacgc aaatgggcgg taggcgtgta cggtgggagg tctatataag cagagctcgt 1140
   ttagtgaacc gggcactcag attctgcggt ctgagtccct tctctgctgg gctgaaaagg 1200
   cctttgtaat aaatataatt ctctactcag tccctgtctc tagtttgtct gttcgagatc 1260
   ctacagttgg cgcccgaaca gggacctgag aggggcgcag accctacctg ttgaacctgg 1320
   ctgatcgtag gatccccggg acagcagagg agaacttaca gaagtcttct ggaggtgttc 1380
   ctggccagaa cacaggagga caggtaagat tgggagaccc tttgacattg gagcaaggcg 1440
   ctcaagaagt tagagaaggt gacggtacaa gggtctcaga aattaactac tggtaactgt 1500
   aattgggcgc taagtctagt agacttattt catgatacca actttgtaaa agaaaaggac 1560
   tggcagctga gggatgtcat tccattgctg gaagatgtaa ctcagacgct gtcaggacaa 1620
   gaaagagagg cctttgaaag aacatggtgg gcaatttctg ctgtaaagat gggcctccag 1680
   attaataatg tagtagatgg aaaggcatca ttccagctcc taagagcgaa atatgaaaag 1740
   aagactgcta ataaaaagca gtctgagccc tctgaagaat atctctagaa ctagtggatc 1800
   ccccgggctg caggagtggg gaggcacgat ggccgctttg gtcgaggcgg atccggccat 1860
   tagccatatt attcattggt tatatagcat aaatcaatat tggctattgg ccattgcata 1920
   cgttgtatcc atatcataat atgtacattt atattggctc atgtccaaca ttaccgccat 1980
   gttgacattg attattgact agttattaat agtaatcaat tacggggtca ttagttcata 2040
   gcccatatat ggagttccgc gttacataac ttacggtaaa tggcccgcct ggctgaccgc 2100
   ccaacgaccc ccgcccattg acgtcaataa tgacgtatgt tcccatagta acgccaatag 2160
   ggactttcca ttgacgtcaa tgggtggagt atttacggta aactgcccac ttggcagtac 2220
   atcaagtgta tcatatgcca agtacgcccc ctattgacgt caatgacggt aaatggcccg 2280
   cctggcatta tgcccagtac atgaccttat gggactttcc tacttggcag tacatctacg 2340
   tattagtcat cgctattacc atggtgatgc ggttttggca gtacatcaat gggcgtggat 2400
   agcggtttga ctcacgggga tttccaagtc tccaccccat tgacgtcaat gggagtttgt 2460
   tttggcacca aaatcaacgg gactttccaa aatgtcgtaa caactccgcc ccattgacgc 2520
   aaatgggcgg taggcatgta cggtgggagg tctatataag cagagctcgt ttagtgaacc 2580
   gtcagatcgc ctggagacgc catccacgct gttttgacct ccatagaaga caccgggacc 2640
   gatccagcct ccgcggcccc aagcttcagc tgctcgagga tctgcggatc cggggaattc 2700
   cccagtctca ggatccacca tgggggatcc cgtcgtttta caacgtcgtg actgggaaaa 2760
   ccctggcgtt acccaactta atcgccttgc agcacatccc cctttcgcca gctggcgtaa 2820
   tagcgaagag gcccgcaccg atcgcccttc ccaacagttg cgcagcctga atggcgaatg 2880
   gcgctttgcc tggtttccgg caccagaagc ggtgccggaa agctggctgg agtgcgatct 2940
   tcctgaggcc gatactgtcg tcgtcccctc aaactggcag atgcacggtt acgatgcgcc 3000
   catctacacc aacgtaacct atcccattac ggtcaatccg ccgtttgttc ccacggagaa 3060
   tccgacgggt tgttactcgc tcacatttaa tgttgatgaa agctggctac aggaaggcca 3120
   gacgcgaatt atttttgatg gcgttaactc ggcgtttcat ctgtggtgca acgggcgctg 3180
   ggtcggttac ggccaggaca gtcgtttgcc gtctgaattt gacctgagcg catttttacg 3240
   cgccggagaa aaccgcctcg cggtgatggt gctgcgttgg agtgacggca gttatctgga 3300
   agatcaggat atgtggcgga tgagcggcat tttccgtgac gtctcgttgc tgcataaacc 3360
   gactacacaa atcagcgatt tccatgttgc cactcgcttt aatgatgatt tcagccgcgc 3420
   tgtactggag gctgaagttc agatgtgcgg cgagttgcgt gactacctac gggtaacagt 3480
   ttctttatgg cagggtgaaa cgcaggtcgc cagcggcacc gcgcctttcg gcggtgaaat 3540
   tatcgatgag cgtggtggtt atgccgatcg cgtcacacta cgtctgaacg tcgaaaaccc 3600
   gaaactgtgg agcgccgaaa tcccgaatct ctatcgtgcg gtggttgaac tgcacaccgc 3660
   cgacggcacg ctgattgaag cagaagcctg cgatgtcggt ttccgcgagg tgcggattga 3720
   aaatggtctg ctgctgctga acggcaagcc gttgctgatt cgaggcgtta accgtcacga 3780
   gcatcatcct ctgcatggtc aggtcatgga tgagcagacg atggtgcagg atatcctgct 3840
   gatgaagcag aacaacttta acgccgtgcg ctgttcgcat tatccgaacc atccgctgtg 3900
   gtacacgctg tgcgaccgct acggcctgta tgtggtggat gaagccaata ttgaaaccca 3960
   cggcatggtg ccaatgaatc gtctgaccga tgatccgcgc tggctaccgg cgatgagcga 4020
   acgcgtaacg cgaatggtgc agcgcgatcg taatcacccg agtgtgatca tctggtcgct 4080
   ggggaatgaa tcaggccacg gcgctaatca cgacgcgctg tatcgctgga tcaaatctgt 4140
   cgatccttcc cgcccggtgc agtatgaagg cggcggagcc gacaccacgg ccaccgatat 4200
   tatttgcccg atgtacgcgc gcgtggatga agaccagccc ttcccggctg tgccgaaatg 4260
   gtccatcaaa aaatggcttt cgctacctgg agagacgcgc ccgctgatcc tttgcgaata 4320
   cgcccacgcg atgggtaaca gtcttggcgg tttcgctaaa tactggcagg cgtttcgtca 4380
   gtatccccgt ttacagggcg gcttcgtctg ggactgggtg gatcagtcgc tgattaaata 4440
   tgatgaaaac ggcaacccgt ggtcggctta cggcggtgat tttggcgata cgccgaacga 4500
   tcgccagttc tgtatgaacg gtctggtctt tgccgaccgc acgccgcatc cagcgctgac 4560
   ggaagcaaaa caccagcagc agtttttcca gttccgttta tccgggcaaa ccatcgaagt 4620
   gaccagcgaa tacctgttcc gtcatagcga taacgagctc ctgcactgga tggtggcgct 4680
   ggatggtaag ccgctggcaa gcggtgaagt gcctctggat gtcgctccac aaggtaaaca 4740
   gttgattgaa ctgcctgaac taccgcagcc ggagagcgcc gggcaactct ggctcacagt 4800
   acgcgtagtg caaccgaacg cgaccgcatg gtcagaagcc gggcacatca gcgcctggca 4860
   gcagtggcgt ctggcggaaa acctcagtgt gacgctcccc gccgcgtccc acgccatccc 4920
   gcatctgacc accagcgaaa tggatttttg catcgagctg ggtaataagc gttggcaatt 4980
   taaccgccag tcaggctttc tttcacagat gtggattggc gataaaaaac aactgctgac 5040
   gccgctgcgc gatcagttca cccgtgcacc gctggataac gacattggcg taagtgaagc 5100
   gacccgcatt gaccctaacg cctgggtcga acgctggaag gcggcgggcc attaccaggc 5160
   cgaagcagcg ttgttgcagt gcacggcaga tacacttgct gatgcggtgc tgattacgac 5220
   cgctcacgcg tggcagcatc aggggaaaac cttatttatc agccggaaaa cctaccggat 5280
   tgatggtagt ggtcaaatgg cgattaccgt tgatgttgaa gtggcgagcg atacaccgca 5340
   tccggcgcgg attggcctga actgccagct ggcgcaggta gcagagcggg taaactggct 5400
   cggattaggg ccgcaagaaa actatcccga ccgccttact gccgcctgtt ttgaccgctg 5460
   ggatctgcca ttgtcagaca tgtatacccc gtacgtcttc ccgagcgaaa acggtctgcg 5520
   ctgcgggacg cgcgaattga attatggccc acaccagtgg cgcggcgact tccagttcaa 5580
   catcagccgc tacagtcaac agcaactgat ggaaaccagc catcgccatc tgctgcacgc 5640
   ggaagaaggc acatggctga atatcgacgg tttccatatg gggattggtg gcgacgactc 5700
   ctggagcccg tcagtatcgg cggaattcca gctgagcgcc ggtcgctacc attaccagtt 5760
   ggtctggtgt caaaaataat aataaccggg caggggggat ccgcagatcc ggctgtggaa 5820
   tgtgtgtcag ttagggtgtg gaaagtcccc aggctcccca gcaggcagaa gtatgcaaag 5880
   catgcctgca ggaattcgat atcaagctta tcgataccgt cgaattggaa gagctttaaa 5940
   tcctggcaca tctcatgtat caatgcctca gtatgtttag aaaaacaagg ggggaactgt 6000
   ggggttttta tgaggggttt tataaatgat tataagagta aaaagaaagt tgctgatgct 6060
   ctcataacct tgtataaccc aaaggactag ctcatgttgc taggcaacta aaccgcaata 6120
   accgcatttg tgacgcgagt tccccattgg tgacgcgtta acttcctgtt tttacagtat 6180
   ataagtgctt gtattctgac aattgggcac tcagattctg cggtctgagt cccttctctg 6240
   ctgggctgaa aaggcctttg taataaatat aattctctac tcagtccctg tctctagttt 6300
   gtctgttcga gatcctacag agctcatgcc ttggcgtaat catggtcata gctgtttcct 6360
   gtgtgaaatt gttatccgct cacaattcca cacaacatac gagccggaag cataaagtgt 6420
   aaagcctggg gtgcctaatg agtgagctaa ctcacattaa ttgcgttgcg ctcactgccc 6480
   gctttccagt cgggaaacct gtcgtgccag ctgcattaat gaatcggcca acgcgcgggg 6540
   agaggcggtt tgcgtattgg gcgctcttcc gcttcctcgc tcactgactc gctgcgctcg 6600
   gtcgttcggc tgcggcgagc ggtatcagct cactcaaagg cggtaatacg gttatccaca 6660
   gaatcagggg ataacgcagg aaagaacatg tgagcaaaag gccagcaaaa ggccaggaac 6720
   cgtaaaaagg ccgcgttgct ggcgtttttc cataggctcc gcccccctga cgagcatcac 6780
   aaaaatcgac gctcaagtca gaggtggcga aacccgacag gactataaag ataccaggcg 6840
   tttccccctg gaagctccct cgtgcgctct cctgttccga ccctgccgct taccggatac 6900
   ctgtccgcct ttctcccttc gggaagcgtg gcgctttctc atagctcacg ctgtaggtat 6960
   ctcagttcgg tgtaggtcgt tcgctccaag ctgggctgtg tgcacgaacc ccccgttcag 7020
   cccgaccgct gcgccttatc cggtaactat cgtcttgagt ccaacccggt aagacacgac 7080
   ttatcgccac tggcagcagc cactggtaac aggattagca gagcgaggta tgtaggcggt 7140
   gctacagagt tcttgaagtg gtggcctaac tacggctaca ctagaaggac agtatttggt 7200
   atctgcgctc tgctgaagcc agttaccttc ggaaaaagag ttggtagctc ttgatccggc 7260
   aaacaaacca ccgctggtag cggtggtttt tttgtttgca agcagcagat tacgcgcaga 7320
   aaaaaaggat ctcaagaaga tcctttgatc ttttctacgg ggtctgacgc tcagtggaac 7380
   gaaaactcac gttaagggat tttggtcatg agattatcaa aaaggatctt cacctagatc 7440
   cttttaaatt aaaaatgaag ttttaaatca atctaaagta tatatgagta aacttggtct 7500
   gacagttacc aatgcttaat cagtgaggca cctatctcag cgatctgtct atttcgttca 7560
   tccatagttg cctgactccc cgtcgtgtag ataactacga tacgggaggg cttaccatct 7620
   ggccccagtg ctgcaatgat accgcgagac ccacgctcac cggctccaga tttatcagca 7680
   ataaaccagc cagccggaag ggccgagcgc agaagtggtc ctgcaacttt atccgcctcc 7740
   atccagtcta ttaattgttg ccgggaagct agagtaagta gttcgccagt taatagtttg 7800
   cgcaacgttg ttgccattgc tacaggcatc gtggtgtcac gctcgtcgtt tggtatggct 7860
   tcattcagct ccggttccca acgatcaagg cgagttacat gatcccccat gttgtgcaaa 7920
   aaagcggtta gctccttcgg tcctccgatc gttgtcagaa gtaagttggc cgcagtgtta 7980
   tcactcatgg ttatggcagc actgcataat tctcttactg tcatgccatc cgtaagatgc 8040
   ttttctgtga ctggtgagta ctcaaccaag tcattctgag aatagtgtat gcggcgaccg 8100
   agttgctctt gcccggcgtc aatacgggat aataccgcgc cacatagcag aactttaaaa 8160
   gtgctcatca ttggaaaacg ttcttcgggg cgaaaactct caaggatctt accgctgttg 8220
   agatccagtt cgatgtaacc cactcgtgca cccaactgat cttcagcatc ttttactttc 8280
   accagcgttt ctgggtgagc aaaaacagga aggcaaaatg ccgcaaaaaa gggaataagg 8340
   gcgacacgga aatgttgaat actcatactc ttcctttttc aatattattg aagcatttat 8400
   cagggttatt gtctcatgag cggatacata tttgaatgta tttagaaaaa taaacaaata 8460
   ggggttccgc gcacatttcc ccgaaaagtg ccacctaaat tgtaagcgtt aatattttgt 8520
   taaaattcgc gttaaatttt tgttaaatca gctcattttt taaccaatag gccgaaatcg 8580
   gcaaaatccc ttataaatca aaagaataga ccgagatagg gttgagtgtt gttccagttt 8640
   ggaacaagag tccactatta aagaacgtgg actccaacgt caaagggcga aaaaccgtct 8700
   atcagggcga tggcccacta cgtgaaccat caccctaatc aagttttttg gggtcgaggt 8760
   gccgtaaagc actaaatcgg aaccctaaag ggagcccccg atttagagct tgacggggaa 8820
   agccaacctg gcttatcgaa attaatacga ctcactatag ggagaccggc            8870
<210> 12
   <211> 12481
   <212> DNA
   <213> Artificial Sequence
<220>
   <223> pONY3.1
<400> 12
   agatcttcaa tattggccat tagccatatt attcattggt tatatagcat aaatcaatat 60
   tggctattgg ccattgcata cgttgtatct atatcataat atgtacattt atattggctc 120
   atgtccaata tgaccgccat gttggcattg attattgact agttattaat agtaatcaat 180
   tacggggtca ttagttcata gcccatatat ggagttccgc gttacataac ttacggtaaa 240
   tggcccgcct ggctgaccgc ccaacgaccc ccgcccattg acgtcaataa tgacgtatgt 300
   tcccatagta acgccaatag ggactttcca ttgacgtcaa tgggtggagt atttacggta 360
   aactgcccac ttggcagtac atcaagtgta tcatatgcca agtccgcccc ctattgacgt 420
   caatgacggt aaatggcccg cctggcatta tgcccagtac atgaccttac gggactttcc 480
   tacttggcag tacatctacg tattagtcat cgctattacc atggtgatgc ggttttggca 540
   gtacaccaat gggcgtggat agcggtttga ctcacgggga tttccaagtc tccaccccat 600
   tgacgtcaat gggagtttgt tttggcacca aaatcaacgg gactttccaa aatgtcgtaa 660
   caactgcgat cgcccgcccc gttgacgcaa atgggcggta ggcgtgtacg gtgggaggtc 720
   tatataagca gagctcgttt agtgaaccgt cagatcacta gaagctttat tgcggtagtt 780
   tatcacagtt aaattgctaa cgcagtcagt gcttctgaca caacagtctc gaacttaagc 840
   tgcagtgact ctcttaaggt agccttgcag aagttggtcg tgaggcactg ggcaggtaag 900
   tatcaaggtt acaagacagg tttaaggaga ccaatagaaa ctgggcttgt cgagacagag 960
   aagactcttg cgtttctgat aggcacctat tggtcttact gacatccact ttgcctttct 1020
   ctccacaggt gtccactccc agttcaatta cagctcttaa ggctagagta cttaatacga 1080
   ctcactatag gctagcctcg aggtcgacgg tatcgcccga acagggacct gagaggggcg 1140
   cagaccctac ctgttgaacc tggctgatcg taggatcccc gggacagcag aggagaactt 1200
   acagaagtct tctggaggtg ttcctggcca gaacacagga ggacaggtaa gatgggagac 1260
   cctttgacat ggagcaaggc gctcaagaag ttagagaagg tgacggtaca agggtctcag 1320
   aaattaacta ctggtaactg taattgggcg ctaagtctag tagacttatt tcatgatacc 1380
   aactttgtaa aagaaaagga ctggcagctg agggatgtca ttccattgct ggaagatgta 1440
   actcagacgc tgtcaggaca agaaagagag gcctttgaaa gaacatggtg ggcaatttct 1500
   gctgtaaaga tgggcctcca gattaataat gtagtagatg gaaaggcatc attccagctc 1560
   ctaagagcga aatatgaaaa gaagactgct aataaaaagc agtctgagcc ctctgaagaa 1620
   tatccaatca tgatagatgg ggctggaaac agaaatttta gacctctaac acctagagga 1680
   tatactactt gggtgaatac catacagaca aatggtctat taaatgaagc tagtcaaaac 1740
   ttatttggga tattatcagt agactgtact tctgaagaaa tgaatgcatt tttggatgtg 1800
   gtacctggcc aggcaggaca aaagcagata ttacttgatg caattgataa gatagcagat 1860
   gattgggata atagacatcc attaccgaat gctccactgg tggcaccacc acaagggcct 1920
   attcccatga cagcaaggtt tattagaggt ttaggagtac ctagagaaag acagatggag 1980
   cctgcttttg atcagtttag gcagacatat agacaatgga taatagaagc catgtcagaa 2040
   ggcatcaaag tgatgattgg aaaacctaaa gctcaaaata ttaggcaagg agctaaggaa 2100
   ccttacccag aatttgtaga cagactatta tcccaaataa aaagtgaggg acatccacaa 2160
   gagatttcaa aattcttgac tgatacactg actattcaga acgcaaatga ggaatgtaga 2220
   aatgctatga gacatttaag accagaggat acattagaag agaaaatgta tgcttgcaga 2280
   gacattggaa ctacaaaaca aaagatgatg ttattggcaa aagcacttca gactggtctt 2340
   gcgggcccat ttaaaggtgg agccttgaaa ggagggccac taaaggcagc acaaacatgt 2400
   tataactgtg ggaagccagg acatttatct agtcaatgta gagcacctaa agtctgtttt 2460
   aaatgtaaac agcctggaca tttctcaaag caatgcagaa gtgttccaaa aaacggaaag 2520
   caaggggctc aagggaggcc ccagaaacaa actttcccga tacaacagaa gagtcagcac 2580
   aacaaatctg ttgtacaaga gactcctcag actcaaaatc tgtacccaga tctgagcgaa 2640
   ataaaaaagg aatacaatgt caaggagaag gatcaagtag aggatctcaa cctggacagt 2700
   ttgtgggagt aacatataat ctagagaaaa ggcctactac aatagtatta attaatgata 2760
   ctcccttaaa tgtactgtta gacacaggag cagatacttc agtgttgact actgcacatt 2820
   ataataggtt aaaatataga gggagaaaat atcaagggac gggaataata ggagtgggag 2880
   gaaatgtgga aacattttct acgcctgtga ctataaagaa aaagggtaga cacattaaga 2940
   caagaatgct agtggcagat attccagtga ctattttggg acgagatatt cttcaggact 3000
   taggtgcaaa attggttttg gcacagctct ccaaggaaat aaaatttaga aaaatagagt 3060
   taaaagaggg cacaatgggg ccaaaaattc ctcaatggcc actcactaag gagaaactag 3120
   aaggggccaa agagatagtc caaagactat tgtcagaggg aaaaatatca gaagctagtg 3180
   acaataatcc ttataattca cccatatttg taataaaaaa gaggtctggc aaatggaggt 3240
   tattacaaga tctgagagaa ttaaacaaaa cagtacaagt aggaacggaa atatccagag 3300
   gattgcctca cccgggagga ttaattaaat gtaaacacat gactgtatta gatattggag 3360
   atgcatattt cactataccc ttagatccag agtttagacc atatacagct ttcactattc 3420
   cctccattaa tcatcaagaa ccagataaaa gatatgtgtg gaaatgttta ccacaaggat 3480
   tcgtgttgag cccatatata tatcagaaaa cattacagga aattttacaa ccttttaggg 3540
   aaagatatcc tgaagtacaa ttgtatcaat atatggatga tttgttcatg ggaagtaatg 3600
   gttctaaaaa acaacacaaa gagttaatca tagaattaag ggcgatctta ctggaaaagg 3660
   gttttgagac accagatgat aaattacaag aagtgccacc ttatagctgg ctaggttatc 3720
   aactttgtcc tgaaaattgg aaagtacaaa aaatgcaatt agacatggta aagaatccaa 3780
   cccttaatga tgtgcaaaaa ttaatgggga atataacatg gatgagctca gggatcccag 3840
   ggttgacagt aaaacacatt gcagctacta ctaagggatg tttagagttg aatcaaaaag 3900
   taatttggac ggaagaggca caaaaagagt tagaagaaaa taatgagaag attaaaaatg 3960
   ctcaagggtt acaatattat aatccagaag aagaaatgtt atgtgaggtt gaaattacaa 4020
   aaaattatga ggcaacttat gttataaaac aatcacaagg aatcctatgg gcaggtaaaa 4080
   agattatgaa ggctaataag ggatggtcaa cagtaaaaaa tttaatgtta ttgttgcaac 4140
   atgtggcaac agaaagtatt actagagtag gaaaatgtcc aacgtttaag gtaccattta 4200
   ccaaagagca agtaatgtgg gaaatgcaaa aaggatggta ttattcttgg ctcccagaaa 4260
   tagtatatac acatcaagta gttcatgatg attggagaat gaaattggta gaagaaccta 4320
   catcaggaat aacaatatac actgatgggg gaaaacaaaa tggagaagga atagcagctt 4380
   atgtgaccag taatgggaga actaaacaga aaaggttagg acctgtcact catcaagttg 4440
   ctgaaagaat ggcaatacaa atggcattag aggataccag agataaacaa gtaaatatag 4500
   taactgatag ttattattgt tggaaaaata ttacagaagg attaggttta gaaggaccac 4560
   aaagtccttg gtggcctata atacaaaata tacgagaaaa agagatagtt tattttgctt 4620
   gggtacctgg tcacaaaggg atatatggta atcaattggc agatgaagcc gcaaaaataa 4680
   aagaagaaat catgctagca taccaaggca cacaaattaa agagaaaaga gatgaagatg 4740
   cagggtttga cttatgtgtt ccttatgaca tcatgatacc tgtatctgac acaaaaatca 4800
   tacccacaga tgtaaaaatt caagttcctc ctaatagctt tggatgggtc actgggaaat 4860
   catcaatggc aaaacagggg ttattaatta atggaggaat aattgatgaa ggatatacag 4920
   gagaaataca agtgatatgt actaatattg gaaaaagtaa tattaaatta atagagggac 4980
   aaaaatttgc acaattaatt atactacagc atcactcaaa ttccagacag ccttgggatg 5040
   aaaataaaat atctcagaga ggggataaag gatttggaag tacaggagta ttctgggtag 5100
   aaaatattca ggaagcacaa gatgaacatg agaattggca tacatcacca aagatattgg 5160
   caagaaatta taagatacca ttgactgtag caaaacagat aactcaagaa tgtcctcatt 5220
   gcactaagca aggatcagga cctgcaggtt gtgtcatgag atctcctaat cattggcagg 5280
   cagattgcac acatttggac aataagataa tattgacttt tgtagagtca aattcaggat 5340
   acatacatgc tacattattg tcaaaagaaa atgcattatg tacttcattg gctattttag 5400
   aatgggcaag attgttttca ccaaagtcct tacacacaga taacggcact aattttgtgg 5460
   cagaaccagt tgtaaatttg ttgaagttcc taaagatagc acataccaca ggaataccat 5520
   atcatccaga aagtcagggt attgtagaaa gggcaaatag gaccttgaaa gagaagattc 5580
   aaagtcatag agacaacact caaacactgg aggcagcttt acaacttgct ctcattactt 5640
   gtaacaaagg gagggaaagt atgggaggac agacaccatg ggaagtattt atcactaatc 5700
   aagcacaagt aatacatgag aaacttttac tacagcaagc acaatcctcc aaaaaatttt 5760
   gtttttacaa aatccctggt gaacatgatt ggaagggacc tactagggtg ctgtggaagg 5820
   gtgatggtgc agtagtagtt aatgatgaag gaaagggaat aattgctgta ccattaacca 5880
   ggactaagtt actaataaaa ccaaattgag tattgttgca ggaagcaaga cccaactacc 5940
   attgtcagct gtgtttcctg aggtctctag gaattgatta cctcgatgct tcattaagga 6000
   agaagaataa acaaagactg aaggcaatcc aacaaggaag acaacctcaa tatttgttat 6060
   aaggtttgat atatgggagt atttggtaaa ggggtaacat ggtcagcatc gcattctatg 6120
   ggggaatccc agggggaatc tcaaccccta ttacccaaca gtcagaaaaa tctaagtgtg 6180
   aggagaacac aatgtttcaa ccttattgtt ataataatga cagtaagaac agcatggcag 6240
   aatcgaagga agcaagagac caagaaatga acctgaaaga agaatctaaa gaagaaaaaa 6300
   gaagaaatga ctggtggaaa ataggtatgt ttctgttatg cttagcagga actactggag 6360
   gaatactttg gtggtatgaa ggactcccac agcaacatta tatagggttg gtggcgatag 6420
   ggggaagatt aaacggatct ggccaatcaa atgctataga atgctggggt tccttcccgg 6480
   ggtgtagacc atttcaaaat tacttcagtt atgagaccaa tagaagcatg catatggata 6540
   ataatactgc tacattatta gaagctttaa ccaatataac tgctctataa ataacaaaac 6600
   agaattagaa acatggaagt tagtaaagac ttctggcata actcctttac ctatttcttc 6660
   tgaagctaac actggactaa ttagacataa gagagatttt ggtataagtg caatagtggc 6720
   agctattgta gccgctactg ctattgctgc tagcgctact atgtcttatg ttgctctaac 6780
   tgaggttaac aaaataatgg aagtacaaaa tcatactttt gaggtagaaa atagtactct 6840
   aaatggtatg gatttaatag aacgacaaat aaagatatta tatgctatga ttcttcaaac 6900
   acatgcagat gttcaactgt taaaggaaag acaacaggta gaggagacat ttaatttaat 6960
   tggatgtata gaaagaacac atgtattttg tcatactggt catccctgga atatgtcatg 7020
   ggacatttta aatgagtcaa cacaatggga tgactgggta agcaaaatgg aagatttaaa 7080
   tcaagagata ctaactacac ttcatggagc caggaacaat ttggcacaat ccatgataac 7140
   attcaataca ccagatagta tagctcaatt tggaaaagac ctttggagtc atattggaaa 7200
   ttggattcct ggattgggag cttccattat aaaatatata gtgatgtttt tgcttattta 7260
   tttgttacta acctcttcgc ctaagatcct cagggccctc tggaaggtga ccagtggtgc 7320
   agggtcctcc ggcagtcgtt acctgaagaa aaaattccat cacaaacatg catcgcgaga 7380
   agacacctgg gaccaggccc aacacaacat acacctagca ggcgtgaccg gtggatcagg 7440
   ggacaaatac tacaagcaga agtactccag gaacgactgg aatggagaat cagaggagta 7500
   caacaggcgg ccaaagagct gggtgaagtc aatcgaggca tttggagaga gctatatttc 7560
   cgagaagacc aaaggggaga tttctcagcc tggggcggct atcaacgagc acaagaacgg 7620
   ctctgggggg aacaatcctc accaagggtc cttagacctg gagattcgaa gcgaaggagg 7680
   aaacatttat gactgttgca ttaaagccca agaaggaact ctcgctatcc cttgctgtgg 7740
   atttccctta tggctatttt ggggactagt aattatagta ggacgcatag caggctatgg 7800
   attacgtgga ctcgctgtta taataaggat ttgtattaga ggcttaaatt tgatatttga 7860
   aataatcaga aaaatgcttg attatattgg aagagcttta aatcctggca catctcatgt 7920
   atcaatgcct cagtatgttt agaaaaacaa ggggggaact gtggggtttt tatgaggggt 7980
   tttataaatg attataagag taaaaagaaa gttgctgatg ctctcataac cttgtataac 8040
   ccaaaggact agctcatgtt gctaggcaac taaaccgcaa taaccgcatt tgtgacgcga 8100
   gttccccatt ggtgacgcgt ggtacctcta gagtcgaccc gggcggccgc ttccctttag 8160
   tgagggttaa tgcttcgagc agacatgata agatacattg atgagtttgg acaaaccaca 8220
   actagaatgc agtgaaaaaa atgctttatt tgtgaaattt gtgatgctat tgctttattt 8280
   gtaaccatta taagctgcaa taaacaagtt aacaacaaca attgcattca ttttatgttt 8340
   caggttcagg gggagatgtg ggaggttttt taaagcaagt aaaacctcta caaatgtggt 8400
   aaaatccgat aaggatcgat ccgggctggc gtaatagcga agaggcccgc accgatcgcc 8460
   cttcccaaca gttgcgcagc ctgaatggcg aatggacgcg ccctgtagcg gcgcattaag 8520
   cgcggcgggt gtggtggtta cgcgcagcgt gaccgctaca cttgccagcg ccctagcgcc 8580
   cgctcctttc gctttcttcc cttcctttct cgccacgttc gccggctttc cccgtcaagc 8640
   tctaaatcgg gggctccctt tagggttccg atttagagct ttacggcacc tcgaccgcaa 8700
   aaaacttgat ttgggtgatg gttcacgtag tgggccatcg ccctgataga cggtttttcg 8760
   ccctttgacg ttggagtcca cgttctttaa tagtggactc ttgttccaaa ctggaacaac 8820
   actcaaccct atctcggtct attcttttga tttataaggg attttgccga tttcggccta 8880
   ttggttaaaa aatgagctga tttaacaaat atttaacgcg aattttaaca aaatattaac 8940
   gtttacaatt tcgcctgatg cggtattttc tccttacgca tctgtgcggt atttcacacc 9000
   gcatacgcgg atctgcgcag caccatggcc tgaaataacc tctgaaagag gaacttggtt 9060
   aggtaccttc tgaggcggaa agaaccagct gtggaatgtg tgtcagttag ggtgtggaaa 9120
   gtccccaggc tccccagcag gcagaagtat gcaaagcatg catctcaatt agtcagcaac 9180
   caggtgtgga aagtccccag gctccccagc aggcagaagt atgcaaagca tgcatctcaa 9240
   ttagtcagca accatagtcc cgcccctaac tccgcccatc ccgcccctaa ctccgcccag 9300
   ttccgcccat tctccgcccc atggctgact aatttttttt atttatgcag aggccgaggc 9360
   cgcctcggcc tctgagctat tccagaagta gtgaggaggc ttttttggag gcctaggctt 9420
   ttgcaaaaag cttgattctt ctgacacaac agtctcgaac ttaaggctag agccaccatg 9480
   attgaacaag atggattgca cgcaggttct ccggccgctt gggtggagag gctattcggc 9540
   tatgactggg cacaacagac aatcggctgc tctgatgccg ccgtgttccg gctgtcagcg 9600
   caggggcgcc cggttctttt tgtcaagacc gacctgtccg gtgccctgaa tgaactgcag 9660
   gacgaggcag cgcggctatc gtggctggcc acgacgggcg ttccttgcgc agctgtgctc 9720
   gacgttgtca ctgaagcggg aagggactgg ctgctattgg gcgaagtgcc ggggcaggat 9780
   ctcctgtcat ctcaccttgc tcctgccgag aaagtatcca tcatggctga tgcaatgcgg 9840
   cggctgcata cgcttgatcc ggctacctgc ccattcgacc accaagcgaa acatcgcatc 9900
   gagcgagcac gtactcggat ggaagccggt cttgtcgatc aggatgatct ggacgaagag 9960
   catcaggggc tcgcgccagc cgaactgttc gccaggctca aggcgcgcat gcccgacggc 10020
   gaggatctcg tcgtgaccca tggcgatgcc tgcttgccga atatcatggt ggaaaatggc 10080
   cgcttttctg gattcatcga ctgtggccgg ctgggtgtgg cggaccgcta tcaggacata 10140
   gcgttggcta cccgtgatat tgctgaagag cttggcggcg aatgggctga ccgcttcctc 10200
   gtgctttacg gtatcgccgc tcccgattcg cagcgcatcg ccttctatcg ccttcttgac 10260
   gagttcttct gagcgggact ctggggttcg aaatgaccga ccaagcgacg cccaacctgc 10320
   catcacgatg gccgcaataa aatatcttta ttttcattac atctgtgtgt tggttttttg 10380
   tgtgaatcga tagcgataag gatccgcgta tggtgcactc tcagtacaat ctgctctgat 10440
   gccgcatagt taagccagcc ccgacacccg ccaacacccg ctgacgcgcc ctgacgggct 10500
   tgtctgctcc cggcatccgc ttacagacaa gctgtgaccg tctccgggag ctgcatgtgt 10560
   cagaggtttt caccgtcatc accgaaacgc gcgagacgaa agggcctcgt gatacgccta 10620
   tttttatagg ttaatgtcat gataataatg gtttcttaga cgtcaggtgg cacttttcgg 10680
   ggaaatgtgc gcggaacccc tatttgttta tttttctaaa tacattcaaa tatgtatccg 10740
   ctcatgagac aataaccctg ataaatgctt caataatatt gaaaaaggaa gagtatgagt 10800
   attcaacatt tccgtgtcgc ccttattccc ttttttgcgg cattttgcct tcctgttttt 10860
   gctcacccag aaacgctggt gaaagtaaaa gatgctgaag atcagttggg tgcacgagtg 10920
   ggttacatcg aactggatct caacagcggt aagatccttg agagttttcg ccccgaagaa 10980
   cgttttccaa tgatgagcac ttttaaagtt ctgctatgtg gcgcggtatt atcccgtatt 11040
   gacgccgggc aagagcaact cggtcgccgc atacactatt ctcagaatga cttggttgag 11100
   tactcaccag tcacagaaaa gcatcttacg gatggcatga cagtaagaga attatgcagt 11160
   gctgccataa ccatgagtga taacactgcg gccaacttac ttctgacaac gatcggagga 11220
   ccgaaggagc taaccgcttt tttgcacaac atgggggatc atgtaactcg ccttgatcgt 11280
   tgggaaccgg agctgaatga agccatacca aacgacgagc gtgacaccac gatgcctgta 11340
   gcaatggcaa caacgttgcg caaactatta actggcgaac tacttactct agcttcccgg 11400
   caacaattaa tagactggat ggaggcggat aaagttgcag gaccacttct gcgctcggcc 11460
   cttccggctg gctggtttat tgctgataaa tctggagccg gtgagcgtgg gtctcgcggt 11520
   atcattgcag cactggggcc agatggtaag ccctcccgta tcgtagttat ctacacgacg 11580
   gggagtcagg caactatgga tgaacgaaat agacagatcg ctgagatagg tgcctcactg 11640
   attaagcatt ggtaactgtc agaccaagtt tactcatata tactttagat tgatttaaaa 11700
   cttcattttt aatttaaaag gatctaggtg aagatccttt ttgataatct catgaccaaa 11760
   atcccttaac gtgagttttc gttccactga gcgtcagacc ccgtagaaaa gatcaaagga 11820
   tcttcttgag atcctttttt tctgcgcgta atctgctgct tgcaaacaaa aaaaccaccg 11880
   ctaccagcgg tggtttgttt gccggatcaa gagctaccaa ctctttttcc gaaggtaact 11940
   ggcttcagca gagcgcagat accaaatact gtccttctag tgtagccgta gttaggccac 12000
   cacttcaaga actctgtagc accgcctaca tacctcgctc tgctaatcct gttaccagtg 12060
   gctgctgcca gtggcgataa gtcgtgtctt accgggttgg actcaagacg atagttaccg 12120
   gataaggcgc agcggtcggg ctgaacgggg ggttcgtgca cacagcccag cttggagcga 12180
   acgacctaca ccgaactgag atacctacag cgtgagctat gagaaagcgc cacgcttccc 12240
   gaagggagaa aggcggacag gtatccggta agcggcaggg tcggaacagg agagcgcacg 12300
   agggagcttc cagggggaaa cgcctggtat ctttatagtc ctgtcgggtt tcgccacctc 12360
   tgacttgagc gtcgattttt gtgatgctcg tcaggggggc ggagcctatg gaaaaacgcc 12420
   agcaacgcgg cctttttacg gttcctggcc ttttgctggc cttttgctca catggctcga 12480
   c                                                                 12481
<210> 13
   <211> 6395
   <212> DNA
   <213> Artificial Sequence
<220>
   <223> pCIneoERev
<400> 13
   tgaataataa aatgtgtgtt tgtccgaaat acgcgttttg agatttctgt cgccgactaa 60
   attcatgtcg cgcgatagtg gtgtttatcg ccgatagaga tggcgatatt ggaaaaattg 120
   atatttgaaa atatggcata ttgaaaatgt cgccgatgtg agtttctgtg taactgatat 180
   cgccattttt ccaaaagtga tttttgggca tacgcgatat ctggcgatag cgcttatatc 240
   gtttacgggg gatggcgata gacgactttg gtgacttggg cgattctgtg tgtcgcaaat 300
   atcgcagttt cgatataggt gacagacgat atgaggctat atcgccgata gaggcgacat 360
   caagctggca catggccaat gcatatcgat ctatacattg aatcaatatt ggccattagc 420
   catattattc attggttata tagcataaat caatattggc tattggccat tgcatacgtt 480
   gtatccatat cgtaatatgt acatttatat tggctcatgt ccaacattac cgccatgttg 540
   acattgatta ttgactagtt attaatagta atcaattacg gggtcattag ttcatagccc 600
   atatatggag ttccgcgtta cataacttac ggtaaatggc ccgcctggct gaccgcccaa 660
   cgacccccgc ccattgacgt caataatgac gtatgttccc atagtaacgc caatagggac 720
   tttccattga cgtcaatggg tggagtattt acggtaaact gcccacttgg cagtacatca 780
   agtgtatcat atgccaagtc cgccccctat tgacgtcaat gacggtaaat ggcccgcctg 840
   gcattatgcc cagtacatga ccttacggga ctttcctact tggcagtaca tctacgtatt 900
   agtcatcgct attaccatgg tgatgcggtt ttggcagtac accaatgggc gtggatagcg 960
   gtttgactca cggggatttc caagtctcca ccccattgac gtcaatggga gtttgttttg 1020
   gcaccaaaat caacggactt ttccaaaatg tcgtaacaac tgcgatcgcc cgccccgttg 1080
   acgcaaatgg gcggtaggcg tgtacggtgg gaggtctata taagcagagc tcgtttagtg 1140
   aaccgtcaga tcactagaag ctttattgcg gtagtttatc acagttaaat tgctaacgca 1200
   gtcagtgctt ctgacacaac agtctcgaac ttaagctgca gtgactctct taaggtagcc 1260
   ttgcagaagt tggtcgtgag gcactgggca ggtaagtatc aaggttacaa gacaggttta 1320
   aggagaccaa tagaaactgg gcttgtcgag acagagaaga ctcttgcgtt tctgataggc 1380
   acctattggt cttactgaca tccactttgc ctttctctcc acaggtgtcc actcccagtt 1440
   caattacagc tcttaaggct agagtactta atacgactca ctataggcta gtaacggccg 1500
   ccagtgtgct ggaattcggc ttatggcaga atcgaaggaa gcaagagacc aagaaatgaa 1560
   cctgaaagaa gaatctaaag aagaaaaaag aagaaatgac tggtggaaaa tagatcctca 1620
   gggccctctg gaaggtgacc agtggtgcag ggtcctccgg cagtcgttac ctgaagaaaa 1680
   aattccatca caaacatgca tcgcgagaag acacctggga ccaggcccaa cacaacatac 1740
   acctagcagg cgtgaccggt ggatcagggg acaaatacta caagcagaag tactccagga 1800
   acgactggaa tggagaatca gaggagtaca acaggcggcc aaagagctgg gtgaagtcaa 1860
   tcgaggcatt tggagagagc tatatttccg agaagaccaa aggggagatt tctcagcctg 1920
   gggcggctat caacgagcac aagaacggct ctggggggaa caatcctcac caagggtcct 1980
   tagacctgga gattcgaagc gaaggaggaa acatttatga agccgaattc tgcagatatc 2040
   catcacactg gcggccgctt ccctttagtg agggttaatg cttcgagcag acatgataag 2100
   atacattgat gagtttggac aaaccacaac tagaatgcag tgaaaaaaat gctttatttg 2160
   tgaaatttgt gatgctattg ctttatttgt aaccattata agctgcaata aacaagttaa 2220
   caacaacaat tgcattcatt ttatgtttca ggttcagggg gagatgtggg aggtttttta 2280
   aagcaagtaa aacctctaca aatgtggtaa aatccgataa ggatcgatcc gggctggcgt 2340
   aatagcgaag aggcccgcac cgatcgccct tcccaacagt tgcgcagcct gaatggcgaa 2400
   tggacgcgcc ctgtagcggc gcattaagcg cggcgggtgt ggtggttacg cgcagcgtga 2460
   ccgctacact tgccagcgcc ctagcgcccg ctcctttcgc tttcttccct tcctttctcg 2520
   ccacgttcgc cggctttccc cgtcaagctc taaatcgggg gctcccttta gggttccgat 2580
   ttagagcttt acggcacctc gaccgcaaaa aacttgattt gggtgatggt tcacgtagtg 2640
   ggccatcgcc ctgatagacg gtttttcgcc ctttgacgtt ggagtccacg ttctttaata 2700
   gtggactctt gttccaaact ggaacaacac tcaaccctat ctcggtctat tcttttgatt 2760
   tataagggat tttgccgatt tcggcctatt ggttaaaaaa tgagctgatt taacaaatat 2820
   ttaacgcgaa ttttaacaaa atattaacgt ttacaatttc gcctgatgcg gtattttctc 2880
   cttacgcatc tgtgcggtat ttcacaccgc atacgcggat ctgcgcagca ccatggcctg 2940
   aaataacctc tgaaagagga acttggttag gtaccttctg aggcggaaag aaccagctgt 3000
   ggaatgtgtg tcagttaggg tgtggaaagt ccccaggctc cccagcaggc agaagtatgc 3060
   aaagcatgca tctcaattag tcagcaacca ggtgtggaaa gtccccaggc tccccagcag 3120
   gcagaagtat gcaaagcatg catctcaatt agtcagcaac catagtcccg cccctaactc 3180
   cgcccatccc gcccctaact ccgcccagtt ccgcccattc tccgccccat ggctgactaa 3240
   ttttttttat ttatgcagag gccgaggccg cctcggcctc tgagctattc cagaagtagt 3300
   gaggaggctt ttttggaggc ctaggctttt gcaaaaagct tgattcttct gacacaacag 3360
   tctcgaactt aaggctagag ccaccatgat tgaacaagat ggattgcacg caggttctcc 3420
   ggccgcttgg gtggagaggc tattcggcta tgactgggca caacagacaa tcggctgctc 3480
   tgatgccgcc gtgttccggc tgtcagcgca ggggcgcccg gttctttttg tcaagaccga 3540
   cctgtccggt gccctgaatg aactgcagga cgaggcagcg cggctatcgt ggctggccac 3600
   gacgggcgtt ccttgcgcag ctgtgctcga cgttgtcact gaagcgggaa gggactggct 3660
   gctattgggc gaagtgccgg ggcaggatct cctgtcatct caccttgctc ctgccgagaa 3720
   agtatccatc atggctgatg caatgcggcg gctgcatacg cttgatccgg ctacctgccc 3780
   attcgaccac caagcgaaac atcgcatcga gcgagcacgt actcggatgg aagccggtct 3840
   tgtcgatcag gatgatctgg acgaagagca tcaggggctc gcgccagccg aactgttcgc 3900
   caggctcaag gcgcgcatgc ccgacggcga ggatctcgtc gtgacccatg gcgatgcctg 3960
   cttgccgaat atcatggtgg aaaatggccg cttttctgga ttcatcgact gtggccggct 4020
   gggtgtggcg gaccgctatc aggacatagc gttggctacc cgtgatattg ctgaagagct 4080
   tggcggcgaa tgggctgacc gcttcctcgt gctttacggt atcgccgctc ccgattcgca 4140
   gcgcatcgcc ttctatcgcc ttcttgacga gttcttctga gcgggactct ggggttcgaa 4200
   atgaccgacc aagcgacgcc caacctgcca tcacgatggc cgcaataaaa tatctttatt 4260
   ttcattacat ctgtgtgttg gttttttgtg tgaatcgata gcgataagga tccgcgtatg 4320
   gtgcactctc agtacaatct gctctgatgc cgcatagtta agccagcccc gacacccgcc 4380
   aacacccgct gacgcgccct gacgggcttg tctgctcccg gcatccgctt acagacaagc 4440
   tgtgaccgtc tccgggagct gcatgtgtca gaggttttca ccgtcatcac cgaaacgcgc 4500
   gagacgaaag ggcctcgtga tacgcctatt tttataggtt aatgtcatga taataatggt 4560
   ttcttagacg tcaggtggca cttttcgggg aaatgtgcgc ggaaccccta tttgtttatt 4620
   tttctaaata cattcaaata tgtatccgct catgagacaa taaccctgat aaatgcttca 4680
   ataatattga aaaaggaaga gtatgagtat tcaacatttc cgtgtcgccc ttattccctt 4740
   ttttgcggca ttttgccttc ctgtttttgc tcacccagaa acgctggtga aagtaaaaga 4800
   tgctgaagat cagttgggtg cacgagtggg ttacatcgaa ctggatctca acagcggtaa 4860
   gatccttgag agttttcgcc ccgaagaacg ttttccaatg atgagcactt ttaaagttct 4920
   gctatgtggc gcggtattat cccgtattga cgccgggcaa gagcaactcg gtcgccgcat 4980
   acactattct cagaatgact tggttgagta ctcaccagtc acagaaaagc atcttacgga 5040
   tggcatgaca gtaagagaat tatgcagtgc tgccataacc atgagtgata acactgcggc 5100
   caacttactt ctgacaacga tcggaggacc gaaggagcta accgcttttt tgcacaacat 5160
   gggggatcat gtaactcgcc ttgatcgttg ggaaccggag ctgaatgaag ccataccaaa 5220
   cgacgagcgt gacaccacga tgcctgtagc aatggcaaca acgttgcgca aactattaac 5280
   tggcgaacta cttactctag cttcccggca acaattaata gactggatgg aggcggataa 5340
   agttgcagga ccacttctgc gctcggccct tccggctggc tggtttattg ctgataaatc 5400
   tggagccggt gagcgtgggt ctcgcggtat cattgcagca ctggggccag atggtaagcc 5460
   ctcccgtatc gtagttatct acacgacggg gagtcaggca actatggatg aacgaaatag 5520
   acagatcgct gagataggtg cctcactgat taagcattgg taactgtcag accaagttta 5580
   ctcatatata ctttagattg atttaaaact tcatttttaa tttaaaagga tctaggtgaa 5640
   gatccttttt gataatctca tgaccaaaat cccttaacgt gagttttcgt tccactgagc 5700
   gtcagacccc gtagaaaaga tcaaaggatc ttcttgagat cctttttttc tgcgcgtaat 5760
   ctgctgcttg caaacaaaaa aaccaccgct accagcggtg gtttgtttgc cggatcaaga 5820
   gctaccaact ctttttccga aggtaactgg cttcagcaga gcgcagatac caaatactgt 5880
   ccttctagtg tagccgtagt taggccacca cttcaagaac tctgtagcac cgcctacata 5940
   cctcgctctg ctaatcctgt taccagtggc tgctgccagt ggcgataagt cgtgtcttac 6000
   cgggttggac tcaagacgat agttaccgga taaggcgcag cggtcgggct gaacgggggg 6060
   ttcgtgcaca cagcccagct tggagcgaac gacctacacc gaactgagat acctacagcg 6120
   tgagctatga gaaagcgcca cgcttcccga agggagaaag gcggacaggt atccggtaag 6180
   cggcagggtc ggaacaggag agcgcacgag ggagcttcca gggggaaacg cctggtatct 6240
   ttatagtcct gtcgggtttc gccacctctg acttgagcgt cgatttttgt gatgctcgtc 6300
   aggggggcgg agcctatgga aaaacgccag caacgcggcc tttttacggt tcctggcctt 6360
   ttgctggcct tttgctcaca tggctcgaca gatct                            6395
<210> 14
   <211> 5961
   <212> DNA
   <213> Artificial Sequence
<220>
   <223> pESYNREV
<400> 14
   tcaatattgg ccattagcca tattattcat tggttatata gcataaatca atattggcta 60
   ttggccattg catacgttgt atctatatca taatatgtac atttatattg gctcatgtcc 120
   aatatgaccg ccatgttggc attgattatt gactagttat taatagtaat caattacggg 180
   gtcattagtt catagcccat atatggagtt ccgcgttaca taacttacgg taaatggccc 240
   gcctggctga ccgcccaacg acccccgccc attgacgtca ataatgacgt atgttcccat 300
   agtaacgcca atagggactt tccattgacg tcaatgggtg gagtatttac ggtaaactgc 360
   ccacttggca gtacatcaag tgtatcatat gccaagtccg ccccctattg acgtcaatga 420
   cggtaaatgg cccgcctggc attatgccca gtacatgacc ttacgggact ttcctacttg 480
   gcagtacatc tacgtattag tcatcgctat taccatggtg atgcggtttt ggcagtacac 540
   caatgggcgt ggatagcggt ttgactcacg gggatttcca agtctccacc ccattgacgt 600
   caatgggagt ttgttttggc accaaaatca acgggacttt ccaaaatgtc gtaacaactg 660
   cgatcgcccg ccccgttgac gcaaatgggc ggtaggcgtg tacggtggga ggtctatata 720
   agcagagctc gtttagtgaa ccgtcagatc actagaagct ttattgcggt agtttatcac 780
   agttaaattg ctaacgcagt cagtgcttct gacacaacag tctcgaactt aagctgcagt 840
   gactctctta aggtagcctt gcagaagttg gtcgtgaggc actgggcagg taagtatcaa 900
   ggttacaaga caggtttaag gagaccaata gaaactgggc ttgtcgagac agagaagact 960
   cttgcgtttc tgataggcac ctattggtct tactgacatc cactttgcct ttctctccac 1020
   aggtgtccac tcccagttca attacagctc ttaaggctag agtacttaat acgactcact 1080
   ataggctagc ctcgagaatt cgccaccatg gctgagagca aggaggccag ggatcaagag 1140
   atgaacctca aggaagagag caaagaggag aagcgccgca acgactggtg gaagatcgac 1200
   ccacaaggcc ccctggaggg ggaccagtgg tgccgcgtgc tgagacagtc cctgcccgag 1260
   gagaagattc ctagccagac ctgcatcgcc agaagacacc tcggccccgg tcccacccag 1320
   cacacaccct ccagaaggga taggtggatt aggggccaga ttttgcaagc cgaggtcctc 1380
   caagaaaggc tggaatggag aattaggggc gtgcaacaag ccgctaaaga gctgggagag 1440
   gtgaatcgcg gcatctggag ggagctctac ttccgcgagg accagagggg cgatttctcc 1500
   gcatggggag gctaccagag ggcacaagaa aggctgtggg gcgagcagag cagcccccgc 1560
   gtcttgaggc ccggagactc caaaagacgc cgcaaacacc tgtgaagtcg acccgggcgg 1620
   ccgcttccct ttagtgaggg ttaatgcttc gagcagacat gataagatac attgatgagt 1680
   ttggacaaac cacaactaga atgcagtgaa aaaaatgctt tatttgtgaa atttgtgatg 1740
   ctattgcttt atttgtaacc attataagct gcaataaaca agttaacaac aacaattgca 1800
   ttcattttat gtttcaggtt cagggggaga tgtgggaggt tttttaaagc aagtaaaacc 1860
   tctacaaatg tggtaaaatc cgataaggat cgatccgggc tggcgtaata gcgaagaggc 1920
   ccgcaccgat cgcccttccc aacagttgcg cagcctgaat ggcgaatgga cgcgccctgt 1980
   agcggcgcat taagcgcggc gggtgtggtg gttacgcgca gcgtgaccgc tacacttgcc 2040
   agcgccctag cgcccgctcc tttcgctttc ttcccttcct ttctcgccac gttcgccggc 2100
   tttccccgtc aagctctaaa tcgggggctc cctttagggt tccgatttag agctttacgg 2160
   cacctcgacc gcaaaaaact tgatttgggt gatggttcac gtagtgggcc atcgccctga 2220
   tagacggttt ttcgcccttt gacgttggag tccacgttct ttaatagtgg actcttgttc 2280
   caaactggaa caacactcaa ccctatctcg gtctattctt ttgatttata agggattttg 2340
   ccgatttcgg cctattggtt aaaaaatgag ctgatttaac aaatatttaa cgcgaatttt 2400
   aacaaaatat taacgtttac aatttcgcct gatgcggtat tttctcctta cgcatctgtg 2460
   cggtatttca caccgcatac gcggatctgc gcagcaccat ggcctgaaat aacctctgaa 2520
   agaggaactt ggttaggtac cttctgaggc ggaaagaacc agctgtggaa tgtgtgtcag 2580
   ttagggtgtg gaaagtcccc aggctcccca gcaggcagaa gtatgcaaag catgcatctc 2640
   aattagtcag caaccaggtg tggaaagtcc ccaggctccc cagcaggcag aagtatgcaa 2700
   agcatgcatc tcaattagtc agcaaccata gtcccgcccc taactccgcc catcccgccc 2760
   ctaactccgc ccagttccgc ccattctccg ccccatggct gactaatttt ttttatttat 2820
   gcagaggccg aggccgcctc ggcctctgag ctattccaga agtagtgagg aggctttttt 2880
   ggaggcctag gcttttgcaa aaagcttgat tcttctgaca caacagtctc gaacttaagg 2940
   ctagagccac catgattgaa caagatggat tgcacgcagg ttctccggcc gcttgggtgg 3000
   agaggctatt cggctatgac tgggcacaac agacaatcgg ctgctctgat gccgccgtgt 3060
   tccggctgtc agcgcagggg cgcccggttc tttttgtcaa gaccgacctg tccggtgccc 3120
   tgaatgaact gcaggacgag gcagcgcggc tatcgtggct ggccacgacg ggcgttcctt 3180
   gcgcagctgt gctcgacgtt gtcactgaag cgggaaggga ctggctgcta ttgggcgaag 3240
   tgccggggca ggatctcctg tcatctcacc ttgctcctgc cgagaaagta tccatcatgg 3300
   ctgatgcaat gcggcggctg catacgcttg atccggctac ctgcccattc gaccaccaag 3360
   cgaaacatcg catcgagcga gcacgtactc ggatggaagc cggtcttgtc gatcaggatg 3420
   atctggacga agagcatcag gggctcgcgc cagccgaact gttcgccagg ctcaaggcgc 3480
   gcatgcccga cggcgaggat ctcgtcgtga cccatggcga tgcctgcttg ccgaatatca 3540
   tggtggaaaa tggccgcttt tctggattca tcgactgtgg ccggctgggt gtggcggacc 3600
   gctatcagga catagcgttg gctacccgtg atattgctga agagcttggc ggcgaatggg 3660
   ctgaccgctt cctcgtgctt tacggtatcg ccgctcccga ttcgcagcgc atcgccttct 3720
   atcgccttct tgacgagttc ttctgagcgg gactctgggg ttcgaaatga ccgaccaagc 3780
   gacgcccaac ctgccatcac gatggccgca ataaaatatc tttattttca ttacatctgt 3840
   gtgttggttt tttgtgtgaa tcgatagcga taaggatccg cgtatggtgc actctcagta 3900
   caatctgctc tgatgccgca tagttaagcc agccccgaca cccgccaaca cccgctgacg 3960
   cgccctgacg ggcttgtctg ctcccggcat ccgcttacag acaagctgtg accgtctccg 4020
   ggagctgcat gtgtcagagg ttttcaccgt catcaccgaa acgcgcgaga cgaaagggcc 4080
   tcgtgatacg cctattttta taggttaatg tcatgataat aatggtttct tagacgtcag 4140
   gtggcacttt tcggggaaat gtgcgcggaa cccctatttg tttatttttc taaatacatt 4200
   caaatatgta tccgctcatg agacaataac cctgataaat gcttcaataa tattgaaaaa 4260
   ggaagagtat gagtattcaa catttccgtg tcgcccttat tccctttttt gcggcatttt 4320
   gccttcctgt ttttgctcac ccagaaacgc tggtgaaagt aaaagatgct gaagatcagt 4380
   tgggtgcacg agtgggttac atcgaactgg atctcaacag cggtaagatc cttgagagtt 4440
   ttcgccccga agaacgtttt ccaatgatga gcacttttaa agttctgcta tgtggcgcgg 4500
   tattatcccg tattgacgcc gggcaagagc aactcggtcg ccgcatacac tattctcaga 4560
   atgacttggt tgagtactca ccagtcacag aaaagcatct tacggatggc atgacagtaa 4620
   gagaattatg cagtgctgcc ataaccatga gtgataacac tgcggccaac ttacttctga 4680
   caacgatcgg aggaccgaag gagctaaccg cttttttgca caacatgggg gatcatgtaa 4740
   ctcgccttga tcgttgggaa ccggagctga atgaagccat accaaacgac gagcgtgaca 4800
   ccacgatgcc tgtagcaatg gcaacaacgt tgcgcaaact attaactggc gaactactta 4860
   ctctagcttc ccggcaacaa ttaatagact ggatggaggc ggataaagtt gcaggaccac 4920
   ttctgcgctc ggcccttccg gctggctggt ttattgctga taaatctgga gccggtgagc 4980
   gtgggtctcg cggtatcatt gcagcactgg ggccagatgg taagccctcc cgtatcgtag 5040
   ttatctacac gacggggagt caggcaacta tggatgaacg aaatagacag atcgctgaga 5100
   taggtgcctc actgattaag cattggtaac tgtcagacca agtttactca tatatacttt 5160
   agattgattt aaaacttcat ttttaattta aaaggatcta ggtgaagatc ctttttgata 5220
   atctcatgac caaaatccct taacgtgagt tttcgttcca ctgagcgtca gaccccgtag 5280
   aaaagatcaa aggatcttct tgagatcctt tttttctgcg cgtaatctgc tgcttgcaaa 5340
   caaaaaaacc accgctacca gcggtggttt gtttgccgga tcaagagcta ccaactcttt 5400
   ttccgaaggt aactggcttc agcagagcgc agataccaaa tactgtcctt ctagtgtagc 5460
   cgtagttagg ccaccacttc aagaactctg tagcaccgcc tacatacctc gctctgctaa 5520
   tcctgttacc agtggctgct gccagtggcg ataagtcgtg tcttaccggg ttggactcaa 5580
   gacgatagtt accggataag gcgcagcggt cgggctgaac ggggggttcg tgcacacagc 5640
   ccagcttgga gcgaacgacc tacaccgaac tgagatacct acagcgtgag ctatgagaaa 5700
   gcgccacgct tcccgaaggg agaaaggcgg acaggtatcc ggtaagcggc agggtcggaa 5760
   caggagagcg cacgagggag cttccagggg gaaacgcctg gtatctttat agtcctgtcg 5820
   ggtttcgcca cctctgactt gagcgtcgat ttttgtgatg ctcgtcaggg gggcggagcc 5880
   tatggaaaaa cgccagcaac gcggcctttt tacggttcct ggccttttgc tggccttttg 5940
   ctcacatggc tcgacagatc t                                           5961
<210> 15
   <211> 4307
   <212> DNA
   <213> Artificial Sequence
<220>
   <223> Codon optimised HIV gag-pol
<400> 15
   atgggcgccc gcgccagcgt gctgtcgggc ggcgagctgg accgctggga gaagatccgc 60
   ctgcgccccg gcggcaaaaa gaagtacaag ctgaagcaca tcgtgtgggc cagccgcgaa 120
   ctggagcgct tcgccgtgaa ccccgggctc ctggagacca gcgaggggtg ccgccagatc 180
   ctcggccaac tgcagcccag cctgcaaacc ggcagcgagg agctgcgcag cctgtacaac 240
   accgtggcca cgctgtactg cgtccaccag cgcatcgaaa tcaaggatac gaaagaggcc 300
   ctggataaaa tcgaagagga acagaataag agcaaaaaga aggcccaaca ggccgccgcg 360
   gacaccggac acagcaacca ggtcagccag aactacccca tcgtgcagaa catccagggg 420
   cagatggtgc accaggccat ctccccccgc acgctgaacg cctgggtgaa ggtggtggaa 480
   gagaaggctt ttagcccgga ggtgataccc atgttctcag ccctgtcaga gggagccacc 540
   ccccaagatc tgaacaccat gctcaacaca gtggggggac accaggccgc catgcagatg 600
   ctgaaggaga ccatcaatga ggaggctgcc gaatgggatc gtgtgcatcc ggtgcacgca 660
   gggcccatcg caccgggcca gatgcgtgag ccacggggct cagacatcgc cggaacgact 720
   agtacccttc aggaacagat cggctggatg accaacaacc cacccatccc ggtgggagaa 780
   atctacaaac gctggatcat cctgggcctg aacaagatcg tgcgcatgta tagccctacc 840
   agcatcctgg acatccgcca aggcccgaag gaaccctttc gcgactacgt ggaccggttc 900
   tacaaaacgc tccgcgccga gcaggctagc caggaggtga agaactggat gaccgaaacc 960
   ctgctggtcc agaacgcgaa cccggactgc aagacgatcc tgaaggccct gggcccagcg 1020
   gctaccctag aggaaatgat gaccgcctgt cagggagtgg gcggacccgg ccacaaggca 1080
   cgcgtcctgg ctgaggccat gagccaggtg accaactccg ctaccatcat gatgcagcgc 1140
   ggcaactttc ggaaccaacg caagatcgtc aagtgcttca actgtggcaa agaagggcac 1200
   acagcccgca actgcagggc ccctaggaaa aagggctgtt ggaaatgtgg aaaggaagga 1260
   caccaaatga aagattgtac tgagagacag gctaattttt tagggaagat ctggccttcc 1320
   cacaagggaa ggccagggaa ttttcttcag agcagaccag agccaacagc cccaccagaa 1380
   gagagcttca ggtttgggga agagacaaca actccctctc agaagcagga gccgatagac 1440
   aaggaactgt atcctttagc ttccctcaga tcactctttg gcagcgaccc ctcgtcacaa 1500
   taaagatagg ggggcagctc aaggaggctc tcctggacac cggagcagac gacaccgtgc 1560
   tggaggagat gtcgttgcca ggccgctgga agccgaagat gatcggggga atcggcggtt 1620
   tcatcaaggt gcgccagtat gaccagatcc tcatcgaaat ctgcggccac aaggctatcg 1680
   gtaccgtgct ggtgggcccc acacccgtca acatcatcgg acgcaacctg ttgacgcaga 1740
   tcggttgcac gctgaacttc cccattagcc ctatcgagac ggtaccggtg aagctgaagc 1800
   ccgggatgga cggcccgaag gtcaagcaat ggccattgac agaggagaag atcaaggcac 1860
   tggtggagat ttgcacagag atggaaaagg aagggaaaat ctccaagatt gggcctgaga 1920
   acccgtacaa cacgccggtg ttcgcaatca agaagaagga ctcgacgaaa tggcgcaagc 1980
   tggtggactt ccgcgagctg aacaagcgca cgcaagactt ctgggaggtt cagctgggca 2040
   tcccgcaccc cgcagggctg aagaagaaga aatccgtgac cgtactggat gtgggtgatg 2100
   cctacttctc cgttcccctg gacgaagact tcaggaagta cactgccttc acaatccctt 2160
   cgatcaacaa cgagacaccg gggattcgat atcagtacaa cgtgctgccc cagggctgga 2220
   aaggctctcc cgcaatcttc cagagtagca tgaccaaaat cctggagcct ttccgcaaac 2280
   agaaccccga catcgtcatc tatcagtaca tggatgactt gtacgtgggc tctgatctag 2340
   agatagggca gcaccgcacc aagatcgagg agctgcgcca gcacctgttg aggtggggac 2400
   tgaccacacc cgacaagaag caccagaagg agcctccctt cctctggatg ggttacgagc 2460
   tgcaccctga caaatggacc gtgcagccta tcgtgctgcc agagaaagac agctggactg 2520
   tcaacgacat acagaagctg gtggggaagt tgaactgggc cagtcagatt tacccaggga 2580
   ttaaggtgag gcagctgtgc aaactcctcc gcggaaccaa ggcactcaca gaggtgatcc 2640
   ccctaaccga ggaggccgag ctcgaactgg cagaaaaccg agagatccta aaggagcccg 2700
   tgcacggcgt gtactatgac ccctccaagg acctgatcgc cgagatccag aagcaggggc 2760
   aaggccagtg gacctatcag atttaccagg agcccttcaa gaacctgaag accggcaagt 2820
   acgcccggat gaggggtgcc cacactaacg acgtcaagca gctgaccgag gccgtgcaga 2880
   agatcaccac cgaaagcatc gtgatctggg gaaagactcc taagttcaag ctgcccatcc 2940
   agaaggaaac ctgggaaacc tggtggacag agtattggca ggccacctgg attcctgagt 3000
   gggagttcgt caacacccct cccctggtga agctgtggta ccagctggag aaggagccca 3060
   tagtgggcgc cgaaaccttc tacgtggatg gggccgctaa cagggagact aagctgggca 3120
   aagccggata cgtcactaac cggggcagac agaaggttgt caccctcact gacaccacca 3180
   accagaagac tgagctgcag gccatttacc tcgctttgca ggactcgggc ctggaggtga 3240
   acatcgtgac agactctcag tatgccctgg gcatcattca agcccagcca gaccagagtg 3300
   agtccgagct ggtcaatcag atcatcgagc agctgatcaa gaaggaaaag gtctatctgg 3360
   cctgggtacc cgcccacaaa ggcattggcg gcaatgagca ggtcgacaag ctggtctcgg 3420
   ctggcatcag gaaggtgcta ttcctggatg gcatcgacaa ggcccaggac gagcacgaga 3480
   aataccacag caactggcgg gccatggcta gcgacttcaa cctgccccct gtggtggcca 3540
   aagagatcgt ggccagctgt gacaagtgtc agctcaaggg cgaagccatg catggccagg 3600
   tggactgtag ccccggcatc tggcaactcg attgcaccca tctggagggc aaggttatcc 3660
   tggtagccgt ccatgtggcc agtggctaca tcgaggccga ggtcattccc gccgaaacag 3720
   ggcaggagac agcctacttc ctcctgaagc tggcaggccg gtggccagtg aagaccatcc 3780
   atactgacaa tggcagcaat ttcaccagtg ctacggttaa ggccgcctgc tggtgggcgg 3840
   gaatcaagca ggagttcggg atcccctaca atccccagag tcagggcgtc gtcgagtcta 3900
   tgaataagga gttaaagaag attatcggcc aggtcagaga tcaggctgag catctcaaga 3960
   ccgcggtcca aatggcggta ttcatccaca atttcaagcg gaaggggggg attggggggt 4020
   acagtgcggg ggagcggatc gtggacatca tcgcgaccga catccagact aaggagctgc 4080
   aaaagcagat taccaagatt cagaatttcc gggtctacta cagggacagc agaaatcccc 4140
   tctggaaagg cccagcgaag ctcctctgga agggtgaggg ggcagtagtg atccaggata 4200
   atagcgacat caaggtggtg cccagaagaa aggcgaagat cattagggat tatggcaaac 4260
   agatggcggg tgatgattgc gtggcgagca gacaggatga ggattag              4307
<210> 16
   <211> 4658
   <212> DNA
   <213> Artificial Sequence
<220>
   <223> Codon optimised EIAV gag-pol
<400> 16
   atgggcgatc ccctcacctg gtccaaagcc ctgaagaaac tggaaaaagt caccgttcag 60
   ggtagccaaa agcttaccac aggcaattgc aactgggcat tgtccctggt ggatcttttc 120
   cacgacacta atttcgttaa ggagaaagat tggcaactca gagacgtgat ccccctcttg 180
   gaggacgtga cccaaacatt gtctgggcag gagcgcgaag ctttcgagcg cacctggtgg 240
   gccatcagcg cagtcaaaat ggggctgcaa atcaacaacg tggttgacgg taaagctagc 300
   tttcaactgc tccgcgctaa gtacgagaag aaaaccgcca acaagaaaca atccgaacct 360
   agcgaggagt acccaattat gatcgacggc gccggcaata ggaacttccg cccactgact 420
   cccaggggct ataccacctg ggtcaacacc atccagacaa acggactttt gaacgaagcc 480
   tcccagaacc tgttcggcat cctgtctgtg gactgcacct ccgaagaaat gaatgctttt 540
   ctcgacgtgg tgccaggaca ggctggacag aaacagatcc tgctcgatgc cattgacaag 600
   atcgccgacg actgggataa tcgccacccc ctgccaaacg cccctctggt ggctccccca 660
   caggggccta tccctatgac cgctaggttc attaggggac tgggggtgcc ccgcgaacgc 720
   cagatggagc cagcatttga ccaatttagg cagacctaca gacagtggat catcgaagcc 780
   atgagcgagg ggattaaagt catgatcgga aagcccaagg cacagaacat caggcagggg 840
   gccaaggaac cataccctga gtttgtcgac aggcttctgt cccagattaa atccgaaggc 900
   caccctcagg agatctccaa gttcttgaca gacacactga ctatccaaaa tgcaaatgaa 960
   gagtgcagaa acgccatgag gcacctcaga cctgaagata ccctggagga gaaaatgtac 1020
   gcatgtcgcg acattggcac taccaagcaa aagatgatgc tgctcgccaa ggctctgcaa 1080
   accggcctgg ctggtccatt caaaggagga gcactgaagg gaggtccatt gaaagctgca 1140
   caaacatgtt ataattgtgg gaagccagga catttatcta gtcaatgtag agcacctaaa 1200
   gtctgtttta aatgtaaaca gcctggacat ttctcaaagc aatgcagaag tgttccaaaa 1260
   aacgggaagc aaggggctca agggaggccc cagaaacaaa ctttcccgat acaacagaag 1320
   agtcagcaca acaaatctgt tgtacaagag actcctcaga ctcaaaatct gtacccagat 1380
   ctgagcgaaa taaaaaagga atacaatgtc aaggagaagg atcaagtaga ggatctcaac 1440
   ctggacagtt tgtgggagta acatacaatc tcgagaagag gcccactacc atcgtcctga 1500
   tcaatgacac ccctcttaat gtgctgctgg acaccggagc cgacaccagc gttctcacta 1560
   ctgctcacta taacagactg aaatacagag gaaggaaata ccagggcaca ggcatcatcg 1620
   gcgttggagg caacgtcgaa accttttcca ctcctgtcac catcaaaaag aaggggagac 1680
   acattaaaac cagaatgctg gtcgccgaca tccccgtcac catccttggc agagacattc 1740
   tccaggacct gggcgctaaa ctcgtgctgg cacaactgtc taaggaaatc aagttccgca 1800
   agatcgagct gaaagagggc acaatgggtc caaaaatccc ccagtggccc ctgaccaaag 1860
   agaagcttga gggcgctaag gaaatcgtgc agcgcctgct ttctgagggc aagattagcg 1920
   aggccagcga caataaccct tacaacagcc ccatctttgt gattaagaaa aggagcggca 1980
   aatggagact cctgcaggac ctgagggaac tcaacaagac cgtccaggtc ggaactgaga 2040
   tctctcgcgg actgcctcac cccggcggcc tgattaaatg caagcacatg acagtccttg 2100
   acattggaga cgcttatttt accatccccc tcgatcctga atttcgcccc tatactgctt 2160
   ttaccatccc cagcatcaat caccaggagc ccgataaacg ctatgtgtgg aagtgcctcc 2220
   cccagggatt tgtgcttagc ccctacattt accagaagac acttcaagag atcctccaac 2280
   ctttccgcga aagataccca gaggttcaac tctaccaata tatggacgac ctgttcatgg 2340
   ggtccaacgg gtctaagaag cagcacaagg aactcatcat cgaactgagg gcaatcctcc 2400
   tggagaaagg cttcgagaca cccgacgaca agctgcaaga agttcctcca tatagctggc 2460
   tgggctacca gctttgccct gaaaactgga aagtccagaa gatgcagttg gatatggtca 2520
   agaacccaac actgaacgac gtccagaagc tcatgggcaa tattacctgg atgagctccg 2580
   gaatccctgg gcttaccgtt aagcacattg ccgcaactac aaaaggatgc ctggagttga 2640
   accagaaggt catttggaca gaggaagctc agaaggaact ggaggagaat aatgaaaaga 2700
   ttaagaatgc tcaagggctc caatactaca atcccgaaga agaaatgttg tgcgaggtcg 2760
   aaatcactaa gaactacgaa gccacctatg tcatcaaaca gtcccaaggc atcttgtggg 2820
   ccggaaagaa aatcatgaag gccaacaaag gctggtccac cgttaaaaat ctgatgctcc 2880
   tgctccagca cgtcgccacc gagtctatca cccgcgtcgg caagtgcccc accttcaaag 2940
   ttcccttcac taaggagcag gtgatgtggg agatgcaaaa aggctggtac tactcttggc 3000
   ttcccgagat cgtctacacc caccaagtgg tgcacgacga ctggagaatg aagcttgtcg 3060
   aggagcccac tagcggaatt acaatctata ccgacggcgg aaagcaaaac ggagagggaa 3120
   tcgctgcata cgtcacatct aacggccgca ccaagcaaaa gaggctcggc cctgtcactc 3180
   accaggtggc tgagaggatg gctatccaga tggcccttga ggacactaga gacaagcagg 3240
   tgaacattgt gactgacagc tactactgct ggaaaaacat cacagagggc cttggcctgg 3300
   agggacccca gtctccctgg tggcctatca tccagaatat ccgcgaaaag gaaattgtct 3360
   atttcgcctg ggtgcctgga cacaaaggaa tttacggcaa ccaactcgcc gatgaagccg 3420
   ccaaaattaa agaggaaatc atgcttgcct accagggcac acagattaag gagaagagag 3480
   acgaggacgc tggctttgac ctgtgtgtgc catacgacat catgattccc gttagcgaca 3540
   caaagatcat tccaaccgat gtcaagatcc aggtgccacc caattcattt ggttgggtga 3600
   ccggaaagtc cagcatggct aagcagggtc ttctgattaa cgggggaatc attgatgaag 3660
   gatacaccgg cgaaatccag gtgatctgca caaatatcgg caaaagcaat attaagctta 3720
   tcgaagggca gaagttcgct caactcatca tcctccagca ccacagcaat tcaagacaac 3780
   cttgggacga aaacaagatt agccagagag gtgacaaggg cttcggcagc acaggtgtgt 3840
   tctgggtgga gaacatccag gaagcacagg acgagcacga gaattggcac acctccccta 3900
   agattttggc ccgcaattac aagatcccac tgactgtggc taagcagatc acacaggaat 3960
   gcccccactg caccaaacaa ggttctggcc ccgccggctg cgtgatgagg tcccccaatc 4020
   actggcaggc agattgcacc cacctcgaca acaaaattat cctgaccttc gtggagagca 4080
   attccggcta catccacgca acactcctct ccaaggaaaa tgcattgtgc acctccctcg 4140
   caattctgga atgggccagg ctgttctctc caaaatccct gcacaccgac aacggcacca 4200
   actttgtggc tgaacctgtg gtgaatctgc tgaagttcct gaaaatcgcc cacaccactg 4260
   gcattcccta tcaccctgaa agccagggca ttgtcgagag ggccaacaga actctgaaag 4320
   aaaagatcca atctcacaga gacaatacac agacattgga ggccgcactt cagctcgccc 4380
   ttatcacctg caacaaagga agagaaagca tgggcggcca gaccccctgg gaggtcttca 4440
   tcactaacca ggcccaggtc atccatgaaa agctgctctt gcagcaggcc cagtcctcca 4500
   aaaagttctg cttttataag atccccggtg agcacgactg gaaaggtcct acaagagttt 4560
   tgtggaaagg agacggcgca gttgtggtga acgatgaggg caaggggatc atcgctgtgc 4620
   ccctgacacg caccaagctt ctcatcaagc caaactga                         4658
<210> 17
   <211> 10392
   <212> DNA
   <213> Artificial Sequence
<220>
   <223> pIRESlhygESYNGP
<400> 17
<210> 18
   <211> 10114
   <212> DNA
   <213> Artificial Sequence
<220>
   <223> pESDSYNGP
<400> 18
   tcaatattgg ccattagcca tattattcat tggttatata gcataaatca atattggcta 60
   ttggccattg catacgttgt atctatatca taatatgtac atttatattg gctcatgtcc 120
   aatatgaccg ccatgttggc attgattatt gactagttat taatagtaat caattacggg 180
   gtcattagtt catagcccat atatggagtt ccgcgttaca taacttacgg taaatggccc 240
   gcctggctga ccgcccaacg acccccgccc attgacgtca ataatgacgt atgttcccat 300
   agtaacgcca atagggactt tccattgacg tcaatgggtg gagtatttac ggtaaactgc 360
   ccacttggca gtacatcaag tgtatcatat gccaagtccg ccccctattg acgtcaatga 420
   cggtaaatgg cccgcctggc attatgccca gtacatgacc ttacgggact ttcctacttg 480
   gcagtacatc tacgtattag tcatcgctat taccatggtg atgcggtttt ggcagtacac 540
   caatgggcgt ggatagcggt ttgactcacg gggatttcca agtctccacc ccattgacgt 600
   caatgggagt ttgttttggc accaaaatca acgggacttt ccaaaatgtc gtaacaactg 660
   cgatcgcccg ccccgttgac gcaaatgggc ggtaggcgtg tacggtggga ggtctatata 720
   agcagagctc gtttagtgaa ccgtcagatc actagaagct ttattgcggt agtttatcac 780
   agttaaattg ctaacgcagt cagtgcttct gacacaacag tctcgaactt aagctgcagt 840
   gactctctta aggtagcctt gcagaagttg gtcgtgaggc actgggcagg taagtatcaa 900
   ggttacaaga caggtttaag gagaccaata gaaactgggc ttgtcgagac agagaagact 960
   cttgcgtttc tgataggcac ctattggtct tactgacatc cactttgcct ttctctccac 1020
   aggtgtccac tcccagttca attacagctc ttaaggctag agtacttaat acgactcact 1080
   ataggctaga gaattccagg taagatgggc gatcccctca cctggtccaa agccctgaag 1140
   aaactggaaa aagtcaccgt tcagggtagc caaaagctta ccacaggcaa ttgcaactgg 1200
   gcattgtccc tggtggatct tttccacgac actaatttcg ttaaggagaa agattggcaa 1260
   ctcagagacg tgatccccct cttggaggac gtgacccaaa cattgtctgg gcaggagcgc 1320
   gaagctttcg agcgcacctg gtgggccatc agcgcagtca aaatggggct gcaaatcaac 1380
   aacgtggttg acggtaaagc tagctttcaa ctgctccgcg ctaagtacga gaagaaaacc 1440
   gccaacaaga aacaatccga acctagcgag gagtacccaa ttatgatcga cggcgccggc 1500
   aataggaact tccgcccact gactcccagg ggctatacca cctgggtcaa caccatccag 1560
   acaaacggac ttttgaacga agcctcccag aacctgttcg gcatcctgtc tgtggactgc 1620
   acctccgaag aaatgaatgc ttttctcgac gtggtgccag gacaggctgg acagaaacag 1680
   atcctgctcg atgccattga caagatcgcc gacgactggg ataatcgcca ccccctgcca 1740
   aacgcccctc tggtggctcc cccacagggg cctatcccta tgaccgctag gttcattagg 1800
   ggactggggg tgccccgcga acgccagatg gagccagcat ttgaccaatt taggcagacc 1860
   tacagacagt ggatcatcga agccatgagc gaggggatta aagtcatgat cggaaagccc 1920
   aaggcacaga acatcaggca gggggccaag gaaccatacc ctgagtttgt cgacaggctt 1980
   ctgtcccaga ttaaatccga aggccaccct caggagatct ccaagttctt gacagacaca 2040
   ctgactatcc aaaatgcaaa tgaagagtgc agaaacgcca tgaggcacct cagacctgaa 2100
   gataccctgg aggagaaaat gtacgcatgt cgcgacattg gcactaccaa gcaaaagatg 2160
   atgctgctcg ccaaggctct gcaaaccggc ctggctggtc cattcaaagg aggagcactg 2220
   aagggaggtc cattgaaagc tgcacaaaca tgttataatt gtgggaagcc aggacattta 2280
   tctagtcaat gtagagcacc taaagtctgt tttaaatgta aacagcctgg acatttctca 2340
   aagcaatgca gaagtgttcc aaaaaacggg aagcaagggg ctcaagggag gccccagaaa 2400
   caaactttcc cgatacaaca gaagagtcag cacaacaaat ctgttgtaca agagactcct 2460
   cagactcaaa atctgtaccc agatctgagc gaaataaaaa aggaatacaa tgtcaaggag 2520
   aaggatcaag tagaggatct caacctggac agtttgtggg agtaacatac aatctcgaga 2580
   agaggcccac taccatcgtc ctgatcaatg acacccctct taatgtgctg ctggacaccg 2640
   gagccgacac cagcgttctc actactgctc actataacag actgaaatac agaggaagga 2700
   aataccaggg cacaggcatc atcggcgttg gaggcaacgt cgaaaccttt tccactcctg 2760
   tcaccatcaa aaagaagggg agacacatta aaaccagaat gctggtcgcc gacatccccg 2820
   tcaccatcct tggcagagac attctccagg acctgggcgc taaactcgtg ctggcacaac 2880
   tgtctaagga aatcaagttc cgcaagatcg agctgaaaga gggcacaatg ggtccaaaaa 2940
   tcccccagtg gcccctgacc aaagagaagc ttgagggcgc taaggaaatc gtgcagcgcc 3000
   tgctttctga gggcaagatt agcgaggcca gcgacaataa cccttacaac agccccatct 3060
   ttgtgattaa gaaaaggagc ggcaaatgga gactcctgca ggacctgagg gaactcaaca 3120
   agaccgtcca ggtcggaact gagatctctc gcggactgcc tcaccccggc ggcctgatta 3180
   aatgcaagca catgacagtc cttgacattg gagacgctta ttttaccatc cccctcgatc 3240
   ctgaatttcg cccctatact gcttttacca tccccagcat caatcaccag gagcccgata 3300
   aacgctatgt gtggaagtgc ctcccccagg gatttgtgct tagcccctac atttaccaga 3360
   agacacttca agagatcctc caacctttcc gcgaaagata cccagaggtt caactctacc 3420
   aatatatgga cgacctgttc atggggtcca acgggtctaa gaagcagcac aaggaactca 3480
   tcatcgaact gagggcaatc ctcctggaga aaggcttcga gacacccgac gacaagctgc 3540
   aagaagttcc tccatatagc tggctgggct accagctttg ccctgaaaac tggaaagtcc 3600
   agaagatgca gttggatatg gtcaagaacc caacactgaa cgacgtccag aagctcatgg 3660
   gcaatattac ctggatgagc tccggaatcc ctgggcttac cgttaagcac attgccgcaa 3720
   ctacaaaagg atgcctggag ttgaaccaga aggtcatttg gacagaggaa gctcagaagg 3780
   aactggagga gaataatgaa aagattaaga atgctcaagg gctccaatac tacaatcccg 3840
   aagaagaaat gttgtgcgag gtcgaaatca ctaagaacta cgaagccacc tatgtcatca 3900
   aacagtccca aggcatcttg tgggccggaa agaaaatcat gaaggccaac aaaggctggt 3960
   ccaccgttaa aaatctgatg ctcctgctcc agcacgtcgc caccgagtct atcacccgcg 4020
   tcggcaagtg ccccaccttc aaagttccct tcactaagga gcaggtgatg tgggagatgc 4080
   aaaaaggctg gtactactct tggcttcccg agatcgtcta cacccaccaa gtggtgcacg 4140
   acgactggag aatgaagctt gtcgaggagc ccactagcgg aattacaatc tataccgacg 4200
   gcggaaagca aaacggagag ggaatcgctg catacgtcac atctaacggc cgcaccaagc 4260
   aaaagaggct cggccctgtc actcaccagg tggctgagag gatggctatc cagatggccc 4320
   ttgaggacac tagagacaag caggtgaaca ttgtgactga cagctactac tgctggaaaa 4380
   acatcacaga gggccttggc ctggagggac cccagtctcc ctggtggcct atcatccaga 4440
   atatccgcga aaaggaaatt gtctatttcg cctgggtgcc tggacacaaa ggaatttacg 4500
   gcaaccaact cgccgatgaa gccgccaaaa ttaaagagga aatcatgctt gcctaccagg 4560
   gcacacagat taaggagaag agagacgagg acgctggctt tgacctgtgt gtgccatacg 4620
   acatcatgat tcccgttagc gacacaaaga tcattccaac cgatgtcaag atccaggtgc 4680
   cacccaattc atttggttgg gtgaccggaa agtccagcat ggctaagcag ggtcttctga 4740
   ttaacggggg aatcattgat gaaggataca ccggcgaaat ccaggtgatc tgcacaaata 4800
   tcggcaaaag caatattaag cttatcgaag ggcagaagtt cgctcaactc atcatcctcc 4860
   agcaccacag caattcaaga caaccttggg acgaaaacaa gattagccag agaggtgaca 4920
   agggcttcgg cagcacaggt gtgttctggg tggagaacat ccaggaagca caggacgagc 4980
   acgagaattg gcacacctcc cctaagattt tggcccgcaa ttacaagatc ccactgactg 5040
   tggctaagca gatcacacag gaatgccccc actgcaccaa acaaggttct ggccccgccg 5100
   gctgcgtgat gaggtccccc aatcactggc aggcagattg cacccacctc gacaacaaaa 5160
   ttatcctgac cttcgtggag agcaattccg gctacatcca cgcaacactc ctctccaagg 5220
   aaaatgcatt gtgcacctcc ctcgcaattc tggaatgggc caggctgttc tctccaaaat 5280
   ccctgcacac cgacaacggc accaactttg tggctgaacc tgtggtgaat ctgctgaagt 5340
   tcctgaaaat cgcccacacc actggcattc cctatcaccc tgaaagccag ggcattgtcg 5400
   agagggccaa cagaactctg aaagaaaaga tccaatctca cagagacaat acacagacat 5460
   tggaggccgc acttcagctc gcccttatca cctgcaacaa aggaagagaa agcatgggcg 5520
   gccagacccc ctgggaggtc ttcatcacta accaggccca ggtcatccat gaaaagctgc 5580
   tcttgcagca ggcccagtcc tccaaaaagt tctgctttta taagatcccc ggtgagcacg 5640
   actggaaagg tcctacaaga gttttgtgga aaggagacgg cgcagttgtg gtgaacgatg 5700
   agggcaaggg gatcatcgct gtgcccctga cacgcaccaa gcttctcatc aagccaaact 5760
   gaacccgggg cggccgcttc cctttagtga gggttaatgc ttcgagcaga catgataaga 5820
   tacattgatg agtttggaca aaccacaact agaatgcagt gaaaaaaatg ctttatttgt 5880
   gaaatttgtg atgctattgc tttatttgta accattataa gctgcaataa acaagttaac 5940
   aacaacaatt gcattcattt tatgtttcag gttcaggggg agatgtggga ggttttttaa 6000
   agcaagtaaa acctctacaa atgtggtaaa atccgataag gatcgatccg ggctggcgta 6060
   atagcgaaga ggcccgcacc gatcgccctt cccaacagtt gcgcagcctg aatggcgaat 6120
   ggacgcgccc tgtagcggcg cattaagcgc ggcgggtgtg gtggttacgc gcagcgtgac 6180
   cgctacactt gccagcgccc tagcgcccgc tcctttcgct ttcttccctt cctttctcgc 6240
   cacgttcgcc ggctttcccc gtcaagctct aaatcggggg ctccctttag ggttccgatt 6300
   tagagcttta cggcacctcg accgcaaaaa acttgatttg ggtgatggtt cacgtagtgg 6360
   gccatcgccc tgatagacgg tttttcgccc tttgacgttg gagtccacgt tctttaatag 6420
   tggactcttg ttccaaactg gaacaacact caaccctatc tcggtctatt cttttgattt 6480
   ataagggatt ttgccgattt cggcctattg gttaaaaaat gagctgattt aacaaatatt 6540
   taacgcgaat tttaacaaaa tattaacgtt tacaatttcg cctgatgcgg tattttctcc 6600
   ttacgcatct gtgcggtatt tcacaccgca tacgcggatc tgcgcagcac catggcctga 6660
   aataacctct gaaagaggaa cttggttagg taccttctga ggcggaaaga accagctgtg 6720
   gaatgtgtgt cagttagggt gtggaaagtc cccaggctcc ccagcaggca gaagtatgca 6780
   aagcatgcat ctcaattagt cagcaaccag gtgtggaaag tccccaggct ccccagcagg 6840
   cagaagtatg caaagcatgc atctcaatta gtcagcaacc atagtcccgc ccctaactcc 6900
   gcccatcccg cccctaactc cgcccagttc cgcccattct ccgccccatg gctgactaat 6960
   ttttttattt tatgcagagg ccgaggccgc ctcggcctct gagctattcc agaagtagtg 7020
   aggaggcttt tttggaggcc taggcttttg caaaaagctt gattcttctg acacaacagt 7080
   ctcgaactta aggctagagc caccatgatt gaacaagatg gattgcacgc aggttctccg 7140
   gccgcttggg tggagaggct attcggctat gactgggcac aacagacaat cggctgctct 7200
   gatgccgccg tgttccggct gtcagcgcag gggcgcccgg ttctttttgt caagaccgac 7260
   ctgtccggtg ccctgaatga actgcaggac gaggcagcgc ggctatcgtg gctggccacg 7320
   acgggcgttc cttgcgcagc tgtgctcgac gttgtcactg aagcgggaag ggactggctg 7380
   ctattgggcg aagtgccggg gcaggatctc ctgtcatctc accttgctcc tgccgagaaa 7440
   gtatccatca tggctgatgc aatgcggcgg ctgcatacgc ttgatccggc tacctgccca 7500
   ttcgaccacc aagcgaaaca tcgcatcgag cgagcacgta ctcggatgga agccggtctt 7560
   gtcgatcagg atgatctgga cgaagagcat caggggctcg cgccagccga actgttcgcc 7620
   aggctcaagg cgcgcatgcc cgacggcgag gatctcgtcg tgacccatgg cgatgcctgc 7680
   ttgccgaata tcatggtgga aaatggccgc ttttctggat tcatcgactg tggccggctg 7740
   ggtgtggcgg accgctatca ggacatagcg ttggctaccc gtgatattgc tgaagagctt 7800
   ggcggcgaat gggctgaccg cttcctcgtg ctttacggta tcgccgctcc cgattcgcag 7860
   cgcatcgcct tctatcgcct tcttgacgag ttcttctgag cgggactctg gggttcgaaa 7920
   tgaccgacca agcgacgccc aacctgccat cacgatggcc gcaataaaat atctttattt 7980
   tcattacatc tgtgtgttgg ttttttgtgt gaatcgatag cgataaggat ccgcgtatgg 8040
   tgcactctca gtacaatctg ctctgatgcc gcatagttaa gccagccccg acacccgcca 8100
   acacccgctg acgcgccctg acgggcttgt ctgctcccgg catccgctta cagacaagct 8160
   gtgaccgtct ccgggagctg catgtgtcag aggttttcac cgtcatcacc gaaacgcgcg 8220
   agacgaaagg gcctcgtgat acgcctattt ttataggtta atgtcatgat aataatggtt 8280
   tcttagacgt caggtggcac ttttcgggga aatgtgcgcg gaacccctat ttgtttattt 8340
   ttctaaatac attcaaatat gtatccgctc atgagacaat aaccctgata aatgcttcaa 8400
   taatattgaa aaaggaagag tatgagtatt caacatttcc gtgtcgccct tattcccttt 8460
   tttgcggcat tttgccttcc tgtttttgct cacccagaaa cgctggtgaa agtaaaagat 8520
   gctgaagatc agttgggtgc acgagtgggt tacatcgaac tggatctcaa cagcggtaag 8580
   atccttgaga gttttcgccc cgaagaacgt tttccaatga tgagcacttt taaagttctg 8640
   ctatgtggcg cggtattatc ccgtattgac gccgggcaag agcaactcgg tcgccgcata 8700
   cactattctc agaatgactt ggttgagtac tcaccagtca cagaaaagca tcttacggat 8760
   ggcatgacag taagagaatt atgcagtgct gccataacca tgagtgataa cactgcggcc 8820
   aacttacttc tgacaacgat cggaggaccg aaggagctaa ccgctttttt gcacaacatg 8880
   ggggatcatg taactcgcct tgatcgttgg gaaccggagc tgaatgaagc cataccaaac 8940
   gacgagcgtg acaccacgat gcctgtagca atggcaacaa cgttgcgcaa actattaact 9000
   ggcgaactac ttactctagc ttcccggcaa caattaatag actggatgga ggcggataaa 9060
   gttgcaggac cacttctgcg ctcggccctt ccggctggct ggtttattgc tgataaatct 9120
   ggagccggtg agcgtgggtc tcgcggtatc attgcagcac tggggccaga tggtaagccc 9180
   tcccgtatcg tagttatcta cacgacgggg agtcaggcaa ctatggatga acgaaataga 9240
   cagatcgctg agataggtgc ctcactgatt aagcattggt aactgtcaga ccaagtttac 9300
   tcatatatac tttagattga tttaaaactt catttttaat ttaaaaggat ctaggtgaag 9360
   atcctttttg ataatctcat gaccaaaatc ccttaacgtg agttttcgtt ccactgagcg 9420
   tcagaccccg tagaaaagat caaaggatct tcttgagatc ctttttttct gcgcgtaatc 9480
   tgctgcttgc aaacaaaaaa accaccgcta ccagcggtgg tttgtttgcc ggatcaagag 9540
   ctaccaactc tttttccgaa ggtaactggc ttcagcagag cgcagatacc aaatactgtc 9600
   cttctagtgt agccgtagtt aggccaccac ttcaagaact ctgtagcacc gcctacatac 9660
   ctcgctctgc taatcctgtt accagtggct gctgccagtg gcgataagtc gtgtcttacc 9720
   gggttggact caagacgata gttaccggat aaggcgcagc ggtcgggctg aacggggggt 9780
   tcgtgcacac agcccagctt ggagcgaacg acctacaccg aactgagata cctacagcgt 9840
   gagctatgag aaagcgccac gcttcccgaa gggagaaagg cggacaggta tccggtaagc 9900
   ggcagggtcg gaacaggaga gcgcacgagg gagcttccag ggggaaacgc ctggtatctt 9960
   tatagtcctg tcgggtttcg ccacctctga cttgagcgtc gatttttgtg atgctcgtca 10020
   ggggggcgga gcctatggaa aaacgccagc aacgcggcct ttttacggtt cctggccttt 10080
   tgctggcctt ttgctcacat ggctcgacag atct                              10114
<210> 19
   <211> 10384
   <212> DNA
   <213> Artificial Sequence
<220>
   <223> pONY8.3G FB29(-)
<400> 19
<210> 20
   <211> 21
   <212> DNA
   <213> Artificial Sequence
<220>
   <223> Sequence flanking the codon-optimised EIAV gag/pol ORF
<400> 20
   tctagagaat tcgccaccat g 21
<210> 21
   <211> 18
   <212> DNA
   <213> Artificial Sequence
<220>
   <223> Sequence flanking the codon-optimised EIAV gag/pol ORF
<400> 21
   tgaacccggg gcggccgc 18
<210> 22
   <211> 11
   <212> DNA
   <213> Artificial Sequence
<220>
   <223> pEV53B
<400> 22
   caggtaagat g 11
<210> 23
   <211> 42
   <212> DNA
   <213> Artificial Sequence
<220>
   <223> Primer
<400> 23
   ggctagagaa ttccaggtaa gatgggcgat cccctcacct gg 42
<210> 24
   <211> 22
   <212> DNA
   <213> Artificial Sequence
<220>
   <223> Primer
<400> 24
   ttgggtactc ctcgctaggt tc 22
<210> 25
   <211> 4307
   <212> DNA
   <213> Artificial Sequence
<220>
   <223> Codon optimised gag-pol sequence (pSYNGP)
<400> 25
   atgggcgccc gcgccagcgt gctgtcgggc ggcgagctgg accgctggga gaagatccgc 60
   ctgcgccccg gcggcaaaaa gaagtacaag ctgaagcaca tcgtgtgggc cagccgcgaa 120
   ctggagcgct tcgccgtgaa ccccgggctc ctggagacca gcgaggggtg ccgccagatc 180
   ctcggccaac tgcagcccag cctgcaaacc ggcagcgagg agctgcgcag cctgtacaac 240
   accgtggcca cgctgtactg cgtccaccag cgcatcgaaa tcaaggatac gaaagaggcc 300
   ctggataaaa tcgaagagga acagaataag agcaaaaaga aggcccaaca ggccgccgcg 360
   gacaccggac acagcaacca ggtcagccag aactacccca tcgtgcagaa catccagggg 420
   cagatggtgc accaggccat ctccccccgc acgctgaacg cctgggtgaa ggtggtggaa 480
   gagaaggctt ttagcccgga ggtgataccc atgttctcag ccctgtcaga gggagccacc 540
   ccccaagatc tgaacaccat gctcaacaca gtggggggac accaggccgc catgcagatg 600
   ctgaaggaga ccatcaatga ggaggctgcc gaatgggatc gtgtgcatcc ggtgcacgca 660
   gggcccatcg caccgggcca gatgcgtgag ccacggggct cagacatcgc cggaacgact 720
   agtacccttc aggaacagat cggctggatg accaacaacc cacccatccc ggtgggagaa 780
   atctacaaac gctggatcat cctgggcctg aacaagatcg tgcgcatgta tagccctacc 840
   agcatcctgg acatccgcca aggcccgaag gaaccctttc gcgactacgt ggaccggttc 900
   tacaaaacgc tccgcgccga gcaggctagc caggaggtga agaactggat gaccgaaacc 960
   ctgctggtcc agaacgcgaa cccggactgc aagacgatcc tgaaggccct gggcccagcg 1020
   gctaccctag aggaaatgat gaccgcctgt cagggagtgg gcggacccgg ccacaaggca 1080
   cgcgtcctgg ctgaggccat gagccaggtg accaactccg ctaccatcat gatgcagcgc 1140
   ggcaactttc ggaaccaacg caagatcgtc aagtgcttca actgtggcaa agaagggcac 1200
   acagcccgca actgcagggc ccctaggaaa aagggctgtt ggaaatgtgg aaaggaagga 1260
   caccaaatga aagattgtac tgagagacag gctaattttt tagggaagat ctggccttcc 1320
   cacaagggaa ggccagggaa ttttcttcag agcagaccag agccaacagc cccaccagaa 1380
   gagagcttca ggtttgggga agagacaaca actccctctc agaagcagga gccgatagac 1440
   aaggaactgt atcctttagc ttccctcaga tcactctttg gcagcgaccc ctcgtcacaa 1500
   taaagatagg ggggcagctc aaggaggctc tcctggacac cggagcagac gacaccgtgc 1560
   tggaggagat gtcgttgcca ggccgctgga agccgaagat gatcggggga atcggcggtt 1620
   tcatcaaggt gcgccagtat gaccagatcc tcatcgaaat ctgcggccac aaggctatcg 1680
   gtaccgtgct ggtgggcccc acacccgtca acatcatcgg acgcaacctg ttgacgcaga 1740
   tcggttgcac gctgaacttc cccattagcc ctatcgagac ggtaccggtg aagctgaagc 1800
   ccgggatgga cggcccgaag gtcaagcaat ggccattgac agaggagaag atcaaggcac 1860
   tggtggagat ttgcacagag atggaaaagg aagggaaaat ctccaagatt gggcctgaga 1920
   acccgtacaa cacgccggtg ttcgcaatca agaagaagga ctcgacgaaa tggcgcaagc 1980
   tggtggactt ccgcgagctg aacaagcgca cgcaagactt ctgggaggtt cagctgggca 2040
   tcccgcaccc cgcagggctg aagaagaaga aatccgtgac cgtactggat gtgggtgatg 2100
   cctacttctc cgttcccctg gacgaagact tcaggaagta cactgccttc acaatccctt 2160
   cgatcaacaa cgagacaccg gggattcgat atcagtacaa cgtgctgccc cagggctgga 2220
   aaggctctcc cgcaatcttc cagagtagca tgaccaaaat cctggagcct ttccgcaaac 2280
   agaaccccga catcgtcatc tatcagtaca tggatgactt gtacgtgggc tctgatctag 2340
   agatagggca gcaccgcacc aagatcgagg agctgcgcca gcacctgttg aggtggggac 2400
   tgaccacacc cgacaagaag caccagaagg agcctccctt cctctggatg ggttacgagc 2460
   tgcaccctga caaatggacc gtgcagccta tcgtgctgcc agagaaagac agctggactg 2520
   tcaacgacat acagaagctg gtggggaagt tgaactgggc cagtcagatt tacccaggga 2580
   ttaaggtgag gcagctgtgc aaactcctcc gcggaaccaa ggcactcaca gaggtgatcc 2640
   ccctaaccga ggaggccgag ctcgaactgg cagaaaaccg agagatccta aaggagcccg 2700
   tgcacggcgt gtactatgac ccctccaagg acctgatcgc cgagatccag aagcaggggc 2760
   aaggccagtg gacctatcag atttaccagg agcccttcaa gaacctgaag accggcaagt 2820
   acgcccggat gaggggtgcc cacactaacg acgtcaagca gctgaccgag gccgtgcaga 2880
   agatcaccac cgaaagcatc gtgatctggg gaaagactcc taagttcaag ctgcccatcc 2940
   agaaggaaac ctgggaaacc tggtggacag agtattggca ggccacctgg attcctgagt 3000
   gggagttcgt caacacccct cccctggtga agctgtggta ccagctggag aaggagccca 3060
   tagtgggcgc cgaaaccttc tacgtggatg gggccgctaa cagggagact aagctgggca 3120
   aagccggata cgtcactaac cggggcagac agaaggttgt caccctcact gacaccacca 3180
   accagaagac tgagctgcag gccatttacc tcgctttgca ggactcgggc ctggaggtga 3240
   acatcgtgac agactctcag tatgccctgg gcatcattca agcccagcca gaccagagtg 3300
   agtccgagct ggtcaatcag atcatcgagc agctgatcaa gaaggaaaag gtctatctgg 3360
   cctgggtacc cgcccacaaa ggcattggcg gcaatgagca ggtcgacaag ctggtctcgg 3420
   ctggcatcag gaaggtgcta ttcctggatg gcatcgacaa ggcccaggac gagcacgaga 3480
   aataccacag caactggcgg gccatggcta gcgacttcaa cctgccccct gtggtggcca 3540
   aagagatcgt ggccagctgt gacaagtgtc agctcaaggg cgaagccatg catggccagg 3600
   tggactgtag ccccggcatc tggcaactcg attgcaccca tctggagggc aaggttatcc 3660
   tggtagccgt ccatgtggcc agtggctaca tcgaggccga ggtcattccc gccgaaacag 3720
   ggcaggagac agcctacttc ctcctgaagc tggcaggccg gtggccagtg aagaccatcc 3780
   atactgacaa tggcagcaat ttcaccagtg ctacggttaa ggccgcctgc tggtgggcgg 3840
   gaatcaagca ggagttcggg atcccctaca atccccagag tcagggcgtc gtcgagtcta 3900
   tgaataagga gttaaagaag attatcggcc aggtcagaga tcaggctgag catctcaaga 3960
   ccgcggtcca aatggcggta ttcatccaca atttcaagcg gaaggggggg attggggggt 4020
   acagtgcggg ggagcggatc gtggacatca tcgcgaccga catccagact aaggagctgc 4080
   aaaagcagat taccaagatt cagaatttcc gggtctacta cagggacagc agaaatcccc 4140
   tctggaaagg cccagcgaag ctcctctgga agggtgaggg ggcagtagtg atccaggata 4200
   atagcgacat caaggtggtg cccagaagaa aggcgaagat cattagggat tatggcaaac 4260
   agatggcggg tgatgattgc gtggcgagca gacaggatga ggattag                4307
<210> 26
   <211> 4307
   <212> DNA
   <213> Artificial Sequence
<220>
   <223> pGP-RRE3
<400> 26
   atgggtgcga gagcgtcagt attaagcggg ggagaattag atcgatggga aaaaattcgg 60
   ttaaggccag ggggaaagaa aaaatataaa ttaaaacata tagtatgggc aagcagggag 120
   ctagaacgat tcgcagttaa tcctggcctg ttagaaacat cagaaggctg tagacaaata 180
   ctgggacagc tacaaccatc ccttcagaca ggatcagaag aacttagatc attatataat 240
   acagtagcaa ccctctattg tgtgcatcaa aggatagaga taaaagacac caaggaagct 300
   ttagacaaga tagaggaaga gcaaaacaaa agtaagaaaa aagcacagca agcagcagct 360
   gacacaggac acagcaatca ggtcagccaa aattacccta tagtgcagaa catccagggg 420
   caaatggtac atcaggccat atcacctaga actttaaatg catgggtaaa agtagtagaa 480
   gagaaggctt tcagcccaga agtgataccc atgttttcag cattatcaga aggagccacc 540
   ccacaagatt taaacaccat gctaaacaca gtggggggac atcaagcagc catgcaaatg 600
   ttaaaagaga ccatcaatga ggaagctgca gaatgggata gagtgcatcc agtgcatgca 660
   gggcctattg caccaggcca gatgagagaa ccaaggggaa gtgacatagc aggaactact 720
   agtacccttc aggaacaaat aggatggatg acaaataatc cacctatccc agtaggagaa 780
   atttataaaa gatggataat cctgggatta aataaaatag taagaatgta tagccctacc 840
   agcattctgg acataagaca aggaccaaaa gaacccttta gagactatgt agaccggttc 900
   tataaaactc taagagccga gcaagcttca caggaggtaa aaaattggat gacagaaacc 960
   ttgttggtcc aaaatgcgaa cccagattgt aagactattt taaaagcatt gggaccagcg 1020
   gctacactag aagaaatgat gacagcatgt cagggagtag gaggacccgg ccataaggca 1080
   agagttttgg ctgaagcaat gagccaagta acaaattcag ctaccataat gatgcagaga 1140
   ggcaatttta ggaaccaaag aaagattgtt aagtgtttca attgtggcaa agaagggcac 1200
   acagccagaa attgcagggc ccctaggaaa aagggctgtt ggaaatgtgg aaaggaagga 1260
   caccaaatga aagattgtac tgagagacag gctaattttt tagggaagat ctggccttcc 1320
   tacaagggaa ggccagggaa ttttcttcag agcagaccag agccaacagc cccaccagaa 1380
   gagagcttca ggtctggggt agagacaaca actccccctc agaagcagga gccgatagac 1440
   aaggaactgt atcctttaac ttccctcaga tcactctttg gcaacgaccc ctcgtcacaa 1500
   taaagatagg ggggcaacta aaggaagctc tattagatac aggagcagat gatacagtat 1560
   tagaagaaat gagtttgcca ggaagatgga aaccaaaaat gataggggga attggaggtt 1620
   ttatcaaagt aagacagtat gatcagatac tcatagaaat ctgtggacat aaagctatag 1680
   gtacagtatt agtaggacct acacctgtca acataattgg aagaaatctg ttgactcaga 1740
   ttggttgcac tttaaatttt cccattagcc ctattgagac tgtaccagta aaattaaagc 1800
   caggaatgga tggcccaaaa gttaaacaat ggccattgac agaagaaaaa ataaaagcat 1860
   tagtagaaat ttgtacagag atggaaaagg aagggaaaat ttcaaaaatt gggcctgaaa 1920
   atccatacaa tactccagta tttgccataa agaaaaaaga cagtactaaa tggagaaaat 1980
   tagtagattt cagagaactt aataagagaa ctcaagactt ctgggaagtt caattaggaa 2040
   taccacatcc cgcagggtta aaaaagaaaa aatcagtaac agtactggat gtgggtgatg 2100
   catatttttc agttccctta gatgaagact tcaggaaata tactgcattt accataccta 2160
   gtataaacaa tgagacacca gggattagat atcagtacaa tgtgcttcca cagggatgga 2220
   aaggatcacc agcaatattc caaagtagca tgacaaaaat cttagagcct tttagaaaac 2280
   aaaatccaga catagttatc tatcaataca tggatgattt gtatgtagga tctgacttag 2340
   aaatagggca gcatagaaca aaaatagagg agctgagaca acatctgttg aggtggggac 2400
   ttaccacacc agacaaaaaa catcagaaag aacctccatt cctttggatg ggttatgaac 2460
   tccatcctga taaatggaca gtacagccta tagtgctgcc agaaaaagac agctggactg 2520
   tcaatgacat acagaagtta gtggggaaat tgaattgggc aagtcagatt tacccaggga 2580
   ttaaagtaag gcaattatgt aaactcctta gaggaaccaa agcactaaca gaagtaatac 2640
   cactaacaga agaagcagag ctagaactgg cagaaaacag agagattcta aaagaaccag 2700
   tacatggagt gtattatgac ccatcaaaag acttaatagc agaaatacag aagcaggggc 2760
   aaggccaatg gacatatcaa atttatcaag agccatttaa aaatctgaaa acaggaaaat 2820
   atgcaagaat gaggggtgcc cacactaatg atgtaaaaca attaacagag gcagtgcaaa 2880
   aaataaccac agaaagcata gtaatatggg gaaagactcc taaatttaaa ctgcccatac 2940
   aaaaggaaac atgggaaaca tggtggacag agtattggca agccacctgg attcctgagt 3000
   gggagtttgt taatacccct cctttagtga aattatggta ccagttagag aaagaaccca 3060
   tagtaggagc agaaaccttc tatgtagatg gggcagctaa cagggagact aaattaggaa 3120
   aagcaggata tgttactaat agaggaagac aaaaagttgt caccctaact gacacaacaa 3180
   atcagaagac tgagttacaa gcaatttatc tagctttgca ggattcggga ttagaagtaa 3240
   acatagtaac agactcacaa tatgcattag gaatcattca agcacaacca gatcaaagtg 3300
   aatcagagtt agtcaatcaa ataatagagc agttaataaa aaaggaaaag gtctatctgg 3360
   catgggtacc agcacacaaa ggaattggag gaaatgaaca agtagataaa ttagtcagtg 3420
   ctggaatcag gaaagtacta tttttagatg gaatagataa ggcccaagat gaacatgaga 3480
   aatatcacag taattggaga gcaatggcta gtgattttaa cctgccacct gtagtagcaa 3540
   aagaaatagt agccagctgt gataaatgtc agctaaaagg agaagccatg catggacaag 3600
   tagactgtag tccaggaata tggcaactag attgtacaca tttagaagga aaagttatcc 3660
   tggtagcagt tcatgtagcc agtggatata tagaagcaga agttattcca gcagaaacag 3720
   ggcaggaaac agcatatttt cttttaaaat tagcaggaag atggccagta aaaacaatac 3780
   atacagacaa tggcagcaat ttcaccagtg ctacggttaa ggccgcctgt tggtgggcgg 3840
   gaatcaagca ggaatttgga attccctaca atccccaaag tcaaggagta gtagaatcta 3900
   tgaataaaga attaaagaaa attataggac aggtaagaga tcaggctgaa catcttaaga 3960
   cagcagtaca aatggcagta ttcatccaca attttaaaag aaaagggggg attggggggt 4020
   acagtgcagg ggaaagaata gtagacataa tagcaacaga catacaaact aaagaattac 4080
   aaaaacaaat tacaaaaatt caaaattttc gggtttatta cagggacagc agaaatccac 4140
   tttggaaagg accagcaaag ctcctctgga aaggtgaagg ggcagtagta atacaagata 4200
   atagtgacat aaaagtagtg ccaagaagaa aagcaaagat cattagggat tatggaaaac 4260
   agatggcagg tgatgattgt gtggcaagta gacaggatga ggattag 4307
<210> 27
   <211> 4658
   <212> DNA
   <213> Equine infectious anemia virus
<400> 27
   atgggagacc ctttgacatg gagcaaggcg ctcaagaagt tagagaaggt gacggtacaa 60
   gggtctcaga aattaactac tggtaactgt aattgggcgc taagtctagt agacttattt 120
   catgatacca actttgtaaa agaaaaggac tggcagctga gggatgtcat tccattgctg 180
   gaagatgtaa ctcagacgct gtcaggacaa gaaagagagg cctttgaaag aacatggtgg 240
   gcaatttctg ctgtaaagat gggcctccag attaataatg tagtagatgg aaaggcatca 300
   ttccagctcc taagagcgaa atatgaaaag aagactgcta ataaaaagca gtctgagccc 360
   tctgaagaat atccaatcat gatagatggg gctggaaaca gaaattttag acctctaaca 420
   cctagaggat atactacttg ggtgaatacc atacagacaa atggtctatt aaatgaagct 480
   agtcaaaact tatttgggat attatcagta gactgtactt ctgaagaaat gaatgcattt 540
   ttggatgtgg tacctggcca ggcaggacaa aagcagatat tacttgatgc aattgataag 600
   atagcagatg attgggataa tagacatcca ttaccgaatg ctccactggt ggcaccacca 660
   caagggccta ttcccatgac agcaaggttt attagaggtt taggagtacc tagagaaaga 720
   cagatggagc ctgcttttga tcagtttagg cagacatata gacaatggat aatagaagcc 780
   atgtcagaag gcatcaaagt gatgattgga aaacctaaag ctcaaaatat taggcaagga 840
   gctaaggaac cttacccaga atttgtagac agactattat cccaaataaa aagtgaggga 900
   catccacaag agatttcaaa attcttgact gatacactga ctattcagaa cgcaaatgag 960
   gaatgtagaa atgctatgag acatttaaga ccagaggata cattagaaga gaaaatgtat 1020
   gcttgcagag acattggaac tacaaaacaa aagatgatgt tattggcaaa agcacttcag 1080
   actggtcttg cgggcccatt taaaggtgga gccttgaaag gagggccact aaaggcagca 1140
   caaacatgtt ataactgtgg gaagccagga catttatcta gtcaatgtag agcacctaaa 1200
   gtctgtttta aatgtaaaca gcctggacat ttctcaaagc aatgcagaag tgttccaaaa 1260
   aacgggaagc aaggggctca agggaggccc cagaaacaaa ctttcccgat acaacagaag 1320
   agtcagcaca acaaatctgt tgtacaagag actcctcaga ctcaaaatct gtacccagat 1380
   ctgagcgaaa taaaaaagga atacaatgtc aaggagaagg atcaagtaga ggatctcaac 1440
   ctggacagtt tgtgggagta acatataatc tagagaaaag gcctactaca atagtattaa 1500
   ttaatgatac tcccttaaat gtactgttag acacaggagc agatacttca gtgttgacta 1560
   ctgcacatta taataggtta aaatatagag ggagaaaata tcaagggacg ggaataatag 1620
   gagtgggagg aaatgtggaa acattttcta cgcctgtgac tataaagaaa aagggtagac 1680
   acattaagac aagaatgcta gtggcagata ttccagtgac tattttggga cgagatattc 1740
   ttcaggactt aggtgcaaaa ttggttttgg cacagctctc caaggaaata aaatttagaa 1800
   aaatagagtt aaaagagggc acaatggggc caaaaattcc tcaatggcca ctcactaagg 1860
   agaaactaga aggggccaaa gagatagtcc aaagactatt gtcagaggga aaaatatcag 1920
   aagctagtga caataatcct tataattcac ccatatttgt aataaaaaag aggtctggca 1980
   aatggaggtt attacaagat ctgagagaat taaacaaaac agtacaagta ggaacggaaa 2040
   tatccagagg attgcctcac ccgggaggat taattaaatg taaacacatg actgtattag 2100
   atattggaga tgcatatttc actataccct tagatccaga gtttagacca tatacagctt 2160
   tcactattcc ctccattaat catcaagaac cagataaaag atatgtgtgg aaatgtttac 2220
   cacaaggatt cgtgttgagc ccatatatat atcagaaaac attacaggaa attttacaac 2280
   cttttaggga aagatatcct gaagtacaat tgtatcaata tatggatgat ttgttcatgg 2340
   gaagtaatgg ttctaaaaaa caacacaaag agttaatcat agaattaagg gcgatcttac 2400
   tggaaaaggg ttttgagaca ccagatgata aattacaaga agtgccacct tatagctggc 2460
   taggttatca actttgtcct gaaaattgga aagtacaaaa aatgcaatta gacatggtaa 2520
   agaatccaac ccttaatgat gtgcaaaaat taatggggaa tataacatgg atgagctcag 2580
   ggatcccagg gttgacagta aaacacattg cagctactac taagggatgt ttagagttga 2640
   atcaaaaagt aatttggacg gaagaggcac aaaaagagtt agaagaaaat aatgagaaga 2700
   ttaaaaatgc tcaagggtta caatattata atccagaaga agaaatgtta tgtgaggttg 2760
   aaattacaaa aaattatgag gcaacttatg ttataaaaca atcacaagga atcctatggg 2820
   caggtaaaaa gattatgaag gctaataagg gatggtcaac agtaaaaaat ttaatgttat 2880
   tgttgcaaca tgtggcaaca gaaagtatta ctagagtagg aaaatgtcca acgtttaagg 2940
   taccatttac caaagagcaa gtaatgtggg aaatgcaaaa aggatggtat tattcttggc 3000
   tcccagaaat agtatataca catcaagtag ttcatgatga ttggagaatg aaattggtag 3060
   aagaacctac atcaggaata acaatataca ctgatggggg aaaacaaaat ggagaaggaa 3120
   tagcagctta tgtgaccagt aatgggagaa ctaaacagaa aaggttagga cctgtcactc 3180
   atcaagttgc tgaaagaatg gcaatacaaa tggcattaga ggataccaga gataaacaag 3240
   taaatatagt aactgatagt tattattgtt ggaaaaatat tacagaagga ttaggtttag 3300
   aaggaccaca aagtccttgg tggcctataa tacaaaatat acgagaaaaa gagatagttt 3360
   attttgcttg ggtacctggt cacaaaggga tatatggtaa tcaattggca gatgaagccg 3420
   caaaaataaa agaagaaatc atgctagcat accaaggcac acaaattaaa gagaaaagag 3480
   atgaagatgc agggtttgac ttatgtgttc cttatgacat catgatacct gtatctgaca 3540
   caaaaatcat acccacagat gtaaaaattc aagttcctcc taatagcttt ggatgggtca 3600
   ctgggaaatc atcaatggca aaacaggggt tattaattaa tggaggaata attgatgaag 3660
   gatatacagg agaaatacaa gtgatatgta ctaatattgg aaaaagtaat attaaattaa 3720
   tagagggaca aaaatttgca caattaatta tactacagca tcactcaaat tccagacagc 3780
   cttgggatga aaataaaata tctcagagag gggataaagg atttggaagt acaggagtat 3840
   tctgggtaga aaatattcag gaagcacaag atgaacatga gaattggcat acatcaccaa 3900
   agatattggc aagaaattat aagataccat tgactgtagc aaaacagata actcaagaat 3960
   gtcctcattg cactaagcaa ggatcaggac ctgcaggttg tgtcatgaga tctcctaatc 4020
   attggcaggc agattgcaca catttggaca ataagataat attgactttt gtagagtcaa 4080
   attcaggata catacatgct acattattgt caaaagaaaa tgcattatgt acttcattgg 4140
   ctattttaga atgggcaaga ttgttttcac caaagtcctt acacacagat aacggcacta 4200
   attttgtggc agaaccagtt gtaaatttgt tgaagttcct aaagatagca cataccacag 4260
   gaataccata tcatccagaa agtcagggta ttgtagaaag ggcaaatagg accttgaaag 4320
   agaagattca aagtcataga gacaacactc aaacactgga ggcagcttta caacttgctc 4380
   tcattacttg taacaaaggg agggaaagta tgggaggaca gacaccatgg gaagtattta 4440
   tcactaatca agcacaagta atacatgaga aacttttact acagcaagca caatcctcca 4500
   aaaaattttg tttttacaaa atccctggtg aacatgattg gaagggacct actagggtgc 4560
   tgtggaaggg tgatggtgca gtagtagtta atgatgaagg aaagggaata attgctgtac 4620
   cattaaccag gactaagtta ctaataaaac caaattga                        4658
<210> 28
   <211> 385
   <212> DNA
   <213> Artificial Sequence
<220>
   <223> pONY3.1
<400> 28
   atgggagacc ctttgacatg gagcaaggcg ctcaagaagt tagagaaggt gacggtacaa 60
   gggtctcaga aattaactac tggtaactgt aattgggcgc taagtctagt agacttattt 120
   catgatacca actttgtaaa agaaaaggac tggcagctga gggatgtcat tccattgctg 180
   gaagatgtaa ctcagacgct gtcaggacaa gaaagagagg cctttgaaag aacatggtgg 240
   gcaatttctg ctgtaaagat gggcctccag attaataatg tagtagatgg aaaggcatca 300
   ttccagctcc taagagcgaa atatgaaaag aagactgcta ataaaaagca gtctgagccc 360
   tctgaagaat atccaatcat gatag                                      385
<210> 29
   <211> 385
   <212> DNA
   <213> Artificial Sequence
<220>
   <223> pONY3.2opti
<400> 29
   atgggcgatc ccctcacctg gtccaaagcc ctgaaaaaac tggaaaaagt caccgttcag 60
   ggtagccaaa agcttaccac aggcaattgc aactgggcat tgtccctggt ggatcttttc 120
   cacgacacta atttcgttaa ggagaaagat tggcaactca gagacgtgat ccccctcttg 180
   gaggacgtga cccaaacatt gtctgggcag gagcgcgaag ctttcgagcg cacctggtgg 240
   gccatcagcg cagtcaaaat ggggctgcaa atcaacaacg tggttgacgg taaagctagc 300
   tttcaactgc tccgcgctaa gtacgagaaa aaaaccgcca acaagaaaca atccgaacct 360
   agcgaggagt acccaatcat gatag                                      385
<210> 30
   <211> 12
   <212> DNA
   <213> Human immunodeficiency virus
<400> 30
   atgggtgcga ga 12
<210> 31
   <211> 12
   <212> DNA
   <213> Human immunodeficiency virus
<400> 31
   gatgaggatt ag 12
<210> 32
   <211> 12
   <212> DNA
   <213> Artificial Sequence
<220>
   <223> gagpol-SYNgp
<400> 32
   atgggcgccc gc 12
<210> 33
   <211> 12
   <212> DNA
   <213> Artificial Sequence
<220>
   <223> gagpol-SYNgp
<400> 33
   gatgaggatt ag 12
<210> 34
   <211> 12
   <212> DNA
   <213> Human immunodeficiency virus
<400> 34
   atgagagtga ag 12
<210> 35
   <211> 12
   <212> DNA
   <213> Human immunodeficiency virus
<400> 35
   gctttgctat aa 12
<210> 36
   <211> 12
   <212> DNA
   <213> Artificial Sequence
<220>
   <223> synGP160mn
<400> 36
   atgagggtga ag 12
<210> 37
   <211> 12
   <212> DNA
   <213> Artificial Sequence
<220>
   <223> synGP160mn
<400> 37
   gcgctgctgt aa 12